(19) Europäisches Patentamt
European Patent Office
Office européen des brevets

(11) EP 4 047 012 A1

# (12) EUROPEAN PATENT APPLICATION

(43) Date of publication:
**24.08.2022 Bulletin 2022/34**

(21) Application number: **21305198.0**

(22) Date of filing: **17.02.2021**

(51) International Patent Classification (IPC):
***C07K 14/00*** *(2006.01)* ***A61K 47/00*** *(2006.01)*
***A61P 31/04*** *(2006.01)* *A61K 38/00 (2006.01)*
*A61L 27/00 (2006.01)*

(52) Cooperative Patent Classification (CPC):
(C-Sets available)
**C07K 14/001; A61L 27/34; A61L 27/54; A61L 29/085; A61L 29/16; A61L 31/10; A61L 31/16; A61P 31/04;** A61K 38/00; A61L 2300/252; A61L 2300/404; A61L 2430/16

(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Université de Bordeaux**
  **33000 Bordeaux (FR)**
- **Centre national de la recherche scientifique**
  **75016 Paris (FR)**
- **Institut Polytechnique de Bordeaux**
  **33402 Talence Cedex (FR)**
- **Université Paul Sabatier Toulouse 3**
  **31400 Toulouse (FR)**
- **Institut Pasteur**
  **75724 Paris Cedex 15 (FR)**

(72) Inventors:
- **BONDUELLE, Colin**
  **33380 MARCHEPRIME (FR)**
- **VERHAEGHE, Pierre**
  **31400 TOULOUSE (FR)**
- **DUPUY, Bruno**
  **75010 PARIS (FR)**
- **SALAS-AMBROSIO, Pedro**
  **33400 TALENCE (FR)**
- **TRONNET, Antoine**
  **31400 TOULOUSE (FR)**

(74) Representative: **Novagraaf Technologies**
**Chemistry & Life Sciences**
**2, rue Sarah Bernhardt**
**CS90017**
**92665 Asnières-sur-Seine Cedex (FR)**

## (54) ANTIMICROBIAL CATIONIC PEPTOID AND N-SUBTITUTED PEPTIDIC COPOLYMERS, PREPARATION AND USES THEREOF

(57) The present invention provides cationic peptoid / N-substituted peptidic copolymers, and pharmaceutical compositions thereof, as described generally and in subclasses herein, which compounds are useful for the treatment of microbial infections.

EP 4 047 012 A1

(52) Cooperative Patent Classification (CPC): (Cont.)

C-Sets
**A61L 27/34, C08L 77/02;**
**A61L 29/085, C08L 77/02;**
**A61L 31/10, C08L 77/02**

## Description

### FIELD OF THE INVENTION

The present invention relates to cationic peptoid / N-substituted peptidic copolymers with antimicrobial activity, method of preparation and uses thereof.

### BACKGROUND OF THE INVENTION

There is growing concern worldwide that extensive use of antibiotics is resulting in the development of antibiotic resistance among pathogenic bacteria. In particular, antibiotic overuse in livestock feeds compromises the effectiveness of current therapies via dissemination of antibiotic resistance genes to both disease-causing and commensal microorganisms. The explosion of bacterial resistance to traditional antibiotics and a rapid increase in the incidence of multidrug resistant microbes have created an urgency to develop new classes of antimicrobial agents. There are now "Superbugs" resistant to most or all antibiotics. Accordingly, there is a great need for additional therapeutic antimicrobial treatments effective against drug-resistant organisms.

Most organisms produce gene encoded antimicrobial peptides (AMPs) as innate defenses to prevent colonization and infection by multiple microbial pathogens. AMPs are produced by bacteria, fungi, plants, insects, amphibians, crustaceans, fish and mammals, including humans, either constitutively or in response to the presence of a microbe. As such, AMPs are nature’s antibiotics and have been the subject of intense research development, particularly against drug resistant microorganisms. AMPs are small, often positively-charged, peptides with high antimicrobial activity. The activity of AMPs can be broad, efficiently acting on many Gram-positive and Gram-negative bacteria species. Because their mode of action apparently involves non-specific interactions with the cytoplasmic membrane of bacteria, bacteria rarely develop resistance to them. Unfortunately, native AMPs lack specificity between prokaryotic and eukaryotic cells, and are therefore too toxic to be used for systemic treatment of bacterial infections. This toxicity, which manifests as drug- and dose-limiting hemolysis of human red blood cells, has limited the development of a new class of antimicrobial agents based on these AMPs. In addition, many of the AMPs under development suffer from weak activity, nonspecific cytotoxicity, and susceptibility to proteolysis. Moreover, the current production, purification and delivery methods available for these peptides have numerous limitations. For example, solid state peptide synthesis and peptide production and purification from cell culture are both costly and time consuming. Additionally, the subsequent targeted delivery of active amounts of these compounds can be challenging. Generally, AMPs cannot be administered orally as they are quickly degraded before they are able to reach their target. AMPs cannot be administered systemically either, as they are rapidly identified and targeted for clearance by the immune system before they can reach the site of infection. Moreover, high peptide concentrations are required to achieve a therapeutic effect which would be cost-prohibitive and would, more importantly, cause severe toxic side-effects. Taken together, these limitations have thus far stifled the development of AMP-based therapeutics.

Accordingly, there is a need for new and effective antimicrobial compounds.

### BRIEF DESCRIPTION OF THE DRAWINGS

FIGs. 1A-B represent the $^1$H NMR spectra obtained in $CDCl_3$ of *N*-Boc-*N*-benzyl-glycine (FIG. 1A) and *N*-benzyl-*N*-carboxyanhydride (FIG. 1B) of example 1.
FIG. 2 represents the $^1$H NMR spectra obtained in $CDCl_3$ of *N*-isopropyl-*N*-carboxyanhydride of example 2.
FIG. 3 represents the $^1$H NMR spectra obtained in $CDCl_3$ of *N*-cyclohexyl-*N*-carboxyanhydride of example 3.
FIG. 4 represents the $^1$H NMR spectra obtained in $CDCl_3$ of *N*-methyl-*N*-carboxyanhydride of example 4.
FIG. 5 represents the $^1$H NMR spectra obtained in $CDCl_3$ of *N*-(p-nitrobenzyl)-*N*-carboxyanhydride of example 5.
FIG. 6 represents the $^1$H NMR spectra obtained in $CDCl_3$ of *N*-(2-(methylthio)ethyl)-*N*-carboxyanhydride of example 6.
FIG. 7 represents the $^1$H NMR spectra obtained in $CDCl_3$ of *N*-trifluoroethyl-*N*-carboxyanhydride of example 7.
FIG. 8 represents the $^1$H NMR spectra obtained in $CDCl_3$ of *N*-Cbz-aminoethyl-*N*-carboxyanhydride of example 8.
FIG. 9 represents the $^1$H NMR spectra obtained in $CDCl_3$ of *N*-Cbz-aminobutyl-*N*-carboxyanhydride of example 9.
FIG. 10 represents the $^1$H NMR spectra obtained in $CDCl_3$ of *N*-methyl-L-alanine *N*-carboxyanhydride of example 10.
FIG. 11 represents the $^1$H NMR spectra obtained in $CDCl_3$ of *N*-methyl-D-alanine *N*-carboxyanhydride of example 11.
FIG. 12 represents the $^1$H NMR spectra obtained in $CDCl_3$ of *N*-methyl-L-leucine *N*-carboxyanhydride of example 12.
FIG. 13 represents the $^1$H NMR spectra obtained in $CDCl_3$ of *N*-methyl-L-phenylalanine *N*-carboxyanhydride of example 13.

FIG. 14 represents the $^1$H NMR spectra obtained in $CDCl_3$ of (S)-3-(3-Methyl-2,5-dioxooxazolidin-4-yl)propanenitrile of example 14.

FIG. 15 represents the $^1$H NMR spectra obtained in DMSO-d6 of *N*-methyl-L-lysine(Me, Z) *N*-carboxyanhydride of example 15.

FIGS. 16A-B represent the $^1$H NMR spectra obtained in DMSO-6d of poly(*N*-(Cbz-aminobutyl-glycine)$_{18}$-(*N*-benzyl-glycine)$_2$)$_{20}$ (FIG. 16A) and the Refractive index trace obtained from Size-Exclusion Chromatography in DMF (FIG. 16B) of example 16

FIG. 17 represents the $^1$H NMR spectra obtained in $D_20$ of poly(*N*-(aminobutyl-glycine)$_{18}$-(*N*-benzyl-glycine)$_2$)$_{20}$ of example 17.

FIGS. 18A-B represent the $^1$H NMR spectra obtained in DMSO-6d of poly(*N*-(Cbz-aminobutyl-glycine)$_{10}$-(*N*-benzyl-glycine)$_{10}$)$_{20}$ (FIG. 18A) and the Refractive index trace obtained from Size-Exclusion Chromatography in DMF (FIG. 18B) of example 18

FIG. 19 represents the $^1$H NMR spectra obtained in $D_20$ of poly(*N*-(aminobutyl-glycine)$_{10}$-(*N*-benzyl-glycine)$_{10}$)$_{20}$ of example 19.

FIGS. 20A-B represent the $^1$H NMR spectra obtained in DMSO-6d of poly(*N*-(Cbz-aminobutyl-glycine)$_{15}$-(*N*-benzyl-glycine)$_{15}$)$_{30}$ (FIG. 20A) and the Refractive index trace obtained from Size-Exclusion Chromatography in DMF (FIG. 20B) of example 20

FIG. 21 represents the $^1$H NMR spectra obtained in $D_20$ of poly(*N*(aminobutyl-glycine)$_{15}$-(*N*-benzyl-glycine)$_{15}$)$_{30}$ of example 21.

FIGS. 22A-B represent the $^1$H NMR spectra obtained in DMSO-6d of poly(*N*-(Cbz-aminobutyl-glycine)$_7$-(*N*-isopropyl-glycine)$_3$)$_{10}$ (FIG. 22A) and the Refractive index trace obtained from Size-Exclusion Chromatography in DMF (FIG. 22B) of example 22

FIG. 23 represents the $^1$H NMR spectra obtained in $D_20$ of poly(*N*-(aminobutyl-glycine)$_7$-(*N*-isopropyl-glycine)$_3$)$_{10}$ of example 23.

FIGS. 24A-B represent the $^1$H NMR spectra obtained in DMSO-6d of poly(*N*-(Cbz-aminobutyl-glycine)$_7$-(*N*-isopropyl-glycine)$_3$)$_{10}$ initiated with Decylamine (FIG. 24A) and the Refractive index trace obtained from Size-Exclusion Chromatography in DMF (FIG. 24B) of example 24.

FIG. 25 represents the $^1$H NMR spectra obtained in $D_20$ of poly(*N*-(aminobutyl-glycine)$_7$-(*N*-isopropyl-glycine)$_3$)$_{10}$ initiated with of example 25.

FIG. 26 represents the $^1$H NMR spectra obtained in $CDCl_3$ of poly[(*N*-methyl-L-lysine(Me, Z))$_{10}$-(*N*-methyl-L-alanine)$_{10}$)$_{20}$ of example 26.

FIGS. 27A-B represent the $^1$H NMR spectra obtained in DMSO-6d of poly[(*N*-Cbz-aminobutyl-glycine)$_{25}$-*random*-(*N*-benzyl-glycine)$_{25}$]$_{50}$ (FIG. 27A) and the Refractive index trace obtained from Size-Exclusion Chromatography in DMF (FIG. 27B) of example 27.

FIGS. 28A-B represent the $^1$H NMR spectra obtained in $CDCl_3$ of poly[(*N*-Cbz-aminobutyl-glycine)$_{25}$-block-(*N*-benzyl-glycine)$_{25}$]$_{50}$ (FIG. 28A) and the Refractive index trace obtained from Size-Exclusion Chromatography in DMF (FIG. 28B) of example 28.

FIG. 29 represents the $^1$H NMR spectra obtained in $D_20$ of poly[(*N*-aminobutyl-glycine)$_{25}$-*random*-(*N*-benzyl-glycine)$_{25}$]$_{50}$ of example 29.

FIGS. 30A-B represent the $^1$H NMR spectra obtained in DMSO-6d of poly[(*N*-Cbz-aminobutyl-glycine)$_{25}$-block-(*N*-benzyl-glycine)$_{25}$]$_{50}$ after propylation (FIG. 30A) and the Refractive index trace obtained from Size-Exclusion Chromatography in DMF (FIG. 30B) of example 30.

FIG. 31 represents the dose-response curve of poly(*N*-(aminobutyl-glycine)$_{15}$-(*N*-benzyl-glycine)$_{15}$)$_{30}$ on *Clostridium difficile* of example 31.

FIG. 32 represents the dose-response curve of poly[(*N*-aminobutyl-glycine)$_{25}$-*random*-(*N*-benzyl-glycine)$_{25}$]$_{50}$ on *Clostridium difficile* of example 32.

FIG. 33 represents the cell viability curve of human epithelial cell line in function of polymer concentration of example 34.

FIG. 34 represents the enzymatic resistance of poly[(*N*-aminobutyl-glycine)$_{25}$-*random*-(*N*-benzyl-glycine)$_{25}$]$_{50}$ using trypsin as model of example 35.

FIG. 35 represents the liposome leakage assays using two artificial membranes of example 37.

FIG. 36 depicts a proposed mechanism for the formation of cyclic polypeptoids by LiHMDS-mediated REP: cyclic zwitterionic species are produced in a controlled fashion and can efficiently afford covalent macrocycles.

## DEFINITIONS

It is understood that the compounds, as described herein, may be substituted with any number of substituents or functional moieties. In general, the term "substituted" whether preceded by the term "optionally" or not, and substituents

contained in formulas of this invention, refer to the replacement of hydrogen radicals in a given structure with the radical of a specified substituent. When more than one position in any given structure may be substituted with more than one substituent selected from a specified group, the substituent may be either the same or different at every position. As used herein, the term "substituted" is contemplated to include all permissible substituents of organic compounds. In a broad aspect, the permissible substituents include acyclic and cyclic, branched and unbranched, carbocyclic and heterocyclic, aromatic and non-aromatic, carbon and heteroatom substituents of organic compounds. For purposes of this invention, heteroatoms such as nitrogen may have hydrogen substituents and/or any permissible substituents of organic compounds described herein which satisfy the valencies of the heteroatoms. Furthermore, this invention is not intended to be limited in any manner by the permissible substituents of organic compounds. Combinations of substituents and variables envisioned by this invention are preferably those that result in the formation of stable compounds useful in the treatment and prevention, for example of disorders, as described generally above. Examples of substituents include, but are not limited to aliphatic; heteroaliphatic; alicyclic; heteroalicyclic; aromatic, heteroaromatic; aryl; heteroaryl; alkylaryl; alkylheteroaryl; alkoxy; aryloxy; heteroalkoxy; heteroaryloxy; alkylthio; arylthio; heteroalkylthio; heteroarylthio; F; C1; Br; I; $-NO_2$; -CN; $-CF_3$; $-CH_2CF_3$; - $CHC1_2$; $-CH_2OH$; $-CH_2CH_2OH$; $-CH_2NH_2$; $-CH_2SO_2CH_3$; - or $-GR^{G1}$ wherein G is -O-, -S-, $-NR^{G2}-$, -C(=O)-, -S(=O)-, $-SO_2-$, -C(=O)O-, $-C(=O)NR^{G2}-$, -OC(=O)-, $-NR^{G2}C(=O)-$, - OC(=O)O-, $-OC(=O)NR^{G2}-$, $-NR^{G2}C(=O)O-$, $-NR^{G2}C(=O)NR^{G2}-$, -C(=S)-, -C(=S)S-, - SC(=S)-, -SC(=S)S-, $-C(=NR^{G2})-$, $-C(=NR^{G2})O-$, $-C(=NR^{G2})NR^{G3}-$, $-OC(=NR^{G2})-$, - $NR^{G2}C(=NR^{G3})-$, $-NR^{G2}SO_2-$, $-NR^{G2}SO_2NR^{G3}-$, or $-SO_2NR^{G2}-$, wherein each occurrence of $R^{G1}$, $R^{G2}$ and $R^{G3}$ independently includes, but is not limited to, hydrogen, halogen, or an optionally substituted aliphatic, heteroaliphatic, alicyclic, heteroalicyclic, aromatic, heteroaromatic, aryl, heteroaryl, alkylaryl, or alkylheteroaryl moiety. Additional examples of generally applicable substituents are illustrated by the specific embodiments shown in the Examples that are described herein.

The term "stable", as used herein, preferably refers to compounds which possess stability sufficient to allow manufacture and which maintain the integrity of the compound for a sufficient period of time to be detected and preferably for a sufficient period of time to be useful for the purposes detailed herein. The term "stability" can refer to an ability to resist degradation, to persist in a given environment, and/or to maintain a particular structure. The term "aliphatic", as used herein, includes both saturated and unsaturated, straight chain (*i.e.,* unbranched) or branched aliphatic hydrocarbons, which are optionally substituted with one or more functional groups. As will be appreciated by one of ordinary skill in the art, "aliphatic" is intended herein to include, but is not limited to, alkyl, alkenyl, alkynyl moieties. Thus, as used herein, the term "alkyl" includes straight and branched alkyl groups. An analogous convention applies to other generic terms such as "alkenyl", "alkynyl" and the like. Furthermore, as used herein, the terms "alkyl", "alkenyl", "alkynyl" and the like encompass both substituted and unsubstituted groups. In certain embodiments, as used herein, "lower alkyl" is used to indicate those alkyl groups (substituted, unsubstituted, branched or unbranched) having about 1-6 carbon atoms.

In certain embodiments, the alkyl, alkenyl and alkynyl groups employed in the invention contain about 1-20 aliphatic carbon atoms. In certain other embodiments, the alkyl, alkenyl, and alkynyl groups employed in the invention contain about 1-10 aliphatic carbon atoms. In yet other embodiments, the alkyl, alkenyl, and alkynyl groups employed in the invention contain about 1-8 aliphatic carbon atoms. In still other embodiments, the alkyl, alkenyl, and alkynyl groups employed in the invention contain about 1-6 aliphatic carbon atoms. In yet other embodiments, the alkyl, alkenyl, and alkynyl groups employed in the invention contain about 1-4 carbon atoms. Illustrative aliphatic groups thus include, but are not limited to, for example, methyl, ethyl, n-propyl, isopropyl, allyl, n-butyl, sec-butyl, isobutyl, tert-butyl, n-pentyl, sec-pentyl, isopentyl, tert-pentyl, n-hexyl, sec-hexyl, moieties and the like, which again, may bear one or more substituents. Alkenyl groups include, but are not limited to, for example, ethenyl, propenyl, butenyl, 1-methyl-2-buten-1-yl, and the like. Representative alkynyl groups include, but are not limited to, ethynyl, 2-propynyl (propargyl), 1-propynyl and the like.

The term "alicyclic", as used herein, refers to compounds which combine the properties of aliphatic and cyclic compounds and include but are not limited to cyclic, or polycyclic aliphatic hydrocarbons and bridged cycloalkyl compounds, which are optionally substituted with one or more functional groups. As will be appreciated by one of ordinary skill in the art, "alicyclic" is intended herein to include, but is not limited to, cycloalkyl, cycloalkenyl, and cycloalkynyl moieties, which are optionally substituted with one or more functional groups. Illustrative alicyclic groups thus include, but are not limited to, for example, cyclopropyl, $-CH_2$-cyclopropyl, cyclobutyl, $-CH_2$-cyclobutyl, cyclopentyl, $-CH_2$-cyclopentyl-n, cyclohexyl, $-CH_2$-cyclohexyl, cyclohexenylethyl, cyclohexanylethyl, norborbyl moieties and the like, which again, may bear one or more substituents.

The term "cycloalkyl", as used herein, refers specifically to cyclic alkyl groups having three to seven, preferably three to ten carbon atoms. Suitable cycloalkyls include, but are not limited to cyclopropyl, cyclobutyl, cyclopentyl, cyclohexyl, cycloheptyl and the like, which, as in the case of aliphatic, heteroaliphatic or heterocyclic moieties, may optionally be substituted. An analogous convention applies to other generic terms such as "cycloalkenyl", "cycloalkynyl" and the like.

The term "heteroaliphatic", as used herein, refers to aliphatic moieties in which one or more carbon atoms in the main chain have been substituted with a heteroatom. Thus, a heteroaliphatic group refers to an aliphatic chain which contains one or more oxygen, sulfur, nitrogen, phosphorus or silicon atoms, *i.e.,* in place of carbon atoms. Thus, a 1-6

atom heteroaliphatic linker having at least one N atom in the heteroaliphatic main chain, as used herein, refers to a $C_{1-6}$aliphatic chain wherein at least one carbon atom is replaced with a nitrogen atom, and wherein any one or more of the remaining 5 carbon atoms may be replaced by an oxygen, sulfur, nitrogen, phosphorus or silicon atom. As used herein, a 1-atom heteroaliphatic linker having at least one N atom in the heteroaliphatic main chain refers to -NH- or -NR- where R is aliphatic, heteroaliphatic, acyl, aromatic, heteroaromatic or an amine protecting group. Heteroaliphatic moieties may be branched or linear unbranched. In certain embodiments, heteroaliphatic moieties are substituted by independent replacement of one or more of the hydrogen atoms thereon with one or more moieties including, any of the substituents described above. The term "heteroalicyclic", "heterocycloalkyl" or "heterocyclic", as used herein, refers to compounds which combine the properties of heteroaliphatic and cyclic compounds and include but are not limited to saturated and unsaturated mono- or polycyclic heterocycles such as morpholino, pyrrolidinyl, furanyl, thiofuranyl, pyrrolyl etc., which are optionally substituted with one or more functional groups, as defined herein. In certain embodiments, the term "heterocyclic" refers to a non-aromatic 5-, 6- or 7- membered ring or a polycyclic group, including, but not limited to a bi- or tri-cyclic group comprising fused six-membered rings having between one and three heteroatoms independently selected from oxygen, sulfur and nitrogen, wherein (i) each 5-membered ring has 0 to 2 double bonds and each 6-membered ring has 0 to 2 double bonds, (ii) the nitrogen and sulfur heteroatoms may optionally be oxidized, (iii) the nitrogen heteroatom may optionally be quaternized, and (iv) any of the above heterocyclic rings may be fused to an aryl or heteroaryl ring. Representative heterocycles include, but are not limited to, pyrrolidinyl, pyrazolinyl, pyrazolidinyl, imidazolinyl, imidazolidinyl, piperidinyl, piperazinyl, oxazolidiny1, isoxazolidinyl, morpholinyl, thiazolidinyl, isothiazolidinyl, and tetrahydrofuryl. Additionally, it will be appreciated that any of the alicyclic or heteroalicyclic moieties described above and herein may comprise an aryl or heteroaryl moiety fused thereto. Additional examples of generally applicable substituents are illustrated by the specific embodiments shown in the Examples that are described herein.

In general, the term "aromatic moiety", as used herein, refers to stable substituted or unsubstituted unsaturated mono- or polycyclic hydrocarbon moieties having preferably 3-14 carbon atoms, comprising at least one ring satisfying the Huckel rule for aromaticity. Examples of aromatic moieties include, but are not limited to, phenyl, indanyl, indenyl, naphthyl, phenanthryl and anthracyl.

In general, the term "heteroaromatic moiety", as used herein, refers to stable substituted or unsubstituted unsaturated mono-heterocyclic or polyheterocyclic moieties having preferably 3-14 carbon atoms, comprising at least one ring satisfying the Huckel rule for aromaticity. Examples of heteroaromatic moieties include, but are not limited to, pyridyl, quinolinyl, dihydroquinolinyl, isoquinolinyl, quinazolinyl, dihydroquinazolyl, and tetrahydroquinazolyl.

It will also be appreciated that aromatic and heteroaromatic moieties, as defined herein, may be attached via an aliphatic *(e.g.,* alkyl) or heteroaliphatic *(e.g.,* heteroalkyl) moiety and thus also include moieties such as -(aliphatic)aromatic, -(heteroaliphatic)aromatic, - (aliphatic)heteroaromatic, -(heteroaliphatic)heteroaromatic, -(alkyl)aromatic, - (heteroalkyl)aromatic, -(alkyl)heteroaromatic, and -(heteroalkyl)heteroaromatic moieties. Thus, as used herein, the phrases "aromatic or heteroaromatic moieties" and "aromatic, heteroaromatic, -(alkyl)aromatic, -(heteroalkyl)aromatic, -(heteroalkyl)heteroaromatic, and -(heteroalkyl)heteroaromatic" are interchangeable. Substituents include, but are not limited to, any of the previously mentioned substituents resulting in the formation of a stable compound.

In general, the term "aryl" refers to aromatic moieties, as described above, excluding those attached via an aliphatic *(e.g.,* alkyl) or heteroaliphatic *(e.g.,* heteroalkyl) moiety. In certain embodiments of the present invention, "aryl" refers to a mono- or bicyclic carbocyclic ring system having one or two rings satisfying the Huckel rule for aromaticity, including, but not limited to, phenyl, naphthyl, tetrahydronaphthyl, indanyl, indenyl and the like.

Similarly, the term "heteroaryl" refers to heteroaromatic moieties, as described above, excluding those attached via an aliphatic *(e.g.,* alkyl) or heteroaliphatic *(e.g.,* heteroalkyl) moiety. In certain embodiments of the present invention, the term "heteroaryl", as used herein, refers to a cyclic unsaturated radical having from about five to about ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furany1, quinolinyl, isoquinolinyl, and the like.

Substituents for aryl and heteroaryl moieties include, but are not limited to, any of the previously mentioned substitutents, *i.e.,* the substituents recited for aliphatic moieties, or for other moieties as disclosed herein, resulting in the formation of a stable compound. The terms "alkoxy" (or "alkyloxy"), and "thioalkyl" as used herein refers to an alkyl group, as previously defined, attached to the parent molecular moiety through an oxygen atom ("alkoxy") or through a sulfur atom ("thioalkyl"). In certain embodiments, the alkyl group contains about 1-20 aliphatic carbon atoms. In certain other embodiments, the alkyl group contains about 1-10 aliphatic carbon atoms. In yet other embodiments, the alkyl group contains about 1-8 aliphatic carbon atoms. In still other embodiments, the alkyl group contains about 1-6 aliphatic carbon atoms. In yet other embodiments, the alkyl group contains about 1-4 aliphatic carbon atoms. Examples of alkoxy groups, include but are not limited to, methoxy, ethoxy, propoxy, isopropoxy, n-butoxy, tert-butoxy, neopentoxy and n-hexoxy. Examples of thioalkyl groups include, but are not limited to, methylthio, ethylthio, propylthio, isopropylthio, n-butylthio, and the like.

The term "amine" refers to a group having the structure $-N(R)_2$ wherein each occurrence of R is independently hydrogen, or an aliphatic, heteroaliphatic, aromatic or heteroaromatic moiety, or the R groups, taken together, may form a heterocyclic moiety. The term "alkylamino" refers to a group having the structure -NHR'wherein R' is alkyl, as defined herein. The term "aminoalkyl" refers to a group having the structure $NH_2R'$-, wherein R' is alkyl, as defined herein. In certain embodiments, the alkyl group contains about 1-20 aliphatic carbon atoms. In certain other embodiments, the alkyl group contains about 1-10 aliphatic carbon atoms. In yet other embodiments, the alkyl, alkenyl, and alkynyl groups employed in the invention contain about 1-8 aliphatic carbon atoms. In still other embodiments, the alkyl group contains about 1-6 aliphatic carbon atoms. In yet other embodiments, the alkyl group contains about 1-4 aliphatic carbon atoms. Examples of alkylamino include, but are not limited to, methylamino, ethylamino, isopropylamino and the like.

"Amine protecting group", as used herein, unless otherwise indicated, refers to a substituent that is commonly employed to block or protect an amine on the compound thereby protecting its functionality while allowing for the reaction of other functional groups on the compound. Non-exclusive examples of an amine-protecting group include: acyl groups (e.g., formyl, acetyl, chloroacetyl, trichloroacetyl,O-nitrophenylacetyl,O-nitrophenoxyacetyl, trifluoroacetyl, acetoacetyl, 4-chlorobutyryl, isobutyryl,O-nitrocinnamoyl, picolinoyl, acylisothiocyanate, aminocaproyl, benzoyl, and the like), acyloxy groups (e.g., 1-tert-butyloxycarbonyl (Boc), methoxycarbonyl, 9-fluorenylmethoxycarbonyl, 2,2,2-trifluoroethoxycarbonyl, 2-trimethylsilylethxoycarbonyl, vinyloxycarbonyl, allyloxycarbonyl, 1,1 -dimethyl-propynyloxycarbonyl, benzyloxycarbonyl, p-nitrobenzyloxycarbony, 2,4-dichlorobenzyloxycarbonyl, and the like), diphenylmethane, and benzylcarbamates.

The terms "halo" and "halogen" as used herein refer to an atom selected from fluorine, chlorine, bromine and iodine.

The term "halogenated" denotes a moiety having one, two, or three halogen atoms attached thereto.

The term "haloalkyl" denotes an alkyl group, as defined above, having one, two, or three halogen atoms attached thereto and is exemplified by such groups as chloromethyl, bromoethyl, trifluoromethyl, and the like.

The term "acyloxy", as used herein, does not substantially differ from the common meaning of this term in the art, and refers to a moiety of structure $-OC(O)R_X$, wherein $R_X$ is a substituted or unsubstituted aliphatic, alicyclic, heteroaliphatic, heteroalicyclic, aryl or heteroaryl moiety.

The term "acyl", as used herein, does not substantially differ from the common meaning of this term in the art, and refers to a moiety of structure $-C(O)R_X$, wherein $R_X$ is a substituted or unsubstituted, aliphatic, alicyclic, heteroaliphatic, heteroalicyclic, aryl or heteroaryl moiety.

The term "imino", as used herein, does not substantially differ from the common meaning of this term in the art, and refers to a moiety of structure $-C(=NR_X)R_Y$, wherein $R_X$ is hydrogen or an optionally substituted aliphatic, alicyclic, heteroaliphatic, heteroalicyclic, aryl or heteroaryl moiety; and $R_Y$ is an optionally substituted aliphatic, alicyclic, heteroaliphatic, heteroalicyclic, aryl or heteroaryl moiety.

"Sulfonate leaving group", as used herein, unless otherwise indicated, refers to anions with the general formula $RSO_2O$-. Non limiting examples of a sulfonate leaving group include: mesylate ($R=CH_3$), triflate ($R=CF_3$), tosylate ($R=CH_3C_6H_4$), besylate ($R=C_6H_5$), tresylate ($R=CH_2CF_3$), and the like.

The term "$C_{1-6}$alkylene", as used herein, refers to a substituted or unsubstituted, linear or branched saturated divalent radical consisting solely of carbon and hydrogen atoms, having from one to six carbon atoms, having a free valence "-" at both ends of the radical. The term "$C_{2-6}$alkenylene", as used herein, refers to a substituted or unsubstituted, linear or branched unsaturated divalent radical consisting solely of carbon and hydrogen atoms, having from two to six carbon atoms, having a free valence "-" at both ends of the radical, and wherein the unsaturation is present only as double bonds and wherein a double bond can exist between the first carbon of the chain and the rest of the molecule.

As used herein, the terms "aliphatic", "heteroaliphatic", "alkyl", "alkenyl", "alkynyl", "heteroalkyl", "heteroalkenyl", "heteroalkynyl", and the like encompass substituted and unsubstituted, saturated and unsaturated, and linear and branched groups. Similarly, the terms "alicyclic", "heterocyclic", "heterocycloalkyl", "heterocycle" and the like encompass substituted and unsubstituted, and saturated and unsaturated groups. Additionally, the terms "cycloalkyl", "cycloalkenyl", "cycloalkynyl", "heterocycloalkyl", "heterocycloalkenyl", "heterocycloalkynyl", "aromatic", "heteroaromatic", "aryl", "heteroaryl" and the like, used alone or as part of a larger moiety, encompass both substituted and unsubstituted groups.

As used herein, the term "isolated", when applied to the compounds of the present invention, refers to such compounds that are (i) separated from at least some components with which they are associated in nature or when they are made and/or (ii) produced, prepared or manufactured by the hand of man.

The phrase, "pharmaceutically acceptable derivative", as used herein, denotes any pharmaceutically acceptable salt, ester, or salt of such ester, of such compound, or any other adduct or derivative which, upon administration to a patient, is capable of providing (directly or indirectly) a compound as otherwise described herein, or a metabolite or residue thereof. Pharmaceutically acceptable derivatives thus include among others pro-drugs. A pro-drug is a derivative of a compound, usually with significantly reduced pharmacological activity, which contains an additional moiety that is susceptible to removal *in vivo* yielding the parent molecule as the pharmacologically active species. An example of a pro-drug is an ester which is cleaved *in vivo* to yield a compound of interest. Pro-drugs of a variety of compounds, and

materials and methods for derivatizing the parent compounds to create the pro-drugs, are known and may be adapted to the present invention. Certain exemplary pharmaceutical compositions and pharmaceutically acceptable derivatives will be discussed in more detail herein below.

As used herein, the term "Microbial infections" refers to an infection caused by bacteria, fungi and/or protozoans.

The term "antimicrobial activity" refers to the ability of a compound to modify a function or metabolic process of a target microorganism, for example to at least partially affect replication, vegetative growth, toxin production, survival, viability in a quiescent state, or other attribute. The term relates to inhibition of growth of a microorganism. In aspects of the claimed peptoid / N-substituted peptidic copolymers and methods, antimicrobial activity relates to the ability of a peptoid / N-substituted peptidic copolymer to kill at least one bacterial species. The bacterial species may be a Gram-negative bacteria or Gram-positive bacteria. The term can be manifested as microbicidal or microbistatic inhibition of microbial growth.

The term "microorganism" herein refers broadly to bacteria, fungi, viruses, and protozoa. In particular, the term is applicable for a microorganism having a cellular or structural component of a lipid bilayer membrane. The membrane may be a cytoplasmic membrane. Pathogenic bacteria, fungi, viruses, and protozoa as known in the art are generally encompassed. Bacteria can include Gram-negative and Gram-positive bacteria in addition to organisms classified in orders of the class Mollicutes and the like, such as species of the Mycoplasma and Acholeplasma genera. Specific examples of Gram-negative bacteria include, but are not limited to, Escherichia coli, Pseudomonas aeruginosa, Acinetobacter baumannii, Salmonella spp., Haemophilus influenzae, Neisseria spp Vibrio cholerae, Vibrio parahaemolyticus and Helicobacter pylori. Examples of Gram-positive bacteria include, but are not limited to, Staphylococcus aureus, Staphylococcus epidermidis, Streptococcus agalactiae, Group A Streptococcus, Streptococcus pyogenes, Enterococcus faecalis, Group B Gram-positive Streptococcus, Corynebacterium xerosis, and Listeria monocytogenes.

Examples of viruses include measles virus, herpes simplex virus (HSV-1 and -2), herpes family members (HIV), hepatitis C, vesicular stomatitis virus (VSV), visna virus, and cytomegalovirus (CMV).

Exemplary fungi include, but are not limited to, Aspergillus species, Dermatophytes, Blastomyces derinatitidis, Candida species, including C. albicans and C. krusei; Malassezia furfur, Exophiala werneckii, Piedraia hortai, Trichosporon beigelii, Pseudallescheria boydii, Madurella grisea, Histoplasma capsulatum, Sporothrix schenckii, Histoplasma capsulatum, Tinea species, including T. versicolor, T. pedis T. unguium, T. cruris, T. capitus, T. corporis, T. barbae; Trichophyton species, including T. rubrum, T. interdigitale, T. tonsurans, T. violaceum, T. yaoundei, T. schoenleinii, T. megninii, T. soudanense, T. equinum, T. erinacei, and T. verrucosum; Microsporum species, including M. audouini, M. ferrugineum, M. canis, M. nanum, M. distortum, M. gypseum, M. fulvum, and the like.

Exemplary protozoa include, but are not limited to Giardia, Cryptosporidium, Isospora belli, Toxoplasma gondii, Trichomonas vaginalis, and Cyclospora species.

The term "treating", as used herein generally means that the compounds of the invention can be used in humans or animals with at least a tentative diagnosis of disease. In certain embodiments, compounds of the invention will delay or slow the progression of the disease thereby giving the individual a longer life span.

The term "preventing" as used herein means that the compounds of the present invention are useful when administered to a patient who has not been diagnosed as possibly having the disease at the time of administration, but who would normally be expected to develop the disease or be at increased risk for the disease. The compounds of the invention will slow the development of disease symptoms, delay the onset of disease, or prevent the individual from developing the disease at all. Preventing also includes administration of the compounds of the invention to those individuals thought to be predisposed to the disease due to familial history, genetic or chromosomal abnormalities, and/or due to the presence of one or more biological markers for the disease.

As used herein the term *"biological sample"* includes, without limitation, cell cultures or extracts thereof; biopsied material obtained from an animal (e.g., mammal) or extracts thereof; and blood, saliva, urine, feces, semen, tears, or other body fluids or extracts thereof. For example, the term "biological sample" refers to any solid or fluid sample obtained from, excreted by or secreted by any living organism, including single-celled micro-organisms (such as bacteria and yeasts) and multicellular organisms (such as plants and animals, for instance a vertebrate or a mammal, and in particular a healthy or apparently healthy human subject or a human patient affected by a condition or disease to be diagnosed or investigated). The biological sample can be in any form, including a solid material such as a tissue, cells, a cell pellet, a cell extract, cell homogenates, or cell fractions; or a biopsy, or a biological fluid. The biological fluid may be obtained from any site (e.g. blood, saliva (or a mouth wash containing buccal cells), tears, plasma, serum, urine, bile, cerebrospinal fluid, amniotic fluid, peritoneal fluid, and pleural fluid, or cells therefrom, aqueous or vitreous humor, or any bodily secretion), a transudate, an exudate (e.g. fluid obtained from an abscess or any other site of infection or inflammation), or fluid obtained from a joint (e.g. a normal joint or a joint affected by disease such as rheumatoid arthritis, osteoarthritis, gout or septic arthritis). The biological sample can be obtained from any organ or tissue (including a biopsy or autopsy specimen) or may comprise cells (whether primary cells or cultured cells) or medium conditioned by any cell, tissue or organ. Biological samples may also include sections of tissues such as frozen sections taken for histological purposes. Biological samples also include mixtures of biological molecules including proteins, lipids, carbohydrates and nucleic

acids generated by partial or complete fractionation of cell or tissue homogenates. Although the sample is preferably taken from a human subject, biological samples may be from any animal, plant, bacteria, virus, yeast, etc. The term *animal,* as used herein, refers to humans as well as non-human animals, at any stage of development, including, for example, mammals, birds, reptiles, amphibians, fish, worms and single cells. Cell cultures and live tissue samples are considered to be pluralities of animals. In certain exemplary embodiments, the non-human animal is a mammal (e.g., a rodent, a mouse, a rat, a rabbit, a monkey, a dog, a cat, a sheep, cattle, a primate, or a pig). An animal may be a transgenic animal or a human clone. If desired, the biological sample may be subjected to preliminary processing, including preliminary separation techniques. Conditions that are "suitable" for an event to occur, such as production of a product, or "suitable" conditions are conditions that do not prevent such events from occurring. Thus, these conditions permit, enhance, facilitate, and/or are conducive to the event.

The term "and/or" means one or all of the listed elements or a combination of any two or more of the listed elements.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, "comprising" is synonymous with "including," "containing," or "characterized by," and is inclusive or open-ended and does not exclude additional, unrecited elements or method steps. As used herein, "consisting of" excludes any element, step, or ingredient not specified in the claim element. As used herein, "consisting essentially of" does not exclude materials or steps that do not materially affect the basic and novel characteristics of the claim. In each instance herein, any of the terms "comprising", "consisting essentially of" and "consisting of" may be optionally replaced with either of the other two terms, thus describing alternative aspects of the scope of the subject matter. The invention illustratively described herein suitably may be practiced in the absence of any element or elements, limitation or limitations which is not specifically disclosed herein.

Unless otherwise specified, "a," "an," "the," and "at least one" are used interchangeably and mean one or more than one.

Whenever a range of values is given in the specification, for example, a temperature range, a time range, or a composition or concentration range, all intermediate ranges and subranges, as well as all individual values included in the ranges given are intended to be included in the disclosure (e.g., 1 to 5 includes 1, 1.5, 2, 2.75, 3, 3.80, 4, 5, etc.). As used herein, ranges specifically include the values provided as endpoint values of the range. For example, a range of 1 to 100 specifically includes the end point values of 1 and 100. Any subranges or individual values in a range or subrange that are included in this description can be excluded from the claims herein.

For any method disclosed herein that includes discrete steps, the steps may be conducted in any feasible order. And, as appropriate, any combination of two or more steps may be conducted simultaneously.

When used herein, the term "amino acid" is intended to refer to any natural or unnatural amino acid, whether made naturally or synthetically, including those in the L- or D-enantiomeric configurations. In general, the L-enantiomeric configuration is preferred. The term can also encompass amino acid analog compounds used in peptidomimetics or in peptoids. The term can include a modified or unusual amino acid or a synthetic derivative of an amino acid, e.g. diamino butyric acid and diamino propionic acid and the like. Exemplary amino acids include: A, Ala, Alanine; M, Met, Methionine; C, Cys, Cysteine; D, Asp, Aspartic Acid; E, Glu, Glutamic Acid; F, Phe, Phenylalanine; G, Gly, Glycine; H, His, Histidine; I, IIe, Isoleucine; K, Lys, Lysine; L, Leu, Leucine; N, Asn, Asparagine; O, Orn, Ornithine; P, Pro, Proline; Q, Gin, Glutamine; R, Arg, Arginine; S, Ser, Serine; T, Thr, Threonine; V, Val, Valine; W, Trp, Tryptophan; Y, Tyr, Tyrosine; Dbu, 2,4-Diaminobutyric acid; Dpr, 2,3-Diaminopropionic acid.

"Peptoids" are a specific subclass of peptidomimetics and refer to N-substituted glycine polymers in which the side chains are appended to the backbone nitrogen. They are closely related to their natural peptide counterparts, but differ chemically in that one or more or all side chains are appended to nitrogen atoms along the molecule's backbone, rather than to the $\alpha$-carbons (as they are in natural amino acids).

"Therapeutically effective amount" as used herein, refers to an amount of formulation, composition, or reagent in a pharmaceutically acceptable carrier or a physiologically acceptable salt of an active compound that is of sufficient quantity to ameliorate the undesirable state of the patient, animal, material, or object so treated. "Ameliorate" refers to a lessening of the detrimental effect of the disease state or disorder, or reduction in contamination, in the receiver of the treatment.

"Pharmaceutical agent or drug" as used herein, refers to a chemical compound or composition capable of inducing a desired therapeutic or prophylactic effect when properly administered to a subject.

"Pharmaceutically acceptable carrier" as used herein, refers to conventional pharmaceutical carriers useful in the methods disclosed herein. Remington's Pharmaceutical Sciences, by E. W. Martin, Mack Publishing Co., Easton, Pa., 15th Edition (1975), describes compositions and formulations suitable for pharmaceutical delivery of compounds and additional pharmaceutical agents. In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e.g., powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain non-toxic auxiliary substances, such as wetting or emulsifying agents, preservatives, salts, amino acids, and pH buffering agents and the like, for example sodium or potassium chloride or phosphate, Tween, sodium acetate or sorbitan monolaurate.

As used herein the term "medical device" refers to any implant, instrument, apparatus, implement, machine, device or any other similar or related object (including any component or accessory), which is intended for use in the diagnosis, treatment, cure or prevention of disease or other conditions.

As used herein, the term "implant" refers to any object intended for placement in a human body that is not a living tissue. The implant may be temporary or permanent. An implant can be a medical device or article comprising artificial components, such as catheters or pacemakers. Implants can also include naturally derived objects that have been processed so that their living tissues have been devitalized. As an example, bone grafts may be processed so that their living cells are removed (acellularized), but so that their shape is retained to serve as a template for ingrowth of bone from a host. As another example, naturally occurring coral can be processed to yield hydroxyapatite preparations that can be applied to the body for certain orthopedic and dental therapies. The term "copolymer" as used herein does not deviate from the conventional meaning of the term in the chemical art, and refers to a mixture of at least two monomers in a macromolecular structure obtained by a polymerization reaction.

As used herein, the expression « N-carboxyanhydride monomer" refers to N-containing carboxylic acid anhydride monomers having the general structure:

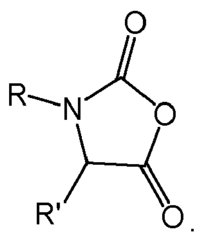

**DETAILED DESCRIPTION OF CERTAIN EXEMPLARY EMBODIMENTS OF THE INVENTION**

As noted above, there has been increasing interest in recent years in the development of new and effective antimicrobial compounds. Compounds of this invention include those generally set forth above and described specifically herein, and are illustrated in part by the various classes, subgenera and species disclosed herein. Additionally, the present invention provides pharmaceutically acceptable derivatives of the inventive compounds, and methods of treating a subject using these compounds, pharmaceutical compositions thereof, or either of these in combination with one or more additional therapeutic agents.

**1) Compounds and Process for their preparation**

In one aspect, the present invention provides a process for the preparation of cationic peptoid / N-substituted peptidic copolymers comprising :

A) at least one step of reacting in a polar solvent:

(i) at least a first N-carboxyanhydride monomer having the structure (I):

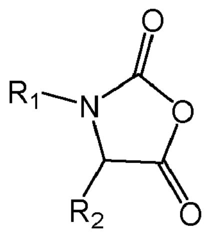

**(I)**

wherein

$R_1$ independently represents a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl or $C_2$-$_{12}$heteroalkynyl group; each of the foregoing alkyl, alkenyl, alkynyl, heteroalkyl, heteroalkenyl, heteroalkynyl group, optionally bearing one or more substituents selected from F; Cl; Br; I; $-NO_2$; -CN; $-CF_3$; $-CH_2CF_3$; $CHCl_2$; or $-GR^{A1}$ wherein G is -O-, -S-, -$NR^{B1}$-, -C(=O)-, -S(=O)-, $-SO_2-$, -C(=O)O-, -C(=O)$NR^{B1}$-, -OC(=O)-, -$NR^{B1}$C(=O)-, wherein each occurrence of $R^{A1}$ and $R^{B1}$ independently represents hydrogen, or a $C_{1-12}$alkyl, $C_2$-$_{12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl or $C_{2-12}$heteroalkynyl group; and

$\mathbf{R_2}$ independently represents H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; $-OR^{A2}$; - C(=O)$R^{A2}$; or $-SR^{A2}$; wherein each occurrence of $R^{A2}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl group may optionally bear one or more substitutents selected from F; Cl; Br; I; $-NO_2$; -CN; $-CF_3$; $-CH_2CF_3$; $CHCl_2$; or $-GR^{B2}$ wherein G is-O-, -S-, -$NR^{C2}$-, -C(=O)-, -S(=O)-, $-SO_2-$, -C(=O)O-, -C(=O)$NR^{C2}$-, -OC(=O)-, -$NR^{C2}$C(=O)-, wherein each occurrence of $R^{B2}$ and $R^{C2}$ independently represents independently represents hydrogen, or a $C_{1-12}$alkyl, $C_{2-12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl, C2-$_{12}$heteroalkynyl, $C_{6-10}$aryl or $C_{4-10}$heteroaryl group; wherein at least one of $R_1$ or $R_2$ bears at least one cationic moiety or protected form thereof;

(ii) at least a second N-carboxyanhydride monomer having the structure (II):

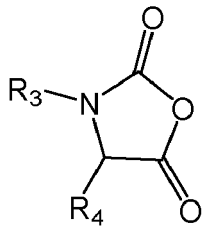

**(II)**

wherein

$R_3$ independently represents a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, or $C_{2-12}$heteroalkynyl; each of the foregoing alkyl, alkenyl, alkynyl, heteroalkyl, heteroalkenyl or heteroalkynyl group optionally bearing one or more substituents selected from $C_{1-12}$alkyl; $C_{2-12}$alkenyl; $C_{2-12}$alkynyl; $C_{1-12}$heteroalkyl; $C_{2-12}$heteroalkenyl; $C_{2-12}$heteroalkynyl; $C_{6-10}$aryl, $C_{6-10}$heteroaryl; F; Cl; Br; I; $-NO_2$; -CN; $-CF_3$; $-CH_2CF_3$; $CHCl_2$; or $-GR^{A3}$ wherein G is-O-, -S-, -$NR^{B3}$-, -C(=O)-, -S(=O)-, $-SO_2-$, -C(=O)O-, -C(=O)$NR^{B3}$-, -OC(=O)-, -$NR^{B3}$C(=O)-, wherein each occurrence of $R^{A3}$ and $R^{B3}$ independently represents hydrogen, or a $C_{1-12}$alkyl, $C_{2-12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl or $C_{2-12}$heteroalkynyl; and

$R_4$ independently represents H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; $-OR^{A4}$; - C(=O)$R^{A4}$; or $-SR^{A4}$; wherein each occurrence of $R^{A4}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl, group may optionally bear one or more substitutents selected from $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl, $C_{4-10}$heteroaryl, F; Cl; Br; I; $-NO_2$; -CN; $-CF_3$; $-CH_2CF_3$; $CHCl_2$; or $-GR^{B4}$ wherein G is -O-, -S-, $-NR^{C4}-$, -C(=O)-, -S(=O)-, $-SO_2-$, - C(=O)O-, $-C(=O)NR^{C4}-$, -OC(=O)-, $-NR^{C4}C(=O)-$, wherein each occurrence of $R^{B4}$ and $R^{C4}$ independently represents independently represents hydrogen, or a $C_{1-12}$alkyl, $C_{2-12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl, $C_{2-12}$heteroalkynyl, $C_{6-10}$aryl or $C_{4-10}$heteroaryl group;

(iii) in the presence of at least one nucleophilic compound initiator and/or at least one base;
wherein the base and the nucleophilic compound initiator may be different compounds or one and the same compound; and

B) when $R_1$ or $R_2$ bears a protected form of at least one cationic moiety, the process further comprises at least one deprotection step of the protected form of the at least one cationic moiety to yield cationic peptoid / N-substituted peptidic copolymers; such as acidic or basic treatment.

Section A

$\mathbf{R_1}$ may independently represent a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl or $C_{2-12}$heteroalkynyl group; each of the foregoing alkyl, alkenyl, alkynyl, heteroalkyl, heteroalkenyl, heteroalkynyl group, optionally bearing one or more substituents selected from F; Cl, Br, I, $-NO_2$, -CN, $-CF_3$, $-CH_2CF_3$, $CHCl_2$, or $-GR^{A1}$ wherein G is -O-, -S-, - $NR^{B1}-$, wherein each occurrence of $R^{A1}$ and $R^{B1}$ independently represents hydrogen, or a $C_{1-12}$alkyl or $C_{1-12}$haloalkyl moiety. $\mathbf{R_1}$ may independently represent a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl or $C_{2-12}$heteroalkynyl group; each of the foregoing alkyl, alkenyl, alkynyl, heteroalkyl, heteroalkenyl, heteroalkynyl group, optionally bearing one or more substituents selected from F; Cl, Br, I, $-NO_2$, -CN, $-CF_3$, $-OCF_3$, $OCH_3$, $-CH_2CF_3$ or $CHCl_2$. $R_1$ may bear at least one cationic moiety or protected form thereof. For example, $R_1$ may independently represent a cyclic or acyclic, substituted or unsubstituted, linear or branched $C_{1-12}$alkyl bearing at least one cationic moiety or protected form thereof. For example, $R_1$ may independently represent an optionally protected cationic side chain of a natural or modified amino acid. For example, $R_1$ may independently represent $-C_{1-6}$alkyl-$NHP_1$, wherein $P_1$ represents an amine protecting group, such as -(C=O)OPh or Cbz. For example, $R_1$ may independently represent $-(CH_2)_2-NHP_1$, $-(CH_2)_3-NHP_1$, $-(CH_2)_4-NHP_1$, $-(CH_2)_5-NHP_1$ or-$(CH_2)_6-NHP_1$, wherein $P_1$ represents an amine protecting group, such as -(C=O)OPh or Cbz. Preferably $P_1$ may represent -(C=O)OPh.

Section B

$\mathbf{R_2}$ may independently represent H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; $-OR^{A2}$; - $C(=O)R^{A2}$; or $-SR^{A2}$; wherein each occurrence of $R^{A2}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl group may optionally bear one or more substitutents selected from F; Cl, Br, I, $-NO_2$, -CN, $-CF_3$, $-OCF_3$, $OCH_3$, $-CH_2CF_3$, $CHCl_2$ or $-GR^{B2}$ wherein G is -O-, -S-, $-NR^{C2}-$, wherein each occurrence of $R^{B2}$ and $R^{C2}$ independently represents independently represents hydrogen, or a $C_{1-12}$alkyl or $C_{1-12}$haloalkyl moiety. $\mathbf{R_2}$ may independently represent H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; $-OR^{A2}$; $-C(=O)R^{A2}$; or $-SR^{A2}$; wherein each occurrence of $R^{A2}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl group may optionally bear one or more substitutents selected from F; Cl, Br, I, -NO2, -CN, $-CF_3$, $-OCF_3$, $OCH_3$, $-CH_2CF_3$ or $CHCl_2$. $R_2$ may bear at least one cationic moiety or protected form thereof. For example, $R_2$ may independently represent a cyclic or acyclic, substituted or unsubstituted, linear or branched $C_{1-12}$alkyl bearing at least one cationic moiety or protected form thereof. For example, $R_2$ may independently represent an optionally protected cationic side chain of a natural or modified amino acid. For example, $R_2$ may independently represent $-C_{1-6}$alkyl-$NHP_1$, wherein $P_1$ represents an amine protecting group, such as -(C=O)OPh or Cbz. For example, $R_2$ may independently

represent $-(CH_2)_2$-NHP$_1$, $-(CH_2)_3$-NHP$_1$, $-(CH_2)_4$-NHP$_1$, $-(CH_2)_5$-NHP$_1$ or $-(CH_2)_6$-NHP$_1$, wherein $P_1$ represents an amine protecting group, such as -(C=O)OPh or Cbz. Preferably $P_1$ may represent -(C=O)OPh.

In exemplary embodiments, $R_1$ may bear at least one cationic moiety or protected form thereof, and $R_2$ may independently represent H, or a cyclic or acyclic, substituted or unsubstituted, linear or branched $C_{1-12}$alkyl, optionally substituted with F; Cl, Br, I, $-NO_2$, - CN, $-CF_3$, $-OCF_3$, $OCH_3$, $-CH_2CF_3$ or $CHCl_2$. Preferably, $R_2$ may represent H, and $R_1$ may independently represent a cyclic or acyclic, substituted or unsubstituted, linear or branched $C_{1-12}$alkyl bearing at least one cationic moiety or protected form thereof. For example, $R_2$ may represent H, and $R_1$ may independently represent an optionally protected cationic side chain of a natural or modified amino acid. For example, $R_2$ may represent H, and $R_1$ may independently represent $-C_{1-6}$alkyl-NHP$_1$, wherein $P_1$ represents an amine protecting group, such as -(C=O)OPh or Cbz. For example, $R_2$ may represent H, and $R_1$ may independently represent $-(CH_2)_2$-NHP$_1$, $-(CH_2)_3$-NHP$_1$, $-(CH_2)_4$-NHP$_1$, $-(CH_2)_5$-NHP$_1$ or-$(CH_2)_6$-NHP$_1$, wherein $P_1$ represents an amine protecting group, such as -(C=O)OPh or Cbz. Preferably $P_1$ may represent -(C=O)OPh.

In exemplary embodiments, $R_2$ may bear at least one cationic moiety or protected form thereof, and $R_1$ may independently represent a cyclic or acyclic, substituted or unsubstituted, linear or branched $C_{1-12}$alkyl, optionally substituted with F; Cl, Br, I, $-NO_2$, - CN, $-CF_3$, $-OCF_3$, $OCH_3$, $-CH_2CF_3$ or $CHCl_2$. Preferably, $R_1$ may represent linear or branched $C_{1-12}$alkyl, and $R_2$ may independently represent a cyclic or acyclic, substituted or unsubstituted, linear or branched $C_{1-12}$alkyl bearing at least one cationic moiety or protected form thereof. For example, $R_1$ may represent linear or branched $C_{1-6}$alkyl, and $R_2$ may independently represent an optionally protected cationic side chain of a natural or modified amino acid. For example, $R_1$ may represent linear or branched $C_{1-6}$alkyl, and $R_2$ may independently represent $-C_{1-6}$alkyl-NHP$_1$, wherein $P_1$ represents an amine protecting group, such as -(C=O)OPh or Cbz. For example, $R_1$ may represent methyl, ethyl, n-propyl, i-propyl, n-butyl, i-butyl or tert-butyl, preferably methyl, and $R_2$ may independently represent $-(CH_2)_2$-NHP$_1$, $-(CH_2)_3$-NHP$_1$, $-(CH_2)_4$-NHP$_1$, $-(CH_2)_5$-NHP$_1$ or-$(CH_2)_6$-NHP,, wherein $P_1$ represents an amine protecting group, such as -(C=O)OPh or Cbz. Preferably $P_1$ may represent -(C=O)OPh.

Section C

$\mathbf{R_3}$ may independently represent a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, or $C_{2-12}$heteroalkynyl; wherein each of the foregoing alkyl, alkenyl, alkynyl, heteroalkyl, heteroalkenyl or heteroalkynyl group may optionally bear one or more substituents selected from F; Cl, Br, I, $-NO_2$, -CN, $-CF_3$, $-CH_2CF_3$, $CHCl_2$ , $C_{6-10}$aryl, $C_{6-10}$heteroaryl or-GR$^{A3}$ wherein G is -O-, -S-, or -NR$^{B3}$-, wherein each occurrence of $R^{A3}$ and $R^{B3}$ independently represents independently represents hydrogen, or a $C_{1-12}$alkyl or $C_{1-12}$haloalkyl moiety. $\mathbf{R_3}$ may independently represent a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, or $C_{2-12}$heteroalkynyl; wherein each of the foregoing alkyl, alkenyl, alkynyl, heteroalkyl, heteroalkenyl or heteroalkynyl group may optionally bear one or more substituents selected from substituted or unsubstituted phenyl, -OR$^{A3}$, -SR$^{A3}$, F, Cl, Br, I, $-NO_2$, -CN, $-CF_3$, $-OCF_3$, $OCH_3$, $-CH_2CF_3$, or $CHCl_2$; wherein each occurrence of $R^{A3}$ independently represents $C_{1-12}$alkyl. $\mathbf{R_3}$ may independently represent cyclic or acyclic, linear or branched $C_{1-6}$alkyl, $C_{1-6}$heteroalkyl, $C_{1-6}$haloalkyl, $-C_{1-6}$alkyl-OC$_{1-6}$alkyl, $-C_{1-6}$alkyl-SC$_{1-6}$alkyl substituted or unsubstituted benzyl. $\mathbf{R_3}$ may independently represent methyl, ethyl, n-propyl, i-propyl, n-butyl, i-butyl, tert-butyl, cyclopropyl, cyclobutyl, cyclopentyl, cyclohexyl, benzyl, p-nitrobenzyl, $-C_{1-6}$alkyl-OC$_{1-6}$alkyl such as-$(CH_2)_2$-OMe, $-(CH_2)_3$- OMe, $-(CH_2)_4$- OMe, $-(CH_2)_5$- OMe or $-(CH_2)_6$- OMe, or $-C_{1-6}$alkyl-SC$_{1-6}$alkyl such as-$(CH_2)_2$-SMe, $-(CH_2)_3$-SMe, $-(CH_2)_4$-SMe, $-(CH_2)_5$-SMe or $-(CH_2)_6$-SMe.

Section D

$\mathbf{R_4}$ may independently represent H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; -OR$^{A4}$; - C(=O)R$^{A4}$; or -SR$^{A4}$; wherein each occurrence of $R^{A4}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl, group may optionally bear one or more substitutents selected from $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl, $C_{4-10}$heteroaryl, F; Cl; Br; I; $-NO_2$; -CN; $-CF_3$; $-CH_2CF_3$; $CHCl_2$; or -GR$^{B4}$ wherein G is -O-, -S-, or -NR$^{C4}$-, wherein each occurrence of $R^{B4}$ and $R^{C4}$ independently represents independently represents hydrogen, or a $C_{1-12}$alkyl or $C_{1-12}$haloalkyl group. $\mathbf{R_4}$ may independently represent H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; -OR$^{A4}$; -C(=O)R$^{A4}$; or -SR$^{A4}$; wherein each occurrence of $R^{A4}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group;

wherein each of the foregoing alkyl, alkenyl, alkynyl, heteroalkyl, heteroalkenyl, heteroalkynyl, aryl or heteroaryl, group may optionally bear one or more substitutents selected from $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl, $C_{4-10}$heteroaryl, F; Cl; Br; I; $-NO_2$; -CN; $-CF_3$; $-OCF_3$, $-OCH_3$, $-CH_2CF_3$ or $CHCl_2$. **$R_4$** may independently represent H or cyclic or acyclic, linear or branched $C_{1-6}$alkyl, $C_{1-6}$heteroalkyl, $C_{1-6}$haloalkyl or benzyl. Preferably, **$R_4$** may independently represent H, $C_{1-12}$alkyl or benzyl. Preferably, **$R_4$** may independently represent H, $C_{1-6}$alkyl or benzyl. **$R_4$** may independently represent H, methyl, ethyl, n-propyl, i-propyl, n-butyl, i-butyl, tert-butyl, cyclopropyl, cyclobutyl, cyclopentyl, cyclohexyl or benzyl. **$R_4$** may independently represent H, methyl, ethyl, n-propyl, i-propyl, n-butyl, i-butyl, or tert-butyl, or benzyl.

In exemplary embodiments, **$R_3$** may independently represent linear or branched $C_{1-6}$alkyl, and **$R_4$** may independently represent H or cyclic or acyclic, linear or branched $C_{1-6}$alkyl, $C_{1-6}$heteroalkyl, $C_{1-6}$haloalkyl or benzyl. For example **$R_3$** may independently represent methyl, ethyl, n-propyl, i-propyl, n-butyl, i-butyl, tert-butyl, and **$R_4$** may independently represent H, methyl, ethyl, n-propyl, i-propyl, n-butyl, i-butyl, tert-butyl, cyclopropyl, cyclobutyl, cyclopentyl, cyclohexyl or benzyl. For example **$R_3$** may independently represent methyl, and **$R_4$** may independently represent H, methyl, ethyl, n-propyl, i-propyl, n-butyl, i-butyl, tert-butyl, cyclopropyl, cyclobutyl, cyclopentyl, cyclohexyl or benzyl.

In other exemplary embodiments, **$R_3$** may independently represent cyclic or acyclic, linear or branched $C_{1-6}$alkyl, $C_{1-6}$heteroalkyl, $C_{1-6}$haloalkyl, $-C_{1-6}$alkyl-$OC_{1-6}$alkyl, $-C_{1-6}$alkyl-$SC_{1-6}$alkyl substituted or unsubstituted benzyl, and **$R_4$** may independently represent H. For example **$R_3$** may independently represent methyl, ethyl, n-propyl, i-propyl, n-butyl, i-butyl, tert-butyl, cyclopropyl, cyclobutyl, cyclopentyl, cyclohexyl, benzyl, p-nitrobenzyl, $-C_{1-6}$alkyl-$OC_{1-6}$alkyl such as$-(CH_2)_2$-OMe, $-(CH_2)_3$- OMe, $-(CH_2)_4$- OMe, $-(CH_2)_5$- OMe or - $(CH_2)_6$- OMe, or $-C_{1-6}$alkyl-$SC_{1-6}$alkyl such as$-(CH_2)_2$-SMe, $-(CH_2)_3$-SMe, $-(CH_2)_4$-SMe, - $(CH_2)_5$-SMe or $-(CH_2)_6$-SMe, and **$R_4$** may independently represent H.

Copolymer topologies

The process according to the present invention allows access to a great variety of cationic peptoid / N-substituted peptidic copolymers of different topologies. For example, the process may lead to linear cationic peptoid / N-substituted peptidic copolymers, macrocyclic cationic peptoid / N-substituted peptidic copolymers, dendritic cationic peptoid / N-substituted peptidic copolymers, or cationic peptoid / N-substituted peptidic copolymer-drug conjugates.

The different copolymer topologies may be obtained depending on the reaction conditions used.

For example :

- linear cationic peptoid / N-substituted peptidic copolymers may be obtained by carrying out the process in the presence of a nucleophilic base ;
- macrocyclic cationic peptoid / N-substituted peptidic copolymers may be obtained by carrying out the process in the presence of a strong base ;
- dendritic cationic peptoid / N-substituted peptidic copolymers may be obtained by carrying out the process in the presence of a base such as a tertiary amine and a dendritic nucleophile ; Alternatively, if the dendritic nucleophile is basic, the process may be carried out without an additional base such as a tertiary amine ;
- cationic peptoid / N-substituted peptidic copolymer-drug conjugates may be obtained by carrying out the process in the presence of a base such as a tertiary amine and a drug nucleophile ; Alternatively, if the drug nucleophile is basic, the process may be carried out without an additional base such as a tertiary amine.

Thus, the presence of the base, as additional component, may not be necessary of the initiator molecule is basic.

In the process according to the invention, the first and second N-carboxyanhydride monomers may have the structure

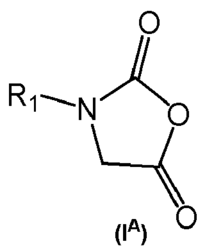
($I^A$) and
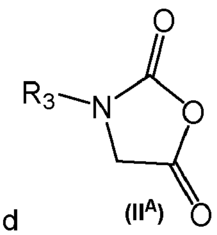
($II^A$),

respectively;

wherein $R_1$ represents an optionally protected cationic side chain of a natural or modified amino acid, and $R_3$ represents an optionally protected hydrophobic side chain of a natural or modified amino acid.

In the process according to the invention, the first N-carboxyanhydride monomers may have the structure

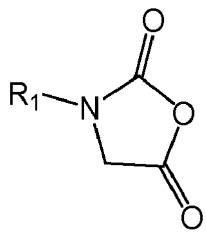

wherein $R_1$ represents $-C_{1-6}alkyl-NHP_1$, wherein $P_1$ represents an amine protecting group, such as -(C=O)OPh.

In the process according to the invention, the first and second N-carboxyanhydride monomers may have the structure respectively;

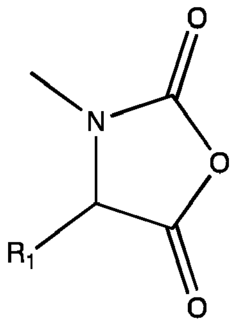

and

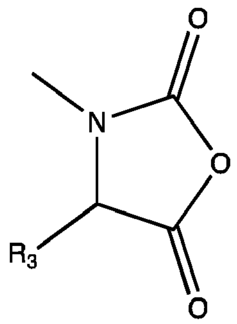

wherein $R_1$ represents an optionally protected cationic side chain of a natural or modified amino acid, and $R_3$ represents an optionally protected hydrophobic side chain of a natural or modified amino acid.

In the process according to the invention, the first N-carboxyanhydride monomers may have the structure

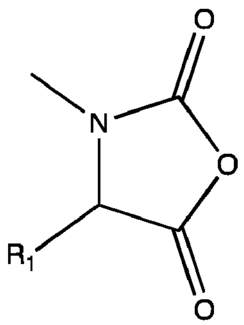

wherein $R_1$ represents $-C_{1-6}alkyl-NHP_1$, wherein $P_1$ represents an amine protecting group, such as -(C=O)OPh.

In the process according to the invention, the molar ratio first N-carboxyanhydride monomer: second N-carboxyanhydride monomer may range between 0 : 100 and 100 : 0, preferably 50:50 and preferentially with cationic content higher than 50% . In other words, the ratio x:y may range between 0:1 and 1:0; for example between 0.1:0.9 and 1:0, for example between 0.2:0.8 and 1:0, for example between 0.3:0.7 and 1:0, for example between 0.4:0.6 and 1:0, for example between 0.5:0.5 and 1:0, for example between 0.6:0.4 and 1:0, for example between 0.7:0.3 and 1:0, for example between 0.8:0.2 and 1:0, for example between 0.9:0.1 and 1:0, for example 0.5:0.5, most preferably $1 \geq x \geq 0.5$. The process according to the present disclosure may be carried out with only one type of N-carboxyanhydride monomers: non-cationic or cationic N-carboxyanhydride monomers (i.e., x=0, or y=0, respectively). Preferably, the process involves at least cationic N-carboxyanhydride monomers (i.e., $x \neq 0$). Preferably, the process involves at least cationic N-carboxyanhydride monomers ("first N-carboxyanhydride monomer") and a second type of N-carboxyanhydride monomers ("second N-carboxyanhydride monomer") (i.e., $x \neq 0$ and $y \neq 0$).

In the process according to the invention, the polar organic solvent may be a non aqueous polar organic solvent, such as such as a solvent with a dielectric constant of greater than 7, preferably greater than 10, preferably greater than 15. For example, the polar solvent may be an aprotic organic solvent such as DCM (dichloromethane), DMF (N,N-dimethylformamide), Dioxane, DMSO (dimethyl sulfoxide), Benzonitrile, THF (tetrahydrofuran) or a mixture of two or more thereof.

Linear peptoid/N-substituted peptidic copolymers

The process according to the invention may be for the preparation of linear peptoid / N-substituted peptidic copolymers, and the process may be carried out in the presence of at least one nucleophilic compound initiator selected from primary amines. Suitable primary amines include aromatic, cycloaliphatic, heterocyclic or aliphatic primary amines. For example, the primary amine may be cyclic or acyclic, substituted or unsubstituted, linear or branched $C_{1-12}$alkyl-$NH_2$, $C_{2-12}$alkenyl-$NH_2$, $C_{2-12}$alkynyl-$NH_2$, $C_{1-12}$heteroalkyl-$NH_2$, $C_{2-12}$heteroalkenyl-$NH_2$, $C_{2-12}$heteroalkynyl-$NH_2$, substituted or unsubstituted $C_{6-10}$aryl-$NH_2$ or $C_{4-10}$heteroaryl-$NH_2$. As exemplary primary amines may be mentioned allylamine, n-hexylamine, n-decylamine, n-dodecylamine or n-octadecylamine.

Accordingly, the linear peptoid / N-substituted peptidic copolymer may have the following structure:

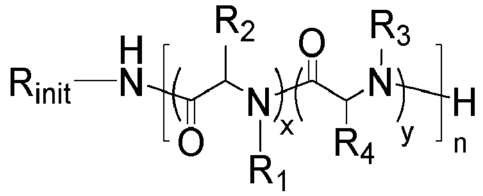

**($V_{linear}$)**

wherein $R_1$, $R_2$, $R_3$ and $R_4$ may be as defined in any classes, subclasses and variants above;
$R_{init}$ represents the radical of the at least one nucleophilic compound initiator, such as allyl, n-hexyl, n-decyl, n-dodecyl or n-octadecyl;
x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer; and
n represents the length of the copolymer (the number of first and second N-carboxyanhydride monomer unit in the copolymer).

For example, $R_1$, $R_2$, $R_3$ and $R_4$ may be as defined in sections A, B, C and D, respectively.

For example, $R_{init}$ may represent an aromatic, cycloaliphatic, heterocyclic or aliphatic group. $R_{init}$ may represent a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl. For example, $R_{init}$ may represent allyl, n-hexyl, n-decyl, n-dodecyl or n-octadecyl.

For example, n may range from 5-100, for example 5-80, for example 5-60, for example 5-50, for example 5-40, for example 5-30, for example 5-20, for example 5-10.

Macrocylic peptoid/N-substituted peptidic copolymers

In another aspect, the process according to the invention may be for the preparation of macrocylic peptoid/N-substituted peptidic copolymers, and the process may be carried out in the presence of at least a strong base selected from lithium, sodium or potassium amides, lithium, sodium or potassium hydrides, or phosphazenes. For example, the strong base may be a lithium amide, such as lithium bis(trimethylsilyl) amide. Accordingly, the macrocylic peptoid / N-substituted peptidic copolymer may have the following structure:

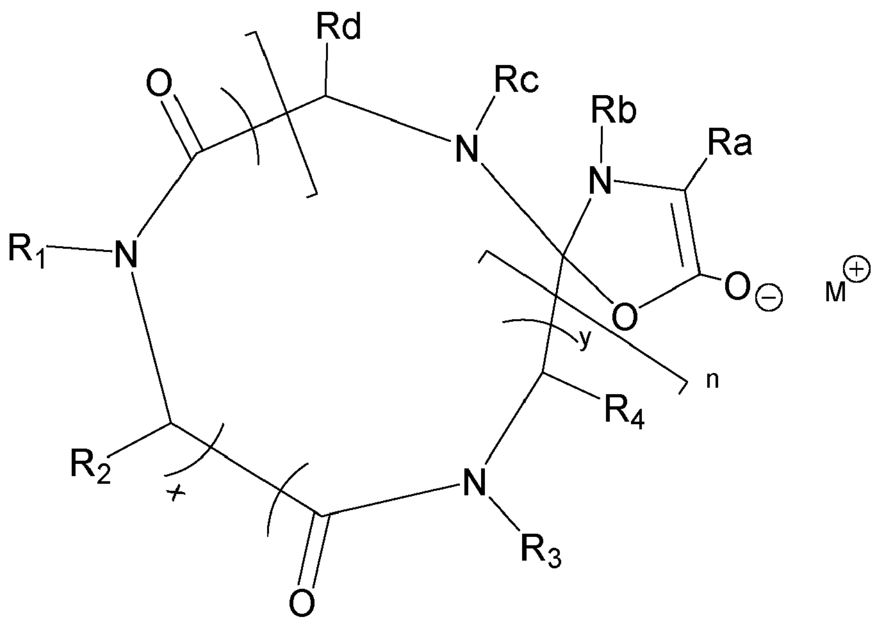

**($V^{A}_{macrocyclic}$)**

or

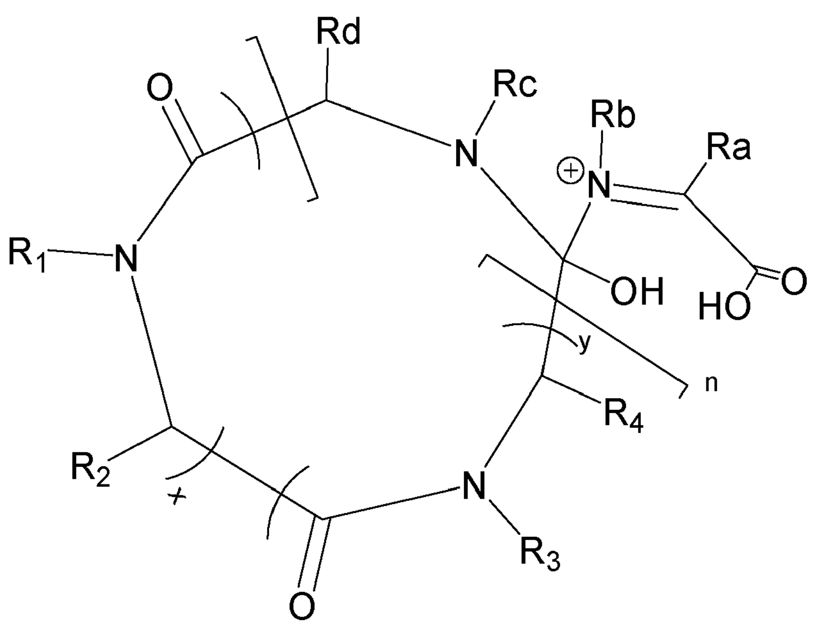

**($V^{B}_{macrocyclic}$)**

wherein $R_1$, $R_2$, $R_3$ and $R_4$ may be as defined in any classes, subclasses and variants above;
(Ra, Rb) may represent ($R_1$, $R_2$) or ($R_3$, $R_4$), respectively;
independently of (Ra, Rb) , (Rc, Rd) may represent ($R_1$, $R_2$) or ($R_3$, $R_4$), respectively;
$M^+$ represents a counterion;
x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer; and
n represents the number of first and second N-carboxyanhydride monomer unit in the copolymer chain.

For example, $R_1$, $R_2$, $R_3$ and $R_4$ may be as defined in sections A, B, C and D, respectively. For example, n

may range from 5-100, for example 5-80, for example 5-60, for example 5-50, for example 5-40, for example 5-30, for example 5-20, for example 5-10.

In the above macrocyclic structure,

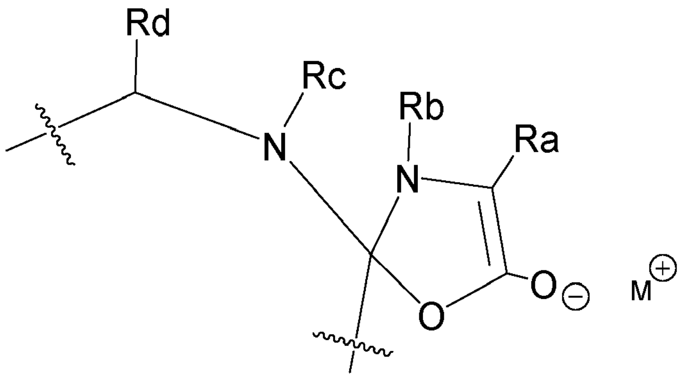

represents the structure resulting from the initiator species. See proposed mechanism of action in FIG. 36.

For example $M^+$ may represent $H^+$, Li+, $Na^+$, $K^+$ or an alkali metal salt such as LiHMDS.

For example, in the macrocylic peptidic copolymer, $R_1$ and $R_3$ may be different. For example, in the macrocylic peptidic copolymer, $R_2$ and $R_4$ may each be a hydrogen atom. For example, in the macrocylic peptidic copolymer, $R_2$ and $R_4$ may each be a hydrogen atom;

$R_1$ may represent a linear or branched $C_{1-12}$alkyl, $C_{2-12}$alkenyl, $C_{2-12}$alkynyl, $C_{1-12}$heteroalkyl, $C_{2-12}$heteroalkenyl or $C_{2-12}$heteroalkynyl group bearing an $NH_3^+$ moiety; such as $-(CH_2)_4-NH_3^+$ (the cationic form of a lysine residue side-chain); and $R_3$ may represent a hydrophobic cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; each of the foregoing alkyl, alkenyl, alkynyl, heteroalkyl, heteroalkenyl, heteroalkynyl, aryl or heteroaryl group; such as the $-CH_2Ph$ side-chain of a phenylalanine residue.

Dendritic peptoid/N-substituted peptidic copolymers

In another aspect, the process according to the invention may be for the preparation of dendritic peptoid / N-substituted peptidic copolymers, and the process may be carried out in the presence of at least one dendritic synthon comprising at least three nucleophilic moieties selected from amino, thio or hydroxyl groups, and optionally at least one base selected from tertiary amines such as triethylamine or N, N-Diisopropylethylamine. A tertiary amine base may be used when the nucleophilic moieties of the dendritic synthon are not basic, for example when a dendritic synthon comprising at least three nucleophilic moieties selected from thio or hydroxyl groups is used. The dendritic synthon may comprise 3-20 nucleophilic moieties selected from amino, thio or hydroxyl groups; preferably 4-20, for example 4-16, nucleophilic moieties selected from primary amino groups. For example, the dendritic synthon may be a PAMAM synthon having one of the following structures:

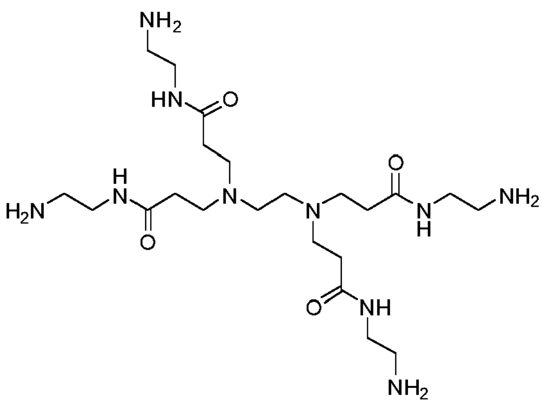

**(PAMAM)-4-arms-NH $_2$**

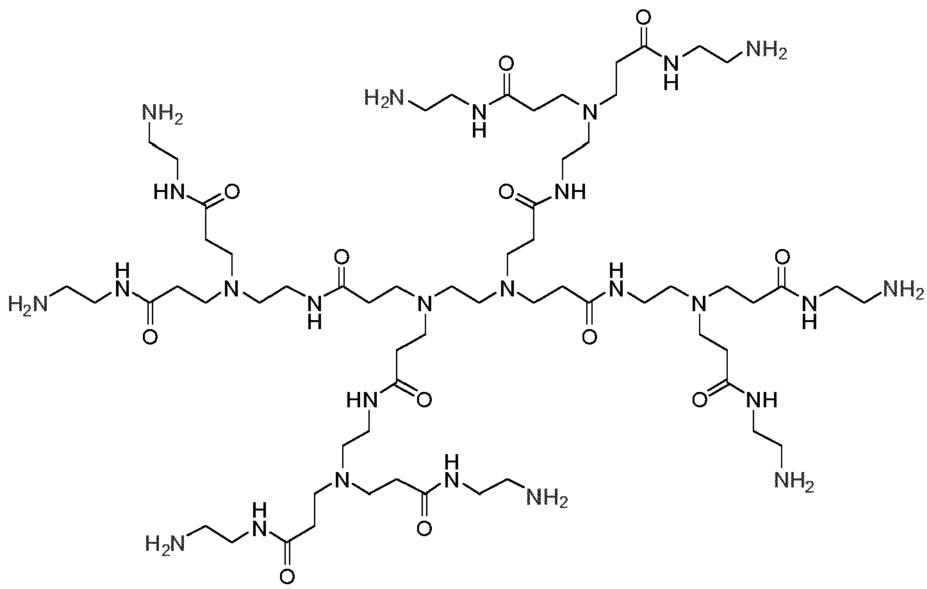

**(PAMAM)-8-arms-NH 2**

,

or

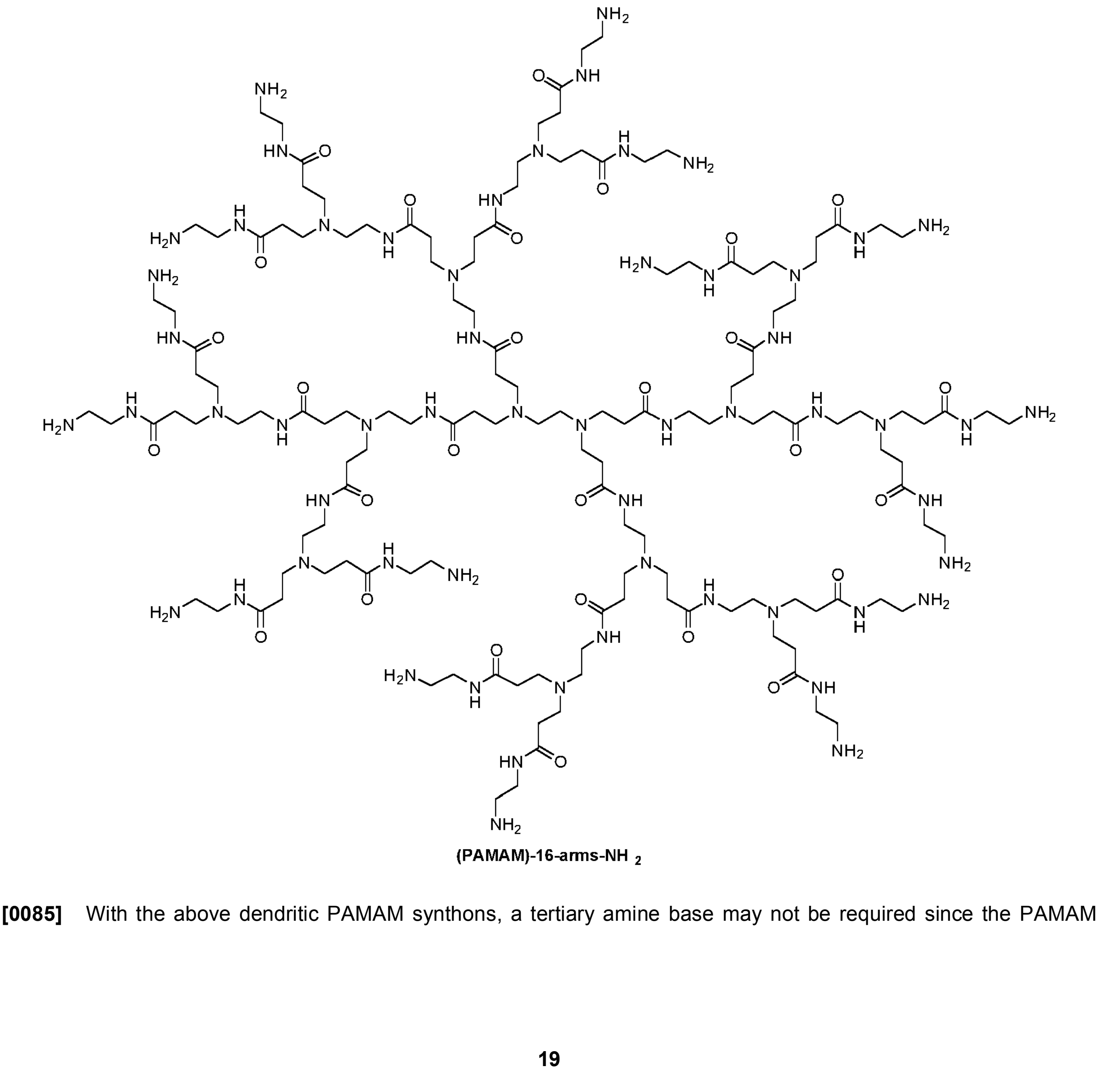

**(PAMAM)-16-arms-NH 2**

With the above dendritic PAMAM synthons, a tertiary amine base may not be required since the PAMAM

dendritic synthons contains basic amino groups.

Accordingly, the dendritic peptoid / N-substituted peptidic copolymer may have the following structure:

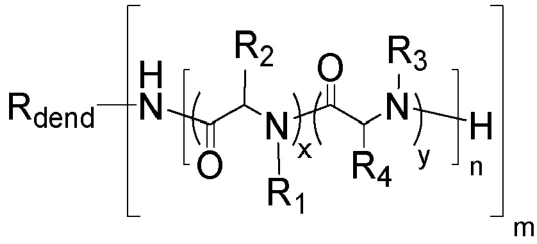

**($V_{dend}$)**

wherein $R_1$, $R_2$, $R_3$ and $R_4$ may be as defined in any classes, subclasses and variants above;
$R_{dend}$ represents a dendritic synthon radical having one of the following structures:

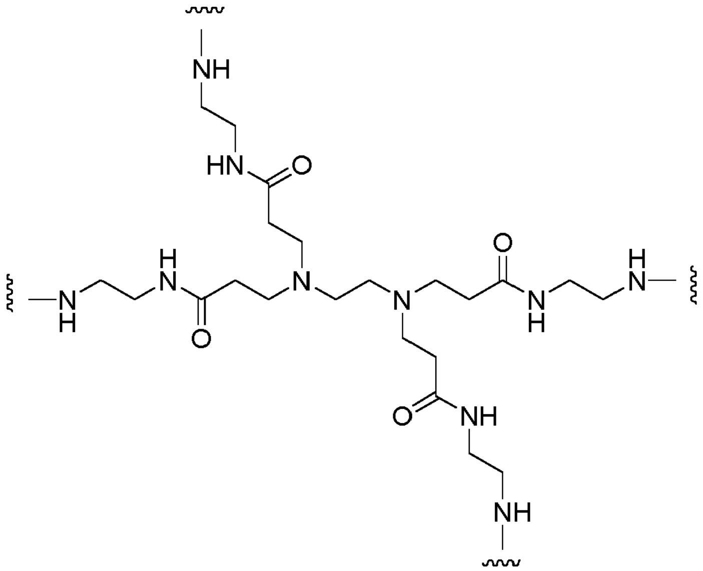

,

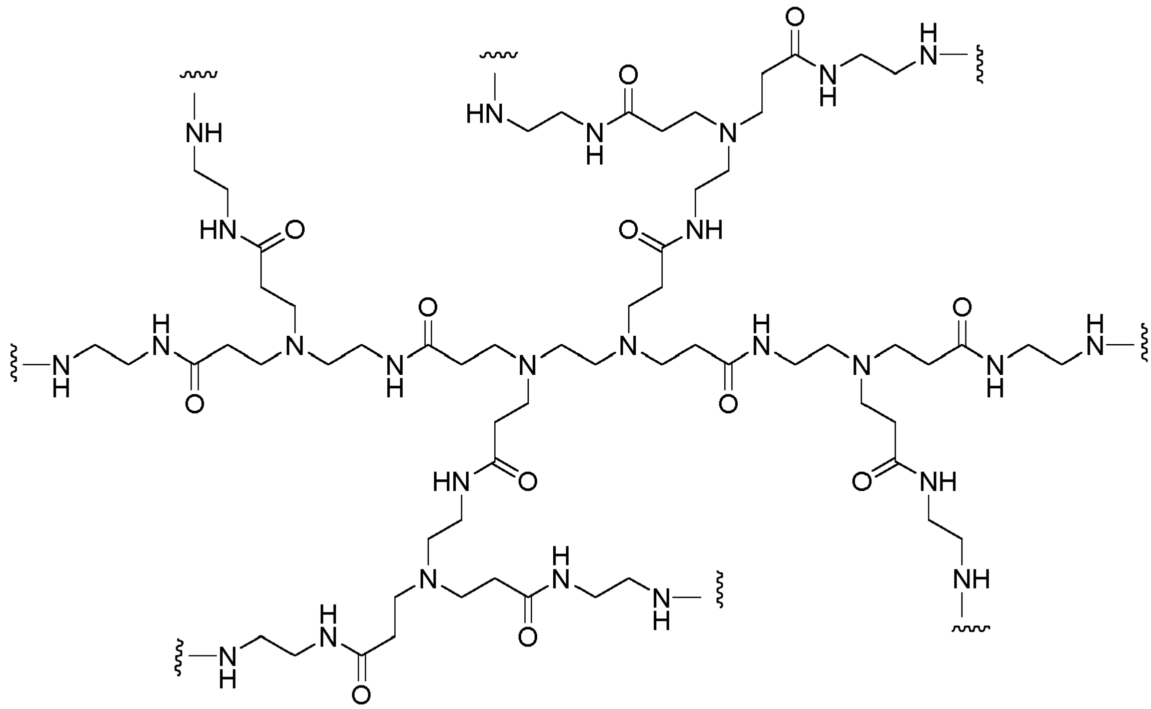

or

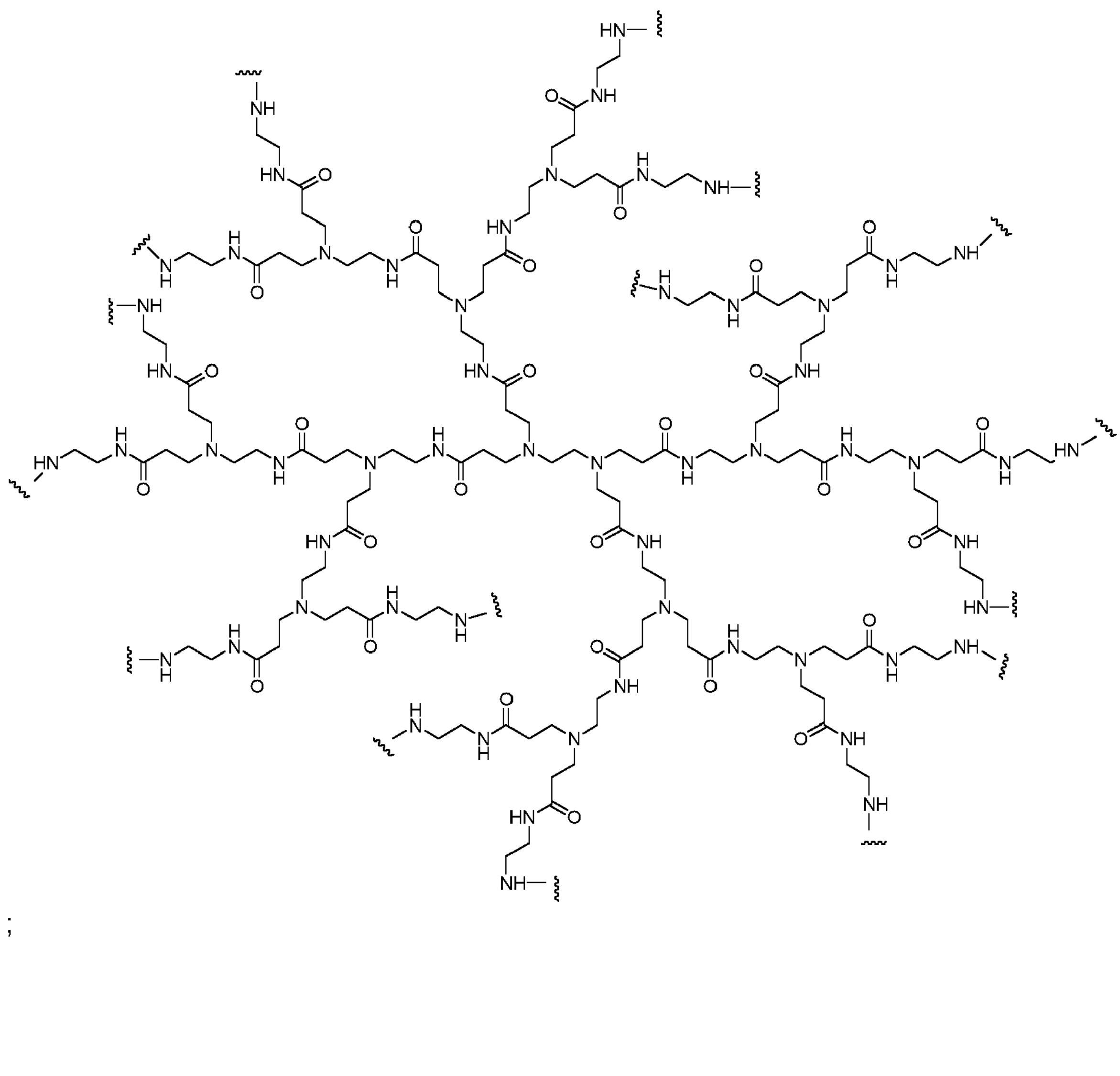

;

x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer;
each occurrence of n independently the number of first and second N-carboxyanhydride monomer unit in the copolymer branch; and
m represents the number of copolymer units covalently bound to the dendrimer synthon. It is understood that for each occurrence of the bracketed structure m, n may differ (i.e., the copolymer branches present on the dendrimer may not all have the same length). For example, $R_1$, $R_2$, $R_3$ and $R_4$ may be as defined in sections A, B, C and D, respectively.

For example, n may range from 5-100, for example 5-80, for example 5-60, for example 5-50, for example 5-40, for example 5-30, for example 5-20, for example 5-10. For example, m may range from 3-20, for example 4-20, for example 4-16. For example m may be 4, 8 or 16.

Peptoid/N-substituted peptidic copolymer-drug conjugates

In another aspect, the process according to the invention may be for the preparation of peptoid / N-substituted peptidic copolymer-drug conjugates, and the process may be carried out in the presence of at least one drug molecule bearing at least one nucleophilic moiety, and optionally at least one base selected from tertiary amines such as trimethylamine or N, N-Diisopropylethylamine. The at least one nucleophilic moiety present on the drug molecule may be selected from amino, thio or hydroxyl groups. A tertiary amine base may be used when the at least one nucleophilic moiety present on the drug molecule is not basic, for example when a drug molecule comprising at least one nucleophilic moiety selected from thio (-SH) or hydroxyl groups is used. The drug molecule may comprise more than one nucleophilic moieties selected from amino, thio or hydroxyl groups. For example, the at least one drug molecule may bear at least one basic primary amino group. In that case, the process is carried out without a tertiary amine, since the drug molecule already contains at least one basic amino group. For example, the at least one drug molecule may be vancomycin, amoxicilline, kanamycine, cefaclor, amphotericin B, nystatin, amikacin or sulphamethoxazole.

For example, the peptoid / N-substituted peptidic copolymer-drug conjugate may have the following structure:

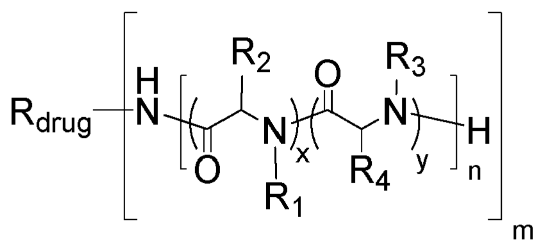

**($V_{conj}$)**

wherein $R_1$, $R_2$, $R_3$ and $R_4$ may be as defined in any classes, subclasses and variants above;
$R_{drug}$ represents the radical of the drug molecule bearing at least one basic primary amino group, such as vancomycin, amoxicilline, kanamycine, cefaclor, amphotericin B, nystatin, amikacin or sulphamethoxazole;
x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer;
each occurrence of n independently the number of first and second N-carboxyanhydride monomer unit in the copolymer; and
m represents the number of copolymer units covalently bound to the drug molecule.

It is understood that for each occurrence of the bracketed structure m, n may differ (i.e., the copolymer branches present on the drug conjugate may not all have the same length).

For example, $R_1$, $R_2$, $R_3$ and $R_4$ may be as defined in sections A, B, C and D, respectively.

For example, n may range from 5-100, for example 5-80, for example 5-60, for example 5-50, for example 5-40, for example 5-30, for example 5-20, for example 5-10. For example, m may range from 1-10, for example 1-8, for example 1-6, for example 1-5, for example 1, 2, 3 or 4. For example m may be 1.

For example, the group $R_{drug}$ in formula ($V_{conj}$) may be derived fom an antiinfective molecule, such as amphotericin B, vancomycin, nystatin, amikacin, or sulphamethoxazole. For example, the group $R_{drug}$ in formula ($V_{conj}$) may have one of the following structures, wherein the arrows designate the point(s) of attachment of the N-substituted

copolymer to the drug moiety $R_{drug}$:

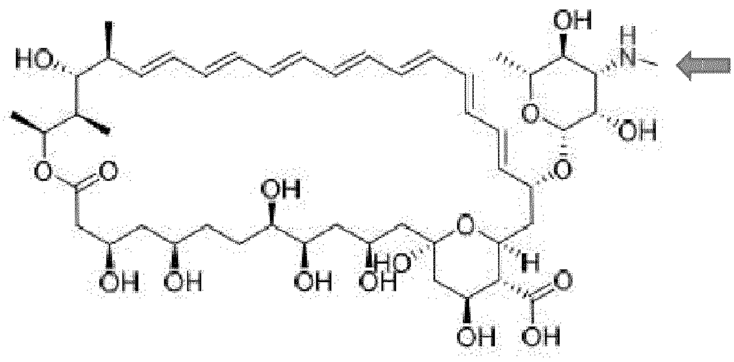

,

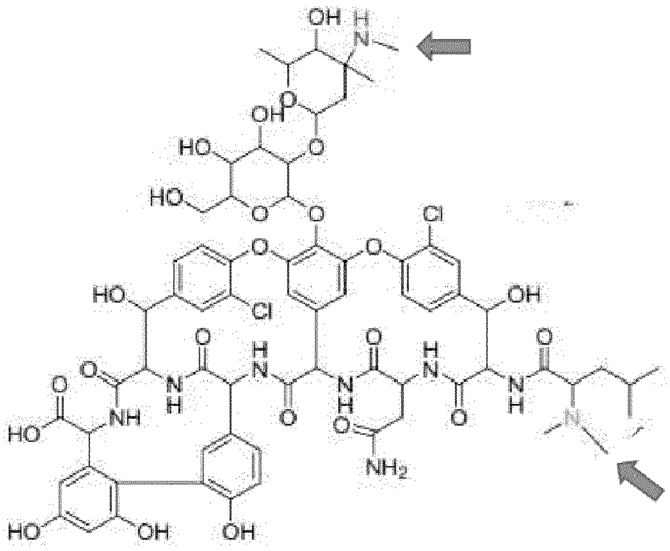

,

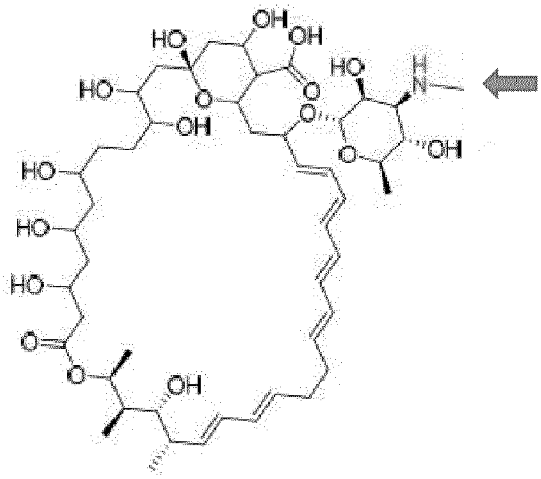

,

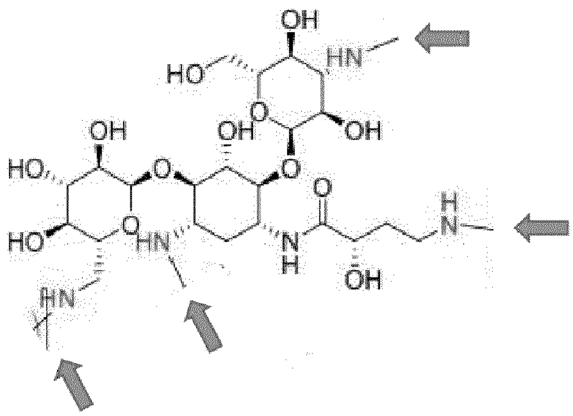

or

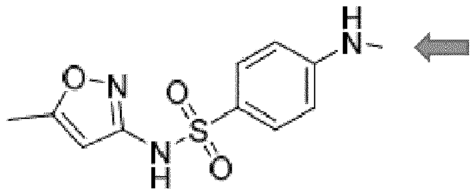

.

For example, the peptoid / N-substituted peptidic copolymer-drug conjugate may have one of the following structures:

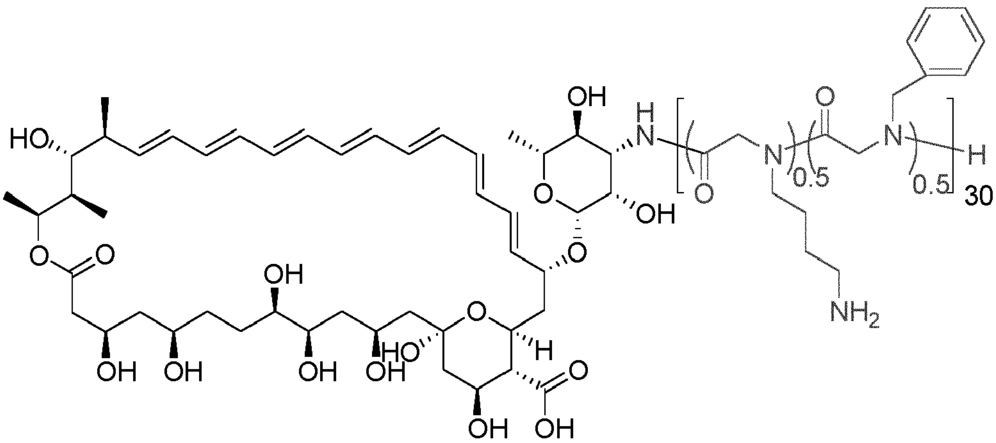

,

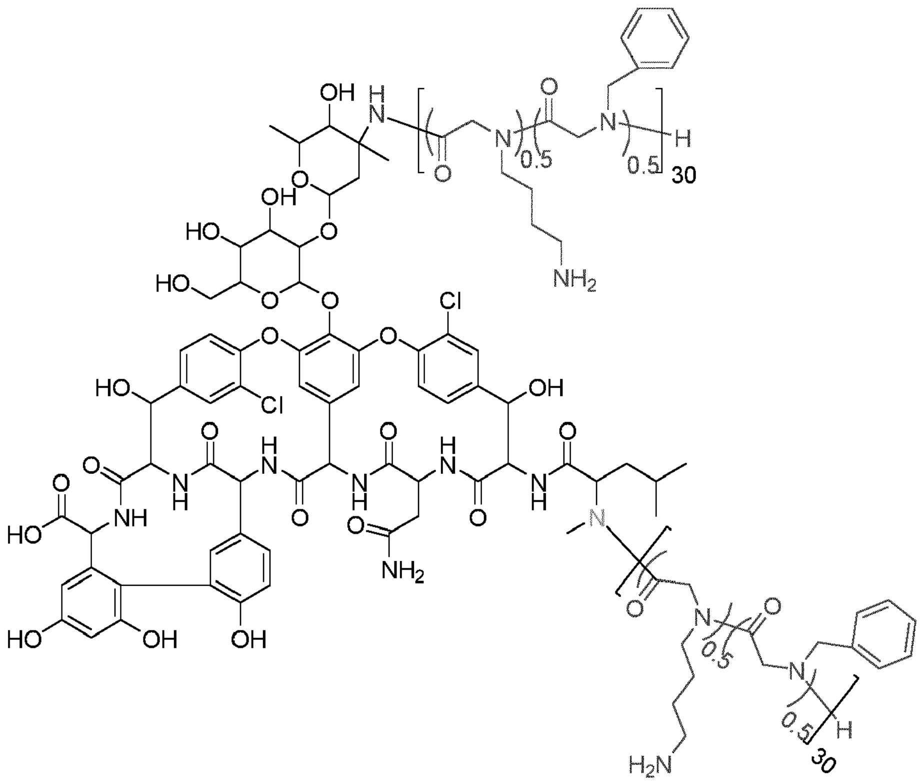

,

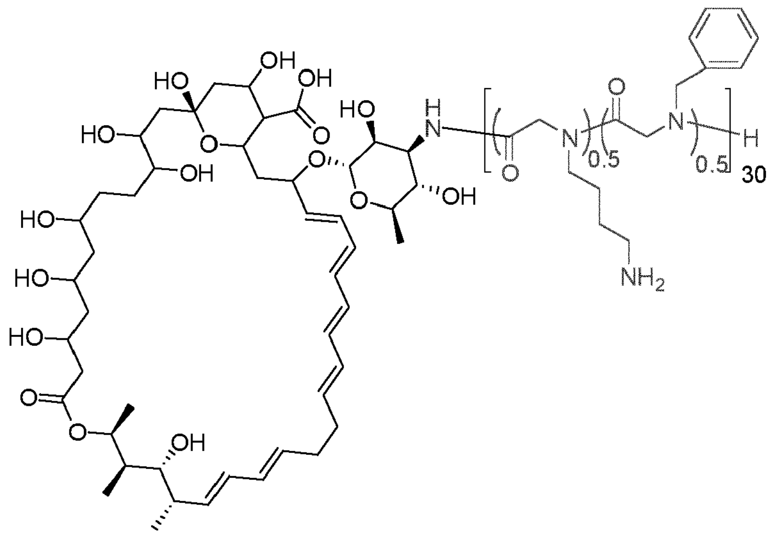

,

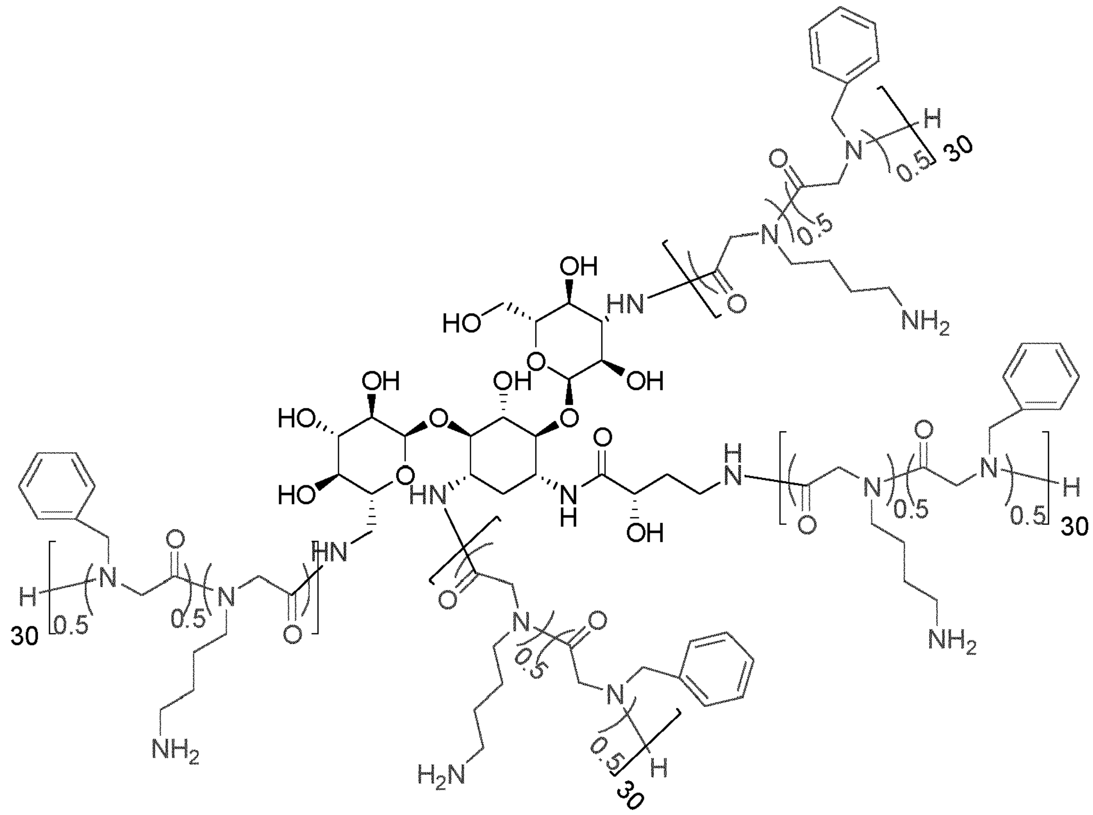

or

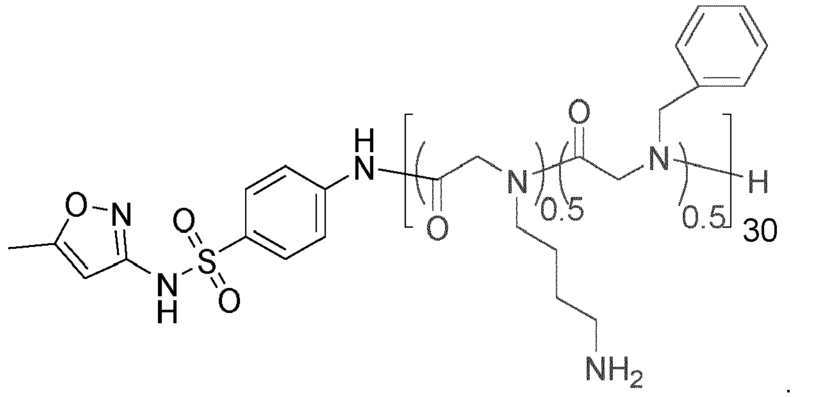

In formulae ($V_{linear}$), ($V^A_{macrocylic}$), ($V^b_{macrocylic}$), ($V_{dend}$) and ($V_{conj}$) described above, $R_1$, $R_2$, $R_3$ and $R_4$ may be as defined in sections A, B, C and D, respectively. For example, in formulae ($V_{linear}$), ($V^A_{macrocylic}$), ($V^b_{macrocylic}$), ($V_{dend}$) and ($V_{conj}$) described above $R_1$ may independently represent a cyclic or acyclic, substituted or unsubstituted, linear or branched $C_{1-12}$alkyl bearing at least one cationic moiety, such as $NH_3^+$, or protected form thereof. For example, $R_1$ may independently represent an optionally protected cationic side chain of a natural or modified amino acid. For example, $R_1$ may independently represent a cationic side chain of a natural or modified amino acid bearing at least one $NH_3^+$ moiety. For example, $R_1$ may independently represent $-C_{1-6}$alkyl-$NHP_1$, wherein $P_1$ represents an amine protecting group, such as -(C=O)OPh or Cbz. For example, $R_1$ may independently represent-$(CH_2)_2$-$NHP_1$, -$(CH_2)_3$-$NHP_1$, -$(CH_2)_4$-$NHP_1$, -$(CH_2)_5$-$NHP_1$ or-$(CH_2)_6$-$NHP_1$, wherein $P_1$ represents an amine protecting group, such as -(C=O)OPh or Cbz. Preferably $P_1$ may represent -(C=O)OPh. For example, $R_1$ may independently represent $-C_{1-6}$alkyl- $NH_3^+$ For example, $R_1$ may independently represent -$(CH_2)_2$- $NH_3^+$, -$(CH_2)_3$- $NH_3^+$, -$(CH_2)_4$- $NH_3^+$, -$(CH_2)_5$- $NH_3^+$ or-$(CH_2)_6$- $NH_3^+$.

For example, in formulae ($V_{linear}$), ($V^A_{macrocylic}$), ($V^b_{macrocylic}$), ($V_{dend}$) and ($V_{conj}$) described above $R_2$ may independently represent a cyclic or acyclic, substituted or unsubstituted, linear or branched $C_{1-12}$alkyl bearing at least one cationic moiety or protected form thereof. For example, $R_2$ may independently represent an optionally protected cationic side chain of a natural or modified amino acid. For example, $R_2$ may independently represent a cationic side

chain of a natural or modified amino acid bearing at least one $NH_3^+$ moiety. For example, $R_2$ may independently represent $-C_{1-6}$alkyl-$NHP_1$, wherein $P_1$ represents an amine protecting group, such as -(C=O)OPh or Cbz. For example, $R_2$ may independently represent $-(CH_2)_2-NHP_1$, $-(CH_2)_3-NHP_1$, $-(CH_2)_4-NHP_1$, $-(CH_2)_5-NHP_1$ or$-(CH_2)_6-NHP_1$, wherein $P_1$ represents an amine protecting group, such as -(C=O)OPh or Cbz. Preferably $P_1$ may represent -(C=O)OPh. For example, $R_2$ may independently represent $-C_{1-6}$alkyl- $NH_3^+$ For example, $R_1$ may independently represent $-(CH_2)_2$- $NH_3^+$, $-(CH_2)_3$-$NH_3^+$, $-(CH_2)_4$- $NH_3^+$, $-(CH_2)_5$- $NH_3^+$ or $-(CH_2)_6$- $NH_3^+$.

In exemplary embodiments, in formulae ($V_{linear}$), ($V^b$ $_{macrocylic}$), ($V^b$ $_{macrocylic}$), ($V_{dend}$) and ($V_{conj}$) described above $R_1$ may bear at least one cationic moiety or protected form thereof, and $R_2$ may independently represent H, or a cyclic or acyclic, substituted or unsubstituted, linear or branched $C_{1-12}$alkyl, optionally substituted with F; Cl, Br, I, $-NO_2$, -CN, $-CF_3$, - $OCF_3$, $OCH_3$, $-CH_2CF_3$ or $CHCl_2$. Preferably, $R_2$ may represent H, and $R_1$ may independently represent a cyclic or acyclic, substituted or unsubstituted, linear or branched $C_{1-12}$alkyl bearing at least one cationic moiety or protected form thereof. For example, $R_2$ may represent H, and $R_1$ may independently represent an optionally protected cationic side chain of a natural or modified amino acid. For example, $R_2$ may represent H, and $R_1$ may independently represent a cationic side chain of a natural or modified amino acid bearing a $NH_3^+$ moiety. For example, $R_2$ may represent H, and $R_1$ may independently represent $-C_{1-6}$alkyl-$NHP_1$, wherein $P_1$ represents an amine protecting group, such as -(C=O)OPh or Cbz. For example, $R_2$ may represent H, and $R_1$ may independently represent $-(CH_2)_2-NHP_1$, $-(CH_2)_3-NHP_1$, $-(CH_2)_4-NHP_1$, $-(CH_2)_5-NHP_1$ or$-(CH_2)_6-NHP_1$, wherein $P_1$ represents an amine protecting group, such as -(C=O)OPh or Cbz. Preferably $P_1$ may represent -(C=O)OPh. For example, $R_2$ may represent H, and $R_1$ may independently represent $-C_{1-6}$alkyl- $NH_3^+$. For example, $R_2$ may represent H, and $R_1$ may independently represent $-(CH_2)_2$- $NH_3^+$, $-(CH_2)_3$- $NH_3^+$, $-(CH_2)_4$- $NH_3^+$, $-(CH_2)_5-NH_3^+$ or $-(CH_2)_6$- $NH_3^+$..

In exemplary embodiments, in formulae ($V_{linear}$), ($V^A{}_{macrocylic}$), ($V^b{}_{macrocylic}$), ($V_{dend}$) and ($V_{conj}$) described above $R_2$ may bear at least one cationic moiety or protected form thereof, and $R_1$ may independently represent a cyclic or acyclic, substituted or unsubstituted, linear or branched $C_{1-12}$alkyl, optionally substituted with F; Cl, Br, I, $-NO_2$, -CN, $-CF_3$, $-OCF_3$, $OCH_3$, $-CH_2CF_3$ or $CHCl_2$. Preferably, $R_1$ may represent linear or branched $C_{1-12}$alkyl, and $R_2$ may independently represent a cyclic or acyclic, substituted or unsubstituted, linear or branched $C_{1-12}$alkyl bearing at least one cationic moiety or protected form thereof. For example, $R_1$ may represent linear or branched $C_{1-6}$alkyl, and $R_2$ may independently represent an optionally protected cationic side chain of a natural or modified amino acid.. For example, $R_1$ may represent linear or branched $C_{1-6}$alkyl, and $R_2$ may independently represent a cationic side chain of a natural or modified amino acid bearing a $NH_3^+$ moiety. For example, $R_1$ may represent linear or branched $C_{1-6}$alkyl, and $R_2$ may independently represent $-C_{1-6}$alkyl-$NHP_1$, wherein $P_1$ represents an amine protecting group, such as - (C=O)OPh or Cbz. For example, $R_1$ may represent methyl, ethyl, n-propyl, i-propyl, n-butyl, i-butyl or tert-butyl, preferably methyl, and $R_2$ may independently represent - $(CH_2)_2-NHP_1$, $-(CH_2)_3-NHP_1$, $-(CH_2)_4-NHP_1$, $-(CH_2)_5-NHP_1$ or $-(CH_2)_6-NHP_1$, wherein $P_1$ represents an amine protecting group, such as -(C=O)OPh or Cbz. Preferably $P_1$ may represent -(C=O)OPh. For example, $R_1$ may represent linear or branched $C_{1-6}$alkyl, and $R_2$ may independently represent $-C_{1-6}$alkyl-$NH_3^+$. For example, $R_1$ may represent methyl, ethyl, n-propyl, i-propyl, n-butyl, i-butyl or tert-butyl, preferably methyl, and $R_2$ may independently represent $-(CH_2)_2$- $NH_3^+$., $-(CH_2)_3$- $NH_3^+$., $-(CH_2)_4$- $NH_3^+$., $-(CH_2)_5$- $NH_3^+$. or $-(CH_2)_6$- $NH_3^+$.

In exemplary embodiments, in formulae ($V_{linear}$), ($V^A{}_{macrocylic}$), ($V^b{}_{macrocylic}$), ($V_{dend}$) and ($V_{conj}$) described above, n may range from 5-100, for example 5-80, for example 5-60, for example 5-50, for example 5-40, for example 5-30, for example 5-20, for example 5-10. In exemplary embodiments, in formula ($V_{dend}$) described above m may range from 3-20, for example 4-20, for example 4-16. For example m may be 4, 8 or 16.

In exemplary embodiments, in formula ($V_{conj}$) described above m may range from 1-10, for example 1-8, for example 1-6, for example 1-5, for example 1, 2, 3 or 4. For example m may be 1.

In exemplary embodiments, in formulae ($V_{linear}$), ($V^A{}_{macrocylic}$), ($V^b{}_{macrocylic}$), ($V_{dend}$) and ($V_{conj}$) described above, the ratio x:y may range between 0:1 and 1:0; for example between 0.1:0.9 and 1:0, for example between 0.2:0.8 and 1:0, for example between 0.3:0.7 and 1:0, for example between 0.4:0.6 and 1:0, for example between 0.5:0.5 and 1:0, for example between 0.6:0.4 and 1:0, for example between 0.7:0.3 and 1:0, for example between 0.8:0.2 and 1:0, for example between 0.9:0.1 and 1:0, for example 0.5:0.5, most preferably $1 \geq x \geq 0.5$.

Synthesis of N-carboxyanhydride monomers

a) Glyoxylic acid route

In the process according to the invention, the first or second N-carboxyanhydride monomer may be prepared by a process comprising steps of:

(i) reacting an amine $R-NH_2$ with a carboxylic acid having the structure R'(C=O)-$CO_2$H to form an amino acid compound having the structure (III):

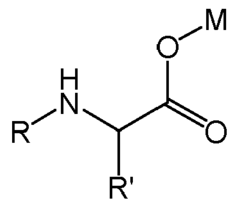

**(III)**

wherein
R represents $R_1$ or $R_3$ as defined in any classes, subclasses and variants above;
R' represents $R_2$ or $R_4$ as defined in any classes, subclasses and variants above, preferably R' may represent H; and
M represents a hydrogen atom or an alkali metal cation selected from Na, Li or K;
(ii) reacting the compound of structure (III) with a nitrogen-protecting group under suitable conditions to form a protected amino acid compound having the structure (IV):

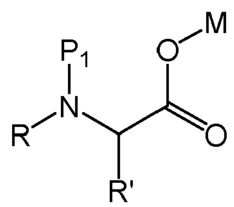

**(IV)**

wherein $P_1$ may represent a suitable amine protecting group, preferably $P_1$ may represent a Boc protecting group; and
(iii) effecting cyclization of compound (IV) under suitable conditions to form a N-carboxyanhydride monomer having the structure:

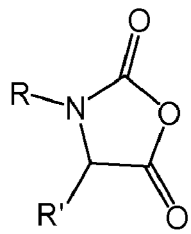

wherein
R represents $R_1$ or $R_3$ as defined in any classes, subclasses and variants above;
; and R' represents $R_2$ or $R_4$ as defined in any classes, subclasses and variants above, preferably R' may represent H.

For example, the resulting N-carboxyanhydride monomer may have one of the flowing structures:

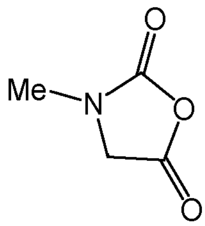 , 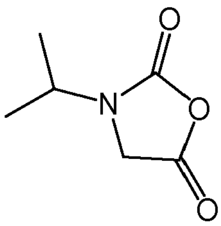 , 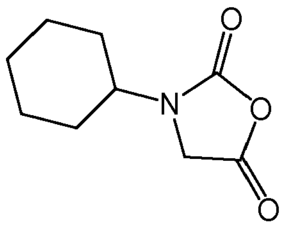 , 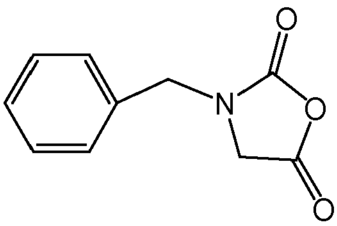 ,

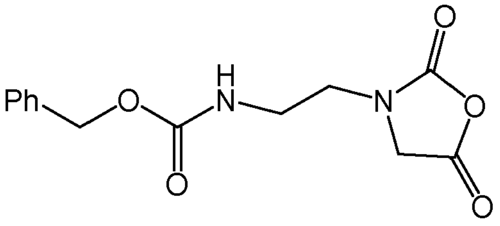,
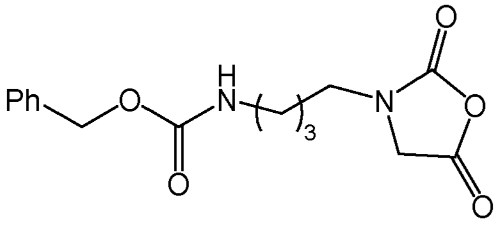,

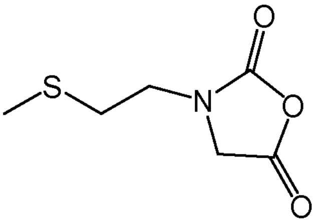,
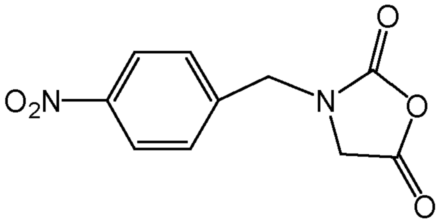 or
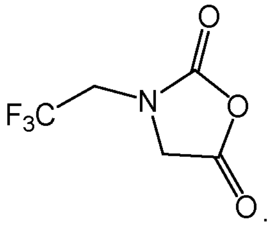.

b) Bio-sourced amino acid route

In the process according to the invention, the first or second N-carboxyanhydride monomer may be prepared by a process comprising steps of:

(i) reacting a natural amino acid

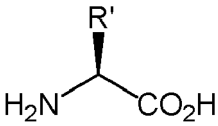

with an amine protecting group, for example $Boc_2O$ or CbzCl, under suitable conditions to form a protected natural amino acid compound having the structure (V):

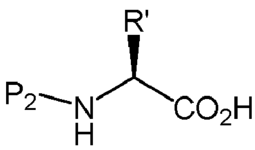

**(V)**

wherein
R' represents $R_2$ or $R_4$ as defined in any classes, subclasses and variants above; and
$P_2$ represents a carbamate protecting group, such as Boc or Cbz;
(ii) reacting the compound of structure (V) with a compound R-X under suitable conditions to form a compound having the structure (VI):

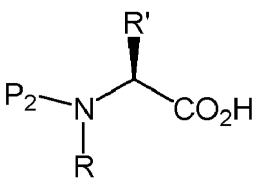

**(VI)**

wherein

R represents $R_1$ or $R_3$ as defined in any classes, subclasses and variants above; and
X represents a suitable leaving group such as a sulfonate leaving group; and
(iii) effecting cyclization of compound (VI) under suitable conditions to form a N-carboxyanhydride monomer having the structure:

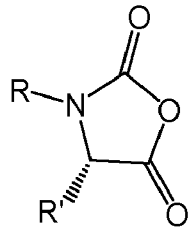

wherein
R represents $R_1$ or $R_3$ as defined in any classes, subclasses and variants above; and R' represents $R_2$ or $R_4$ as defined in any classes, subclasses and variants above.

For example, step (ii) may be carried out with

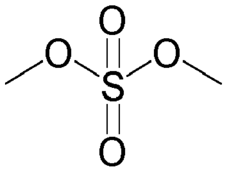

in the presence of NaH.
For example, the resulting N-carboxyanhydride monomer may have one of the flowing structures:

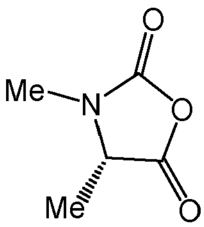,

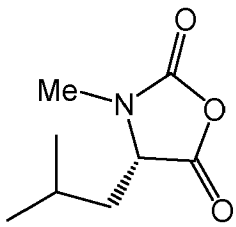,

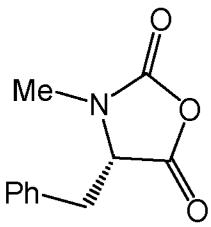

or

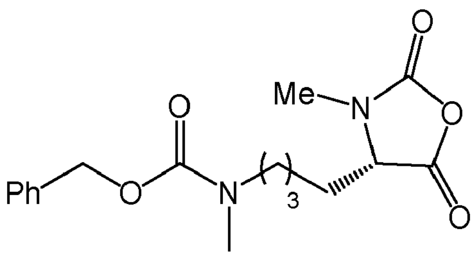.

In the preparation of peptoid / N-substituted peptidic copolymers of the present invention, protection of remote functionality of intermediates from undesired reactions can be accomplished with a protecting group. The term "protecting group" or "Pg" refers to a substituent that is commonly employed to block or protect a particular functionality while reacting other functional groups on the compound. For example, an amine-protecting group is a substituent attached to an amine that blocks or protects the amine-functionality of the compound or intermediate. Suitable amine protecting groups include: 1-tert-butyloxycarbonyl (Boc), acyl groups including: formyl, acetyl, chloroacetyl, trichloroacetyl, o-nitrophenylacetyl, o-nitrophenoxyacetyl, trifluoroacetyl, acetoacetyl, 4-chlorobutyryl, isobutyryl,o-nitrocinnamoyl, picolinoyl,

acylisothiocyanate, aminocaproyl, benzoyl, and the like; and acyloxy groups including: methoxycarbonyl, 9-fluorenylmethoxycarbonyl, 2,2,2-trifluoroethoxycarbonyl, 2-trimethylsilylethxoycarbonyl, vinyloxycarbonyl, allyloxycarbonyl, 1,1-dimethyl-propynyloxycarbonyl, benzyloxycarbonyl, p-nitrobenzyloxycarbony, 2,4-dichlorobenzyloxycarbonyl, and the like. Similarly, diphenylmethane and benzylcarbamates can be used as amine protecting groups. Suitable protecting groups and their respective uses are readily determined by one skilled in the art. For a general description of protecting groups and their use, see T. W. Greene, Protective Groups in Organic Synthesis, John Wiley & Sons, New York, 1991. Peptoid / N-substituted peptidic copolymers obtainable by a process according to the invention are advantageously resistant to peptidases (also named peptide hydrolase or protease) in aqueous medium (the aqueous medium may be *in vivo, in vitro* and/or ex *vivo;* preferably *in vivo).*

Peptoid / N-substituted peptidic copolymers obtainable by a process according to the invention are advantageously effective at killing bacteria. For example, peptoid / N-substituted peptidic copolymers obtainable by a process according to the invention may be advantageously effective at Gram-negative bacteria and Gram-positive bacteria. Peptoid / N-substituted peptidic copolymers obtainable by a process according to the invention advantageously mimick the antimicrobial activity of antimicrobial peptides against *Clostridioïdes difficile, S. aureus meti-S, S. aureus meti-R, Streptococcus pneumoniae, Listeria monocytogenes, Enterococcus faecalis, Escherichia coli, Acinetobacter baumanii, Pseudomonas aeruginosa, Bacteroides fragilis,* and/or *Helicobacter pylori.*

### 2) Pharmaceutical Compositions

As discussed above, the present invention provides antimicrobial cationic peptoid / N-substituted peptidic copolymers, and thus the present copolymers are useful for the treatment of microbial infections. Accordingly, in another aspect of the present invention, pharmaceutically acceptable compositions are provided, wherein these compositions comprise any of the cationic peptoid / N-substituted peptidic copolymers as described herein or pharmaceutically acceptable derivative thereof, and optionally comprise a pharmaceutically acceptable carrier, adjuvant or vehicle. In certain embodiments, these compositions optionally further comprise one or more additional therapeutic agents. Accordingly, the present invention also provides pharmaceutical compositions comprising an effective amount of a peptoid / N-substituted peptidic copolymer obtainable by a process according to the invention or pharmaceutically acceptable derivative thereof, and a pharmaceutically acceptable carrier, adjuvant, or vehicle.

The peptoid / N-substituted peptidic copolymers described herein can be used to prepare therapeutic pharmaceutical compositions, for example, by combining the peptoid / N-substituted peptidic copolymers with a pharmaceutically acceptable diluent, excipient, or carrier. The peptoid / N-substituted peptidic copolymers may be added to a carrier in the form of a salt or solvate. For example, in cases where peptoid / N-substituted peptidic copolymers are sufficiently basic or acidic to form stable non-toxic acid or base salts, administration of the peptoid / N-substituted peptidic copolymers as salts may be appropriate. Examples of pharmaceutically acceptable salts are organic acid addition salts formed with acids that form a physiological acceptable anion, for example, tosylate, methanesulfonate. acetate, citrate, malonate, tartrate, succinate, benzoate, ascorbate, a-ketoglutarate, lactate, phosphate and 3-glycerophosphate. Suitable inorganic salts may also be formed, including hydrochloride, halide, sulfate, nitrate, bicarbonate, and carbonate salts.

Pharmaceutically acceptable salts may be obtained using standard procedures well known in the art, for example by reacting a sufficiently basic compound such as an amine with a suitable acid to provide a physiologically acceptable ionic compound. Alkali metal (for example, sodium, potassium or lithium) or alkaline earth metal (for example, calcium) salts of carboxylic acids can also be prepared by analogous methods.

When employed as pharmaceuticals, especially as antimicrobial agents administered to mammals, the peptoid / N-substituted peptidic copolymers of this disclosure are administered in the form of pharmaceutical compositions. These peptoid / N-substituted peptidic copolymers can be administered by a variety of routes including oral, rectal, transdermal, subcutaneous, intravenous, intramuscular, intrathecal, and intranasal. Such pharmaceutical compositions are prepared in a manner well known in the pharmaceutical art and comprise at least one AMP of this disclosure.

The pharmaceutical compositions of the present invention contain, as the active ingredient, one or more of the peptoid / N-substituted peptidic copolymers of this disclosure, associated with pharmaceutically acceptable formulations. In making the compositions of this invention, the active ingredient is usually mixed with an excipient, diluted by an excipient or enclosed within a carrier which can be in the form of a capsule, sachet, paper or other container. An excipient is usually an inert substance that forms a vehicle for a drug. When the excipient serves as a diluent, it can be a solid, semi-solid, or liquid material, which acts as a vehicle, carrier or medium for the active ingredient. Thus, the compositions can be in the form of solutions, syrups, aerosols (as a solid or in a liquid medium), ointments containing, for example, up to 30% by weight of the active compound, soft and hard gelatin capsules, suppositories, sterile injectable solutions, and sterile packaged powders.

In preparing a formulation, it may be necessary to mill active compounds of this disclosure to provide the appropriate particle size prior to combining with the other ingredients. If the antimicrobial peptoid / N-substituted peptidic copolymers is substantially insoluble, it ordinarily is milled to a particle size of less than 200 mesh. If the peptoid / N-substituted peptidic copolymer is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, gum Arabic, calcium phosphate, alginates, tragacanth, gelatin, calcium silicate, microcrystalline cellulose, polyvinylpyrrolidone, cellulose, sterile water, syrup, and methylcellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of this disclosure can be formulated to provide quick, sustained or delayed release of the active ingredient after administration to the patient by employing procedures known in the art.

For preparing solid compositions such as tablets, the principal active ingredient is mixed with a pharmaceutical excipient to form a solid preformulation composition containing a homogeneous mixture of a peptoid / N-substituted peptidic copolymer of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

Formulations of this disclosure suitable for oral administration may be in the form of capsules, cachets, pills, tablets, powders, granules or as a solution or a suspension in an aqueous or non-aqueous liquid, or an oil-in-water or water-in-oil liquid emulsions, or as an elixir or syrup, or as pastilles (using an inert base, such as gelatin and glycerin, or sucrose and acacia), and the like, each containing a predetermined amount of a peptoid / N-substituted peptidic copolymer or peptoid / N-substituted peptidic copolymers of the present invention as an active ingredient. A peptoid / N-substituted peptidic copolymer of the present invention may also be administered as bolus, electuary or paste.

In solid dosage forms of this disclosure for oral administration (capsules, tablets, pills, dragees, powders, granules and the like), the active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monosterate; (8) absorbents, such as kaolin and bentonite clay; (9) lubricants, such as talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may be employed as fillers in soft and hard-filled gelatin capsules using such excipients as lactose or milk sugars, as well as high molecular weight polyethylene glycols and the like.

A tablet may be made by compression or molding optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent. The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter. These compositions may also optionally contain opacifying agents and may release the active ingredient only, or preferentially, in a certain portion of the gastrointestinal tract, optionally, in a delayed manner. Examples of embedding compositions which can be used include polymeric substances and waxes. The active ingredient can also be in microencapsulated form. The tablets or pills of this disclosure may be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Liquid dosage forms for oral administration of the peptoid / N-substituted peptidic copolymers of this disclosure include pharmaceutically-acceptable emulsions, microemulsions, solutions, suspensions, syrups and elixirs. In addition to the active ingredient, the liquid dosage forms may contain inert diluents commonly used in the art, such as water or

other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming, and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of this disclosure for rectal or vaginal administration may be presented as a suppository, which may be prepared by mixing one or more peptoid / N-substituted peptidic copolymers of this disclosure with one or more suitable nonirritating excipients or carriers comprising, for example, cocoa butter, polyethylene glycol, a suppository wax or salicylate, and which is solid at room temperature, but liquid at body temperature and, therefore, will melt in the rectum or vagina and release the active compound. Formulations of the present invention which are suitable for vaginal administration also include pessaries, tampons, creams, gels, pastes, foams or spray formulations containing such carriers as are known in the art to be appropriate.

Dosage forms for the topical or transdermal administration of peptoid / N-substituted peptidic copolymers of this invention include powders, sprays, ointments, pastes, creams, lotions, gels, solutions, patches, drops and inhalants. The active ingredient may be mixed under sterile conditions with a pharmaceutically-acceptable carrier, and with any buffers, or propellants which may be required.

The ointments, pastes, creams and gels may contain, in addition to an active ingredient, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to an active ingredient, excipients such as lactose, talc, silicic acid, aluminum hydroxide, calcium silicates and polyamide powder or mixtures of these substances. Sprays can additionally contain customary propellants such as chlorofluorohydrocarbons and volatile unsubstituted hydrocarbons, such as butane and propane.

Transdermal patches have the added advantage of providing controlled delivery of peptoid / N-substituted peptidic copolymers of this disclosure to the body. Such dosage forms can be made by dissolving, dispersing or otherwise incorporating one or more peptoid / N-substituted peptidic copolymers of this disclosure in a proper medium, such as an elastomeric matrix material. Absorption enhancers can also be used to increase the flux of the peptoid / N-substituted peptidic copolymers across the skin. The rate of such flux can be controlled by either providing a rate-controlling membrane or dispersing the peptoid / N-substituted peptidic copolymer in a polymer matrix or gel. Pharmaceutical formulations include those suitable for administration by inhalation or insufflation or for nasal or intraocular administration. For administration to the upper (nasal) or lower respiratory tract by inhalation, the peptoid / N-substituted peptidic copolymers of this disclosure are conveniently delivered from an insufflator, nebulizer or a pressurized pack or other convenient means of delivering an aerosol spray. Pressurized packs may comprise a suitable propellant such as dichlorodifluoromethane, trichlorofluoromethane, dichlorotetrafluoroethane, carbon dioxide, or other suitable gas. In the case of a pressurized aerosol, the dosage unit may be determined by providing a valve to deliver a metered amount.

Alternatively, for administration by inhalation or insufflation, the composition may take the form of a dry powder, for example, a powder mix of one or more peptoid / N-substituted peptidic copolymers of this disclosure and a suitable powder base, such as lactose or starch. The powder composition may be presented in unit dosage form in, for example, capsules or cartridges, or, e.g., gelatin or blister packs from which the powder may be administered with the aid of an inhalator, insufflator or a metered-dose inhaler.

For intranasal administration, peptoid / N-substituted peptidic copolymers of this disclosure may be administered by means of nose drops or a liquid spray, such as by means of a plastic bottle atomizer or metered-dose inhaler. Typical of atomizers are the Mistometer (Wintrop) and Medihaler (Riker).

Drops, such as eye drops or nose drops, may be formulated with an aqueous or nonaqueous base also comprising one or more dispersing agents, solubilizing agents or suspending agents. Liquid sprays are conveniently delivered from pressurized packs. Drops can be delivered by means of a simple eye dropper-capped bottle or by means of a plastic bottle adapted to deliver liquid contents dropwise by means of a specially shaped closure.

Pharmaceutical compositions of this invention suitable for parenteral administration comprise one or more peptoid / N-substituted peptidic copolymers of this disclosure in combination with one or more pharmaceutically-acceptable, sterile isotonic aqueous or non-aqueous solutions, dispersions, suspensions or emulsions, or sterile powders which may be reconstituted into sterile injectable solutions or dispersions just prior to use, which may contain antioxidants, buffers, solutes which render the formulation isotonic with the blood of the intended recipient or suspending or thickening agents.

Examples of suitable aqueous and nonaqueous carriers which may be employed in the pharmaceutical compositions of this disclosure include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as wetting agents, emulsifying agents, and dispersing agents. It may also be desirable to include isotonic agents, such as sugars, sodium chloride, and the like in the compositions. In addition, prolonged absorption of the injectable pharmaceutical form may be brought about by the inclusion of agents which delay absorption such as aluminum monosterate and gelatin.

In some cases, to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug is accomplished by dissolving or suspending the drug in an oil vehicle.

Injectable depot forms are made by forming microencapsule matrices of the drug in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue. The injectable materials can be sterilized for example, by filtration through a bacterial-retaining filter.

The formulations may be presented in unit-dose or multi-dose sealed containers, for example, ampules and vials, and may be stored in a lyophilized condition requiring only the addition of the sterile liquid carrier, for example water for injection, immediately prior to use. Extemporaneous injection solutions and suspensions may be prepared from sterile powders, granules and tablets of the type described above.

Suitable alkalinizing agents include alkali metal salts and alkaline earth metal salts. The alkali metal salts include sodium carbonate, sodium hydroxide, sodium silicate, disodium hydrogen orthophosphate, sodium aluminate, and other suitable alkali metal salts or mixtures thereof. Suitable alkaline metal salts include calcium carbonate, calcium hydroxide, magnesium carbonate, magnesium hydroxide, magnesium silicate, magnesium aluminate, aluminum magnesium hydroxide or mixture thereof. More particularly, calcium carbonate, potassium bicarbonate, calcium hydroxide, and/or sodium carbonate may be used as alkalinizing agents to obtain a formulation pH within the desired pH range of pH 8 to pH 13. The concentration of the alkalinizing agent is selected to obtain the desired pH, varying from about 0.1% to about 30%, by weight, and more preferably from about 12.5% to about 30%, by weight, of the total weight of the dosage formulation.

Suitable antioxidants may be selected from amongst one or more pharmaceutically acceptable antioxidants known in the art. Examples of pharmaceutically acceptable antioxidants include butylated hydroxyanisole (BHA), sodium ascorbate, butylated hydroxytoluene (BHT), sodium sulfite, citric acid, malic acid and ascorbic acid. The antioxidants may be present in the dosage formulations of the present invention at a concentration between about 0.001% to about 5%, by weight, of the dosage formulation. Suitable chelating agents may be selected from amongst one or more chelating agents known in the art. Examples of suitable chelating agents include disodium edetate (EDTA), edetic acid, citric acid and combinations thereof. The chelating agents may be present in a concentration between about 0.001% and about 5%, by weight, of the dosage formulation.

## 3) Therapeutic Methods

Methods for Preventing and Treating Microbial Infection

Another aspect of this disclosure provides methods for preventing and treating a microbial infection. These methods include administering to a subject in need thereof a therapeutically effective amount of a peptoid / N-substituted peptidic copolymer or composition of this disclosure or pharmaceutically acceptable derivative thereof that kills or inhibits the growth of infectious microbes, thereby inhibiting or treating the microbial infections.

As such, the present invention provides a peptoid / N-substituted peptidic copolymer obtainable by a process according to the invention or pharmaceutically acceptable derivative thereof, for use as antimicrobial agent.

According to the present invention, there is provided a method of treating, ameliorating, reversing, causing the remission of, and/or preventing or acting as a prophylaxis, or preventing the initiation of a microbial infection, comprising: administering to a subject in need thereof an effective amount of peptoid / N-substituted peptidic copolymer obtainable by a process according to the invention or pharmaceutically acceptable derivative thereof.

Aspects of the disclosure include antimicrobial compositions that find use in a variety of applications. In some instances, the pharmaceutical composition according to the invention finds use as an antimicrobial preservative, disinfectant or sterile storage medium. Applications of interest include use as a disinfectant or storage medium for an ocular

device, such as a contact lens, an intraocular lens implant or a drug-eluting ocular device. In certain instances, the pharmaceutical composition according ot the invention can be provided to a subject, as a solid or liquid composition, for use in a solution for storing, soaking and/or rinsing a contact lens. The solutions are well suited for ophthalmic use and are effective in cleaning, disinfecting, and sterilizing the contact lens upon exposure to the composition without the need for physical or thermal treatment of the lens. Any convenient carriers, excipients and/or diluents that find use in sterile or disinfecting solutions can be adapted for inclusion in the subject antimicrobial compositions. See e.g., U.S. Pat. No. 6,482,799.

Alternatively, the antimicrobial cationic peptoid / N-substituted peptidic copolymers of the present disclosure can be coated on the surface of medical devices, such as implantable medical devices, surgical instruments and indwelling medical devices (e.g., pacemakers, catheters, artificial joints, and the like), as a means of preventing infection.

A subject may have other clinical indications that have associated infection or inflammation treatable or preventable with the compositions and methods of the present invention, which include without limitation those associated with implantable, indwelling, or similar medical devices, such as intravascular catheters (e.g., intravenous and intraarterial), right heart flow-directed, catheters, Hickman catheters, arteriovenus fistulae, catheters used in hemodialysis and peritoneal dialysis (e.g., silastic, central venous, Tenckhoff, and teflon catheters), vascular access ports, indwelling urinary catheters, urinary catheters, silicone catheters, ventricular catheters, synthetic vascular prostheses (e.g., aortofemoral and femoropopliteal), prosthetic heart valves, prosthetic joints, orthopedic implants, penile implants, shunts (e.g., Scribner, Torkildsen, central nervous system, portasystemic, ventricular, ventriculoperitoneal), intrauterine devices, tampons, contact lenses, dental implants, ureteral stents, pacemakers, implantable defibrillators, tubing, cannulas, probes, blood monitoring devices, needles, and the like.

The pharmaceutical composition may be administered to the subject by applying the composition to a surface of a medical device prior to inserting the medical device into the subject.

For example, the subject may be selected from a human, a domesticated animal, a farm animal and a zoo animal.

In another aspect, the antimicrobial peptoid / N-substituted peptidic copolymers of the present application may be used for treating surfaces of medical devices, natural or synthetic implants, prosthetics, surgical instruments and implements, dental products, and the like. As used herein, unless otherwise noted, the term "surface" is used interchangeably with the term "solid support." As such, the antimicrobial peptoid / N-substituted peptidic copolymer compositions may be used to coat the inner and outer aspects of various instruments and devices, whether disposable or intended for repeated uses. Such surfaces can include a broad spectrum of articles adapted for medical use, including without limitation, scalpels, needles, scissors and other devices used in invasive surgical, therapeutic or diagnostic procedures; blood filters, and the like.

By coating surfaces of medical devices, implants and the like, the peptoid / N-substituted peptidic copolymer compositions of the present disclosure can prevent adherence of microorganisms thereto so as to reduce/eliminate any possible cell aggregation and biofilm formation known to occur following implantation. Device-related infections usually result from the introduction of microorganisms, primarily bacteria, during the device insertion or implantation procedure, or from attachment of blood-borne organisms to the newly inserted device and their subsequent propagation on its surface. Coating the medical device with the compositions of the present application can therefore inhibit biofilm formation of one or more microbial species, prevent medical device related infections, and consequently can reduce the need of antibiotic treatment or removal of the medical device from the subject.

Exemplary medical devices, instruments, and implants whose surfaces are susceptible to biofilm formation include, but are not limited to, any object that is capable of being implanted temporarily or permanently into a mammalian organism, such as a human. Representative medical devices, instruments, and implants that may be used according to the present application include, for example, central venous catheters, urinary catheters, endotracheal tubes, mechanical heart valves, pacemakers, vascular grafts, stents and prosthetic joints. Medical devices that may be coated according to the teachings of the present application may further include artificial blood vessels, catheters and other devices for the removal or delivery of fluids to patients, artificial hearts, artificial kidneys, orthopedic pins, prosthetic joints, plates and implants; catheters and other tubes (including urological and biliary tubes, endotracheal tubes, intubation tubes, peripherally insertable central venous catheters, dialysis catheters, long term tunneled central venous catheters, peripheral venous catheters, short term central venous catheters, arterial catheters, pulmonary catheters, Swan-Ganz catheters, urinary catheters, peritoneal catheters), urinary devices (including long term urinary devices, tissue bonding urinary devices, artificial urinary sphincters, urinary dilators), shunts (including ventricular or arterio-venous shunts); prostheses (including breast implants, penile prostheses, vascular grafting prostheses, aneurysm repair devices, mechanical heart valves, artificial joints, artificial larynxes, otological implants), intrauterine devices, anastomotic devices, vascular catheter ports, vascular stents, clamps, embolic devices, wound drain tubes, ocular lenses, dental implants, dentures, dental crowns, dental caps, hydrocephalus shunts, pacemakers and implantable defibrillators, needleless connectors, voice prostheses and the like.

The invention also relates to an article coated with the antimicrobial the peptoid / N-substituted peptidic copolymer compositions of the present disclosure. The article may be a medical device. For example, the medical device

may be an implant, a tongue depressor, a medical thermometer, a blood sugar meter, a medical robot, a surgical tool or a neuroprosthesis. For example, the medical device may be a microchip implant. The invention therefore relates to antimicrobial-coated medical device articles, and encompasses medical device articles comprising a coating including the antimicrobial the peptoid / N-substituted peptidic copolymer compositions of the present disclosure. Antimicrobial peptoid / N-substituted peptidic copolymer compositions of the present disclosure can be applied as a coating to a variety of substrates. Useful substrates include, for example, flexible and/or elastomeric articles that may comprise and/or be coated with a layer comprising non-siliceous ceramic materials (e.g., carbides, borides, nitrides), siliceous materials such as glasses and siliceous ceramic materials (e.g., silicides), metals (e.g., stainless steel, titanium, gold, silver, chromium, cobalt, tantulum as well as alloys of these metals and alloys of these metals with other metals such as, for example, cobalt-chromium alloys and titanium alloys used in orthopedic implants), and/or metal oxides. The substrate may be films, sheets, tubes, fibers, and the like formed of polymeric materials that are either thermoplastic polymers or thermoset polymers. Exemplary polymeric substrates include, but are not limited to, thermoplastic and thermoset polymers that are optionally plactisized and include polyolefins, polyethylene, polypropylene, fluoropolymers, polyamides, polyethers, epoxies, polyvinyl chloride and plasticized polyvinylchloride, polyisoprene, polyisobutylene, block copolymers such as styrene-butadene styrene and styrene-isoprene styrene, hydrogenated versions of these available from Kraton Polymers, metallocene polyolefins, rayon polyester, polyethylene terephthalate (PET), poly(meth)acrylates, polycarbonates, polystyrenes, polystyrene copolymers such as styrene acrylonitrile copolymers, polyesters, polyethersulfone, acrylics and acrylic copolymers, polyacrylamides, and polyurethanes, silicones such as two part cured polydiemthylsiloxanes, natural rubber latex, polyisoprene, nitrile rubber, and the like, as well as combinations thereof including blends thereof and laminates thereof. Suitable degradeable polymer substrates include, for example, polylactic acid (PLA), polyglycolic acid (PGA), and combinations thereof.

The substrates can be used to fabricate a variety of useful articles (e.g., as a part, a portion, or the entirety of the article). The surface of the substrate may comprise one or more of a variety of surface topographies including, for example, substantially planar surfaces, contoured surfaces, microreplicated structures, and a combination of any two or more of the foregoing. The articles (e.g., medical devices) comprise a variety of surfaces that may be deliberately or incidentally contacted with microbiologically-contaminated items during routine use. Suitable articles may be found in health care environments (e.g., patient care rooms, countertops, bedrails, patient care equipment such as instruments and stethoscopes (including, for example, stethoscope diaphragms and/or tubing), and in-dwelling medical devices such as venous access catheters, nasal gastric tubes, shunts, myringotomy tubes, intrauterine devices, urinary catheters, patient feeding tubes, ventilator tubing, endotracheal tubes, and other flat, tubular, or shaped flexible medical devices intended for contact with mammalian tissue). Other nonlimiting exemplary medical devices that can be coated with an antimicrobial composition according to the present disclosure include contact lenses, intraocular lenses, artificial corneas, wound dressings, eye bandages, materials for the sustained release of an active compound such as a drug delivery patch, moldings that can be used in surgery, such as heart valves, vascular grafts, catheters, artificial organs, orthopedic implants, encapsulated biologic implants, e.g. pancreatic islets, materials for prostheses such as bone substitutes, or moldings for diagnostics, membranes or biomedical instruments or apparatus.

The present disclosure also provides methods for coating the antimicrobial peptoid / N-substituted peptidic copolymer composition according to the present disclosure onto a substrate. The composition (e.g., the reaction mixture) comprising the antimicrobial peptoid / N-substituted peptidic copolymer in solvent (e.g., an aqueous solvent, an organic solvent) can be contacted with a substrate. The solvent can be evaporated to leave the antimicrobial peptoid / N-substituted peptidic copolymer in the form of a coating on the substrate. The substrate may be heated before and/or during the contacting step to accelerate the evaporation of the solvent. For example, the substrate may be heated to a temperature that does not degrade the function of the peptoid / N-substituted peptidic copolymer or a component of the substrate onto which the peptoid / N-substituted peptidic copolymer is coated. A suitable temperature for contacting the peptoid / N-substituted peptidic copolymer composition on a glass substrate may be from room temperature to about 120° C. A person of ordinary skill in the art will recognize that higher temperatures will facilitate faster removal of organic solvent from the polymer composition.

The antimicrobial peptoid / N-substituted peptidic copolymer can be diluted to a final concentration of 1 wt. % to about 20 wt % in a solvent such as an organic solvent before using the diluted composition to coat the antimicrobial peptoid / N-substituted peptidic copolymer onto a substrate. The antimicrobial peptoid / N-substituted peptidic copolymer may be diluted to a final concentration of 1 wt % to about 5 wt % in the organic solvent before using the diluted composition to coat the antimicrobial peptoid / N-substituted peptidic copolymer onto a substrate. Suitable organic solvents to dilute the polymer have a flashpoint below 150°C. and include ethers, ketones esters and alcohols, for example, isopropyl alcohol.

The infecting microorganism may include Gram-negative and/or Gram-positive bacteria.

The infecting microorganism may be a Gram-negative or Gram-positive bacteria, which may include, but is not limited to, *Clostridioïdes difficile, S. aureus meti-S, S. aureus meti-R, Streptococcus pneumoniae, Listeria monocytogenes, Enterococcus faecalis, Escherichia coli, Acinetobacter baumanii, Pseudomonas aeruginosa, Bacteroides fra-*

*gilis,* and/or *Helicobacter pylori.*

The antimicrobial peptoid / N-substituted peptidic copolymers administered, preferably as a component of a pharmaceutical composition, can include a single antimicrobial peptoid / N-substituted peptidic copolymer of this disclosure, or multiple peptoid / N-substituted peptidic copolymers of this disclosure.

Therapeutic peptoid / N-substituted peptidic copolymers of this disclosure or pharmaceutically acceptable derivative thereof may be administered by a number of routes, including orally, topically, or parenteral administration including intravenous by injection or infusion, intraperitoneal, intramuscular, intradermal, intrathecal, intrasternal, or intraarticular injection. One of skill in the art can readily determine the appropriate route of administration.

The therapeutically effective amounts of the peptoid / N-substituted peptidic copolymers of this disclosure that inhibit or kill an infecting microorganism will depend upon the subject being treated, the severity and type of the infection, and the manner of administration. For example, a therapeutically effective amount of a peptoid / N-substituted peptidic copolymer of this disclosure can vary from about 1 microgram/injection up to about 10 mg/injection. The exact amount of the peptoid / N-substituted peptidic copolymer is readily determined by one of skill in the art based on the age, weight, sex, and physiological condition of the subject. Effective doses can be extrapolated from dose-response curves derived from in vitro or animal model test systems.

One or more peptoid / N-substituted peptidic copolymers of this disclosure that effectively inhibit or kill an infecting microorganism can be administered in conjunction with one or more additional pharmaceutical agents. The additional pharmaceutical agents can be administered at the same time as, or sequentially with, the peptoid / N-substituted peptidic copolymer(s) of this disclosure. The additional pharmaceutical agent may be an additional antimicrobial agent. When administered at the same time, the additional pharmaceutical agent(s) can be formulated in the same composition that includes the peptoid / N-substituted peptidic copolymer(s) of this disclosure.

In one embodiment, the invention provides a product comprising a peptoid / N-substituted peptidic copolymer of the invention or pharmaceutically acceptable derivative thereof and at least one other therapeutic agent as a combined preparation for simultaneous, separate or sequential use in therapy. In one embodiment, the therapy is the treatment of a microbial infection. Products provided as a combined preparation include a composition comprising the peptoid / N-substituted peptidic copolymer of the invention or pharmaceutically acceptable derivative thereof and the other therapeutic agent(s) together in the same pharmaceutical composition, or the agent of the invention and the other therapeutic agent(s) in separate form, e.g. in the form of a kit.

In the combination therapies of the invention, the peptoid / N-substituted peptidic copolymer of the invention or pharmaceutically acceptable derivative thereof and the other therapeutic agent may be manufactured and/or formulated by the same or different manufacturers. Moreover, the peptoid / N-substituted peptidic copolymer of the invention or pharmaceutically acceptable derivative thereof and the other therapeutic may be brought together into a combination therapy: (i) prior to release of the combination product to physicians (e.g. in the case of a kit comprising the peptoid / N-substituted peptidic copolymer of the invention and the other therapeutic agent); (ii) by the physician themselves (or under the guidance of the physician) shortly before administration; (iii) in the patient themselves, e.g. during sequential administration of the peptoid / N-substituted peptidic copolymer of the invention and the other therapeutic agent. Accordingly, the invention provides the use of peptoid / N-substituted peptidic copolymer(s) of the invention or pharmaceutically acceptable derivative thereof for treating a microbial infection, wherein the medicament is prepared for administration with another therapeutic agent. The invention also provides the use of another therapeutic agent for treating a microbial infection, wherein the medicament is administered with a peptoid / N-substituted peptidic copolymer of the invention or pharmaceutically acceptable derivative thereof.

The invention also provides a peptoid / N-substituted peptidic copolymer of the invention or pharmaceutically acceptable derivative thereof for use in a method of treating a microbial infection, wherein the peptoid / N-substituted peptidic copolymer of the invention is prepared for administration with another therapeutic agent. The invention also provides another therapeutic agent for use in a method of treating a microbial infection, wherein the other therapeutic agent is prepared for administration with a peptoid / N-substituted peptidic copolymer of the invention or pharmaceutically acceptable derivative thereof. The invention also provides a peptoid / N-substituted peptidic copolymer of the invention or pharmaceutically acceptable derivative thereof for use in a method of treating a microbial infection, wherein the peptoid / N-substituted peptidic copolymer of the invention or pharmaceutically acceptable derivative thereof is administered with another therapeutic agent. The invention also provides another therapeutic agent for use in a method of treating a microbial infection, wherein the other therapeutic agent is administered with a peptoid / N-substituted peptidic copolymer of the invention or pharmaceutically acceptable derivative thereof.

The invention also provides the use of a peptoid / N-substituted peptidic copolymer of the invention or pharmaceutically acceptable derivative thereof for treating a microbial infection, wherein the subject has previously (e.g. within 24 hours) been treated with another therapeutic agent. The invention also provides the use of another therapeutic agent for treating a microbial infection, wherein the subject has previously (e.g. within 24 hours) been treated with a peptoid / N-substituted peptidic copolymer of the invention or pharmaceutically acceptable derivative thereof.

Compositions may additionally comprise molecules which assist or augment the action of the agents of the

invention, e.g. an antibiotic, which inhibits bacterial or fungal growth or kills bacteria or fungi. A peptoid / N-substituted peptidic copolymer of the invention or pharmaceutically acceptable derivative thereof may be combined in variable or fixed ratio combinations with antibiotics from any known antibiotic class. Specifically, these antibiotics may include antibiotics of the lincomycin family (a class of antibiotic agents originally recovered from streptomyces lincolnensis); antibiotics of the tetracycline family (a class of antibiotic agents originally recovered from streptomyces aureofaciens); and sulfur-based antibiotics such as the sulfonamides. Beta-lactams that can be combined include penems, carbapenems (imipenem, meropenem, ertapenem, doripenem, panipenem, biapenem, and the like), monobactams (aztreonam, tigimonam, carumonam, BAL30072, and the like), as well as a variety of other beta-lactam cell envelope antibiotics (see: wikipedia.org/wiki/Monobactam).

Specific examples of some suitable antibiotics of the lincomycin family include lincomycin, clindamycin, and clindamycin phosphate.

Specific examples of macrolide antibiotics include erythromycin, azithromycin, clarithromycin, dirithromycin, roxithromycin, carbomycin A, josamycin, kitasamycin, midecamycin/midecamycin acetate and troleandomycin.

Specific examples of ketolide antibiotics include telithromycin, cethromycin, solithromycin, spiramycin, ansamycin, oleandomycin, carbomycin, and tylosin.

Specific examples of antibiotics of the tetracycline family include tetracycline itself, chlortetracycline, oxytetracycline, demeclocycline, rolitetracycline, methacycline and doxycycline.

Specific examples of sulfur-based antibiotics include the sulfonamides, sulfacetamide, sulfabenzamide, sulfadiazine, sulfadoxine, sulfamerazine, sulfamethazine, sulfamethizole, sulfisoxazole, and sulfamethoxazole.

Further examples of combinable antibiotics include the oxazolidinones such as zyvox (linezolid), peptide antibiotics such as the polymixins, quinolones, fluoroquinolones (wikipedia.orgiwiki/Quinolone_antibiotic), aminoglycosides (wikipedia.org/wiki/Aminoglycoside), and rifamycins (wikipedia.org/wiki/Rifamycin). Combinable antibiotics can also include various antibacterial agents, antifungal agents, antimycotic agents and antiviral agents; penicillins such as ampicillin or amoxicillin, cephalosporins such as cephalothin and ceclor (cephachlor), aminoglycosides such as, kanamycin, macrolides such as erythromycin, nystatin, and amphotericin; and the antibiotics amikacin, bacillomycin, chloramphenicol, doxorubicin, doxycycline, ethambutol, gentamicin, isoniazid, kanamycin, carbacephalosporins such as lorabid (loracarbef), mupirocin, neomycin, pyrrolnitrin, rifampin, streptomycin, and vancomycin. Those skilled in the art can determine an appropriate time and duration of therapy that includes the administration of a peptoid / N-substituted peptidic copolymer of this disclosure to achieve the desired preventative or ameliorative effects on the subject treated.

In one embodiment, an antimicrobial peptoid / N-substituted peptidic copolymers of the present disclosure or pharmaceutically acceptable derivative thereof have antimicrobial activity for Gram negative microbes that is greater that the antimicrobial activity it has against Gram positive microbes. In one embodiment, an antimicrobial peptoid / N-substituted peptidic copolymers of the present disclosure or pharmaceutically acceptable derivative thereof has essentially similar antimicrobial activity for Gram negative microbes and Gram positive microbes. In one embodiment, an antimicrobial peptoid / N-substituted peptidic copolymers of the present disclosure or pharmaceutically acceptable derivative thereof has antimicrobial activity for Gram positive microbes that is greater that the antimicrobial activity it has against Gram negative microbes.

Whether an antimicrobial peptoid / N-substituted peptidic copolymer of this disclosure or pharmaceutically acceptable derivative thereof has antimicrobial activity can be determined using different indicator strains. Examples of indicator strains include, but are not limited to, *Clostridioïdes difficile, S. aureus meti-S, S. aureus meti-R, Streptococcus pneumoniae, Listeria monocytogenes, Enterococcus faecalis, Escherichia coli, Acinetobacter baumanii, Pseudomonas aeruginosa, Bacteroides fragilis,* and/or *Helicobacter pylori.* Methods for testing the activity of a peptoid / N-substituted peptidic copolymer of this disclosure or pharmaceutically acceptable derivative thereof include, but are not limited to, the stab-on-agar test as well as other methods useful for evaluating the activity of bacteriocins. Such methods are known in the art and are routine.

In one embodiment, a method includes treating a subject having a pathogenic microbe present in its gastrointestinal tract. In another embodiment, the present invention is directed to methods for treating certain conditions in a subject that may be caused by, or associated with, a microbe. Such conditions include, for instance, Gram negative microbial infections and Gram positive microbial infections of the gastrointestinal tract. Examples of conditions that may be caused by the presence of certain microbes in a subject's gastrointestinal tract include, but are not limited to, diarrhea, gastroenteritis, hemolytic-uremic syndrome, inflammatory bowel disease, irritable bowel disease, and Crohn's Disease.

Treating a subject, such as a subject having a pathogenic microbe or a subject having a condition, can be prophylactic or, alternatively, can be initiated after the need for treatment arises. Treatment that is prophylactic, for instance, initiated before a subject manifests symptoms of a condition caused by a pathogenic microbe, such as *Clostridioïdes difficile, S. aureus meti-S, S. aureus meti-R, Streptococcus pneumoniae, Listeria monocytogenes, Enterococcus faecalis, Escherichia coli, Acinetobacter baumanii, Pseudomonas aeruginosa, Bacteroides fragilis,* and/or *Helicobacter pylori,* is referred to herein as treatment of a subject that is "at risk" of developing the condition. Typically, a subject "at risk" of developing a condition is a subject likely to be exposed to a pathogenic microbe, such as *Clostridioïdes difficile,*

*S. aureus meti-S, S. aureus meti-R, Streptococcus pneumoniae, Listeria monocytogenes, Enterococcus faecalis, Escherichia coli, Acinetobacter baumanii, Pseudomonas aeruginosa, Bacteroides fragilis,* and/or *Helicobacter pylori,* causing the condition. For instance, the subject is present in an area where the condition has been diagnosed in at least one other subject (e.g., a hospital in the case of a nosocomial infection). Accordingly, administration of a composition can be performed before, during, or after the occurrence of a condition caused by a pathogenic microbe. Treatment initiated after the development of a condition may result in decreasing the severity of the symptoms of one of the conditions, or completely removing the symptoms. In this aspect of the invention, an "effective amount" is an amount effective to prevent the manifestation of symptoms of a condition, decrease the severity of the symptoms of a condition, and/or completely remove the symptoms. The potency of a composition described herein can be tested according to routine methods (see, for instance, Stanfield et al., Microb Pathog., 3:155-165 (1987), Fox et al., Am. J. Vet. Res., 48:85-90 (1987), Ruiz-Palacios, Infect. Immun., 34:250-255 (1981), and Humphrey et al., J. Infect. Dis., 151:485-493 (1985)). Methods for determining whether a subject a condition caused by a pathogenic microbe and symptoms associated with the conditions are routine and known to the art.

Another aspect of the invention relates to inhibiting microbial growth in a biological sample or a patient, which method comprises administering to the patient, or contacting said biological sample with a cationic peptoid / N-substituted peptidic copolymer according to the invention, or pharmaceutically acceptable derivative thereof, or a composition comprising said cationic peptoid / N-substituted peptidic copolymer or pharmaceutically acceptable derivative thereof. The term "biological sample", as used herein, includes, without limitation, cell cultures or extracts thereof; biopsied material obtained from a mammal or extracts thereof; and blood, saliva, urine, feces, semen, tears, or other body fluids or extracts thereof.

Inhibition of microbial growth in a biological sample is useful for a variety of purposes that are known to one of skill in the art. Examples of such purposes include, but are not limited to, blood transfusion, organ-transplantation, biological specimen storage, and biological assays.

The peptoid / N-substituted peptidic copolymers of this disclosure may be isolated or purified. As used herein, the term "purified" can be understood in to refer to a state of enrichment or selective enrichment of a particular component relative to an earlier state of crudeness or constituency of another component. This term can be considered to correspond to a material that is at least partially purified as opposed to a state of absolute purity. For example, a peptoid / N-substituted peptidic copolymer composition may be considered purified even if the composition does not reach a level of one hundred percent purity with respect to other components in the composition.

All references cited throughout this application, for example patent documents including issued or granted patents or equivalents; patent application publications; and non-patent literature documents or other source material; are hereby incorporated by reference herein in their entireties, as though individually incorporated by reference, to the extent each reference is at least partially not inconsistent with the disclosure in this application (for example, a reference that is partially inconsistent is incorporated by reference except for the partially inconsistent portion of the reference).

When a group of substituents is disclosed herein, it is understood that all individual members of that group and all subgroups, including any isomers, enantiomers, and diastereomers of the group members, are disclosed separately.

When a Markush group or other grouping is used herein, all individual members of the group and all combinations and subcombinations possible of the group are intended to be individually included in the disclosure. When a compound is described herein such that a particular isomer, enantiomer or diastereomer of the compound is not specified, for example, in a formula or in a chemical name, that description is intended to include each isomer and enantiomer of the compound described individually, or in any combination. Additionally, unless otherwise specified, all isotopic variants of compounds disclosed herein are intended to be encompassed by the disclosure. As a brief illustration, it will be understood that any one or more hydrogens in a molecule disclosed can be replaced with deuterium or tritium.

Isotopic variants of a molecule are generally useful as standards in assays for the molecule and in chemical and biological research related to the molecule or its use. Methods for making such isotopic variants are known in the art.

One of ordinary skill in the art will appreciate that starting materials, biological and chemical materials, biological and chemical reagents, synthetic methods, purification methods, analytical methods, assay methods, and biological methods other than those specifically exemplified can be employed in the practice of the invention without resort to undue experimentation. All art-known functional equivalents, of any such materials and methods are intended to be included in this disclosure.

The terms and expressions which have been employed herein are used as terms of description and not of limitation, and there is no intention in the use of such terms and expressions of excluding any equivalents of the features shown and described or portions thereof, but it is recognized that various modifications are possible within the scope of the invention claimed. Thus, although the invention has been disclosed by various aspects that may include preferred embodiments and aspects, modifications and variations of the concepts herein disclosed may be resorted to by those skilled in the art. Such modifications and variations are considered to be within the scope of this invention as defined by the appended claims.

## TREATMENT KIT

In other embodiments, the present invention relates to a kit for conveniently and effectively carrying out the methods in accordance with the present invention. In general, the pharmaceutical pack or kit comprises one or more containers filled with one or more of the ingredients of the pharmaceutical compositions of the invention. Such kits are especially suited for the delivery of solid oral forms such as tablets or capsules. Such a kit preferably includes a number of unit dosages, and may also include a card having the dosages oriented in the order of their intended use. If desired, a memory aid can be provided, for example in the form of numbers, letters, or other markings or with a calendar insert, designating the days in the treatment schedule in which the dosages can be administered. Alternatively, placebo dosages, or calcium dietary supplements, either in a form similar to or distinct from the dosages of the pharmaceutical compositions, can be included to provide a kit in which a dosage is taken every day. Optionally associated with such container(s) can be a notice in the form prescribed by a governmental agency regulating the manufacture, use or sale of pharmaceutical products, which notice reflects approval by the agency of manufacture, use or sale for human administration.

## EQUIVALENTS

The representative examples that follow are intended to help illustrate the invention, and are not intended to, nor should they be construed to, limit the scope of the invention. Indeed, various modifications of the invention and many further embodiments thereof, in addition to those shown and described herein, will become apparent to those skilled in the art from the full contents of this document, including the examples which follow and the references to the scientific and patent literature cited herein. It should further be appreciated that the contents of those cited references are incorporated herein by reference to help illustrate the state of the art.

The following examples contain important additional information, exemplification and guidance that can be adapted to the practice of this invention in its various embodiments and the equivalents thereof.

## EXEMPLIFICATION

The peptoid / N-substituted peptidic copolymers of this invention and their preparation can be understood further by the examples that illustrate some of the processes by which these peptoid / N-substituted peptidic copolymers are prepared or used. It will be appreciated, however, that these examples do not limit the invention. Variations of the invention, now known or further developed, are considered to fall within the scope of the present invention as described herein and as hereinafter claimed.

In the Examples that follow, "Strategy 1" refers to the "glyoxylic acid route" and "Strategy 2" to the "bio-sourced amino-acid route" described generally above in the present disclosure.

## EXAMPLES

### Example 1: Synthesis of hydrophobic NNCA, strategy 1: *N*-benzyl-*N*-carboxy anhydride

All reagents were purchased from Fluorochem or Sigma-Aldrich.

To a solution of benzylamine (6.51 mL, 91.5 mmol, 1 equiv.) in methanol (200 mL), glyoxylic acid (2.89 g, 30.5 mmol, 0.3 equiv.) was added and the reaction mixture was stirred for 30 min. Then, $NaBH_4$ (1.93 g, 30.5 mmol, 0.3 equiv.) was added and the reaction mixture was stirred for 30 min. The addition of glyoxylic acid and $NaBH_4$ was repeated five times in the same conditions, leading to a total quantity of glyoxylic acid of 14.45 g and a total quantity of $NaBH_4$ of 9.65 g. A last addition of $NaBH_4$ (3.86 g, 61 mmol, 0.6 eq) was performed. The reaction mixture was evaporated and directly involved into the second reaction step without any purification. The crude material was dissolved in a mixture of water/dioxane (1/1, 300 mL) before $K_2CO_3$ (25.53 g, 182.9 mmol, 2 equiv.) and di-*tert*-butyl dicarbonate (30.8 g, 137.1 mmol, 1.5 equiv.) were successively before the reaction mixture was stirred for 12 h. Then, water (100 mL) was added and the reaction mixture was washed 3 times with diethyl ether (100 mL). The aqueous phase was acidified to pH = 3 by dropwise addition of a 6M HCI solution. Then, the aqueous phase was extracted 4 times with diethyl ether (100 mL). The combined organic layers were dried over magnesium sulphate, filtered and evaporated to give *N*-Boc-*N*-benzyl glycine as a powder in 77% yield (2-step synthesis yield). To a solution of this N-Boc-N-benzyl glycine (1.00 g, 3.8 mmol, 1 equiv.) in anhydrous THF (6 mL) under Argon, thionyl chloride (0.27 mL, 3.8 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-benzyl-*N*-carboxy anhydride as a white powder in 79% yield. (FIG. 1)

**Example 2: Synthesis of hydrophobic NNCA, strategy 1: *N*-isopropyl-*N*-carboxy anhydride**

All reagents were purchased from Fluorochem or Sigma-Aldrich. *N*-isopropyl-*N*-Boc-Protected glycine derivatives was synthesized by following the procedure described in the example 1 by varying the R-$NH_2$. To a solution of *N*-Boc-*N*-isopropyl glycine (1 g, 4.6 mmol, 1 equiv.) in anhydrous THF (6 mL) under Argon, thionyl chloride (0.33 mL, 4.6 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-isopropyl-*N*-carboxy anhydride as a white powder in 89% yield. (FIG. 2)

**Example 3: Synthesis of hydrophobic NNCA, strategy 1: *N*-cyclohexyl-*N*-carboxy anhydride**

All reagents were purchased from Fluorochem or Sigma-Aldrich. *N*-cyclohexyl-*N*-Boc-Protected glycine derivatives was synthesized by following the procedure described in the example 1 by varying the R-$NH_2$. To a solution of *N*-Boc-*N*-cyclohexyl glycine (1.00 g, 3.9 mmol, 1 equiv.) in anhydrous THF (6 mL) under Argon, thionyl chloride (0.28 mL, 3.9 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-cyclohexyl-*N*-carboxy anhydride as a white powder in 80% yield. (FIG. 3)

**Example 4: Synthesis of hydrophobic NNCA, strategy 1: *N*-methyl-*N*-carboxy anhydride,**

All reagents were purchased from Fluorochem or Sigma-Aldrich. *N*-methyl-*N*-Boc-Protected glycine derivatives was synthesized by following the procedure described in the example 1 by varying the R-$NH_2$. To a solution of *N*-Boc-*N*-methyl glycine (1 g, 5.3 mmol, 1 equiv.) in anhydrous THF (6 mL) under Argon, thionyl chloride (0.38 mL, 5.3 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-methyl-*N*-carboxy anhydride as a white powder in 72% yield. (FIG. 4)

**Example 5: Synthesis of hydrophobic NNCA, strategy 1: *N*-(*p*-nitrobenzyl)-*N*-carboxy anhydride**

All reagents were purchased from Fluorochem or Sigma-Aldrich. *N*-(p-nitrobenzyl)-*N*-Boc-Protected glycine derivatives was synthesized by following the procedure described in the example 1 by varying the R-$NH_2$. To a solution of *N*-Boc-*N*-(p-nitrobenzyl) glycine (1.00 g, 3.2 mmol, 1 equiv.) in anhydrous THF (6 mL) under Argon, thionyl chloride (0.23 mL, 3.2 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-(*p*-nitrobenzyl)-*N*-carboxy anhydride as a white powder in 65% yield. (FIG. 5)

**Example 6: Synthesis of hydrophobic NNCA, strategy 1: *N*-(2-(methylthio)ethyl)-*N*-carboxy anhydride**

All reagents were purchased from Fluorochem or Sigma-Aldrich. *N*-2-(methylthio)ethyl)-*N*-Boc-Protected glycine derivatives was synthesized by following the procedure described in the example 1 by varying the R-$NH_2$. To a solution of *N*-Boc-*N*-(2-(methylthio)ethyl) glycine (1.00 g, 4 mmol, 1 equiv.) in anhydrous THF (6 mL) under Argon, thionyl chloride (0.28 mL, 3.8 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-(2-(methylthio)ethyl)-*N*-carboxy anhydride as a white powder in 71% yield. (FIG. 6)

**Example 7: Synthesis of hydrophobic NNCA, strategy 1: *N*-trifluoroethyl-*N*-carboxy anhydride**

All reagents were purchased from Fluorochem or Sigma-Aldrich. *N*-trifluoroethyl-*N*-Boc-Protected glycine derivatives was synthesized by following the procedure described in the example 1 by varying the R-$NH_2$. To a solution of *N*-Boc-*N*-trifluoroethyl glycine (1.00 g, 3.9 mmol, 1 equiv.) in anhydrous THF (6 mL) under Argon, thionyl chloride (0.28 mL, 3.9 mmol, 1 equiv.) was added and the reaction mixture was stirred for 4 h. The solvent was evaporated using vacuum and the crude material was washed 3 times with anhydrous hexane (18 mL). Then, DCM (4 mL) was added

and the suspension was filtered over previously dried Celite. Celite was washed 5 times with 5 mL of DCM. The solvent was evaporated under vacuum to give *N*-trifluoroethyl-*N*-carboxy anhydride as a white powder in 46% yield. (FIG. 7)

**Example 8: Synthesis of cationic NNCA, strategy 1: *N*-Cbz-aminoethyl-*N*-carboxy anhydride**

All reagents were purchased from Fluorochem or Sigma-Aldrich. *N*-Cbz-aminoethyl-*N*-Boc-Protected glycine derivatives was synthesized by following the procedure described in the example 1 by varying the $R-NH_2$. To a solution of *N*-Boc-*N*-Cbz-aminoethyl glycine (1.00 g, 2.8 mmol, 1 equiv.) in anhydrous THF (6 mL) under Argon, thionyl chloride (0.20 mL, 2.8 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-Cbz-aminoethyl-*N*-carboxy anhydride as a white powder in 47% yield. (FIG. 8)

**Example 9: Synthesis of cationic NNCA, strategy 1: *N*-Cbz-aminobutyl-*N*-carboxy anhydride**

All reagents were purchased from Fluorochem or Sigma-Aldrich. *N*-Cbz-aminobutyl-*N*-Boc-Protected glycine derivatives was synthesized by following the procedure described in the example 1 by varying the $R-NH_2$. To a solution of N-Boc-N- Cbz-aminobutyl glycine (1.00 g, 2.6 mmol, 1 equiv.) in anhydrous THF (6 mL) under Argon, thionyl chloride (0.19 mL, 2.6 mmol, 1 equiv.) was added and the reaction mixture was stirred for 4 h. The solvent was evaporated using vacuum and the crude material was washed 3 times with anhydrous hexane (18 mL). Then, DCM (4 mL) was added and the suspension was filtered over previously dried Celite. Celite was washed 5 times with 5 mL of DCM. The solvent was evaporated under vacuum to give *N*-Cbz-aminobutyl-*N*-carboxy anhydride as a white powder in 44% yield. (FIG. 9)

**Example 10: Synthesis of hydrophobic NNCA, strategy 2: *N*-methyl-L-alanine NCA**

All reagents were purchased from Fluorochem or Sigma-Aldrich.

To a solution of L-alanine (1.00 g, 11.2 mmol, 1 equiv.) in a mixture of water:dioxane (5:3, 40 mL), $K_2CO_3$ (3.10 g, 22.4 mmol, 2 equiv.) and di-tert-butyl dicarbonate (2.95 g, 13.5 mmol, 1.2 equiv.) were added before the reaction mixture was stirred for 12 h. Then, water (100 mL) was added and the reaction mixture was washed 3 times with diethyl ether (50 mL). The aqueous phase was acidified to pH = 3 by dropwise addition of a 6M HCl solution. Then, the aqueous phase was extracted 4 times with diethyl ether (50 mL). The combined organic layers were dried over magnesium sulphate, filtered and evaporated to give *N*-Boc-L-alanine as a white powder in 90% yield. To a solution of this *N*-Boc-L-alanine (1.00 g, 5.3 mmol, 1 equiv.) in THF (20 mL), 60% sodium hydride (0.65 g, 15.9 mmol, 3 equiv.) was added and the reaction mixture was stirred for 30 min at 0 °C. Then, dimethyl sulfate (0.75 mL, 8 mmol, 1.5 equiv.) was added and the reaction mixture was stirred for 30 min at 0 °C and for 6 h at rt. The reaction was stopped by the addition of diethyl ether (100 mL) followed by the addition of water (50 mL) at 0 °C. The aqueous layer was washed 3 times with diethyl ether (100 mL). The aqueous phase was acidified to pH = 3 by dropwise addition of a 6M HCl solution. Then, the aqueous phase was extracted with diethyl ether (100 mL) three times and washed with brine (100 mL) three times. The combined organic layers were dried over magnesium sulphate, filtered and evaporated to give *N*-Boc-*N*-methyl-L-alanine as a white powder in 65 % yield. To a solution of this *N*-Boc-*N*-Me-L-alanine (1.00 g, 4.9 mmol, 1 equiv.) in anhydrous DCM (20 mL) under Argon, phosphorus trichloride (0.52 mL, 5.9 mmol, 1.2 equiv.) was then added and the reaction mixture was stirred for 3 h at 0 °C. The solvent was evaporated using vacuum and the crude material was solubilized in DCM (20 mL). The solution was transferred, using a cannula, in a new Schlenk flask containing previously dried celite. Then, the solution was filtered in a new Schlenk flask and was dried under vacuum to give *N*-methyl-L-alanine *N*-carboxy anhydride as a white powder in 95% yield. (FIG. 10)

**Example 11: Synthesis of hydrophobic NNCA, strategy 2: *N*-methyl-D-alanine NCA**

All reagents were purchased from Fluorochem or Sigma-Aldrich. *N*-Boc-*N*-Me-D-alanine was synthesized by following the procedure described in the example 10 by varying the amino acid. To a solution *N*-Boc-*N*-Me-D-alanine (1.00 g, 4.92 mmol, 1 equiv.) in anhydrous DCM (20 mL) under Argon, phosphorus trichloride (0.52 mL, 5.9 mmol, 1.2 equiv.) was added and the reaction mixture was stirred for 3 h at 0 °C. The solvent was evaporated using vacuum and the crude material was solubilized in DCM (20 mL). The solution was transferred, using a cannula, in a new Schlenk flask containing previously dried celite. Then, the solution was filtered in a new Schlenk flask and was dried under vacuum to give *N*-methyl-D-alanine *N*-carboxy anhydride as a white powder in 82% yield. (FIG. 11)

**Example 12: Synthesis of hydrophobic NNCA, strategy 2: *N*-methyl-L-leucine NCA**

All reagents were purchased from Fluorochem or Sigma-Aldrich. *N*-Boc-*N*-Me-L-leucine was synthesized by following the procedure described in the example 10 by varying the amino acid. To a solution *N*-Boc-*N*-Me-L-leucine (1.00 g, 4.1 mmol, 1 equiv.) in anhydrous DCM (20 mL) under Argon, phosphorus trichloride (0.43 mL, 4.9 mmol, 1.2 equiv.) was added and the reaction mixture was stirred for 3 h at 0 °C. The solvent was evaporated using vacuum and the crude material was solubilized in DCM (20 mL). The solution was transferred, using a cannula, in a new Schlenk flask containing previously dried celite. Then, the solution was filtered in a new Schlenk flask and was dried under vacuum to give *N*-methyl-L-leucine *N*-carboxy anhydride as a colorless oil in 80% yield (FIG. 12).

**Example 13: Synthesis of hydrophobic NNCA, strategy 2: *N*-methyl-L-phenylalanine NCA**

All reagents were purchased from Fluorochem or Sigma-Aldrich. *N*-Boc-*N*-Me-L-phenylalanine was synthesized by following the procedure described in the example 10 by varying the amino acid. To a solution *N*-Boc-*N*-Me-L-phenylalanine (1.00 g, 3.6 mmol, 1 equiv.) in anhydrous DCM (20 mL) under Argon, phosphorus trichloride (0.38 mL, 4.3 mmol, 1.2 equiv.) was added and the reaction mixture was stirred for 3 h at 0 °C. The solvent was evaporated using vacuum and the crude material was solubilized in DCM (20 mL). The solution was transferred, using a cannula, in a new Schlenk flask containing previously dried celite. Then, the solution was filtered in a new Schlenk flask and was dried under vacuum to give *N*-methyl-L-phenylalanine *N*-carboxy anhydride as a white powder in 95% yield. (FIG. 13)

**Example 14: Synthesis of hydrophobic NNCA, strategy 2: (S)-3-(3-Methyl-2,5-dioxooxazolidin-4-yl)propanenitrile**

All reagents were purchased from Fluorochem or Sigma-Aldrich.

To a solution of *N*-Boc-L-glutamine (7.4 g, 30 mmol, 1equiv.) in pyridine (100 mL), acetic anhydride (3.7 mL, 36 mmol, 1.2 equiv.) was added and the reaction mixture was stirred overnight. Then, the reaction mixture was concentrated and the crude residue was taken up in diethyl ether (100 mL) before the solution was washed with 5% citric acid solution (100 mL) for three times and a brine solution (100 mL) for three times. The organic layer was dried over magnesium sulphate, filtered and evaporated. Without further purification, the crude material was dissolved in THF (100 mL), and 60% sodium hydride (3.6 g, 90 mmol, 3 equiv.) was added before the reaction mixture was stirred for 30 min at 0 °C. Then, dimethyl sulfate (4.2 mL, 45 mmol, 1.5 equiv.) was added and the reaction mixture was stirred for 30 min at 0 °C and 6 h at rt. The reaction was stopped by the addition of diethyl ether (200 mL) followed by the addition of water (100 mL) at 0 °C. The aqueous layer was washed with diethyl ether (200 mL) three times. The aqueous phase was acidified to pH = 3 by dropwise addition of a 6M HCl solution. Then, the aqueous phase was extracted with diethyl ether (200 mL) three times and washed with brine (200 mL) three times. The combined organic layers were dried over magnesium sulphate, filtered and evaporated to give *N*-Boc-2-methylamino-4-cyano-L-butyric acid as a white powder in 60% yield (2-step synthesis yield). To a solution of this *N*-Boc-2-methylamino-4-cyano-L-butyric acid (1.00 g, 4.1 mmol, 1 equiv.) in anhydrous DCM (20 mL) under Argon, phosphorus trichloride (0.44 mL, 5 mmol, 1.2 equiv.) was added and the reaction mixture was stirred for 3 h at 0 °C. The solvent was evaporated using vacuum and the crude material was solubilized in DCM (20 mL). The solution was transferred, using a cannula, in a new Schlenk flask containing previously dried celite. Then, the solution was filtered in a new Schlenk flask and was dried under vacuum to give (S)-3-(3-Methyl-2,5-dioxooxazolidin-4-yl)propanenitrile as a colorless oil in 78% yield. (FIG. 14)

**Example 15: Synthesis of cationic NNCA, strategy 2: *N*-methyl-L-lysine(Me, Z) NCA**

All reagents were purchased from Fluorochem or Sigma-Aldrich. *N*-Boc-*N*-Me-L-lysine (Me, Z) was synthesized by following the procedure described in the example 10 by varying the amino acid. To a solution $N_\alpha$-Boc-$N_\alpha$-Me-L-lysine(Me, Z) (1.00 g, 2.5 mmol, 1 equiv.) in anhydrous DCM (20 mL) under Argon, phosphorus trichloride (0.26 mL, 2.9 mmol, 1.2 equiv.) was added and the reaction mixture was stirred for 3 h at 0 °C. The solvent was evaporated using vacuum and the crude material was solubilized in DCM (20 mL). The solution was transferred, using a cannula, in a new Schlenk flask containing previously dried celite. Then, the solution was filtered in a new Schlenk flask and was dried under vacuum to give *N*-methyl-L-lysine(Me, Z) *N*-carboxy anhydride as a colorless oil in 93% yield. (FIG. 15)

**Example 16: Synthesis of linear topology: poly(*N*-(Cbz-aminobutyl-glycine)$_{18}$-(*N*-benzyl-glycine)$_2$)$_{20}$**

All reagents were purchased from Fluorochem, Sigma-Aldrich or other supplier except those described in the present disclosure .

To a mixture of *N*-Cbz-aminobutyl-*N*-carboxy anhydride (183 mg, $5.9 \times 10^{-4}$ mol, 18 equiv.) and *N*-benzyl-*N*-carboxy anhydride (12 mg, $6.6 \times 10^{-5}$ mol, 2 equiv.) in anhydrous DMF (2 mL), under Ar atmosphere, allylamine 1 M in

anhydrous DMF (33 μL, $3.3x10^{-5}$ mol, 1 equiv.) was added with an argon purged syringe. The solution was stirred at rt under argon until the disappearance of the peak around 1850 $cm^{-1}$ by FTIR. The polymer was then recovered by precipitation in diethyl ether (20 mL), centrifuged at 4000 rpm. After supernatant was removed, the polymer was dried under high vacuum to give poly(*N*-(Cbz-aminobutyl-glycine)$_{18}$-(*N*-benzyl-glycine)$_2$)$_{20}$ as a yellowish powder in 69% yield. (FIG. 16)

**Example 17: Synthesis of linear topology: poly(*N*-(aminobutyl-glycine)$_{18}$-(*N*-benzyl-glycine)$_2$)$_{20}$**

All reagents were purchased from Fluorochem, Sigma-Aldrich or other supplier except those described in the present disclosure.

To a solution of poly(*N*-(Cbz-aminobutyl-glycine)$_{18}$-(*N*-benzyl-glycine)$_2$)$_{20}$ (50 mg, $1.7x10^{-3}$ mol/ Cbz unit, 1 equiv.) in trifluoroacetic acid (0.5 mL), HBr 33% (55.9 μL, $3.4x10^{-3}$ mol, 2 equiv.) was added and the reaction was stirred for 3 h at rt. Then, the polymer was precipitated in diethyl ether (10 mL). After supernatant was removed, the polymer was solubilized in milli-Q water and was neutralized at pH=7 using $NaHC0_3$. The polymer was dialyzed with milli-Q water and lyophilized to give poly(*N*-(aminobutyl-glycine)$_{18}$-(*N*-benzyl-glycine)$_2$)$_{20}$ as a yellowish viscous oil in 74% yield. (FIG. 17)

**Example 18: Synthesis of linear topology: poly(*N*-(Cbz-aminobutyl-glycine)$_{10}$-(*N*-benzyl-glycine)$_{10}$)$_{20}$**

All reagents were purchased from Fluorochem, Sigma-Aldrich or other supplier except those described in the present disclosure.

To a mixture of *N*-Cbz-aminobutyl-*N*-carboxy anhydride (101.9 mg, $3.3x10^{-4}$ mol, 10 equiv.) and *N*-benzyl-*N*-carboxy anhydride (63.6 mg, $3.3x10^{-4}$ mol, 10 equiv.) in anhydrous DMF (2 mL), under Ar atmosphere, allylamine 1 M in anhydrous DMF (33 μL, $3.3x10^{-5}$ mol, 1 equiv.) was added with an argon purged syringe. The solution was stirred at rt under argon until the disappearance of the peak around 1850 $cm^{-1}$ by FTIR. The polymer was then recovered by precipitation in diethyl ether (20 mL), centrifuged at 4000 rpm. After supernatant was removed, the polymer was dried under high vacuum to give poly(*N*-(aminobutyl-glycine)$_{10}$-(*N*-benzyl-glycine)$_{10}$)$_{20}$ as a white powder in 70% yield. (FIG. 18)

**Example 19: Synthesis of linear topology: poly(*N*-(aminobutyl-glycine)$_{10}$-(*N*-benzyl-glycine)$_{10}$)$_{20}$**

All reagents were purchased from Fluorochem, Sigma-Aldrich or other supplier except those described in the present disclosure.

To a solution of poly(*N*-(Cbz-aminobutyl-glycine)$_{10}$-(*N*-benzyl-glycine)$_{10}$)$_{20}$ (20 mg, $3.8x10^{-5}$ mol/ Cbz unit, 1 equiv.) in trifluoroacetic acid (0.2 mL), HBr 33% (12.4 μL, $7.6x10^{-5}$ mmol, 2 equiv.) was added and the reaction was stirred for 3 h at rt. Then, the polymer was precipitated in diethyl ether (10 mL). After supernatant was removed, the polymer was solubilized in milli-Q water and was neutralized at pH=7 using $NaHC0_3$. The polymer was dialyzed with milli-Q water and lyophilized to give poly(*N*-(aminobutyl-glycine)$_{10}$-(*N*-benzyl-glycine)$_{10}$)$_{20}$ as a yellowish powder in 82% yield. (FIG. 19)

**Example 20: Synthesis of linear topology: poly(*N*-(Cbz-aminobutyl-glycine)$_{15}$-(*N*-benzyl-glycine)$_{15}$)$_{30}$**

All reagents were purchased from Fluorochem, Sigma-Aldrich or other supplier except those described in the present disclosure.

To a mixture of *N*-Cbz-aminobutyl-*N*-carboxy anhydride (101.9 mg, $3.3x10^{-4}$ mol, 15 equiv.) and *N*-benzyl-*N*-carboxy anhydride (63.6 mg, $3.3x10^{-4}$ mol, 15 equiv.) in anhydrous DMF (2 mL), under Ar atmosphere, allylamine 1 M in anhydrous DMF (55.4 μL, $2.2x10^{-5}$ mol, 1 equiv.) was added with an argon purged syringe. The solution was stirred at rt under argon until the disappearance of the peak around 1850 $cm^{-1}$ by FTIR. The polymer was then recovered by precipitation in diethyl ether (20 mL), centrifuged at 4000 rpm. After supernatant was removed, the polymer was dried under high vacuum to give poly(*N*-(aminobutyl-glycine)$_{15}$-(*N*-benzyl-glycine)$_{15}$)$_{30}$ as a yellowish powder in 56% yield. (FIG. 20)

**Example 21: Synthesis of linear topology: poly(*N*-(aminobutyl-glycine)$_{15}$-(*N*-benzyl-glycine)$_{15}$)$_{30}$**

All reagents were purchased from Fluorochem, Sigma-Aldrich or other supplier except those described in the present disclosure.

To a solution of poly(*N*-(Cbz-aminobutyl-glycine)$_{15}$-(N-benzyl-glycine)$_{15}$)$_{30}$ (50 mg, $9.6x10^{-5}$ mol/ Cbz unit, 1 equiv.) in trifluoroacetic acid (0.5 mL), HBr 33% (31 μL, $1.9x10^{-4}$ mol, 2 equiv.) was added and the reaction was stirred

for 3 h at rt. Then, the polymer was precipitated in diethyl ether (10 mL). After supernatant was removed, the polymer was solubilized in milli-Q water and was neutralized at pH=7 using $NaHC0_3$. The polymer was dialyzed with milli-Q water and lyophilized to give poly(*N*-(aminobutyl-glycine)$_{15}$-(*N*-benzyl-glycine)$_{15}$)$_{30}$ as a yellowish powder in 30% yield. (FIG. 21)

**Example 22: Synthesis of linear topology: poly(*N*-(Cbz-aminobutyl-glycine)$_7$-(*N*-isopropyl-glycine)$_3$)$_{10}$**

All reagents were purchased from Fluorochem, Sigma-Aldrich or other supplier except those described in the present disclosure.

To a mixture of *N*-Cbz-aminobutyl-*N*-carboxy anhydride (142.7 mg, 4.7x10$^{-4}$ mol, 7 equiv.) and *N*-isopropyl-*N*-carboxy anhydride (28.6 mg, 1.9x10$^{-4}$ mol, 3 equiv.) in anhydrous DMF (2 mL), under Ar atmosphere, allylamine 1 M in anhydrous DMF (66 μL, 6.7x10$^{-5}$ mol, 1 equiv.) was added with an argon purged syringe. The solution was stirred at rt under argon until the disappearance of the peak around 1850 cm$^{-1}$ by FTIR. The polymer was then recovered by precipitation in diethyl ether (20 mL), centrifuged at 4000 rpm. After supernatant was removed, the polymer was dried under high vacuum to give poly(*N*-(aminobutyl-glycine)$_7$-(*N*-ispropyl-glycine)$_3$)$_{20}$ as a yellowish viscous oil in 36% yield. (FIG. 22)

**Example 23: Synthesis of linear topology: poly(*N*-(aminobutyl-glycine)$_7$-(*N*-isopropyl-glycine)$_3$)$_{10}$**

All reagents were purchased from Fluorochem, Sigma-Aldrich or other supplier except those described in the present disclosure.

To a solution of poly(*N*-(Cbz-aminobutyl-glycine)$_7$-(*N*-isopropyl-glycine)$_3$)$_{10}$ (52 mg, 1.4x10$^{-4}$ mol/ Cbz unit, 1 equiv.) in trifluoroacetic acid (0.5 mL), HBr 48% (31 μL, 2.8x10$^{-4}$ mol, 2 equiv.) was added and the reaction was stirred for 3 h at rt. Then, the polymer was precipitated in diethyl ether (10 mL). After supernatant was removed, the polymer was solubilized in milli-Q water and was neutralized at pH=7 using $NaHC0_3$. The polymer was dialyzed with milli-Q water and lyophilized to give poly(*N*-(aminobutyl-glycine)$_7$-(*N*-isopropyl-glycine)$_3$)$_{10}$ as a yellowish viscous oil in 16% yield. (FIG. 23)

**Example 24: Synthesis of linear topology: poly(*N*-(Cbz-aminobutyl-glycine)$_7$-(*N*-isopropyl-glycine)$_3$)$_{10}$ initiated with decylamine**

All reagents were purchased from Fluorochem, Sigma-Aldrich or other supplier except those described in the present disclosure.

To a mixture of *N*-Cbz-aminobutyl-*N*-carboxy anhydride (142 mg, 4.7x10$^{-4}$ mol, 7 equiv.) and *N*-isopropyl-*N*-carboxy anhydride (28.6 mg, 2.0x10$^{-4}$ mol, 3 equiv.) in anhydrous DMF (2 mL), under Ar atmosphere, decylamine 0.5 M in anhydrous DMF (133 μL, 6.7x10$^{-5}$ mol, 1 equiv.) was added with an argon purged syringe. The solution was stirred at rt under argon until the disappearance of the peak around 1850 cm$^{-1}$ by FTIR. The polymer was then recovered by precipitation in diethyl ether (20 mL), centrifuged at 4000 rpm. After supernatant was removed, the polymer was dried under high vacuum to give poly(*N*-(aminobutyl-glycine)$_7$-(*N*-isopropyl-glycine)$_3$)$_{10}$ as a yellowish oil in 41% yield. (FIG. 24)

**Example 25: Synthesis of linear topology: poly(*N*-(aminobutyl-glycine)$_7$-(*N*-isopropyl-glycine)$_3$)$_{10}$ initiated with decylamine**

All reagents were purchased from Fluorochem, Sigma-Aldrich or other supplier except those described in the present disclosure.

To a solution of poly(*N*-(Cbz-aminobutyl-glycine)$_7$-(*N*-isopropyl-glycine)$_3$)$_{10}$ (52 mg, 1.1x10$^{-4}$ mmol/ Cbz unit, 1 equiv.) in trifluoroacetic acid (0.5 mL), HBr 33% (27 μL, 2.3x10$^{-5}$ mol, 2 equiv.) was added and the reaction was stirred for 3 h at rt. Then, the polymer was precipitated in diethyl ether (10 mL). After supernatant was removed, the polymer was solubilized in milli-Q water and was neutralized at pH=7 using $NaHC0_3$. The polymer was dialyzed with milli-Q water and lyophilized to give poly(*N*-(aminobutyl-glycine)$_{10}$-(*N*-benzyl-glycine)$_{10}$)$_{20}$ as a yellowish viscous oil in 81% yield. (FIG. 25)

**Example 26: Synthesis of linear topology: poly[(*N*-methyl-L-lysine(Me, Z))$_{10}$-(*N*-methyl-L-alanine)$_{10}$)$_{20}$**

All reagents were purchased from Fluorochem, Sigma-Aldrich or other supplier except those described in the present disclosure.

To a mixture of *N*-methyl-L-lysine(Me, Z) *N*-carboxy anhydride (200 mg, 0.6 mmol, 10 equiv.) and *N*-methyl-L-alanine *N*-carboxy anhydride (77 mg, 0.6 mmol, 10 equiv.) in anhydrous DMF (1.5 mL), under Ar atmosphere, allylamine

(4.5 μL, 0.06 mmol, 1 equiv.) was added with an argon purged syringe. The solution was stirred at rt under argon until the disappearance of the peak around 1850 $cm^{-1}$ by FTIR. The polymer was then recovered by precipitation in diethyl ether (20 mL), centrifuged at 4000 rpm. After supernatant was removed, the polymer was dried under high vacuum to give poly(*N*-methyl-L-lysine)$_{10}$-(*N*-methyl-L-alanine)$_{10}$)$_{20}$ as a white powder in 67% yield. (FIG. 26)

**Example 27: Synthesis of cyclic topology: poly[(*N*-Cbz-aminobutyl-glycine)$_{25}$-random-(*N*-benzyl-glycine)$_{25}$]$_{50}$.**

All reagents were purchased from Fluorochem, Sigma-Aldrich or other supplier except those described in the present disclosure.

To a mixture of *N*-benzyl-*N*-carboxy anhydride (63 mg, 3.3x10$^{-4}$ mol, 25 equiv.) and N-Cbz-aminobutyl-*N*-carboxy anhydride (101 mg, 3.3x10$^{-4}$ mol, 25 equiv.) in anhydrous DMF (2 mL), under Ar atmosphere, a solution of LiHMDS 0.5 M in anhydrous DMF (27 μL, 1.3x10$^{-5}$ mol, 1 equiv.) was added with an argon purged syringe. The solution was stirred at rt under argon until the disappearance of the peak around 1850 $cm^{-1}$ by FTIR. Then, the polymer was recovered by precipitation in diethyl ether (20 mL), centrifuged at 4000 rpm. After supernatant was removed, the polymer was dried under high vacuum to give cyclic poly[(*N*-Cbz-aminobutyl-glycine)$_{25}$-*random*-(*N*-benzyl-glycine)$_{25}$]$_{50}$ as a white powder in 92% yield. (FIG. 27) A proposed mechanism for the formation of cyclic polypeptoids by LiHMDS-mediated REP is depicted in FIG. 36.

**Example 28: Synthesis of cyclic topology: poly[(*N*-Cbz-aminobutyl-glycine)$_{25}$-*block*-(*N*-benzyl-glycine)$_{25}$]$_{50}$.**

All reagents were purchased from Fluorochem, Sigma-Aldrich or other supplier except those described in the present disclosure.

To a solution of *N*-Cbz-aminobutyl-*N*-carboxy anhydride (101 mg, 3.3x10$^{-4}$ mol, 25 equiv.) in anhydrous DMF (2 mL), under Ar atmosphere, a solution of LiHMDS 0.5 M in anhydrous DMF (27 μL, 1.3x10$^{-5}$ mol, 1 equiv.) was added with an argon purged syringe. The solution was stirred at rt under argon until the disappearance of the peak around 1850 $cm^{-1}$ by FTIR. Then, *N*-benzyl-*N*-carboxy anhydride (63 mg, 3.3x10$^{-4}$ mol, 25 eq) was added under Ar atmosphere and the solution was stirred at rt under argon until the disappearance of the peak around 1850 $cm^{-1}$ by FTIR. The polymer was recovered by precipitation in diethyl ether (20 mL) and centrifuged at 4000 rpm. After supernatant was removed the polymer was dried under high vacuum to give cyclic poly[(*N*-Cbz-aminobutyl-glycine)$_{25}$-*block*-(*N*-benzyl-glycine)$_{25}$]$_{50}$ as a yellowish powder in 50% yield. (FIG. 28)

**Example 29: Synthesis of cyclic topology: poly[(*N*-aminobutyl-glycine)$_{25}$-*random*-(*N*-benzyl-glycine)$_{25}$]$_{50}$.**

All reagents were purchased from Fluorochem, Sigma-Aldrich or other supplier except those described in the present disclosure.

To a solution of cyclic poly[(*N*-Cbz-aminobutyl-glycine)$_{25}$-*random*-(*N*-benzyl-glycine)$_{25}$]$_{50}$ (105 mg, 2.0x10$^{-4}$ mol, 1 equiv.) in trifluoroacetic acid (1 mL), HBr 33% (66.5 μL, 4.0x10$^{-4}$ mol, 2 equiv. /Cbz-aminobutyl unit) was added and the reaction was stirred for 3 h at rt. Then, the polymer was precipitated in diethyl ether (10 mL). After supernatant was removed, the polymer was solubilized in milli-Q water and was neutralized at pH=7 using $NaHC0_3$. The polymer was dialyzed with milli-Q water and lyophilized to give cyclic poly[(*N*-aminobutyl-glycine)$_{25}$-*random*-(*N*-benzyl-glycine)$_{25}$]$_{50}$ as a yellowish powder in 80% yield. (FIG. 29)

**Example 30: Synthesis of linear topology poly[(*N*-aminobutyl-glycine)$_{25}$-*random*-(*N*-benzyl-glycine)$_{25}$]$_{50}$ using propylation.**

All reagents were purchased from Fluorochem, Sigma-Aldrich or other supplier except when otherwise mentioned in this disclosure.

To a solution of cyclic poly[(*N*-aminobutyl-glycine)$_{25}$-*random*-(*N*-benzyl-glycine)$_{25}$]$_{50}$ (150 mg, 2.9x10$^{-5}$ mol, 1 equiv.) in anhydrous DMF (2 mL). Then, propionic anhydride (6.7 μL, 5.8x10$^{-5}$ mol) was added and the reaction was stirred during 24 h at 20 °C. The polymer was precipitated in diethyl ether (40 mL) and dried under vacuum to give linear copolymer poly[(*N*-aminobutyl-glycine)$_{25}$-*random*-(*N*-benzyl-glycine)$_{25}$]$_{50}$ as yelowish powder in 87% yield. (FIG. 30)

**Example 31: Antimicrobial assay with linear polymer on *Clostridium difficile:* poly(*N*-(aminobutyl-glycine)$_{15}$-(*N*-benzyl-glycine)$_{15}$)$_{30}$**

*Clostridium difficile* strain 630Δerm used in this study is described by Hussain *et al,* 2005. C. *difficile* strain was cultured in an anaerobic chamber with an atmosphere of 5% $H_2$, 5% $CO_2$, 90% $N_2$. Cells were cultured on brain-hearth infusion medium at 37 °C. Metronidazole and Vancomycin were purchased from Sigma-Aldrich. Fidaxomicin was purchased from Carbosynth. LL-37 antimicrobial peptide was purchased from sb-peptide. The Minimal Inhibitory Concentration (MIC) of tested polymer and control drugs were determined using the microdilution method. The experiments were performed by triplicates. The uninoculated medium was used as a negative control to test for contamination of the growth medium. The positive control was inoculated with C. *difficile* but no antimicrobial compound was added. MIC reported are the minimal drug concentration that suppressed totally the growth of bacteria after 24 h at 37°C and determined visually.

poly(*N*-(aminobutyl-glycine)$_{15}$-(*N*-benzyl-glycine)$_{15}$)$_{30}$ was solubilized in highly purified water to a concentration of 1000 μg/mL. A twofold serial dilution was performed to give a concentration range from 1000 to 8 μg/mL and 20 μL of each dilution was added to a 96-well plate before the plate was putted in an anaerobic chamber. Then, an overnight bacterial solution of *C. difficile* having an $OD_{600}$ between 0.450 and 0.600 ($10^7$-$10^8$ CFU/mL) was diluted in Brain Heart Infusion (BHI) at $10^5$ CFU/mL, each well was inoculated anaerobically with 180 μL to give a final concentration range for poly(*N*-(aminobutyl-glycine)$_{15}$-(*N*-benzyl-glycine)$_{15}$)$_{30}$ from 100 to 0.8 μg/mL. 96-well plate was incubated overnight at 37°C.

Same methodology was used for the preparation of positive control with a concentration range from 50 to 0.4 μg/mL. (FIG. 31)

### Example 32: Antimicrobial assay with cyclic polymer on *Clostridium difficile:* poly[(*N*-aminobutyl-glycine)$_{25}$-*random*-(N-benzyl-glycine)$_{25}$]$_{50}$

*Clostridium difficile* strain 630Δerm used in this study is described by Hussain *et al,* 2005. C. *difficile* strain was cultured in an anaerobic chamber with an atmosphere of 5% $H_2$, 5% $CO_2$, 90% $N_2$. Cells were cultured on brain-hearth infusion medium at 37 °C. Metronidazole and Vancomycin were purchased from Sigma-Aldrich. Fidaxomicin was purchased from Carbosynth. LL-37 antimicrobial peptide was purchased from sb-peptide. The Minimal Inhibitory Concentration (MIC) of tested polymer and control drugs were determined using the microdilution method. The experiments were performed by triplicates. The uninoculated medium was used as a negative control to test for contamination of the growth medium. The positive control was inoculated with C. *difficile* but no antimicrobial compound was added. MIC reported are the minimal drug concentration that suppressed totally the growth of bacteria after 24 h at 37°C and determined visually.

Poly[(*N*-aminobutyl-glycine)$_{25}$-*random*-(*N*-benzyl-glycine)$_{25}$]$_{50}$ was solubilized in highly purified water to a concentration of 1000 μg/mL. A twofold serial dilution was performed to give a concentration range from 1000 to 8 μg/mL and 20 μL of each dilution was added to a 96-well plate before the plate was putted in an anaerobic chamber. Then, an overnight bacterial solution of *C. difficile* having an $OD_{600}$ between 0.450 and 0.600 ($10^7$-$10^8$ CFU/mL) was diluted in Brain Heart Infusion (BHI) at $10^5$ CFU/mL, each well was inoculated anaerobically with 180 μL to give a final concentration range for Poly[(*N*-aminobutyl-glycine)$_{25}$-*random*-(*N*-benzyl-glycine)$_{25}$]$_{50}$ from 100 to 0.8 μg/mL. 96-well plate was incubated overnight at 37°C.

Same methodology was used for the preparation of positive control with a concentration range from 50 to 0.4 μg/mL. (FIG. 32)

### Example 33: Cytotoxicity assay with linear polymer on human epithelial cell line: poly(*N*-(aminobutyl-glycine)$_{15}$-(*N*-benzyl-glycine)$_{15}$)$_{30}$

Doxorubicin was purchased from Sigma-Aldrich. All other reagents were purchased from Sigma-Aldrich.

The evaluation of the tested peptides cytotoxicity by MTT assay on the Caco-2 cell line (human epithelial cell line) was done according to Mosmann with slight modifications. Briefly, cells (1.$10^5$ cells/mL) in 100 μL of the complete medium [DMEM High Glucose supplemented with 10% fetal calf serum (FCS), 2 mM L-glutamine, antibiotics (100 U/mL penicillin, 100 μg/mL streptomycin) and 1X NEAA] were seeded into each well of 96-well plates and incubated at 37 °C in a humidified 5% $CO_2$ with 95% air atmosphere. After a 24 h incubation, various concentrations of peptides and appropriate controls were then added (100 μL) and the plates were incubated for 72 h at 37 °C. Each well was then microscope-examined for detecting possible precipitate formation before aspiration of the medium. MTT solution (0.5 mg/mL in complete DMEM High Glucose, 100 μl) was then added to each well. Cells were incubated for 2 h at 37 °C. The MTT solution was then removed and DMSO (100 μL) was added to dissolve the resulting formazan crystals. Plates were shaken vigorously (300 rpm) for 5 min. The absorbance was measured at 570 nm with a microplate spectrophotometer (Eon Bio Tek). Water was used as blank and doxorubicin as a positive control. $CC_{50}$ were calculated by nonlinear regression analysis processed on dose-response curves, using TableCurve 2D V5 software. $CC_{50}$ values represent the mean value calculated from three independent experiments. (FIG. 33)

**Example 34: Cytotoxicity assay with cyclic polymer on human epithelial cell line: poly[(*N*-aminobutyl-glycine)$_{25}$-*random*-(*N*-benzyl-glycine)$_{25}$]$_{50}$**

Doxorubicin was purchased from Sigma-Aldrich. All other reagents were purchased from Sigma-Aldrich.

The evaluation of the tested peptides cytotoxicity by MTT assay on the Caco-2 cell line (human epithelial cell line) was done according to Mosmann with slight modifications. Briefly, cells (1.10$^5$ cells/mL) in 100 μL of the complete medium [DMEM High Glucose supplemented with 10% fetal calf serum (FCS), 2 mM L-glutamine, antibiotics (100 U/mL penicillin, 100 μg/mL streptomycin) and 1X NEAA] were seeded into each well of 96-well plates and incubated at 37 °C in a humidified 5% $CO_2$ with 95% air atmosphere. After a 24 h incubation, various concentrations of peptides and appropriate controls were then added (100 μL) and the plates were incubated for 72 h at 37 °C. Each well was then microscope-examined for detecting possible precipitate formation before aspiration of the medium. MTT solution (0.5 mg/mL in complete DMEM High Glucose, 100 μl) was then added to each well. Cells were incubated for 2 h at 37 °C. The MTT solution was then removed and DMSO (100 μL) was added to dissolve the resulting formazan crystals. Plates were shaken vigorously (300 rpm) for 5 min. The absorbance was measured at 570 nm with a microplate spectrophotometer (Eon Bio Tek). Water was used as blank and doxorubicin as a positive control. $CC_{50}$ were calculated by non-linear regression analysis processed on dose-response curves, using TableCurve 2D V5 software. $CC_{50}$ values represent the mean value calculated from three independent experiments. (FIG. 33)

**Example 35: Trypsin digestion assay with cyclic polymer: poly[(*N*-aminobutyl-glycine)$_{25}$-*random*-(*N*-benzyl-glycine)$_{25}$]$_{50}$**

A Poly[(*N*-aminobutyl-glycine)$_{25}$-*random*-(*N*-benzyl-glycine)$_{25}$]$_{50}$ solution was prepared (0.1 mM) in 1 mL of $NaHCO_3$ buffer 50 mM pH=7.8 equilibrated at 37 °C for 10 min and 47 μL of trypsin 42 μM (1 mg/mL) was added. At a certain time, 2.3 μL of the reaction mixture were taken, placed in an Eppendorf tube, quenched with 200 μL acetonitrile and 200 μL sodium borate buffer 0.1 M and kept in an ice bath. 4-Fluoro-7-nitro-benzofurazan (NBDF) (10 μL, acetonitrile solution 10 mM) was added, immediately the solution was incubated at 60 °C for 3 min and 500 rpm in the Eppendorf thermomixer R. The sample was placed on ice and 20 μL HCl 3N were added to stabilize the 4-amino-7-nitro-benzofurazan product. The fluorescence was measured in a Spectra max M2 (Molecular devices) with $\lambda_{ex}$=470 nm and $\lambda_{em}$=540 nm. The values were adjusted using the appropriate blank. The raw data was obtained from SoftMaxPro V5 and analysed in Origin2016 software. (FIG. 34)

**Example 36: Antimicrobial assay with cyclic polymer on *Clostridium difficile* after trypsin treatment: poly[(*N*-aminobutyl-glycine)$_{25}$-*random*-(*N*-benzyl-glycine)$_{25}$]$_{50}$**

*Clostridium difficile* strain 630Δerm used in this study is described by Hussain *et al,* 2005. C. *difficile* strain was cultured in an anaerobic chamber with an atmosphere of 5% $H_2$, 5% $CO_2$, 90% $N_2$. Cells were cultured on brain-hearth infusion medium at 37 °C. LL-37 antimicrobial peptide was purchased from sb-peptide. Trypsin was purchased from Sigma-Aldrich. The Minimal Inhibitory Concentration (MIC) of tested polymer and control drug were determined using the microdilution method. The experiments were performed by triplicates. The uninoculated medium was used as a negative control to test for contamination of the growth medium. The positive control was inoculated with C. *difficile* but no antimicrobial compound was added. MIC reported are the minimal drug concentration that suppressed totally the growth of bacteria after 24 h at 37°C and determined visually.

Solution of Poly[(*N*-aminobutyl-glycine)$_{25}$-*random*-(*N*-benzyl-glycine)$_{25}$]$_{50}$ was prepared in carbonate buffer 50 mM pH=7.8 at 1 mg/mL with 0.3 mg/mL of trypsin. Solution of LL-37 was prepared in carbonate buffer 50 mM pH=7.8 at 2 mg/mL with 0.6 mg/mL of trypsin. After 1h at 37°C, a twofold serial dilution of poly[(*N*-aminobutyl-glycine)$_{25}$-*random*-(*N*-benzyl-glycine)$_{25}$]$_{50}$ and LL-37 were prepared in carbonate buffer 50 mM pH=7.8 to give a final concentration range of 1000 to 8 μg/mL for poly[(*N*-aminobutyl-glycine)$_{25}$-*random*-(*N*-benzyl-glycine)$_{25}$]$_{50}$ and 2000 to 16 μg/mL for LL-37 and 20 μL of each concentration was added to a 96-well plate. Solutions of poly[(*N*-aminobutyl-glycine)$_{25}$-*random*-(*N*-benzyl-glycine)$_{25}$]$_{50}$ and LL-37 in carbonate buffer 50 mM pH=7.8 were also prepared without trypsin as control. Then, an overnight bacterial solution of C. *difficile* having an $OD_{600}$ between 0.450 and 0.600 ($10^7$-$10^8$ CFU/mL) was diluted in Brain Heart Infusion (BHI) at $10^5$ CFU/mL, each well was inoculated anaerobically with 180 μL and incubated at 37°C. The experiments were performed by triplicates.

**Example 37: Liposome destabilization assay with linear polymer: poly(*N*-(aminobutyl-glycine)$_{15}$-(*N*-benzyl-glycine)$_{15}$)$_{30}$**

Liposomes mixtures of SPC:cholesterol (SPC:Chol) or DOPG:cholesterol (PG:Chol) in a molar ratio of 8:2 were formulated. Liposomes were formulated according to the adapted method of the thin lipid film hydration. Lipid solutions

in chloroform/methanol (4:1) were evaporated under nitrogen flow and left under vacuum for 2-3 h to form a lipid film. Carboxufluoresceine (CF) was dissolved in phosphate buffer Saline (PBS) (137 mM NaCl, 2.7 mM KCl, 10 mM $Na_2HPO_4$, 2 mM $KH_2PO_4$) to reach 70 mM, pH was adjusted with concentrated NaOH to 7,4. This thin lipid film was then hydrated in the CF solution and vortexed 1h. At room temperature the yielded multilamellar vesicles (MLVs) were then extruded 13 times with a mini-extruder (Avanti Polar Lipids, Inc., Alabaster, Alabama, USA) through polycarbonate membranes with a pore diameter of 100 nm (Avanti Polar Lipids, Inc.). The obtained LUVs were separated from possible unincorporated CF by passage through a Sepharose® CL-4B loaded (Sigma-Aldrich) column, using PBS buffer as eluent. The LUVs size was assessed by DLS (NanoZS®, Malvern Instruments, Worcestershire, UK) after a 1:100 dilutions in the PBS buffer. Liposomes were stables within 4 month: SPC:Chol having a Z-average 153.1±0.5 nm, PDI=0.1 ±0.02 and Z-potential -3.9±0.9: PG:Chol having a Z-average 138.1±1.7 nm with a PDI=0.1 ±0.04 and Z-potential -33.3±2.2. The entrapment of CF was measured by the dequenching of fluorescence after the addition of 2 μL 20% (v/v) Triton X-100 measured with a Synergy 2 microplate reader (Biotek, Colmar, France) equipped with the appropriate filters ($\lambda_{ex}$=485/20 nm and $\lambda_{em}$=528/20 nm). CF release assay was performed in a final volume of 100 μL, using 10 μM LUVs in PBS buffer. Polypeptoids solubilized in PBS buffer were then added to the solution to reach a final concentration of 90 μg/mL. The fluorescence was recorded immediately (Fo) and for 1.5 h at 25 °C. It was compared with that measured at the end of the experiment after the addition of 2 μL of 20% Triton X-100 solution to achieve complete liposome leakage ($F_{max}$). The percentage of CF release was calculated according to the following equation:

$$\%\ \mathrm{CF\ leakage}_{(t)} = \frac{F_t - F_0}{F_{max} - F_0} \times 100$$

where $F_t$ was the fluorescence intensity at time t, $F_0$ the initial fluorescence intensity, and $F_{max}$ the final fluorescence intensity after adding Triton X-100. Fluorescences of PBS and polypeptoids alone at the same concentration were measured as negative controls. (FIG. 35)

While we have described a number of embodiments of this invention, it is apparent that our basic examples may be altered to provide other embodiments that utilize the peptoid / N-substituted peptidic copolymers and methods of this invention. Therefore, it will be appreciated that the scope of this invention is to be defined by the appended claims rather than by the specific embodiments that have been represented by way of example.

## Claims

**1.** Process for the preparation of cationic peptoid / N-substituted peptidic copolymers comprising :

A) at least one step of reacting in a polar solvent:

(i) at least a first N-carboxyanhydride monomer having the structure (I):

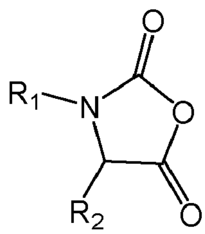

**(I)**

wherein

**$R_1$** independently represents a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl or $C_{2-12}$heteroalkynyl group; each of the foregoing alkyl, alkenyl, alkynyl, heteroalkyl, heteroalkenyl, heteroalkynyl group, optionally bearing one or more substituents selected from F; Cl; Br; I; $-NO_2$; -CN; $-CF_3$; $-CH_2CF_3$; $CHCl_2$; or $-GR^{A1}$ wherein G is -O-, -S-, - $NR^{B1}$-, -C(=O)-, -S(=O)-, $-SO_2$-, -C(=O)O-, -C(=O)$NR^{B1}$-, -OC(=O)-, -$NR^{B1}$C(=O)-, wherein each occurrence of $R^{A1}$ and $R^{B1}$ independently represents hydrogen, or a $C_{1-12}$alkyl, $C_{2-12}$alkenyl, $C_{2-12}$alkynyl,

$C_{1-12}$haloalkyl, $C_{2-12}$haloalkenyl, $C_{2-12}$haloalkynyl, $C_{1-12}$heteroalkyl, $C_{2-12}$heteroalkenyl or $C_{2-12}$heteroalkynyl group; and

**$R_2$** independently represents H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; $-OR^{A2}$; $-C(=O)R^{A2}$; or $-SR^{A2}$; wherein each occurrence of $R^{A2}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl group may optionally bear one or more substitutents selected from F; Cl; Br; I; $-NO_2$; -CN; $-CF_3$; $-CH_2CF_3$; $CHCl_2$; or $-GR^{B2}$ wherein G is -O-, -S-, $-NR^{C2}-$, -C(=O)-, -S(=O)-, $-SO_2-$, -C(=O)O-, $-C(=O)NR^{C2}-$, -OC(=O)-, $-NR^{C2}C(=O)-$, wherein each occurrence of $R^{B2}$ and $R^{C2}$ independently represents independently represents hydrogen, or a $C_{1-12}$alkyl, $C_{2-12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl, $C_{2-12}$heteroalkynyl, $C_{6-10}$aryl or $C_{4-10}$heteroaryl group;

wherein at least one of $R_1$ or $R_2$ bears at least one cationic moiety or protected form thereof;

(ii) at least a second N-carboxyanhydride monomer having the structure (II):

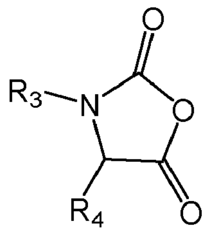

**(II)**

wherein

**$R_3$** independently represents a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, or $C_{2-12}$heteroalkynyl; each of the foregoing alkyl, alkenyl, alkynyl, heteroalkyl, heteroalkenyl or heteroalkynyl group optionally bearing one or more substituents selected from $C_{1-12}$alkyl; $C_{2-12}$alkenyl; $C_{2-12}$alkynyl; $C_{1-12}$heteroalkyl; $C_{2-12}$heteroalkenyl; $C_{2-12}$heteroalkynyl; $C_{6-10}$aryl; $C_{6-10}$heteroaryl; F; Cl; Br; I; $-NO_2$; -CN; $-CF_3$; $-CH_2CF_3$; $CHCl_2$; or $-GR^{A3}$ wherein G is -O-, -S-, $-NR^{B3}-$, -C(=O)-, -S(=O)-, $-SO_2-$, -C(=O)O-, $-C(=O)NR^{B3}-$, -OC(=O)-, $-NR^{B3}C(=O)-$, wherein each occurrence of $R^{A3}$ and $R^{B3}$ independently represents hydrogen, or a $C_{1-12}$alkyl, $C_{2-12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl or $C_{2-12}$heteroalkynyl; and

$R_4$ independently represents H, a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; $-OR^{A4}$; $-C(=O)R^{A4}$; or $-SR^{A4}$; wherein each occurrence of $R^{A4}$ independently represents hydrogen or a cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl or $C_{4-10}$heteroaryl group; wherein each of the foregoing alkyl, alkenyl, alkynyl, heteroalkyl, heteroalkenyl, heteroalkynyl, aryl or heteroaryl, group may optionally bear one or more substitutents selected from $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, $C_{6-10}$aryl, $C_{4-10}$heteroaryl, F; Cl; Br; I; $-NO_2$; -CN; $-CF_3$; $-CH_2CF_3$; $CHCl_2$; or $-GR^{B4}$ wherein G is -O-, -S-, $-NR^{C4}-$, -C(=O)-, -S(=O)-, $-SO_2-$, - C(=O)O-, $-C(=O)NR^{C4}-$, -OC(=O)-, $-NR^{C4}C(=O)-$, wherein each occurrence of $R^{B4}$ and $R^{C4}$ independently represents independently represents hydrogen, or a $C_{1-12}$alkyl, $C_{2-12}$alkenyl, $C_{2-12}$alkynyl, $C_{1-12}$haloalkyl, $C_{2-12}$haloalkenyl, $C_{2-12}$haloalkynyl, $C_{1-12}$heteroalkyl, $C_{2-12}$heteroalkenyl, $C_{2-12}$heteroalkynyl, $C_{6-10}$aryl or $C_{4-10}$heteroaryl group;

(iii) in the presence of at least one nucleophilic compound initiator and/or at least one base;

wherein the base and the nucleophilic compound initiator may be different compounds or one and the same compound; and

B) when $R_1$ or $R_2$ bears a protected form of at least one cationic moiety, the process further comprises at least one deprotection step of the protected form of the at least one cationic moiety to yield cationic peptoid / N-substituted peptidic copolymers; such as acidic or basic treatment.

2. Process according to claim 1, for the preparation of linear peptoid / N-substituted peptidic copolymers, wherein the process is carried out in the presence of at least one nucleophilic compound initiator selected from primary amines, such as allylamine, n-hexylamine, n-decylamine, n-dodecylamine or n-octadecylamine.

3. Process according to claim 1, for the preparation of macrocylic peptoid / N-substituted peptidic copolymers, wherein the process is carried out in the presence of at least a strong base selected from lithium, sodium or potassium amides, lithium, sodium or potassium hydrides, or phosphazenes.

4. Process according to claim 3, wherein the strong base is a lithium amide, such as lithium bis(trimethylsilyl) amide.

5. Process according to claim 1, for the preparation of dendritic peptoid / N-substituted peptidic copolymers, wherein the process is carried out in the presence of at least one dendritic synthon comprising at least three nucleophilic moieties selected from amino, thio or hydroxyl groups, and optionally at least one base selected from tertiary amines such as triethylamine or N, N-Diisopropylethylamine in particular when the at least one dendritic synthon comprises non-basic nucleophilic moieties selected from thio or hydroxyl groups.

6. Process according to claim 5, wherein the at least one dendritic synthon is a PAMAM synthon having one of the following structures:

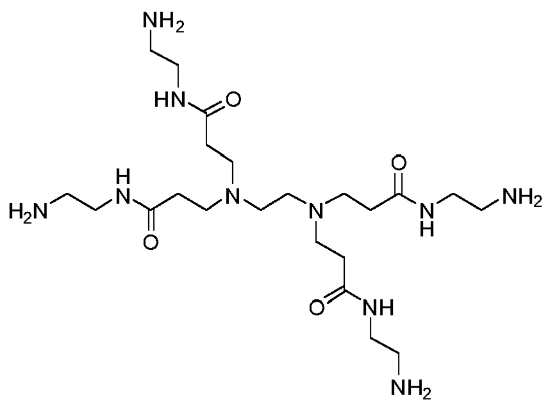

**(PAMAM)-4-arms-NH $_2$**

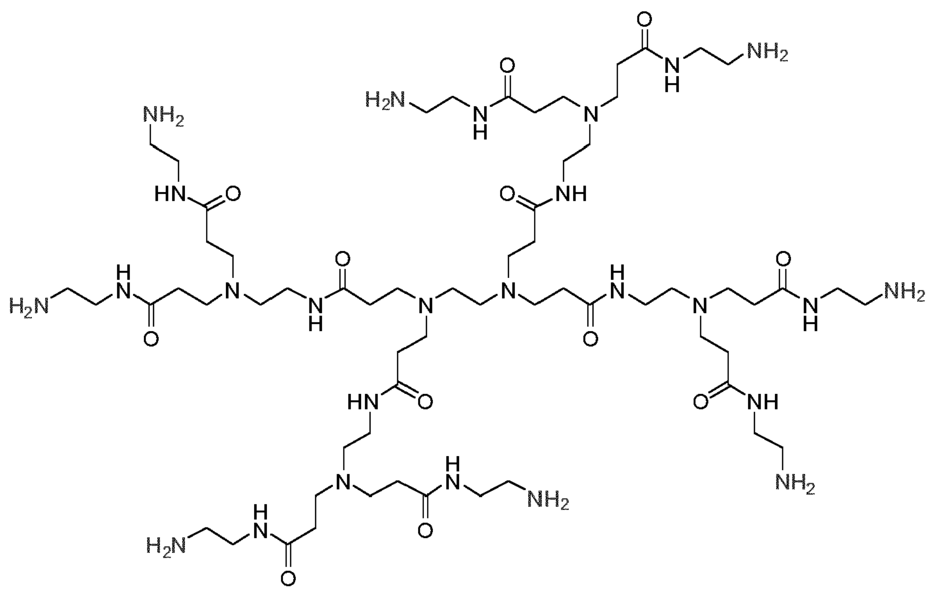

**(PAMAM)-8-arms-NH $_2$**

,

or

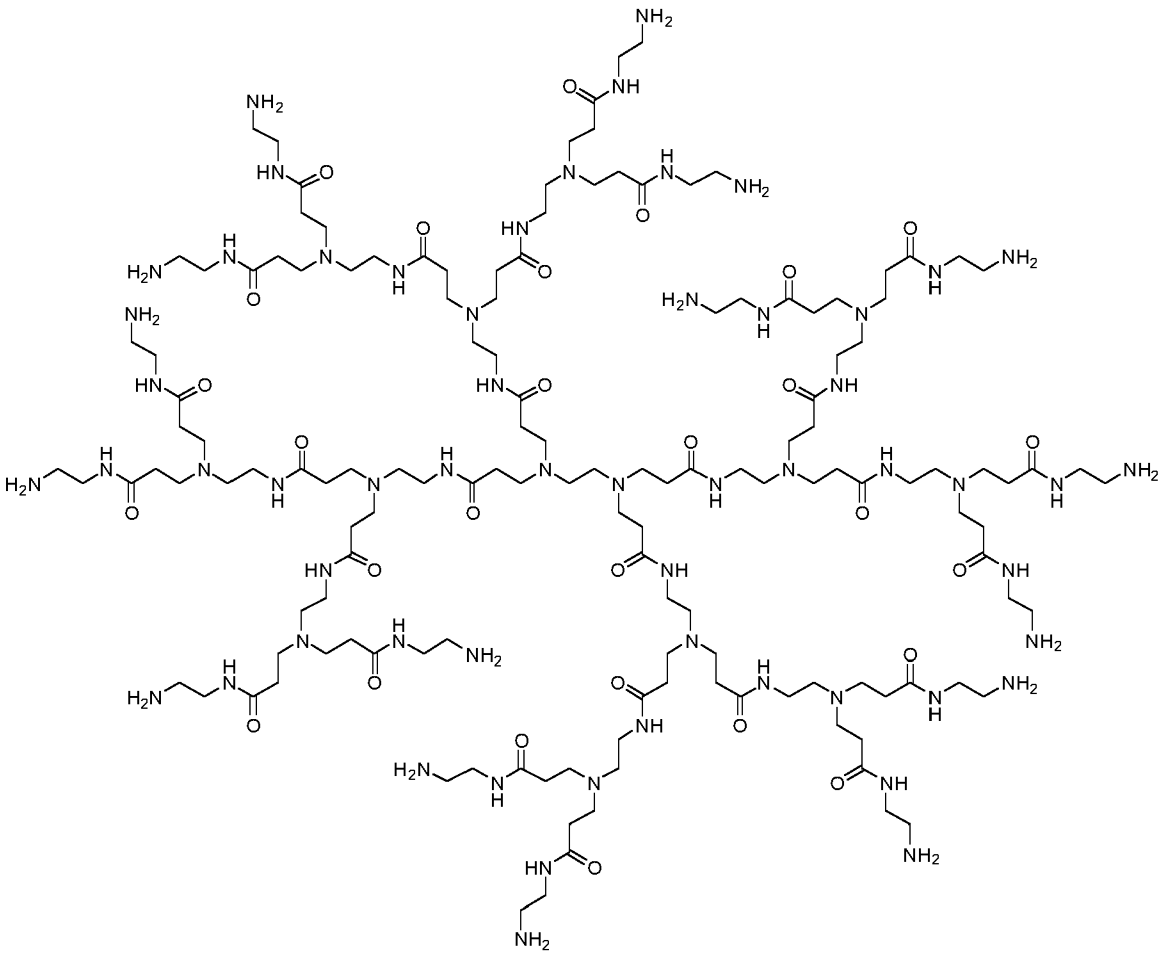

(PAMAM)-16-arms-$NH_2$

7. Process according to claim 1, for the preparation of peptoid / N-substituted peptidic copolymer-drug conjugates, wherein the process is carried out in the presence of at least one drug molecule bearing at least one nucleophilic moiety, and optionally at least one base selected from tertiary amines such as trimethylamine or N, N-Diisopropylethylamine.

8. Process according to claim 7, wherein the at least one drug molecule bears at least one basic primary amino group, such as vancomycin, amoxicilline, kanamycine, cefaclor, amphotericin B, nystatin, amikacin or sulfamethoxazole; and the process is carried out without a tertiary amine.

9. Process according to any one of claims 1-8, wherein the first and second N-carboxyanhydride monomers have the structure

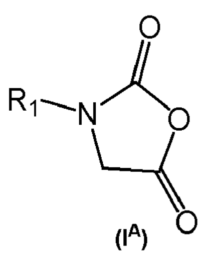
($I^A$) and

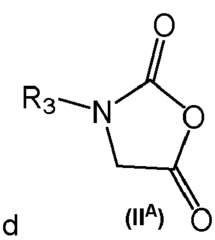
($II^A$),

respectively;
wherein $R_1$ represents an optionally protected cationic side chain of a natural or modified amino acid, and $R_3$ represents an optionally protected hydrophobic side chain of a natural or modified amino acid.

**10.** Process according to any one of claims 1-8, wherein the first N-carboxyanhydride monomers has the structure

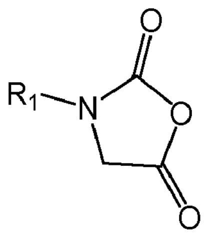

wherein $R_1$ represents $-C_{1-6}$alkyl-$NHP_1$, wherein $P_1$ represents an amine protecting group, such as -(C=O)OPh.

**11.** Process according to any one of claims 1-8, wherein the first and second N-carboxyanhydride monomers have the structure respectively;

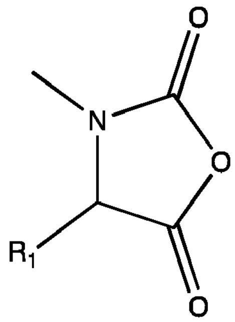

and

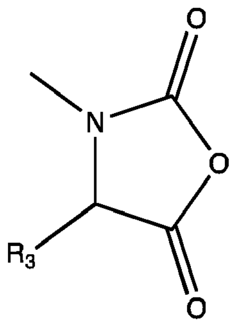

wherein $R_1$ represents an optionally protected cationic side chain of a natural or modified amino acid, and $R_3$ represents an optionally protected hydrophobic side chain of a natural or modified amino acid.

**12.** Process according to any one of claims 1-8, wherein the first N-carboxyanhydride monomers has the structure

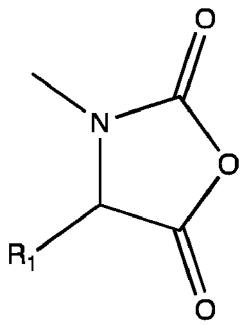

wherein $R_1$ represents $-C_{1-6}$alkyl-$NHP_1$, wherein $P_1$ represents an amine protecting group, such as -(C=O)OPh.

**13.** Process according to any one of claims 1-12, wherein the molar ratio first N-carboxyanhydride monomer: second N-carboxyanhydride monomer ranges between 0 : 100 and 100 : 0, preferably 50:50 and preferentially with cationic content higher than 50%.

**14.** Process according to any one of claims 1-13, wherein the polar organic solvent is an aprotic solvent such as DCM, DMF, Dioxane, DMSO, Benzonitrile, THF or a mixture thereof.

**15.** Process according to any one of claims 1-14, wherein the first or second N-carboxyanhydride monomer is prepared by a process comprising steps of:

(i) reacting an amine $R-NH_2$ with a carboxylic acid having the structure $R'(C=O)-CO_2H$ to form an amino acid

compound having the structure (III):

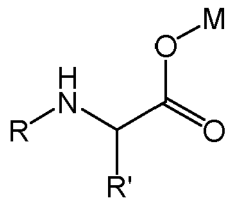

**(III)**

wherein
R represents $R_1$ or $R_3$ as defined in claim 1;
R' represents $R_2$ or $R_4$ as defined in claim 1, preferably R' represents H; and
M represents a hydrogen atom or an alkali metal cation selected from Na, Li or K;
(ii) reacting the compound of structure (III) with a nitrogen-protecting group under suitable conditions to form a protected amino acid compound having the structure (IV):

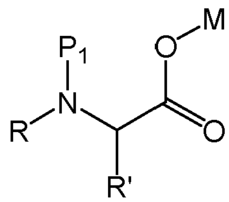

**(IV)**

wherein $P_1$ represents a suitable amine protecting group, preferably $P_1$ represents a Boc protecting group; and
(iii) effecting cyclization of compound (IV) under suitable conditions to form a N-carboxyanhydride monomer having the structure:

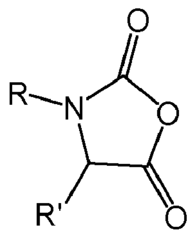

wherein
R represents $R_1$ or $R_3$ as defined in claim 1; and R' represents $R_2$ or $R_4$ as defined in claim 1, preferably R' represents H.

**16.** Process according to claim 15, wherein the resulting N-carboxyanhydride monomer has one of the flowing structures:

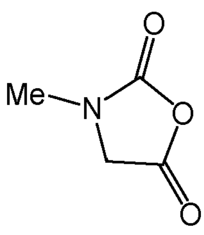 , 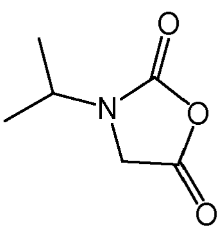 , 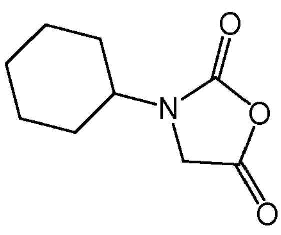 , 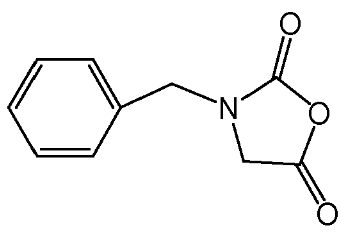 ,

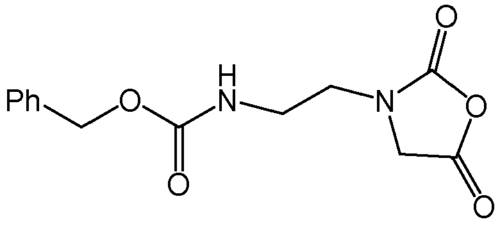,
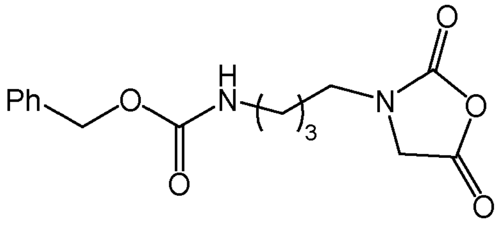,

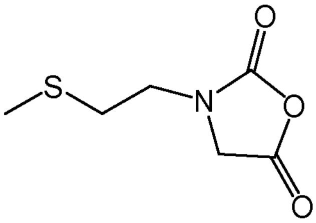,
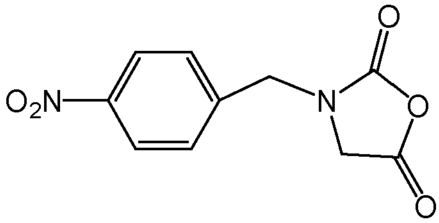 or
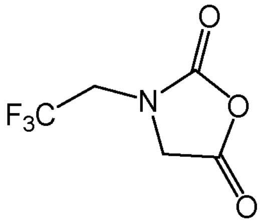.

**17.** Process according to any one of claims 1-14, wherein the first or second N-carboxyanhydride monomer is prepared by a process comprising steps of:

(i) reacting a natural amino acid

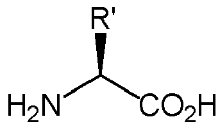

with an amine protecting group, for example $Boc_2O$ or CbzCl, under suitable conditions to form a protected natural amino acid compound having the structure (V):

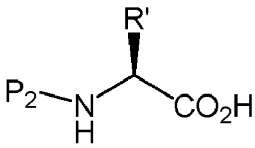

**(V)**

wherein
R' represents $R_2$ or $R_4$ as defined in claim 1; and
$P_2$ represents a carbamate protecting group, such as Boc or Cbz;
(ii) reacting the compound of structure (V) with a compound R-X under suitable conditions to form a compound having the structure (VI):

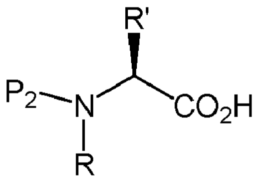

**(VI)**

wherein
R represents $R_1$ or $R_3$ as defined in claim 1;-and

X represents a suitable leaving group such as a sulfonate leaving group; and

(iii) effecting cyclization of compound (VI) under suitable conditions to form a N-carboxyanhydride monomer having the structure:

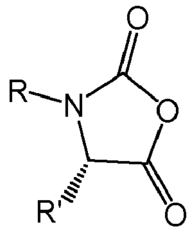

wherein

R represents $R_1$ or $R_3$ as defined in claim 1; and R' represents $R_2$ or $R_4$ as defined in claim 1.

**18.** Process according to claim 17, wherein the resulting N-carboxyanhydride monomer has one of the flowing structures:

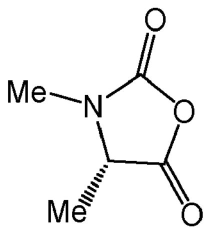
,
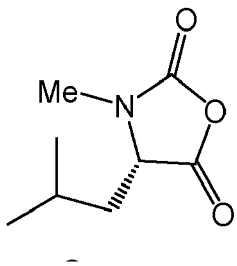
,
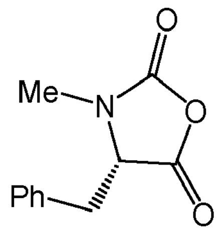

or

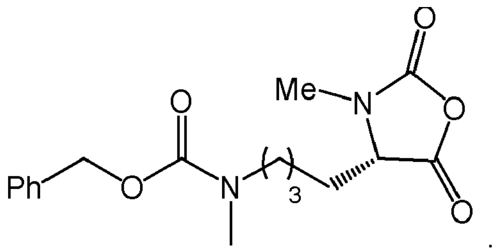
.

**19.** Linear peptoid / N-substituted peptidic copolymer obtainable by a process of claim 1 or 2, wherein the linear peptoid / N-substituted peptidic copolymer has the following structure:

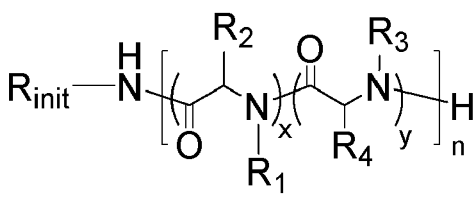

**($V_{linear}$)**

wherein $R_1$, $R_2$, $R_3$ and $R_4$ are as defined in claim 1;

$R_{init}$ represents the radical of a primary amine nucleophilic compound initiator, such as allyl, n-hexyl, n-decyl, n-dodecyl or n-octadecyl;

x ranges between 0 and 1, and represents the molar ratio of first N-carboxyanhydride monomer units in the copolymer;

y ranges between 0 and 1, and represents the molar ratio of second N-carboxyanhydride monomer units in the copolymer;

wherein x + y =1 and the x and y repeat monomer units are randomly distributed in the macrocyclic peptoid / N-substituted peptidic copolymer; and

n represents the number of first and second N-carboxyanhydride monomer unit in the copolymer.

**20.** Macrocylic peptoid / N-substituted peptidic copolymer obtainable by a process of any one of claims 1, 3 and 4, wherein the macrocylic peptoid / N-substituted peptidic copolymer has the following structure:

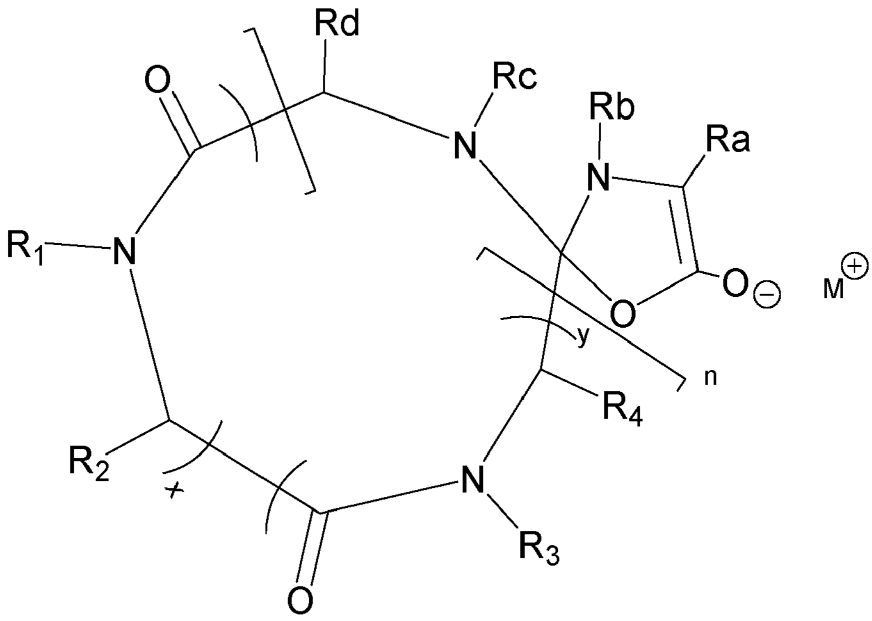

**($V^{A}_{macrocyclic}$)**

or

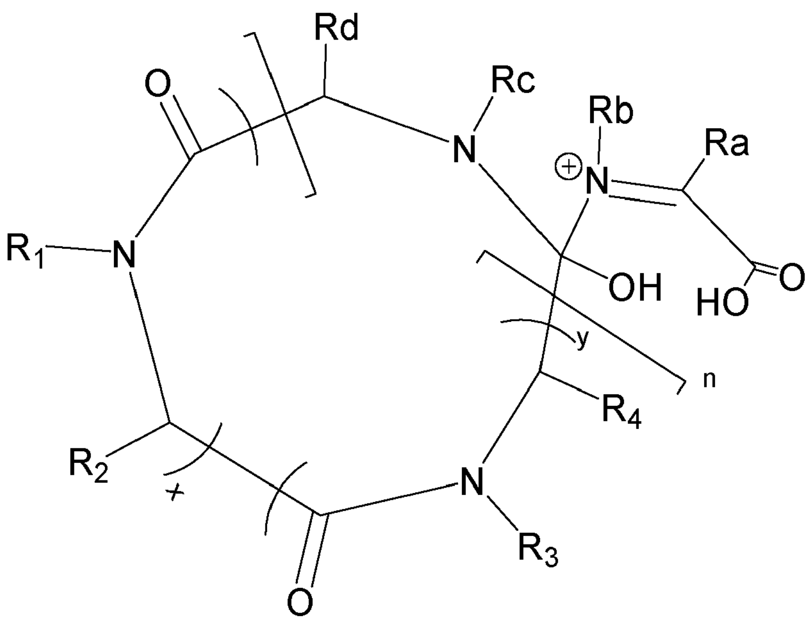

**($V^{B}_{macrocyclic}$)**

wherein $R_1$, $R_2$, $R_3$ and $R_4$ are as defined in claim 1;
(Ra, Rb) may represent ($R_1$, $R_2$) or ($R_3$, $R_4$), respectively;
independently of (Ra, Rb), (Rc, Rd) may represent ($R_1$, $R_2$) or ($R_3$, $R_4$), respectively;
$M^+$ represents a counterion, such as $H^+$, $Li^+$, $Na^+$, $K^+$ or an alkali metal salt such as LiHMDS;

x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer; and
n represents the number of first and second N-carboxyanhydride monomer unit in the copolymer chain.

**21.** Macrocylic peptoid / N-substituted peptidic copolymer according to claim 20, wherein $R_1$ and $R_3$ are different.

**22.** Macrocylic peptoid / N-substituted peptidic copolymer according to claim 20 or 21, wherein $R_2$ and $R_4$ are each a hydrogen atom.

**23.** Macrocylic peptoid / N-substituted peptidic copolymer according to claim 20, 21 or 22 wherein $R_2$ and $R_4$ are each a hydrogen atom;
$R_1$ represents a linear or branched $C_{1-12}$alkyl, $C_{2-12}$alkenyl, $C_{2-12}$alkynyl, $C_{1-12}$heteroalkyl, $C_{2-12}$heteroalkenyl or $C_{2-12}$heteroalkynyl group bearing an $NH_3^+$ moiety; such as $-(CH_2)_4-NH_3^+$ (the cationic form of a lysine residue side-chain); and
$R_3$ represents a hydrophobic cyclic or acyclic, substituted or unsubstituted, linear or branched $C_{1-12}$alkyl, $C_{2-12}$alkenyl, $C_{2-12}$alkynyl, $C_{1-12}$heteroalkyl, $C_{2-12}$heteroalkenyl, $C_{2-12}$heteroalkynyl, substituted or unsubstituted $C_{6-10}$aryl or $C_{4-10}$heteroaryl group; each of the foregoing alkyl, alkenyl, alkynyl, heteroalkyl, heteroalkenyl, heteroalkynyl, aryl or heteroaryl group; such as the $-CH_2Ph$ side-chain of a phenylalanine residue.

**24.** Dendritic peptoid / N-substituted peptidic copolymer obtainable by a process of any one of claims 1, 5 and 6, wherein the dendritic peptoid / N-substituted peptidic copolymer has the following structure:

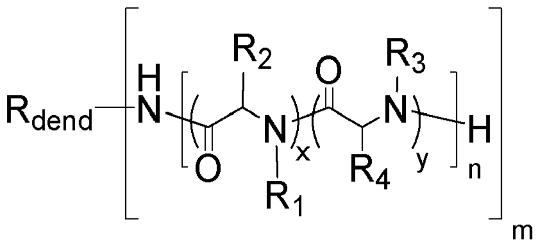

**($V_{dend}$)**

wherein $R_1$, $R_2$, $R_3$ and $R_4$ are as defined in claim 1;
$R_{dend}$ represents a dendritic synthon radical having one of the following structures:

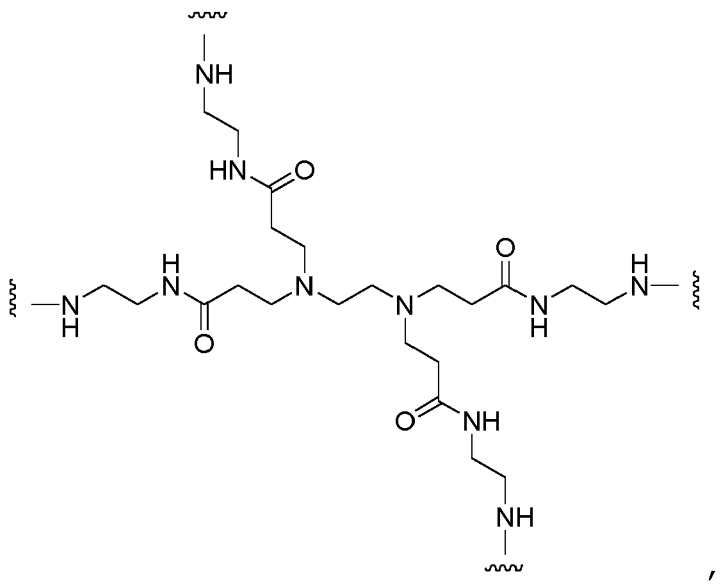

,

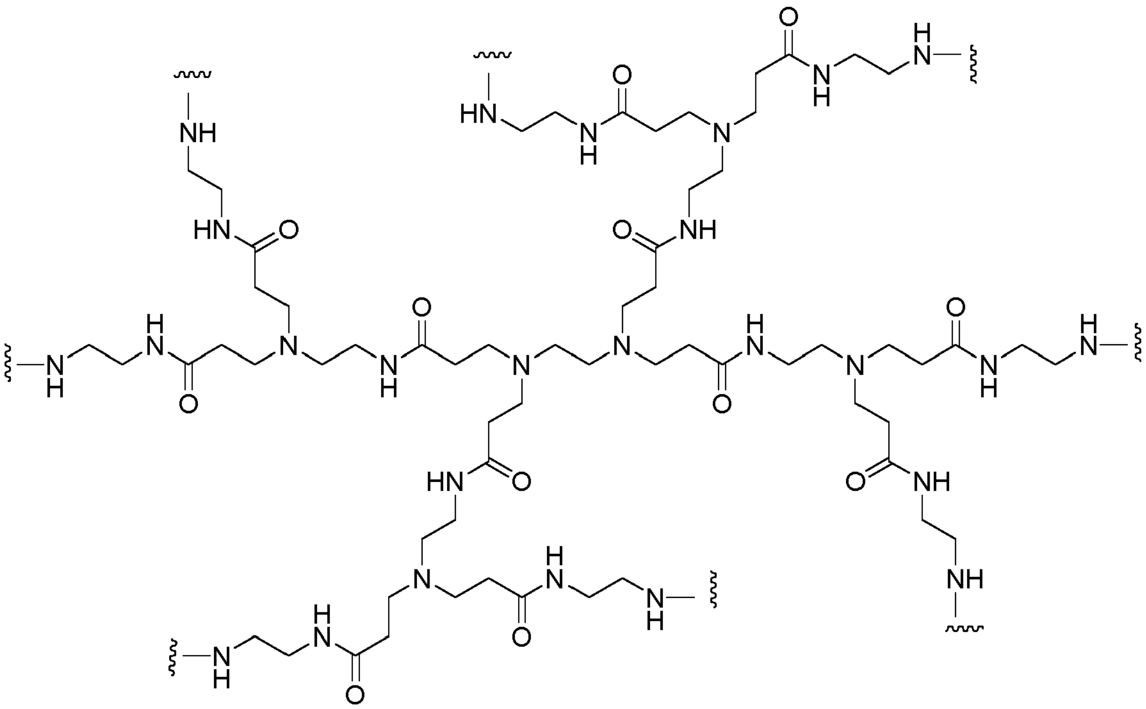

or

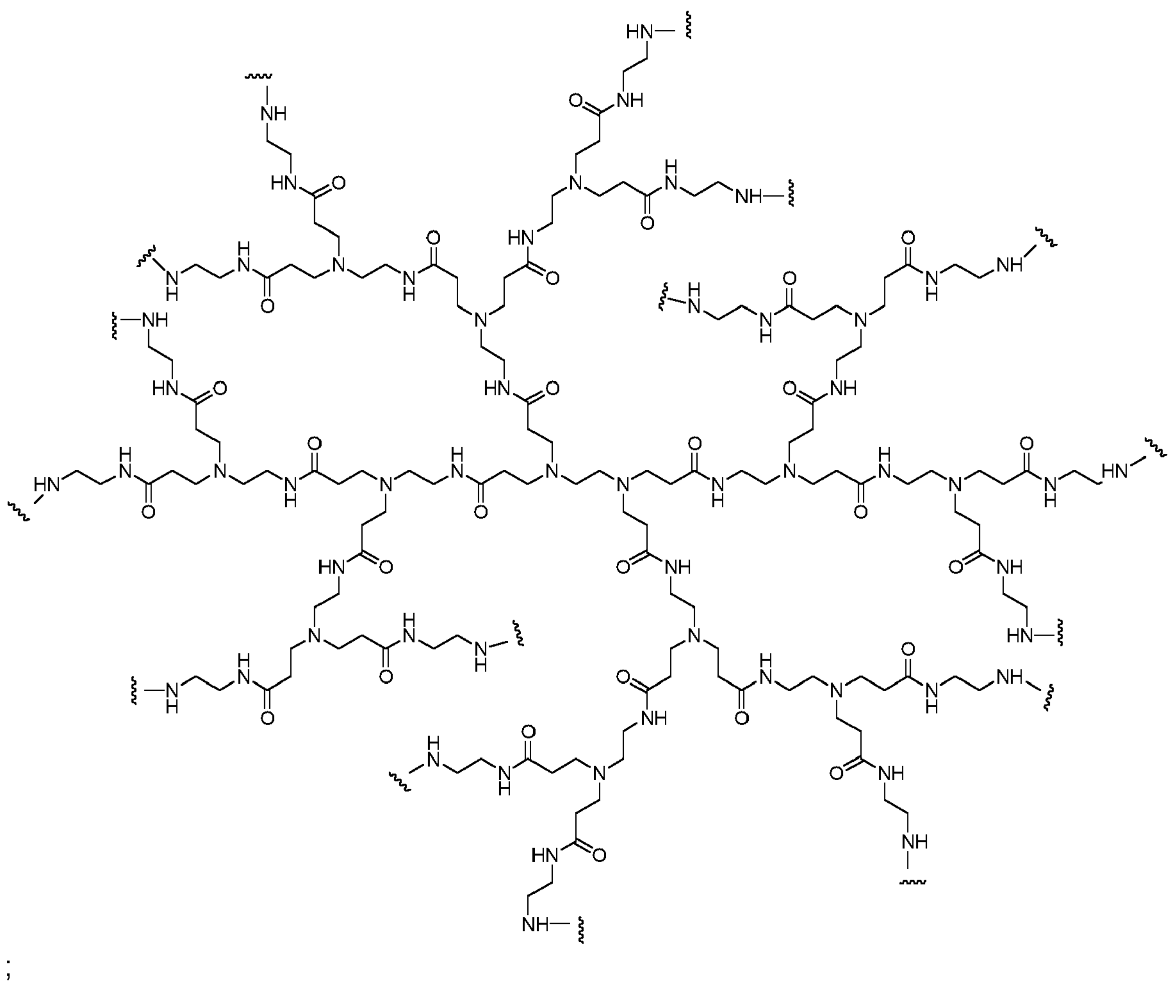

;

x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer;
each occurrence of n independently represents the number of first and second N-carboxyanhydride monomer units in the copolymer branch; and
m represents the number of copolymer units covalently bound to the dendrimer synthon.

**25.** Peptoid / N-substituted peptidic copolymer-drug conjugate obtainable by a process of any one of claims 1, 7 and 8, wherein the peptoid / N-substituted peptidic copolymer-drug conjugate has the following structure:

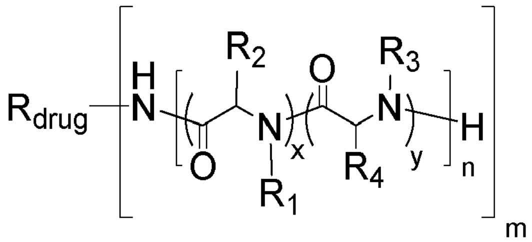

**($V_{conj}$)**

wherein $R_1$, $R_2$, $R_3$ and $R_4$ are as defined in claim 1;
$R_{drug}$ represents the radical of a drug molecule bearing at least one basic primary amino group, such as vancomycin, amoxicilline, kanamycine, cefaclor, amphotericin B, nystatin, amikacin or sulphamethoxazole;
x ranges between 0 and 1, and represents the molar ratio of first N-carboxyanhydride monomer units in the copolymer;
y ranges between 0 and 1, and represents the molar ratio of second N-carboxyanhydride monomer units in the copolymer;
wherein x + y =1 and the x and y repeat monomer units are randomly distributed in the macrocyclic peptoid / N-substituted peptidic copolymer;
each occurrence of n independently the number of first and second N-carboxyanhydride monomer unit in the copolymer; and
m represents the number of copolymer units covalently bound to the drug molecule.

**26.** Peptoid / N-substituted peptidic copolymer-drug conjugate according to claim 25, the group $R_{drug}$ in formula ($V_{conj}$) may have one of the following structures, wherein the arrows designate the point(s) of attachment of the N-substituted copolymer to the group $R_{drug}$:

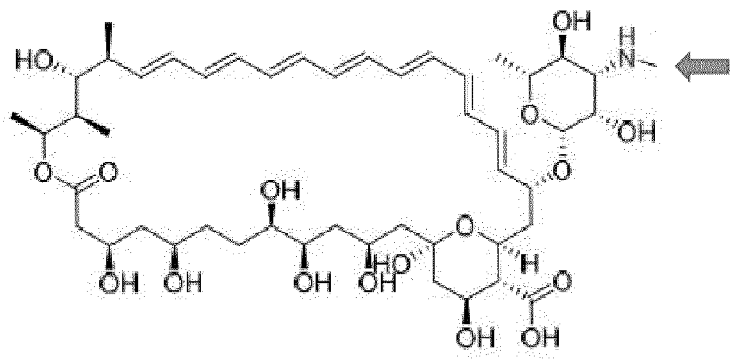

,

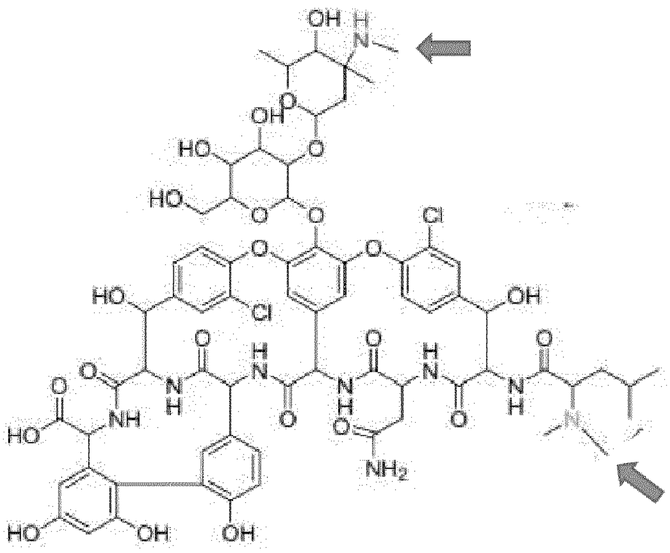

,

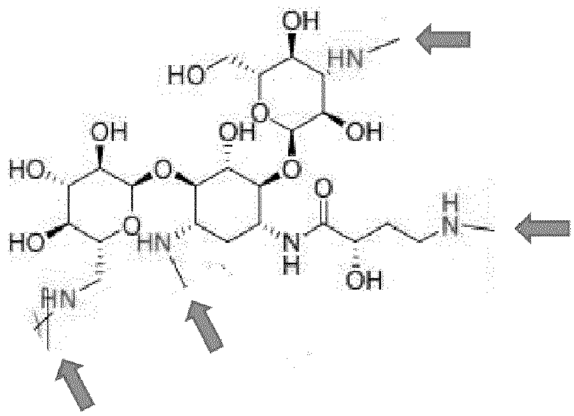

or

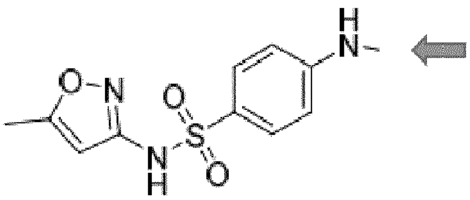

.

**27.** Peptoid / N-substituted peptidic copolymer obtainable by a process according to any one of claims 1-14, wherein the peptoid / N-substituted peptidic copolymer is resistant to peptidases in aqueous medium.

**28.** Peptoid / N-substituted peptidic copolymer obtainable by a process according to any one of claims 1-14, wherein the peptoid / N-substituted peptidic copolymer is effective at killing bacteria.

**29.** Peptoid / N-substituted peptidic copolymer obtainable by a process according to any one of claims 1-14, wherein the peptoid / N-substituted peptidic copolymer mimicks the antimicrobial activity of antimicrobial peptides against *Clostridioïdes difficile, S. aureus meti-S, S. aureus meti-R, Streptococcus pneumoniae, Listeria monocytogenes, Enterococcus faecalis, Escherichia coli, Acinetobacter baumanii, Pseudomonas aeruginosa, Bacteroides fragilis,* and/or *Helicobacter pylori.*

**30.** Pharmaceutical composition comprising an effective amount of a peptoid / N-substituted peptidic copolymer obtainable by a process according to any one of claims 1-12 or pharmaceutically acceptable derivative thereof, and a pharmaceutically acceptable carrier, adjuvant, or vehicle.

**31.** The pharmaceutical composition of claim 30, wherein the composition is formulated to be coated on the surface of an implantable medical device.

**32.** Peptoid / N-substituted peptidic copolymer obtainable by a process according to any one of claims 1-12 or pharmaceutically acceptable derivative thereof, for use as antimicrobial agent.

**33.** An item impregnated with or coated with the composition of claim 30 or 31, wherein the item is selected from the group consisting of a medical device, medical instrument, medical implement, prosthetic, implantable device or material or tissue and wound dressing.

**FIG. 1A**

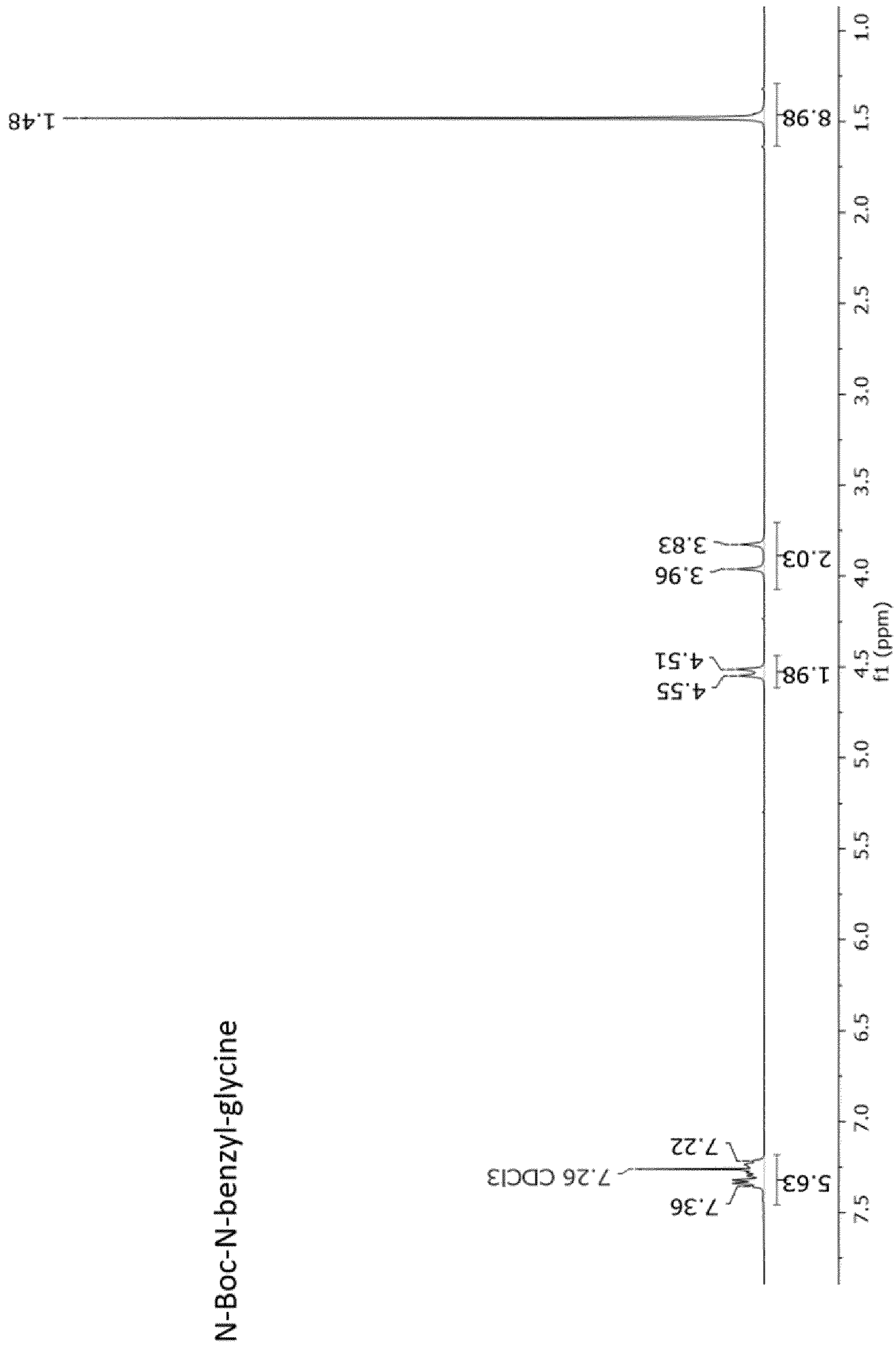
N-Boc-N-benzyl-glycine
1.48
8.98
3.83
3.96
2.03
4.51
4.55
1.98
7.22
7.26 CDCl3
7.36
5.63
1.0
1.5
2.0
2.5
3.0
3.5
4.0
4.5
5.0
5.5
6.0
6.5
7.0
7.5
f1 (ppm)

**FIG. 1B**

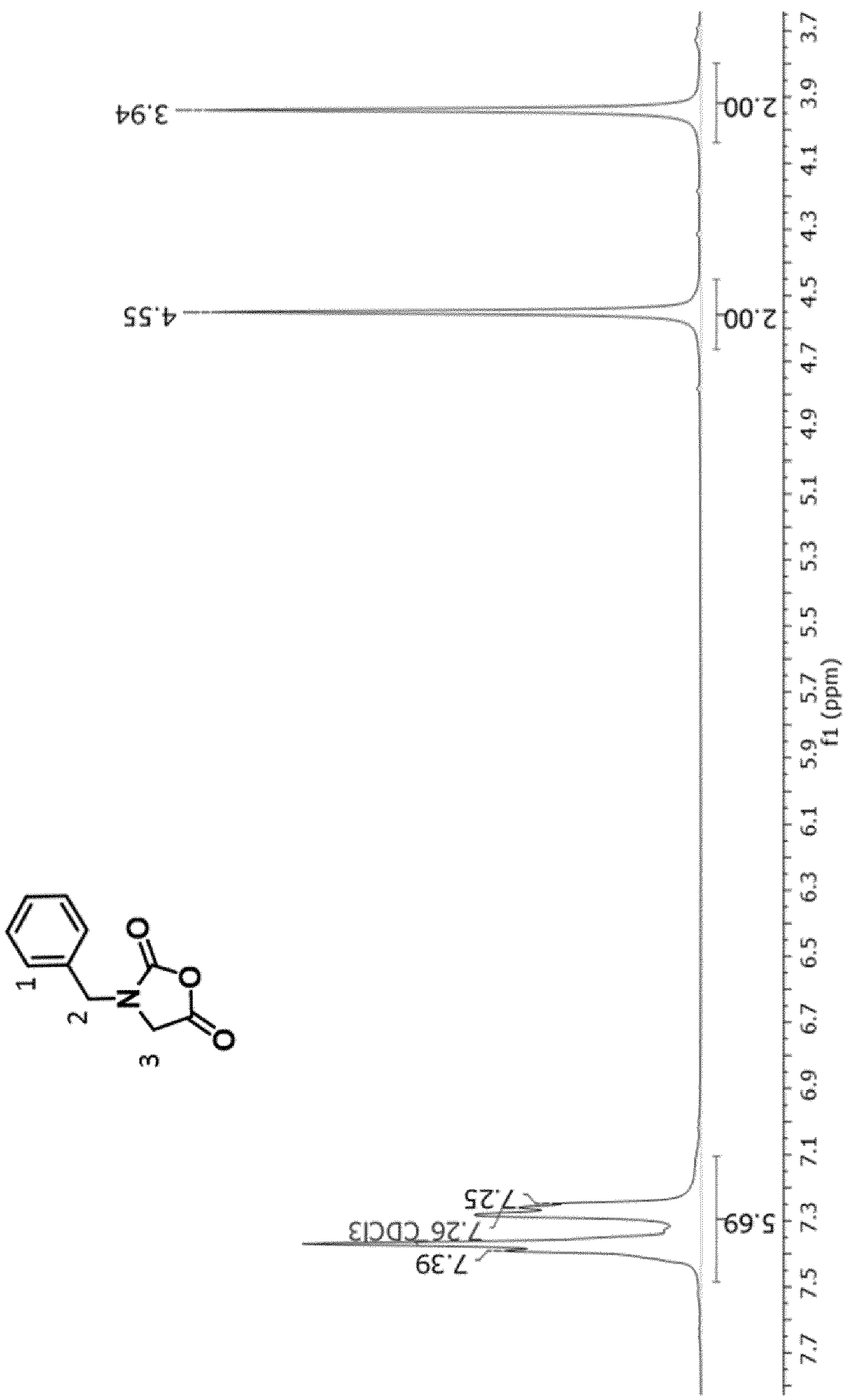

**FIG. 2**

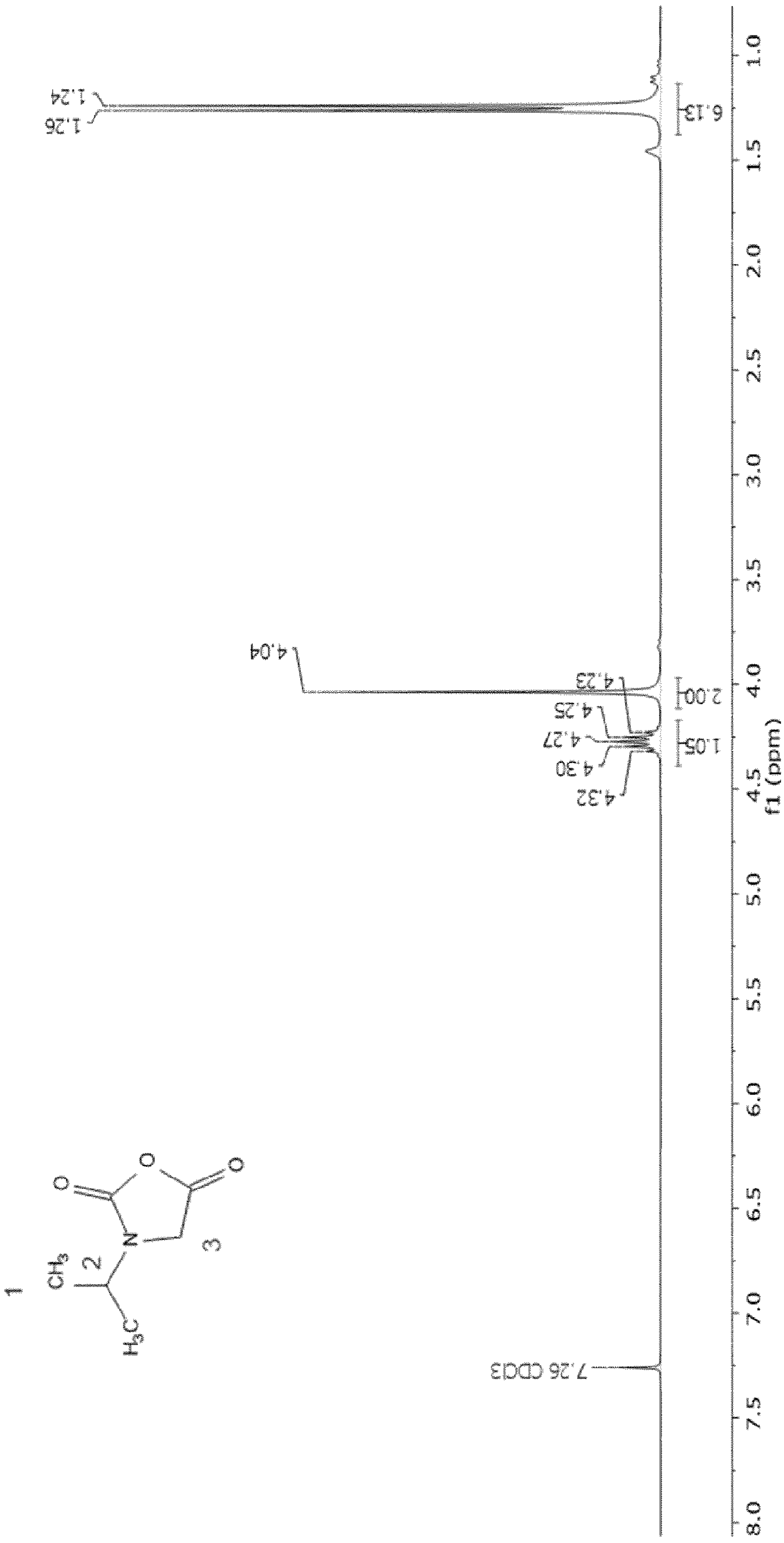
1.24
1.26
6.13
4.04
4.23
4.25
4.27
4.30
4.32
2.00
1.05
7.26 CDCl3
f1 (ppm)
1.0 1.5 2.0 2.5 3.0 3.5 4.0 4.5 5.0 5.5 6.0 6.5 7.0 7.5 8.0

**FIG. 3**

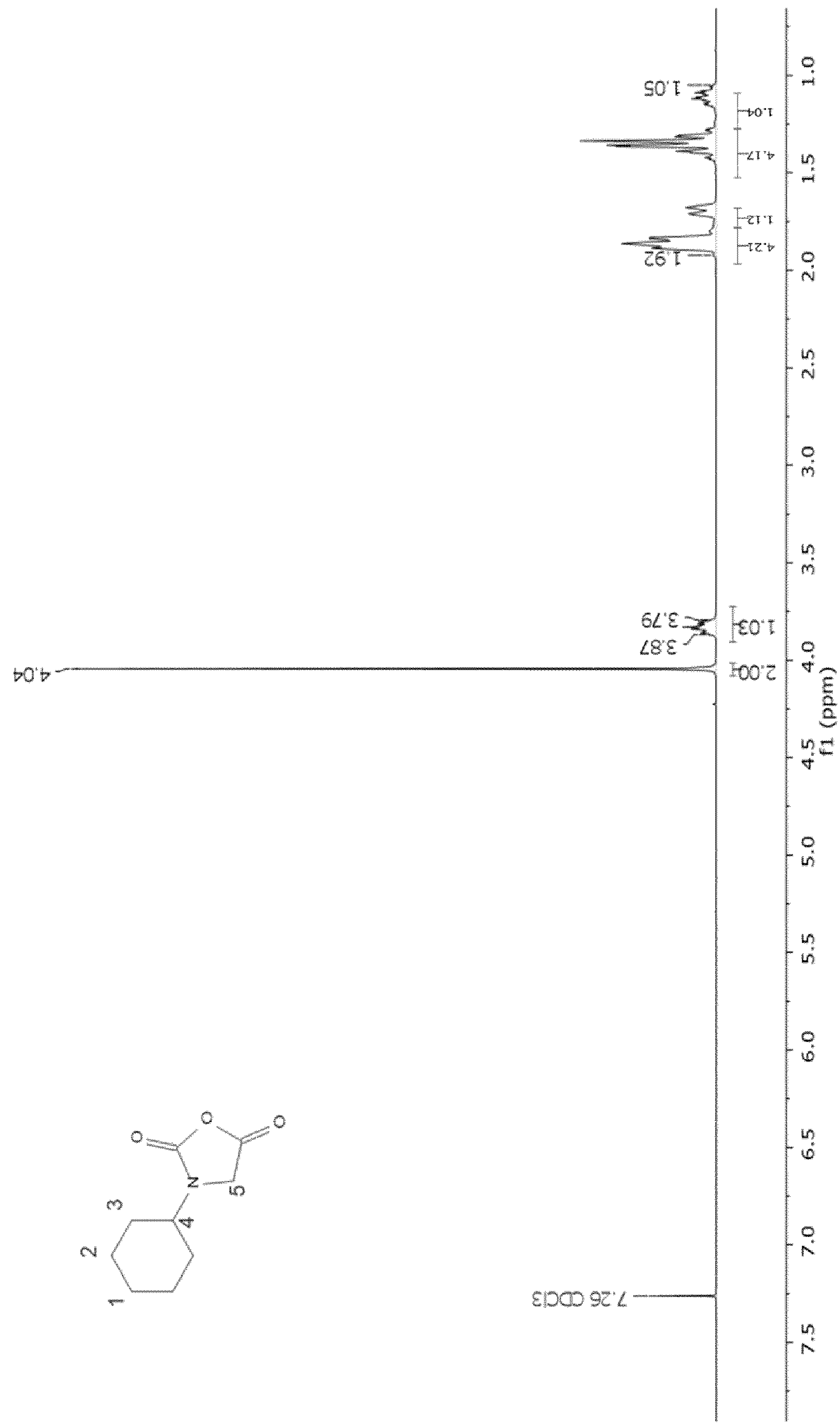

FIG. 4

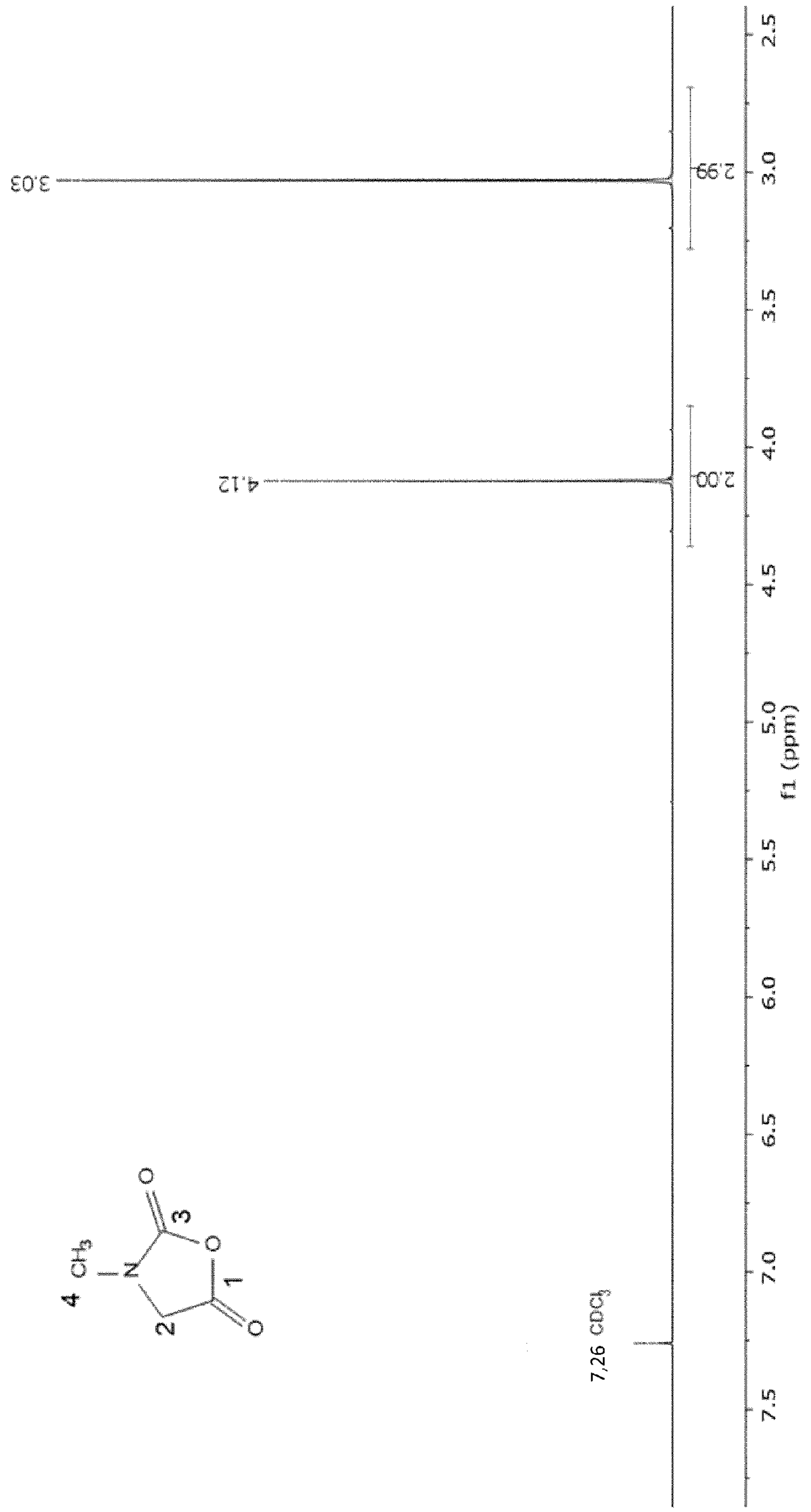

**FIG. 5**

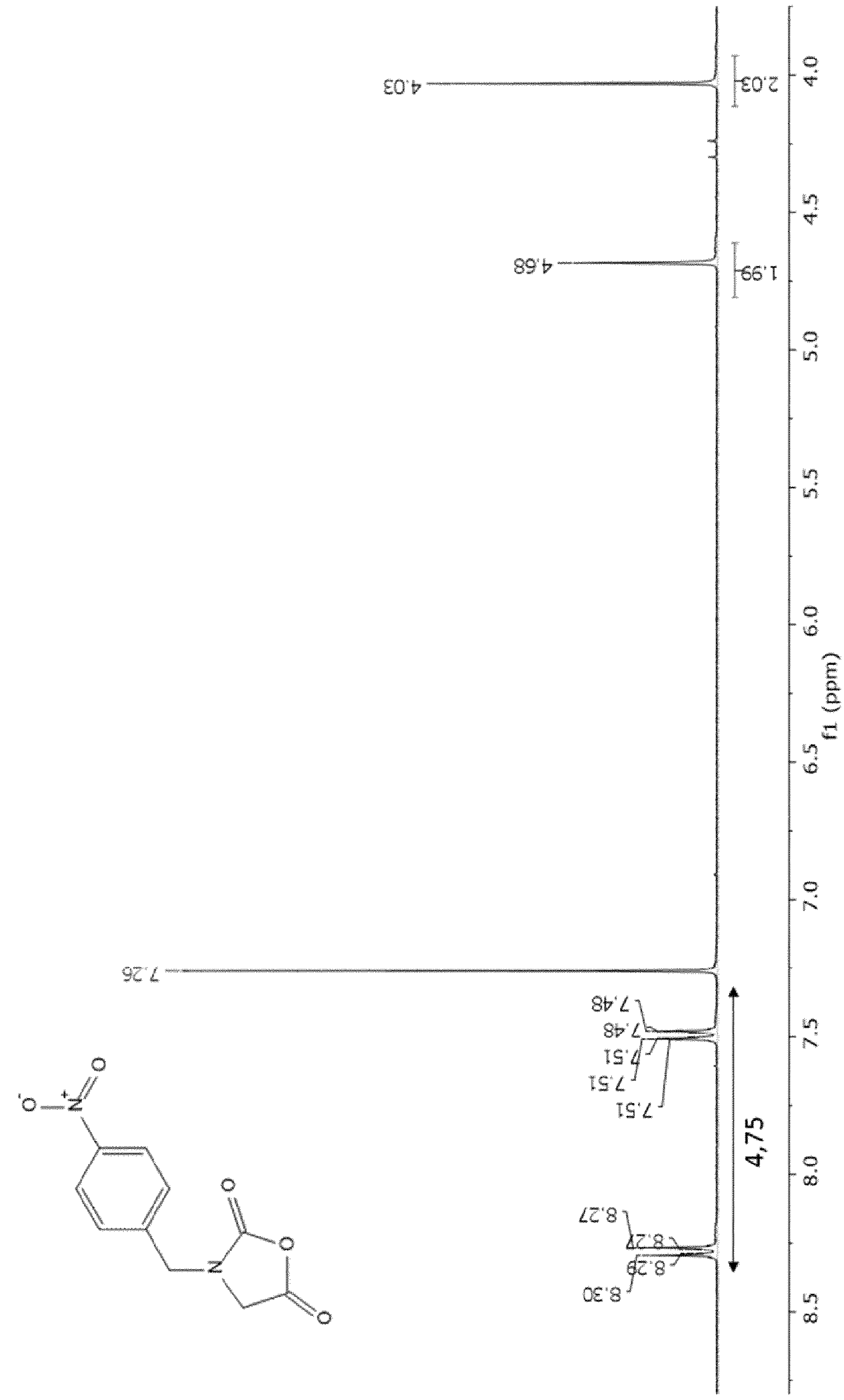
4.03
2.03
4.68
1.99
7.26
7.48
7.48
7.51
7.51
7.51
8.27
8.27
8.29
8.30
4,75
4.0
4.5
5.0
5.5
6.0
6.5
7.0
7.5
8.0
8.5
f1 (ppm)

# FIG. 6

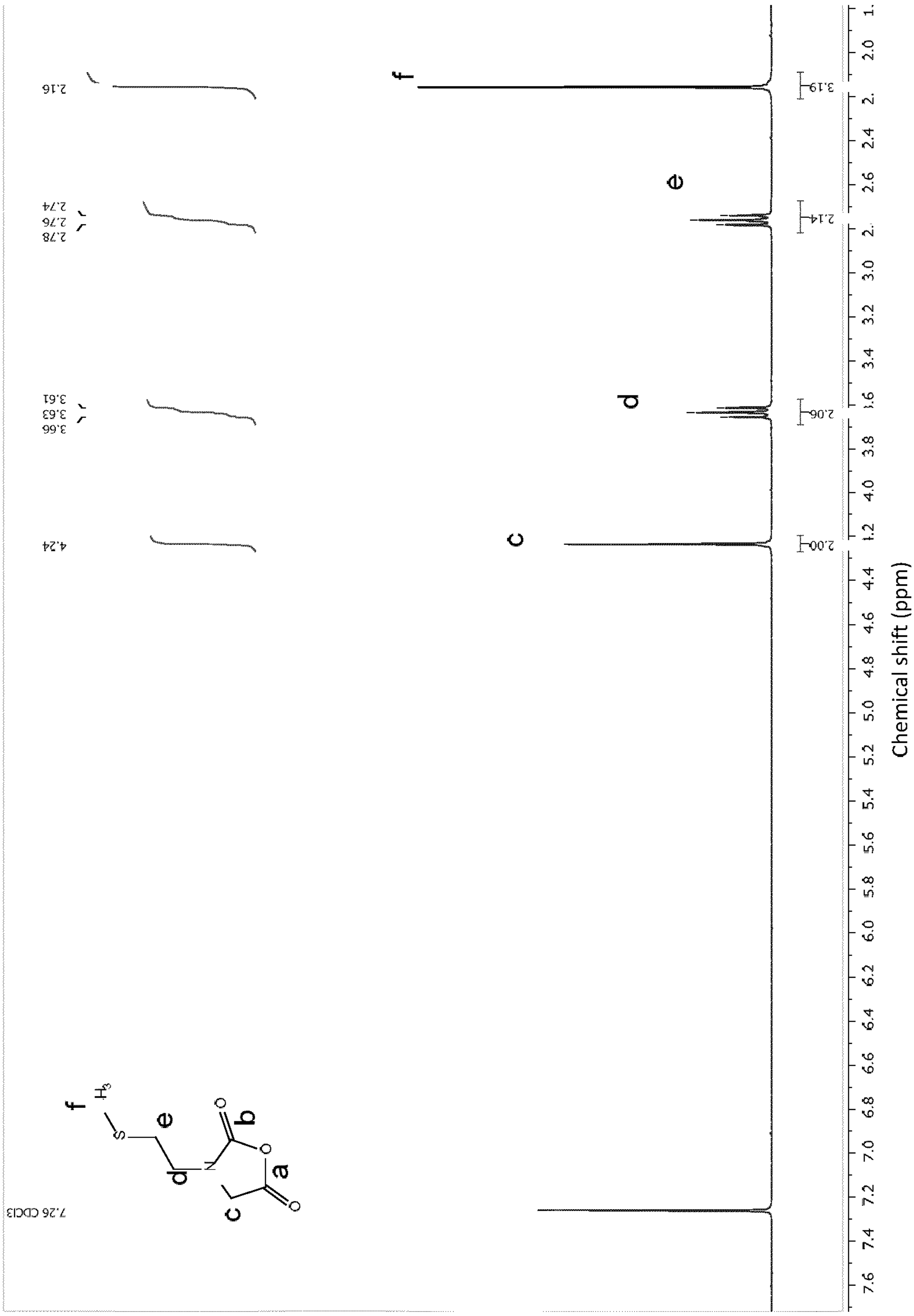
Chemical shift (ppm)
7.26 CDCl3
4.24
3.66
3.63
3.61
2.78
2.76
2.74
2.16
2.00
2.06
2.14
3.19

**FIG. 7**

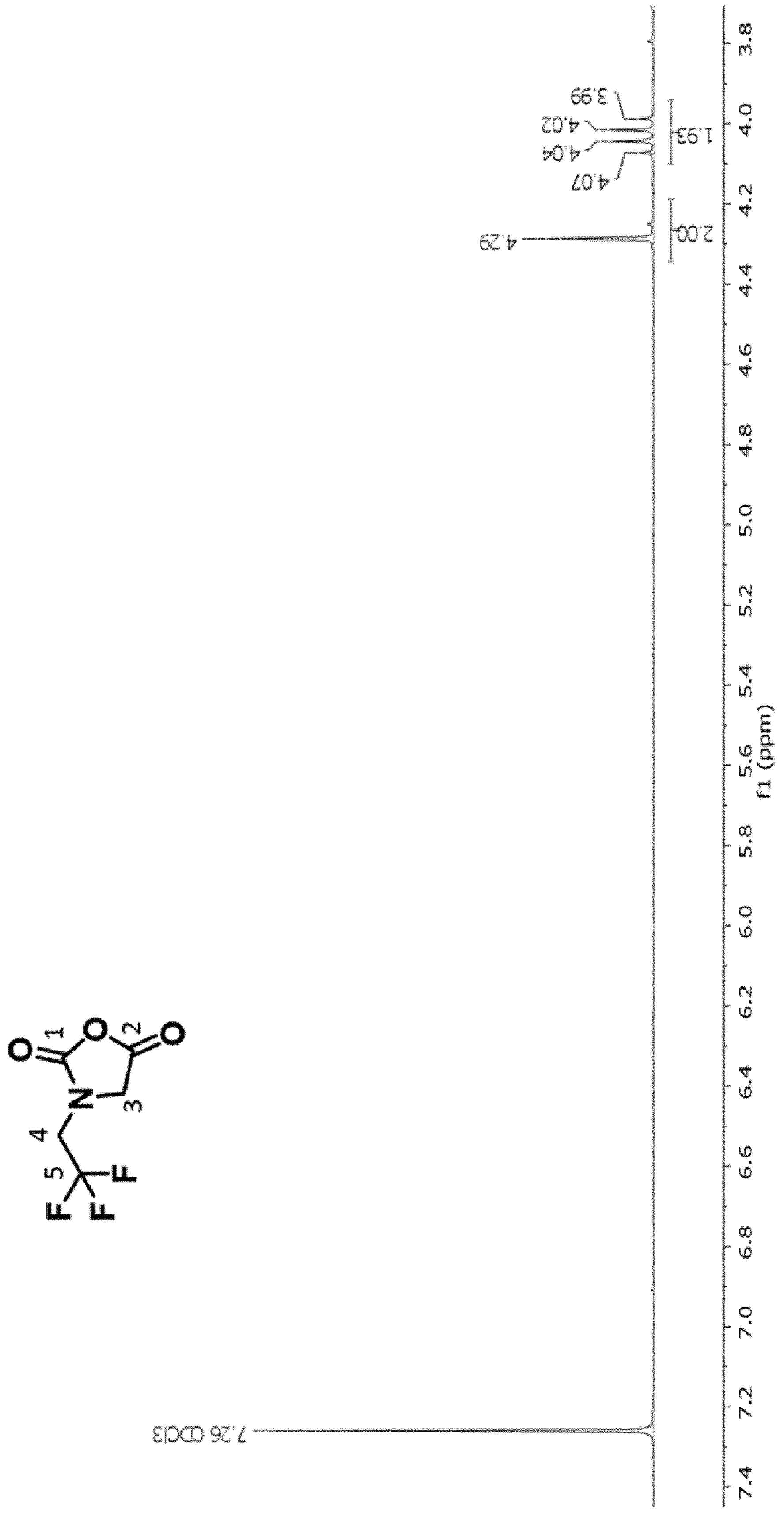
3.99
4.02
4.04
4.07
4.29
1.93
2.00
7.26 CDCl3
3.8
4.0
4.2
4.4
4.6
4.8
5.0
5.2
5.4
5.6
5.8
6.0
6.2
6.4
6.6
6.8
7.0
7.2
7.4
f1 (ppm)

**FIG. 8**

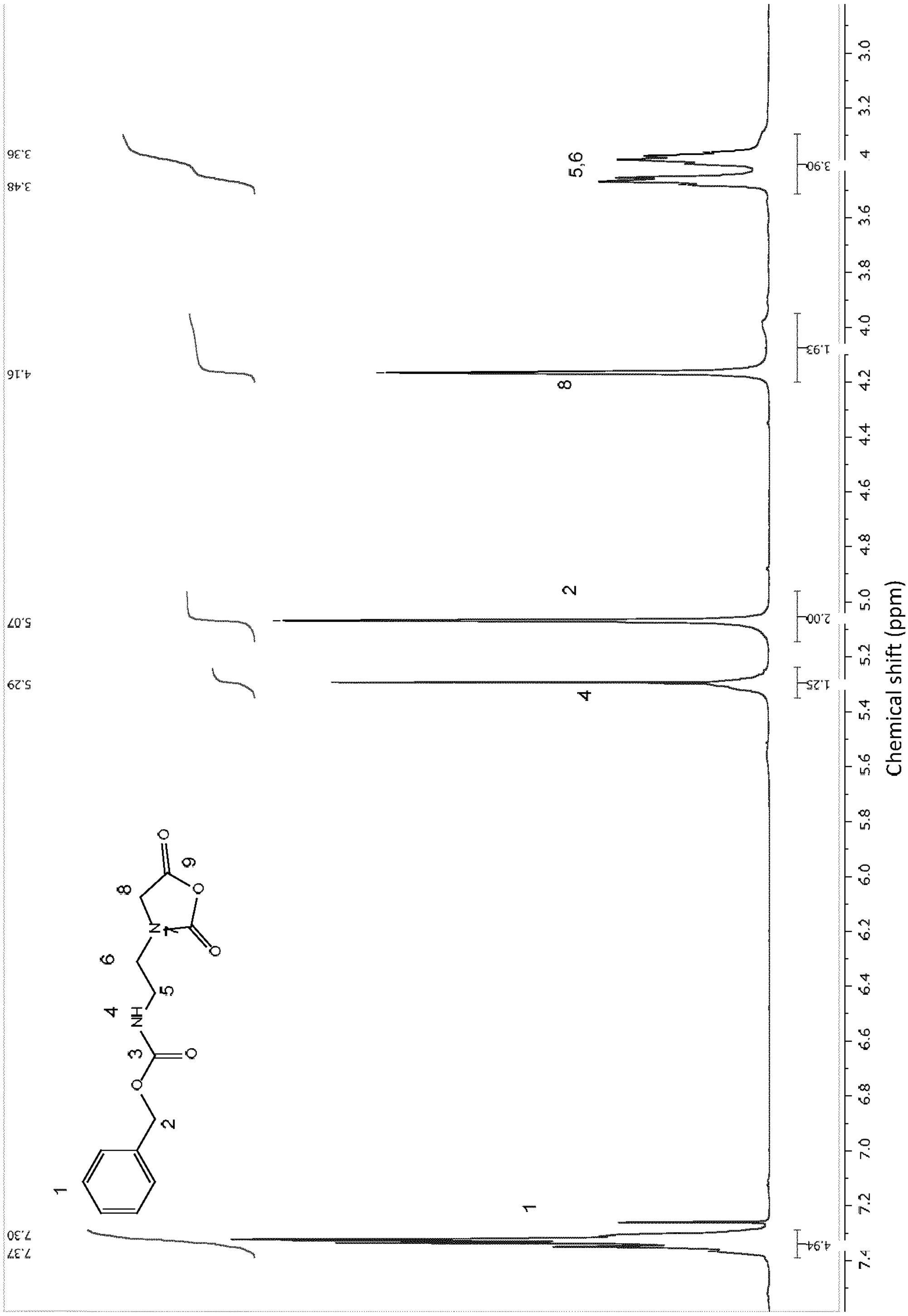

**FIG. 9**

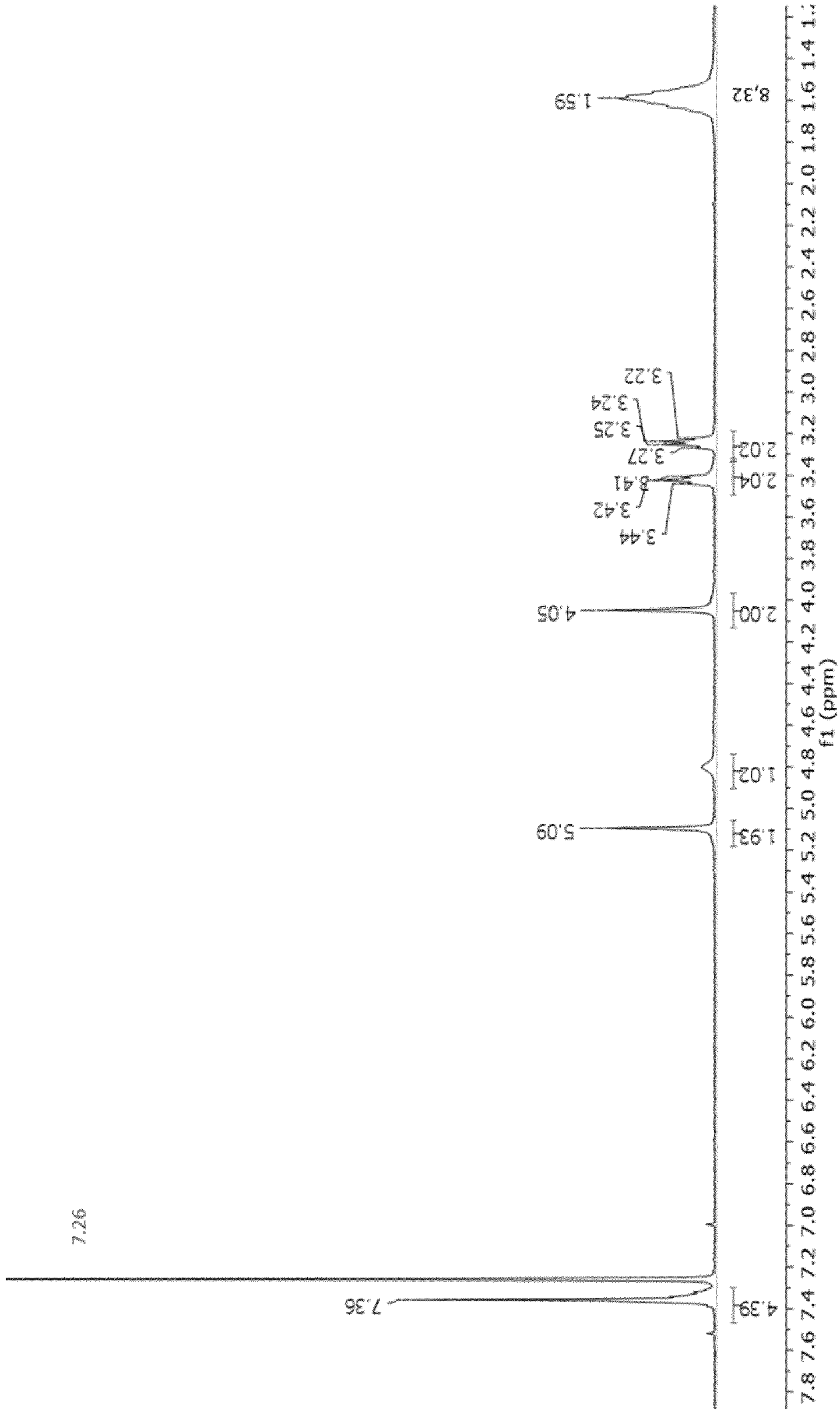
1.59
3.22
3.24
3.25
3.27
3.41
3.42
3.44
4.05
5.09
7.36
7.26
8.32
2.02
2.04
2.00
1.02
1.93
4.39
7.8 7.6 7.4 7.2 7.0 6.8 6.6 6.4 6.2 6.0 5.8 5.6 5.4 5.2 5.0 4.8 4.6 4.4 4.2 4.0 3.8 3.6 3.4 3.2 3.0 2.8 2.6 2.4 2.2 2.0 1.8 1.6 1.4
f1 (ppm)

**FIG. 10**

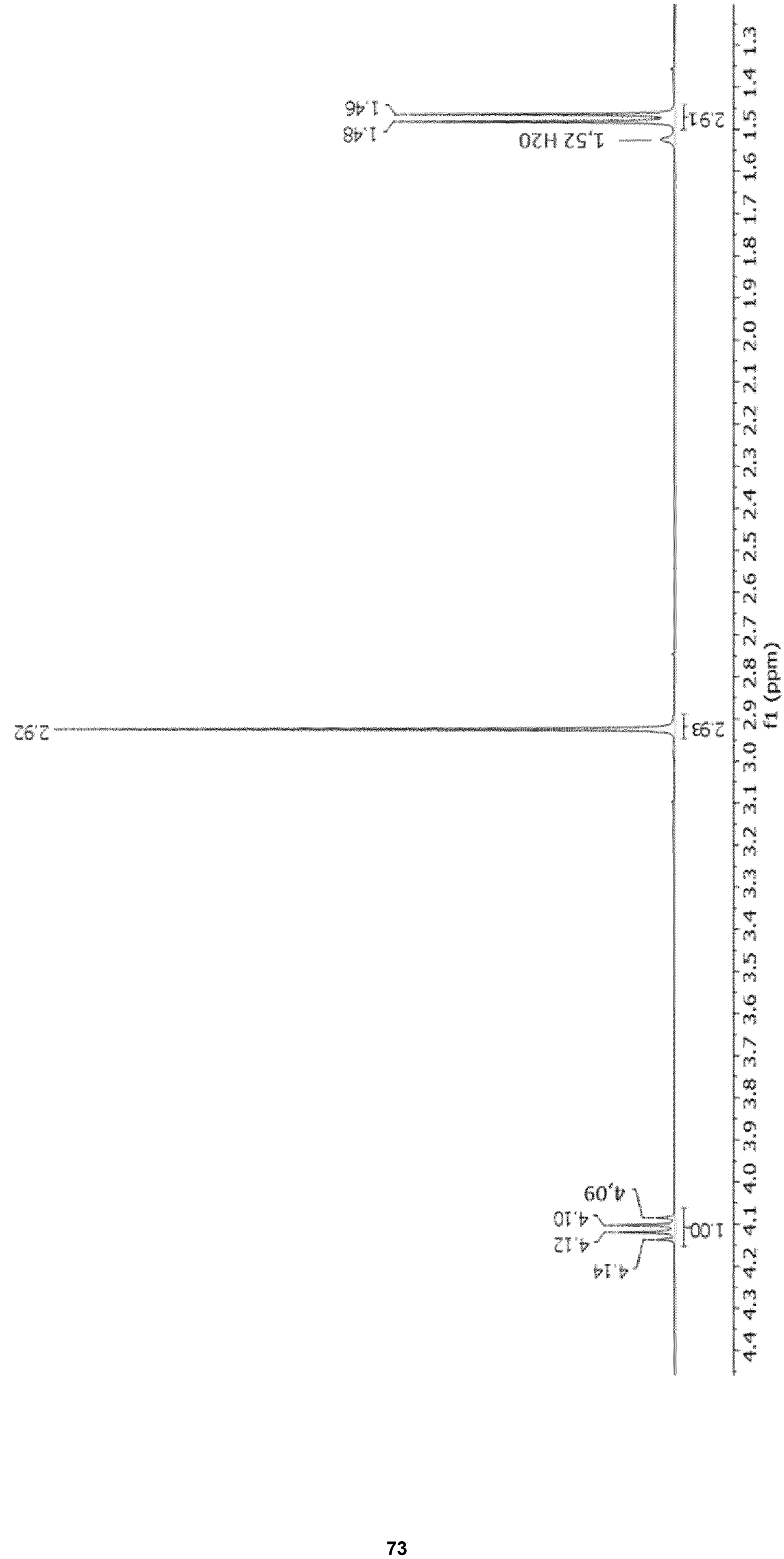

FIG. 11

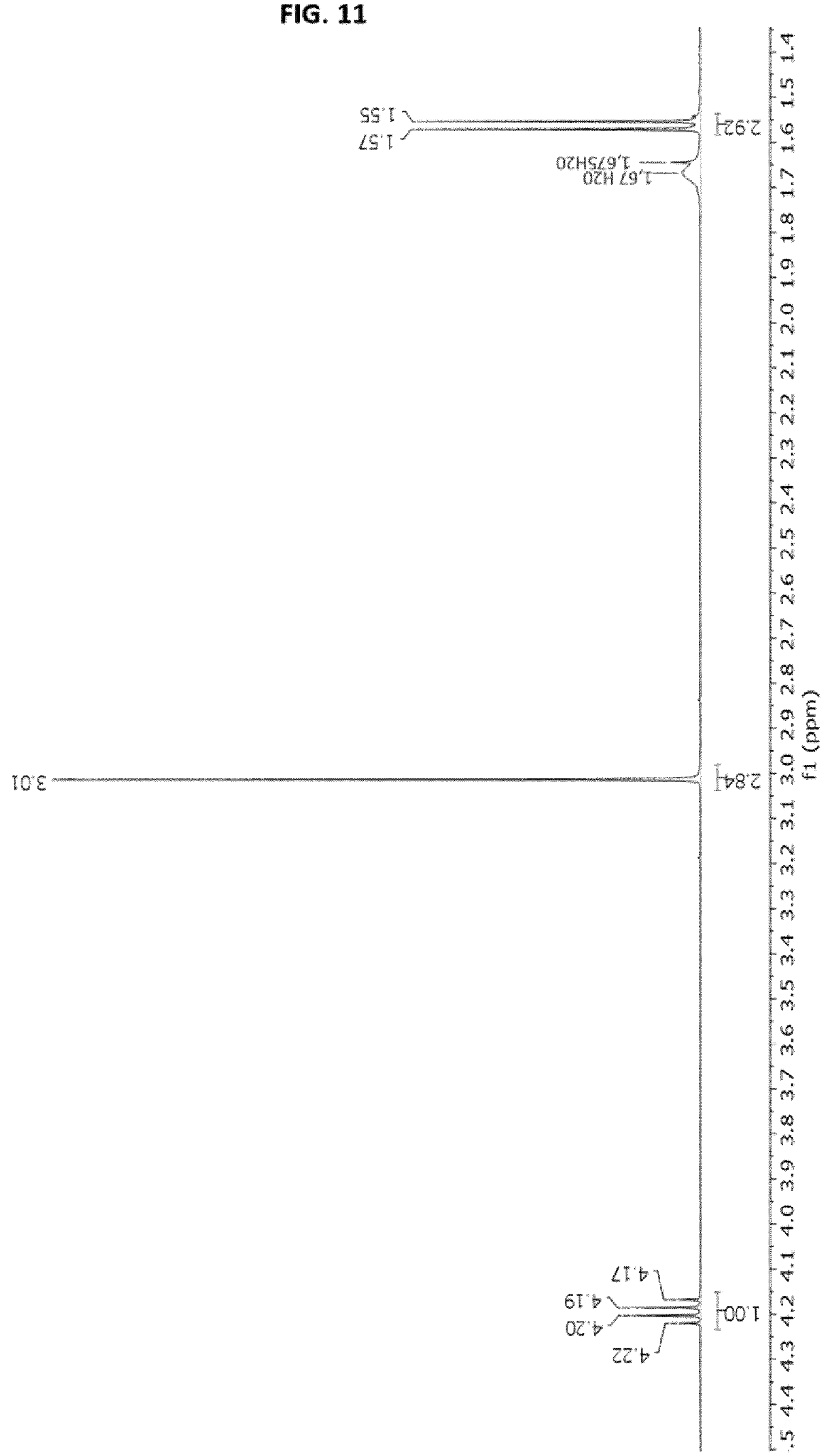

FIG. 12

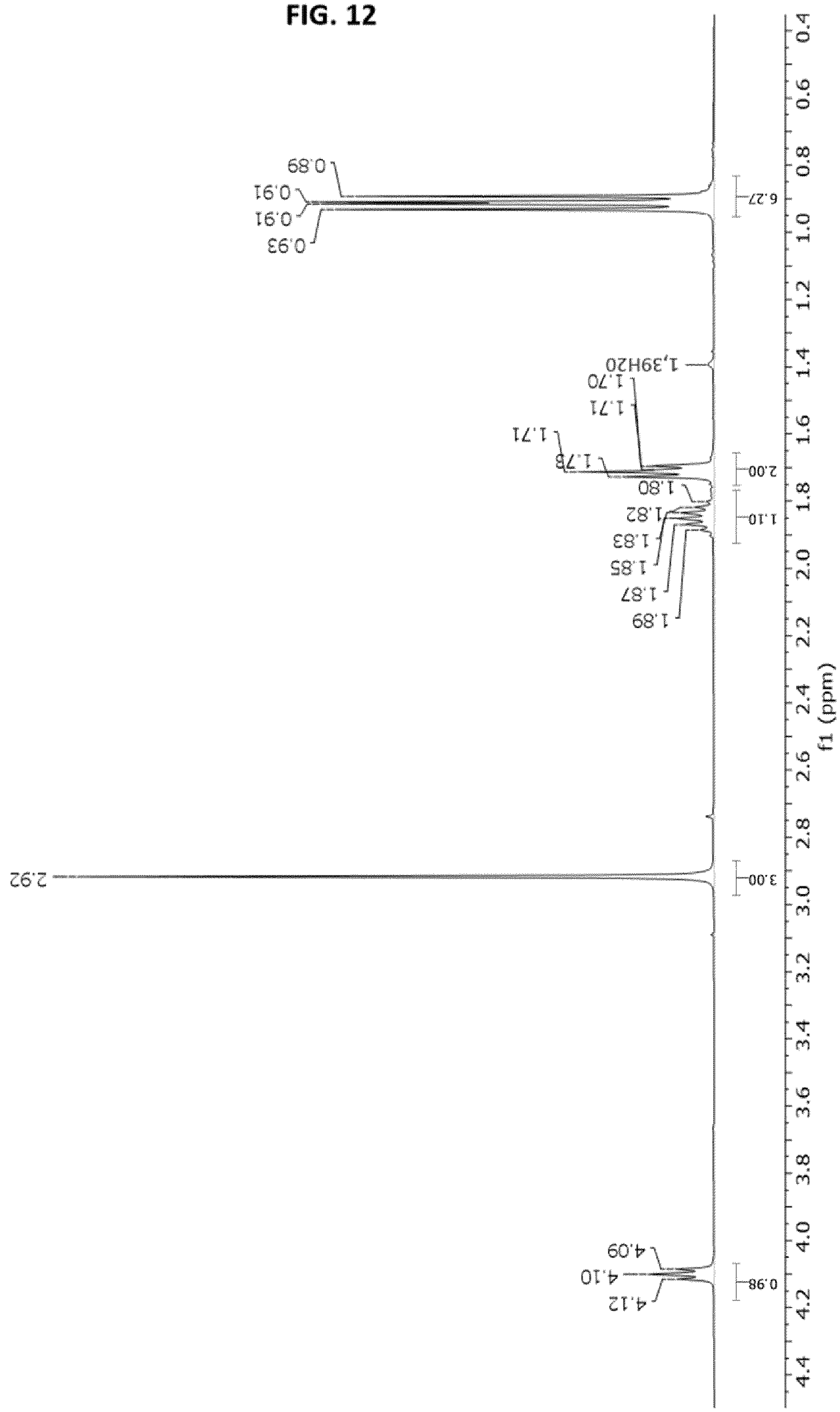
0.89
0.91
0.91
0.93
1,39H2O
1.70
1.71
1.71
1.78
1.80
1.82
1.83
1.85
1.87
1.89
2.92
4.09
4.10
4.12
6.27
2.00
1.10
3.00
0.98
f1 (ppm)

FIG. 13

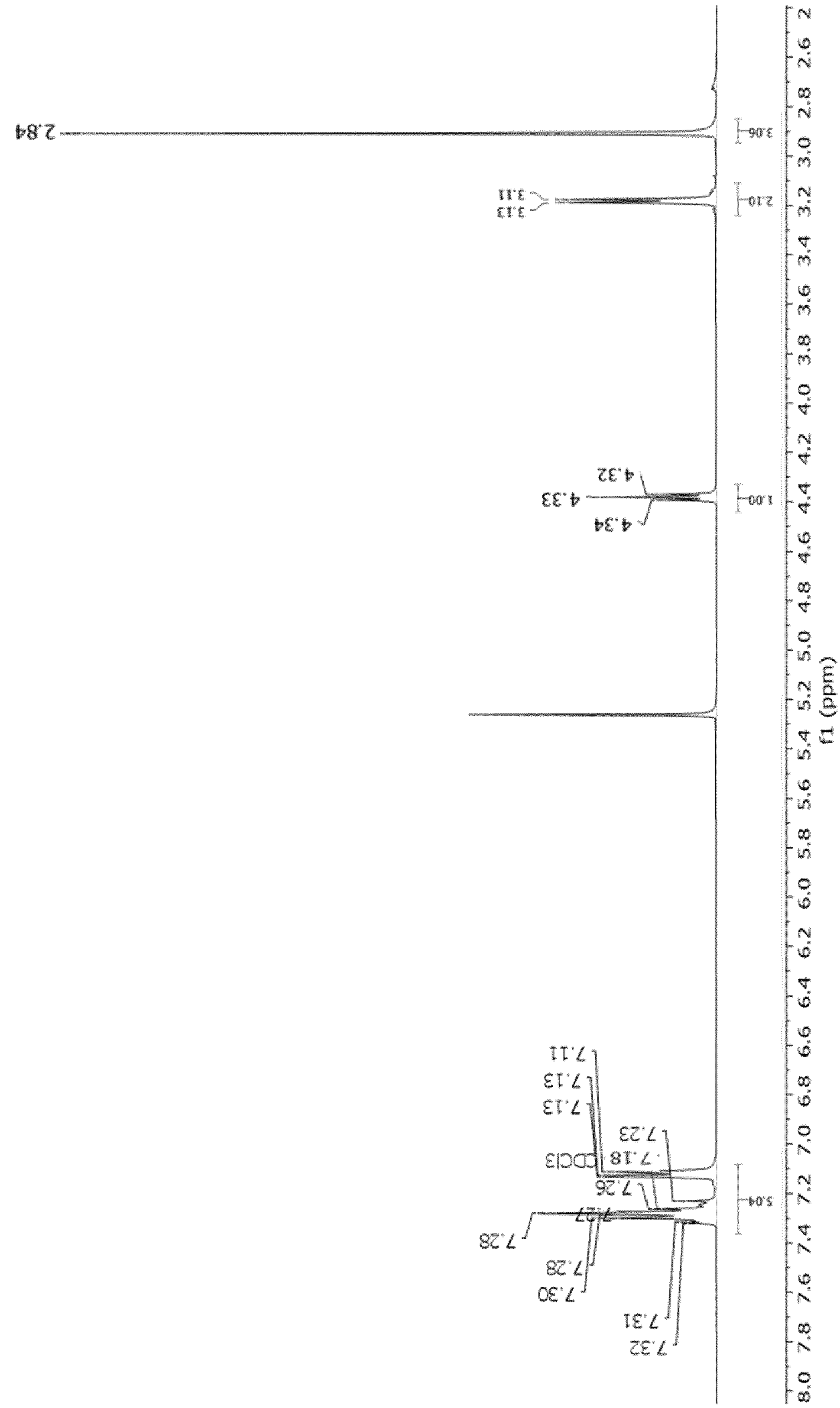
2.84
3.11
3.13
4.32
4.33
4.34
7.11
7.13
7.13
CDCl3
7.18
7.23
7.26
7.27
7.28
7.28
7.30
7.31
7.32
3.06
2.10
1.00
5.04
f1 (ppm)

**FIG. 14**

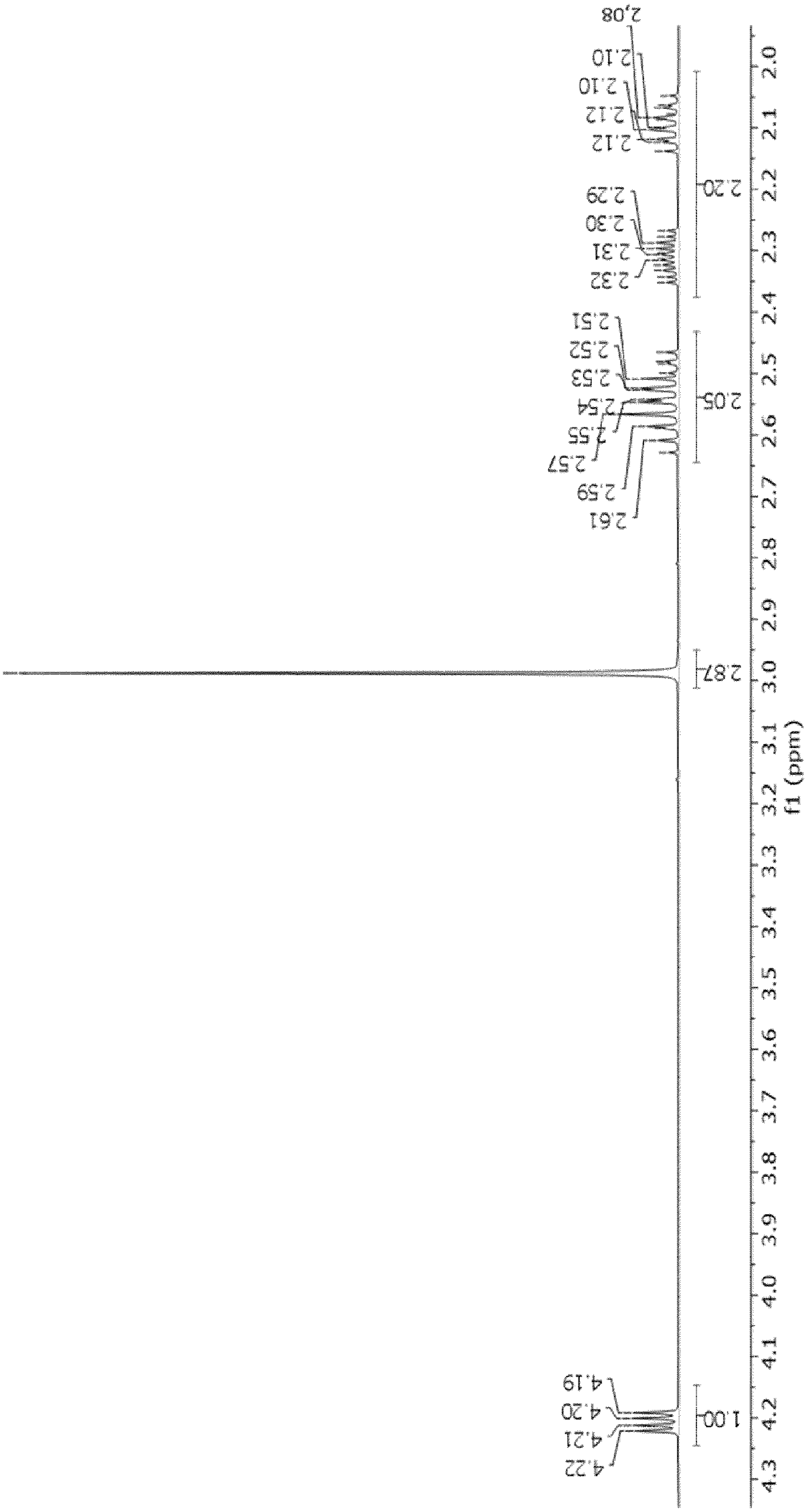
2.08
2.10
2.10
2.12
2.12
2.29
2.30
2.31
2.32
2.51
2.52
2.53
2.54
2.55
2.57
2.59
2.61
2.99
4.19
4.20
4.21
4.22
2.20
2.05
2.87
1.00
2.0
2.1
2.2
2.3
2.4
2.5
2.6
2.7
2.8
2.9
3.0
3.1
3.2
3.3
3.4
3.5
3.6
3.7
3.8
3.9
4.0
4.1
4.2
4.3
f1 (ppm)

**FIG. 15**

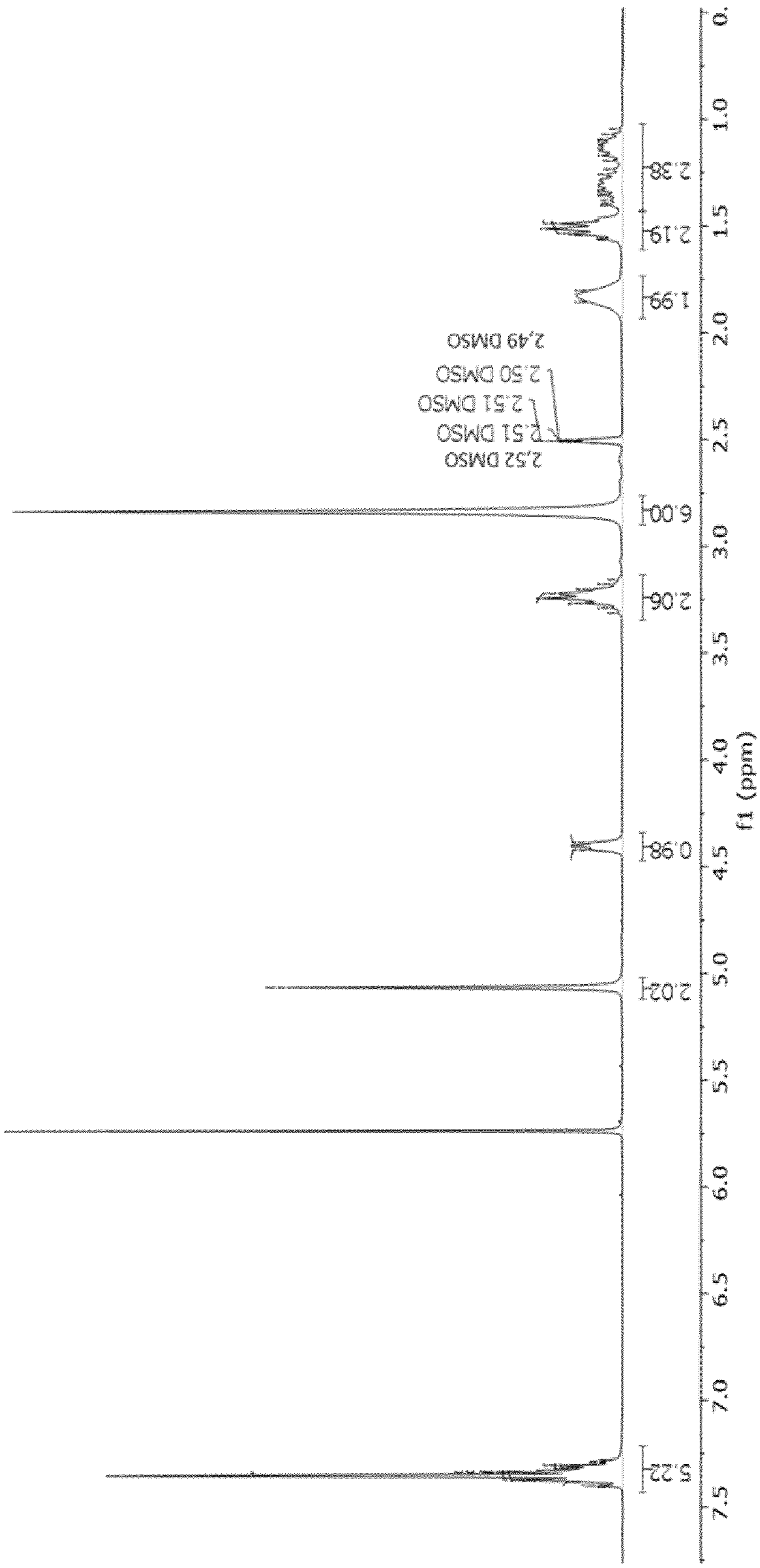
2,49 DMSO
2,50 DMSO
2,51 DMSO
2,51 DMSO
2,52 DMSO
2.38
2.19
1.99
6.00
2.06
0.98
2.02
5.22
f1 (ppm)

FIG. 16A

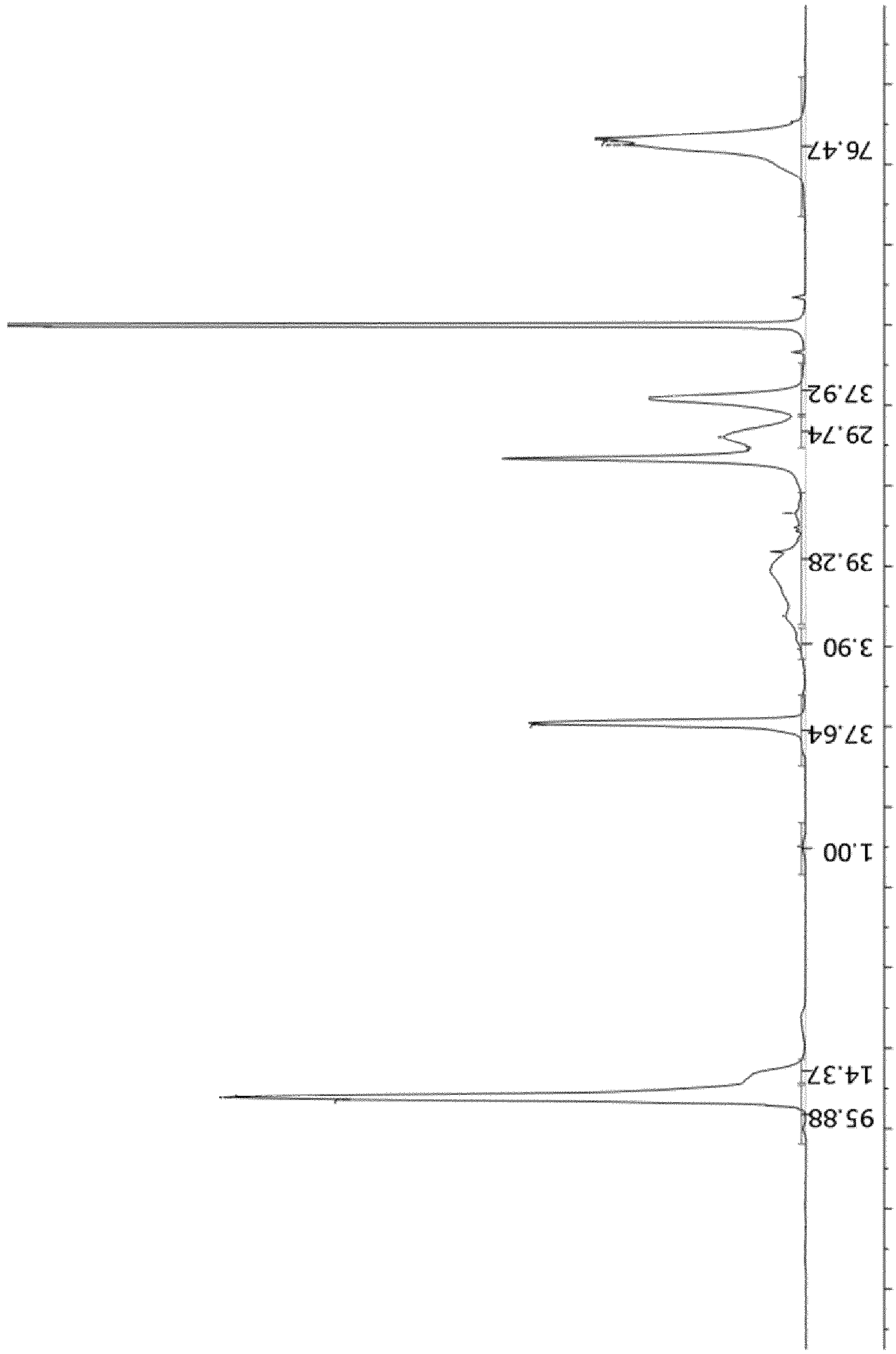

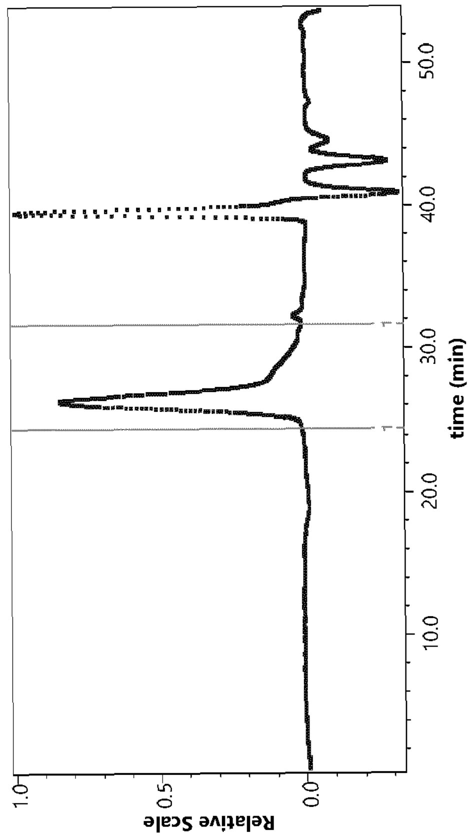


FIG. 16B

**FIG. 17**

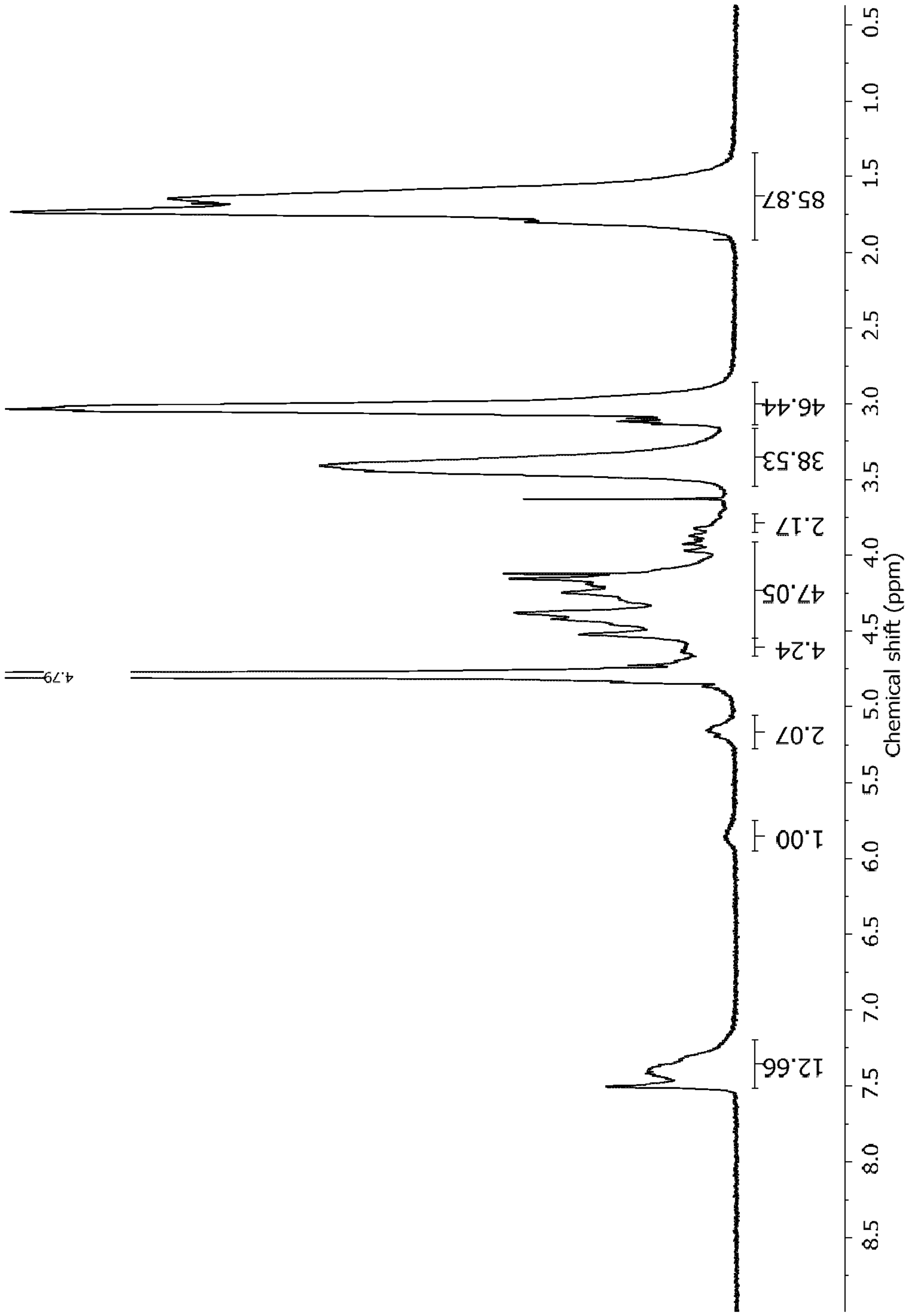
85.87
46.44
38.53
2.17
47.05
4.24
4.79
2.07
1.00
12.66
0.5
1.0
1.5
2.0
2.5
3.0
3.5
4.0
4.5
5.0
5.5
6.0
6.5
7.0
7.5
8.0
8.5
Chemical shift (ppm)

**FIG. 18A**

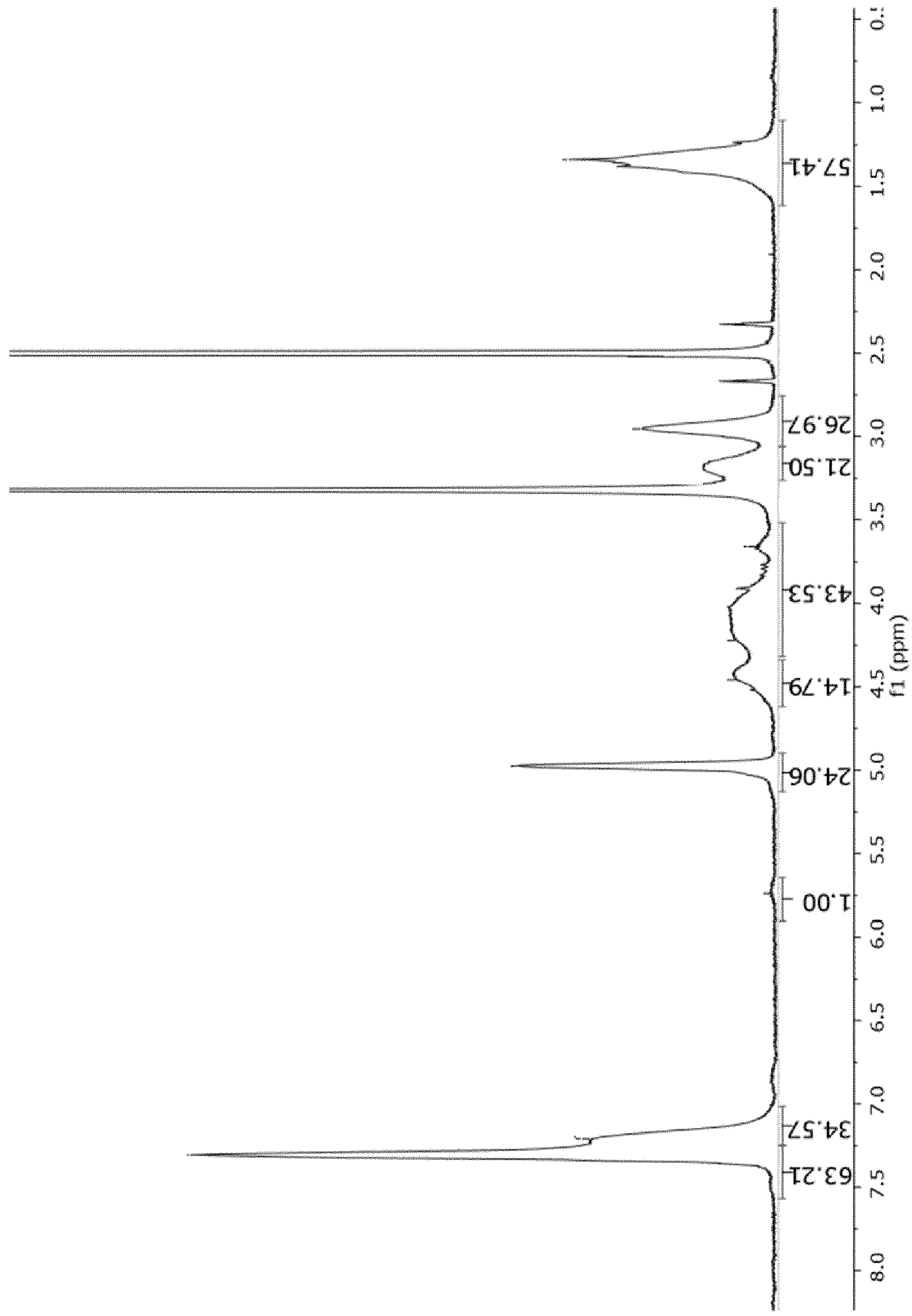
57.41
26.97
21.50
43.53
14.79
24.06
1.00
34.57
63.21
f1 (ppm)
0.5
1.0
1.5
2.0
2.5
3.0
3.5
4.0
4.5
5.0
5.5
6.0
6.5
7.0
7.5
8.0

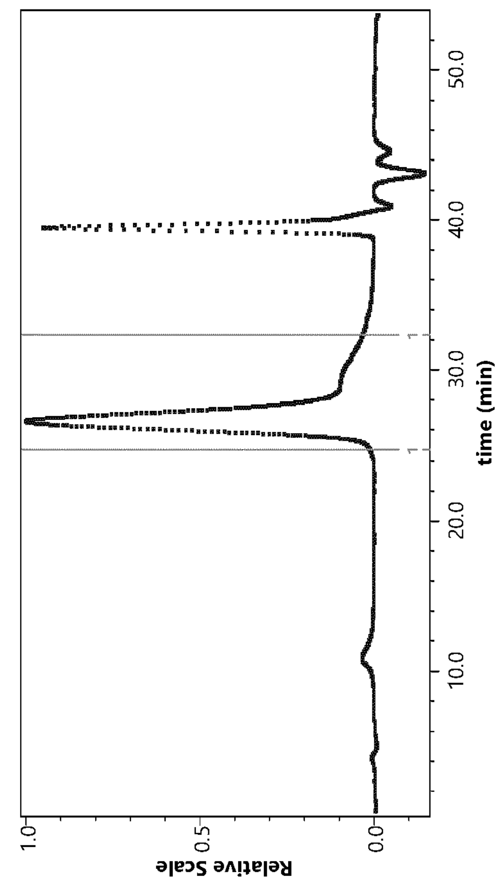


FIG. 18B

**FIG. 19**

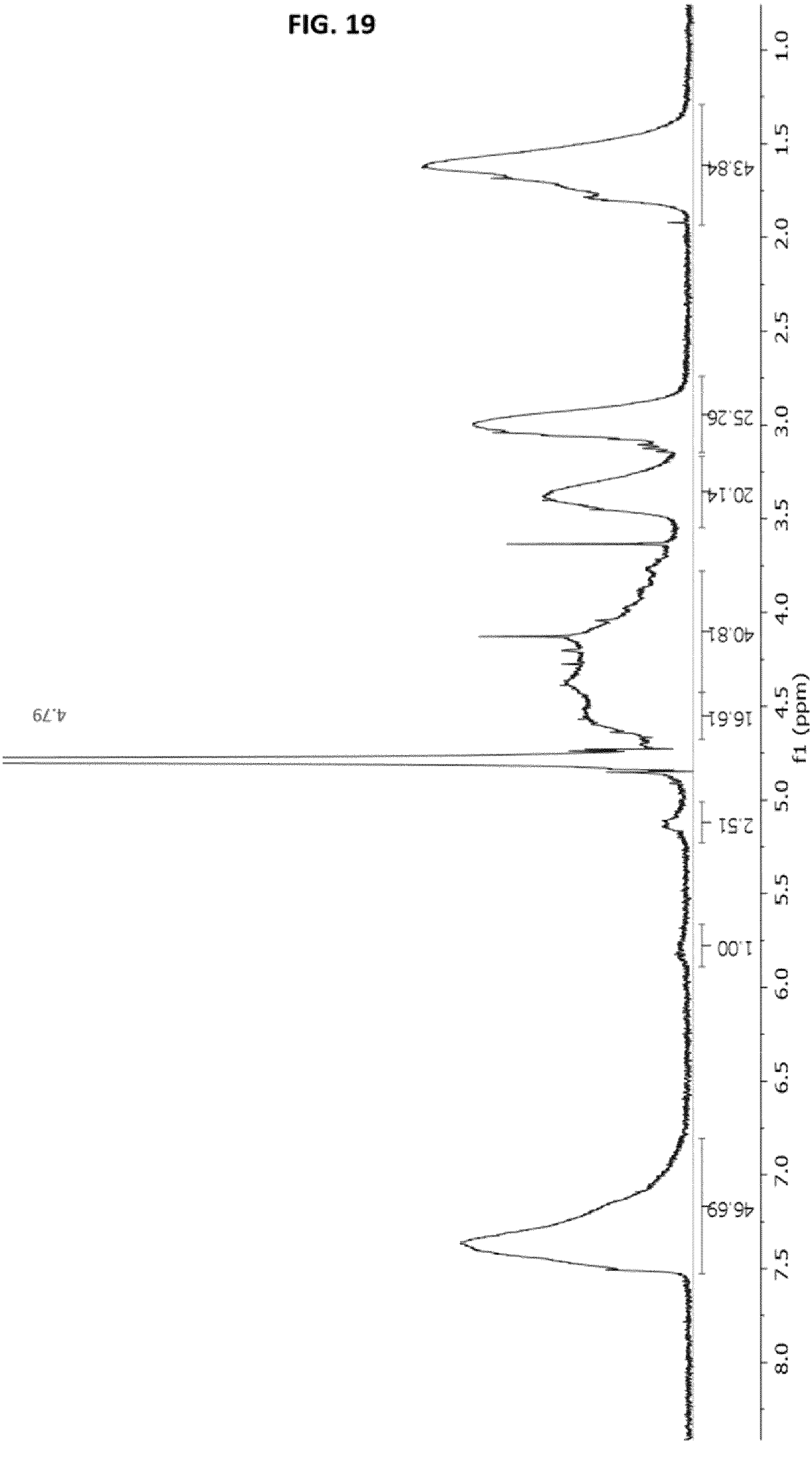
1.0
1.5
2.0
2.5
3.0
3.5
4.0
4.5
5.0
5.5
6.0
6.5
7.0
7.5
8.0
f1 (ppm)
43.84
25.26
20.14
40.81
16.61
2.51
1.00
46.69
4.79

FIG. 20A

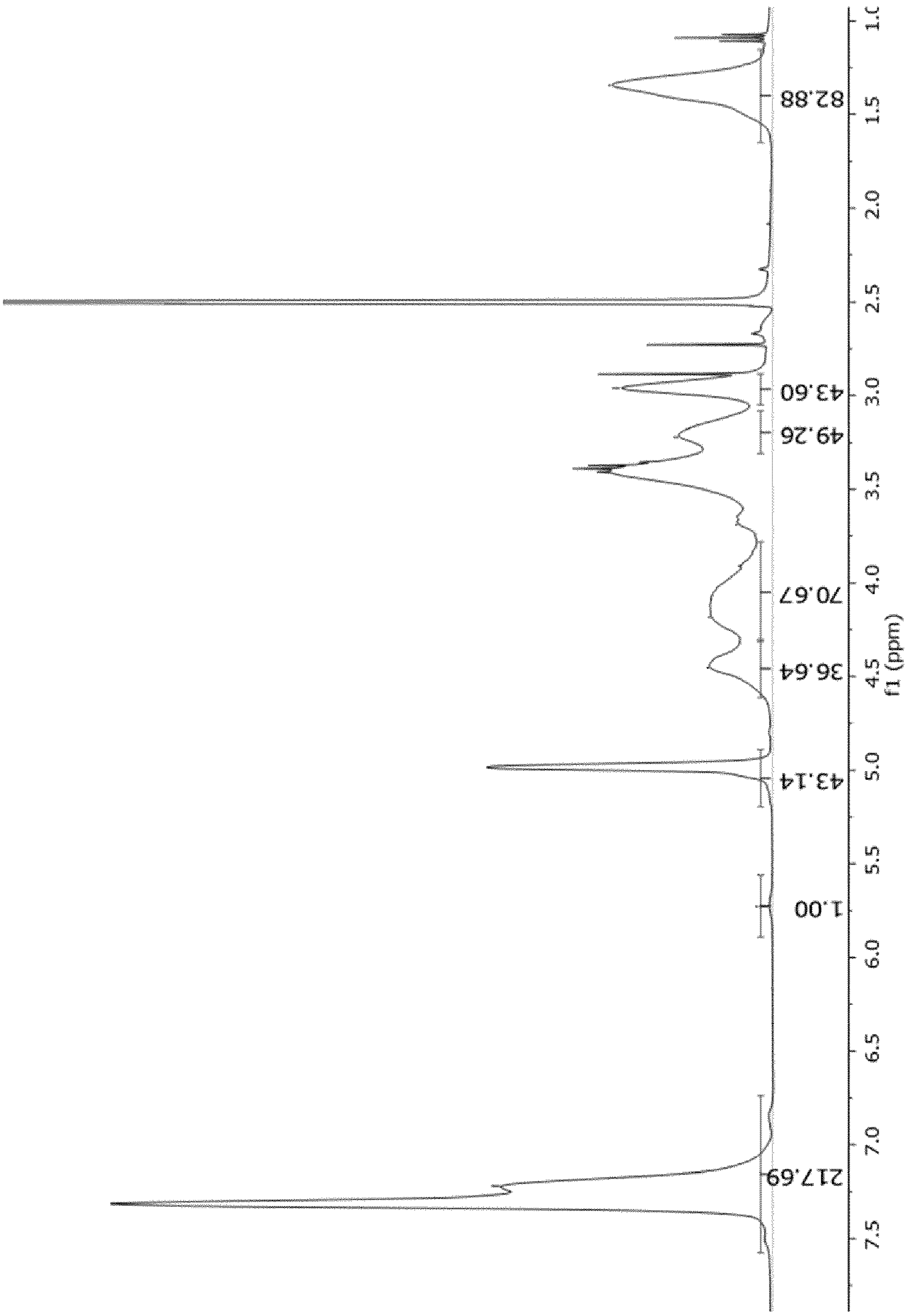
82.88
43.60
49.26
70.67
36.64
43.14
1.00
217.69
1.0
1.5
2.0
2.5
3.0
3.5
4.0
4.5
5.0
5.5
6.0
6.5
7.0
7.5
f1 (ppm)

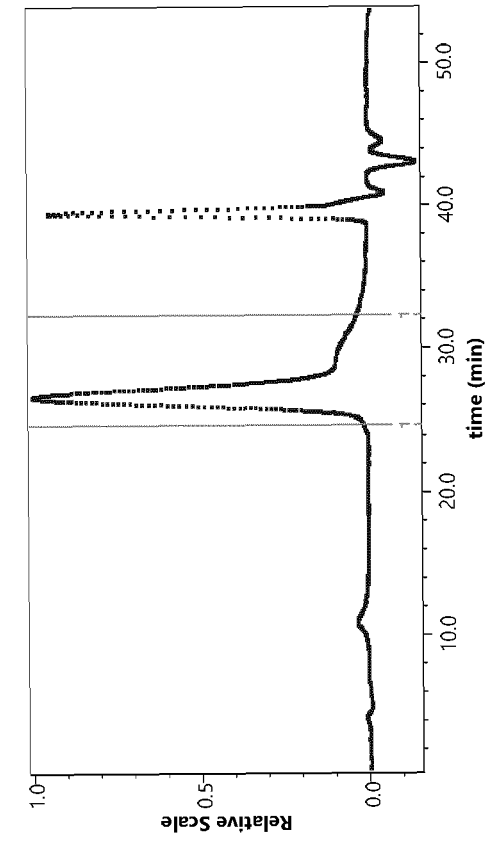


FIG. 20B

**FIG. 21**

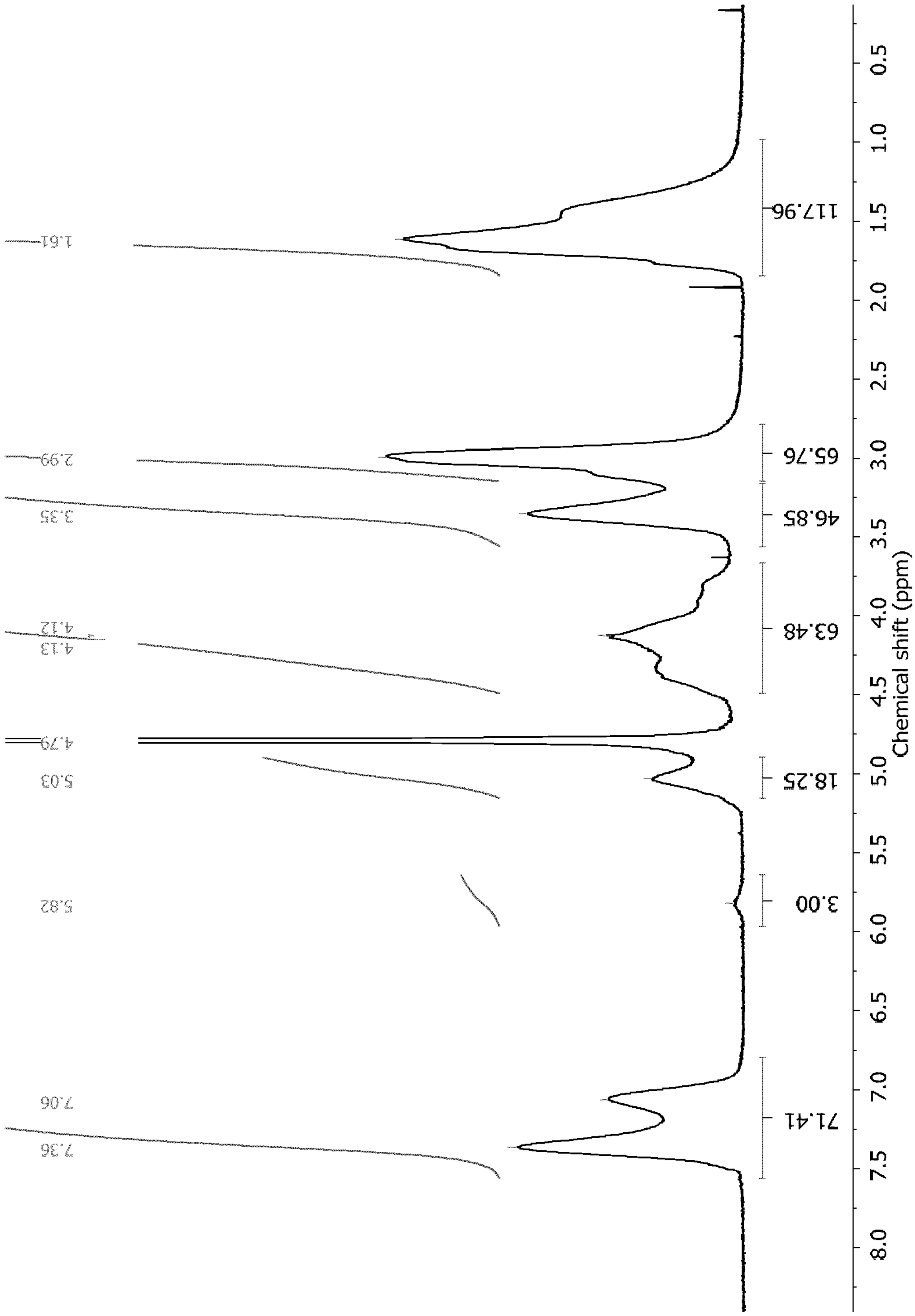
Chemical shift (ppm)
117.96
65.76
46.85
63.48
18.25
3.00
71.41
1.61
2.99
3.35
4.12
4.13
4.79
5.03
5.82
7.06
7.36

FIG. 22A

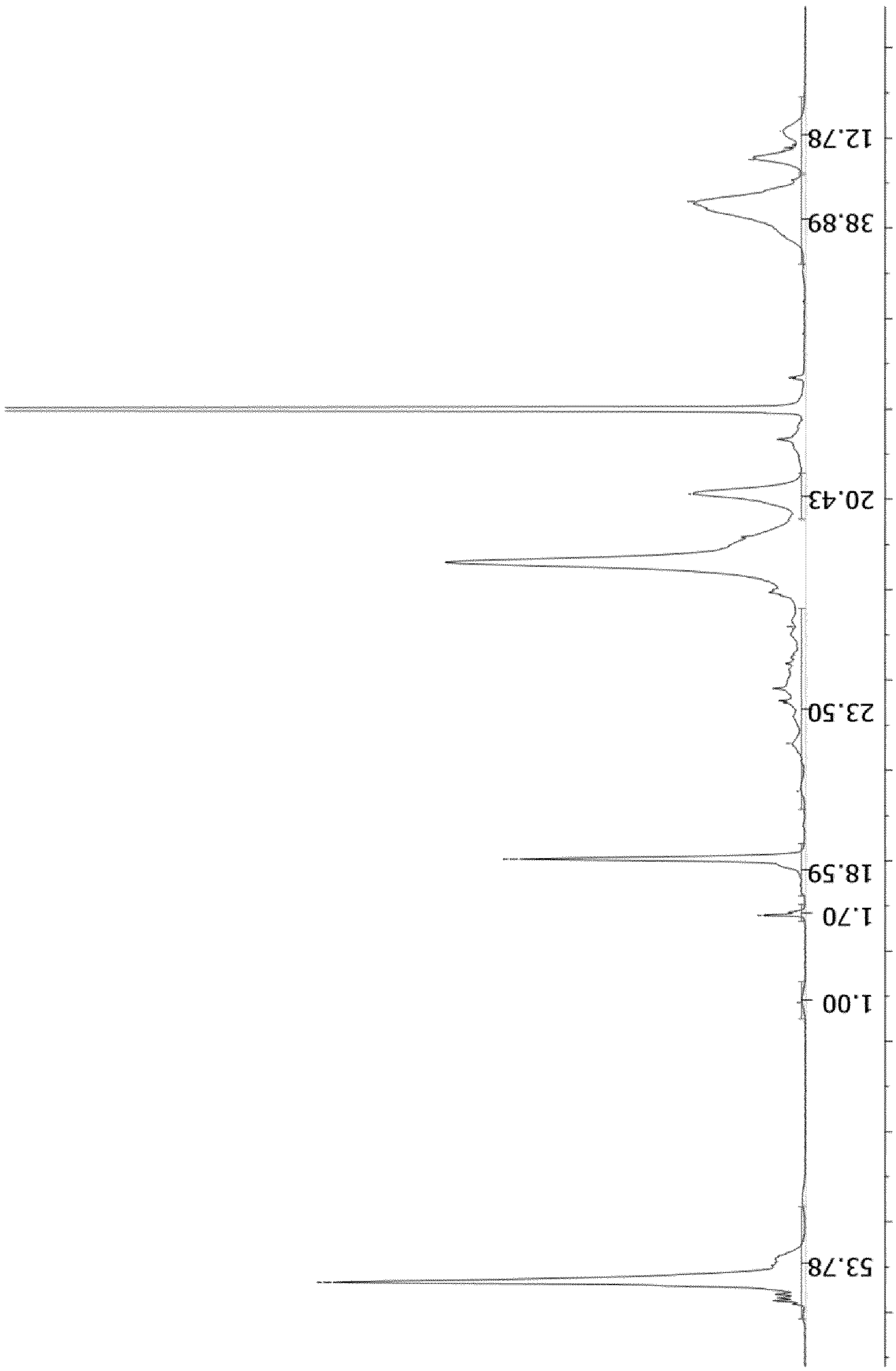
12.78
38.89
20.43
23.50
18.59
1.70
1.00
53.78
0.5
1.0
1.5
2.0
2.5
3.0
3.5
4.0
4.5
5.0
5.5
6.0
6.5
7.0
7.5
f1 (ppm)

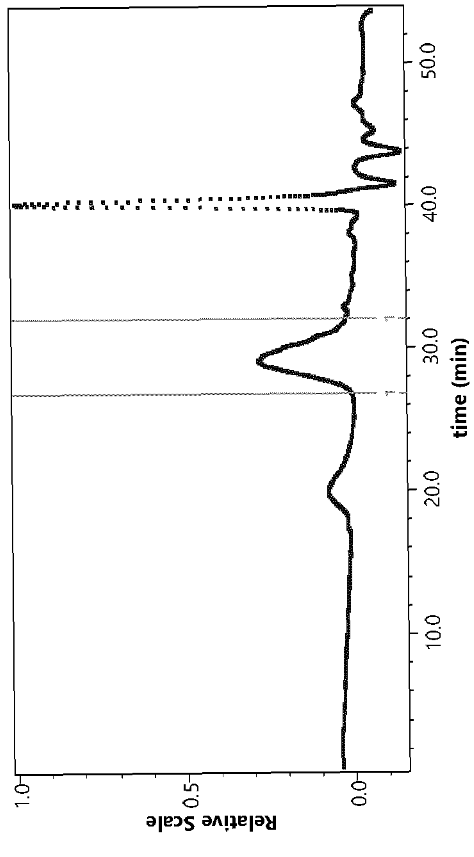


FIG. 22B

FIG. 23

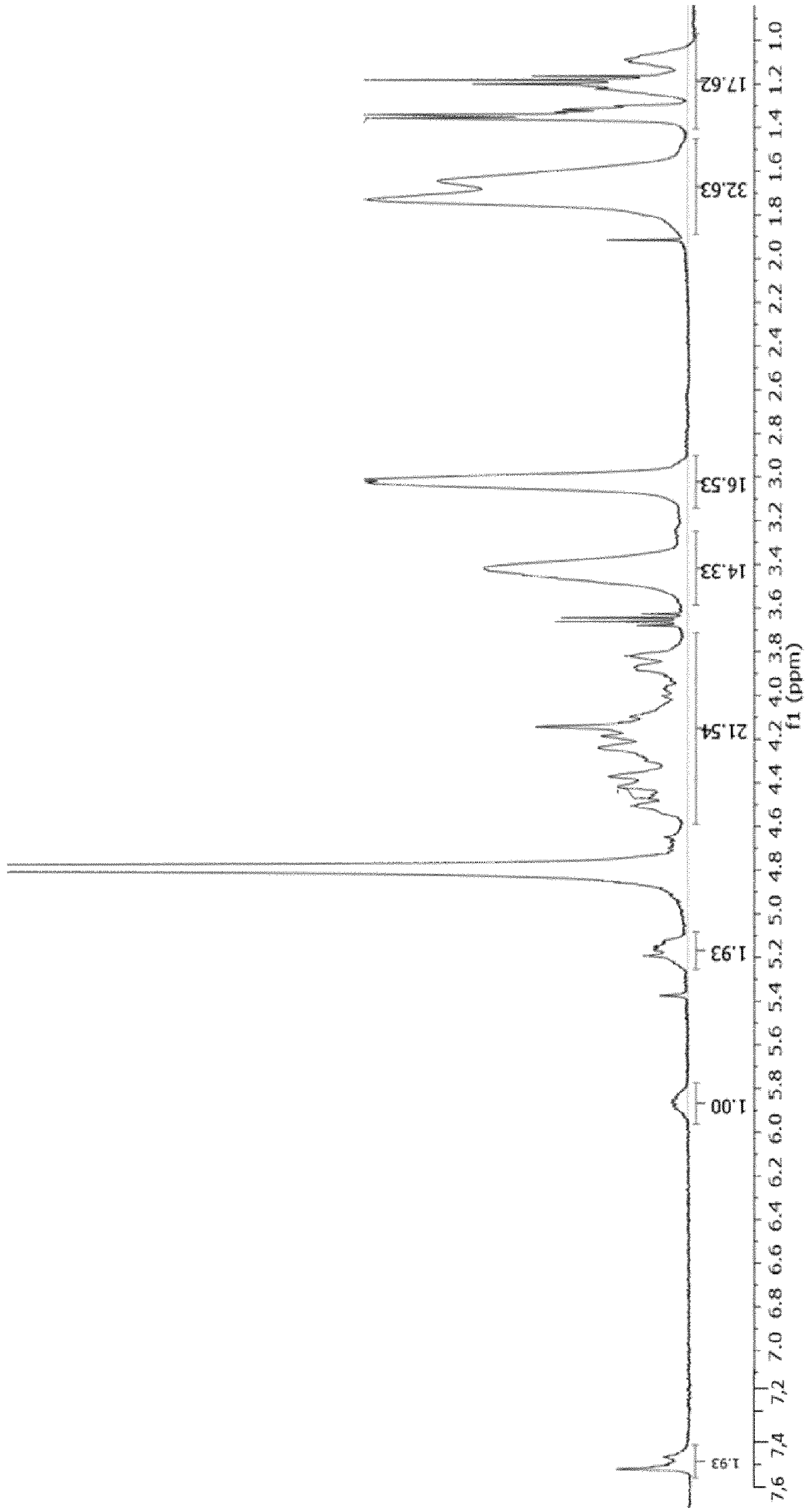
17.62
32.63
16.53
14.33
21.54
1.93
1.00
1.93
1.0 1.2 1.4 1.6 1.8 2.0 2.2 2.4 2.6 2.8 3.0 3.2 3.4 3.6 3.8 4.0 4.2 4.4 4.6 4.8 5.0 5.2 5.4 5.6 5.8 6.0 6.2 6.4 6.6 6.8 7.0 7.2 7.4 7.6
f1 (ppm)

# FIG. 24A

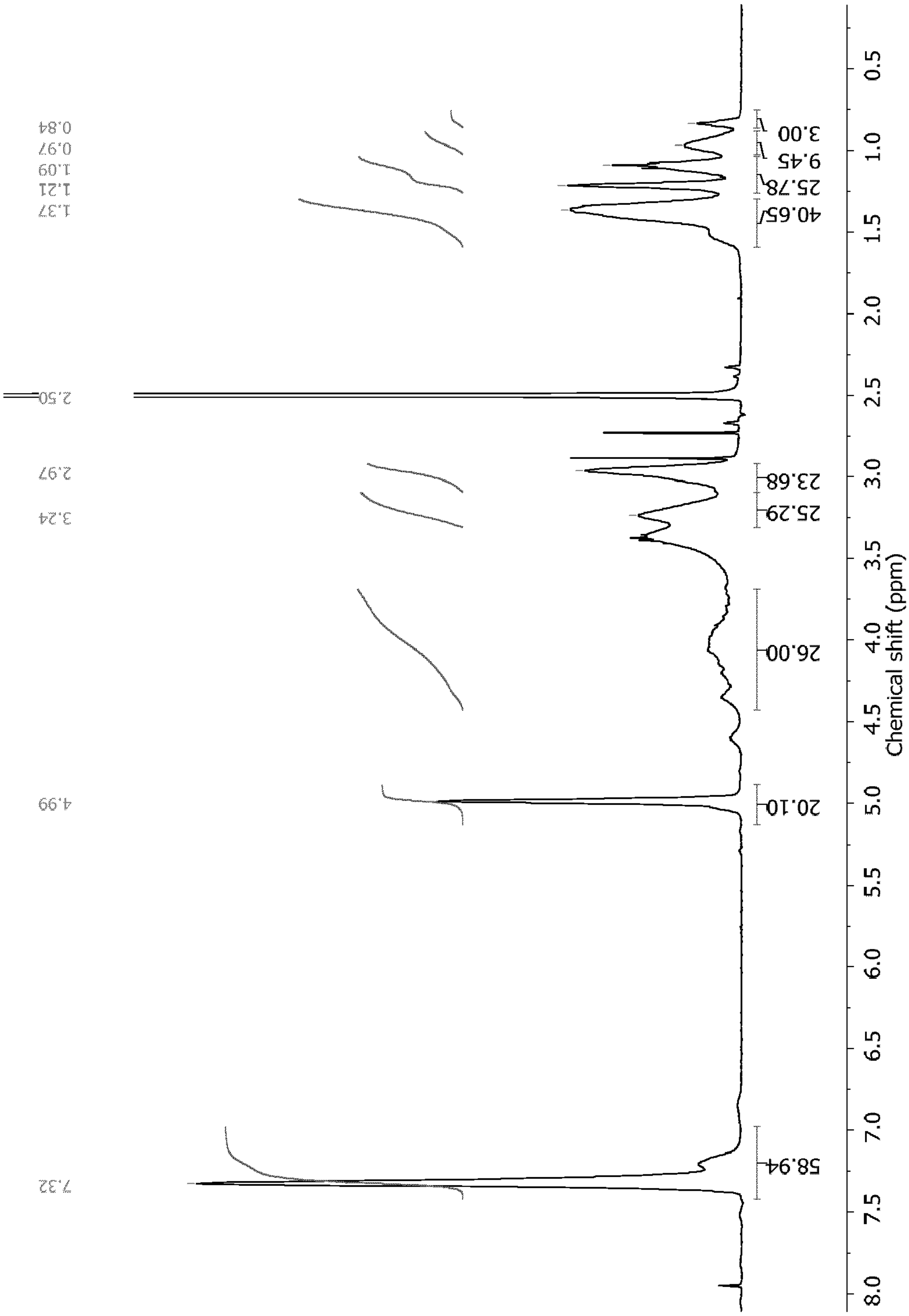
0.84
0.97
1.09
1.21
1.37
2.50
2.97
3.24
4.99
7.32
3.00
9.45
25.78
40.65
23.68
25.29
26.00
20.10
58.94
0.5
1.0
1.5
2.0
2.5
3.0
3.5
4.0
4.5
5.0
5.5
6.0
6.5
7.0
7.5
8.0
Chemical shift (ppm)

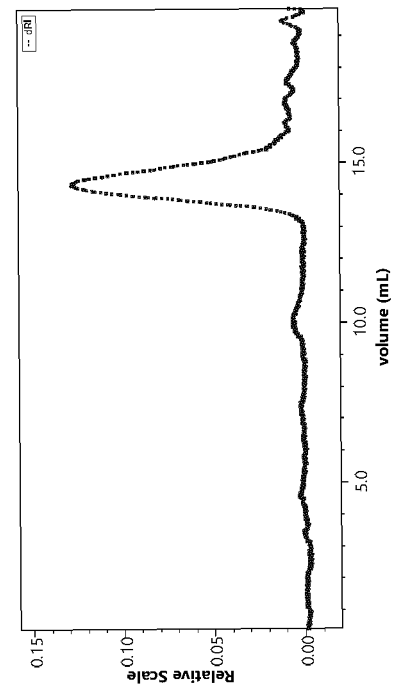


FIG. 24B

# FIG. 25

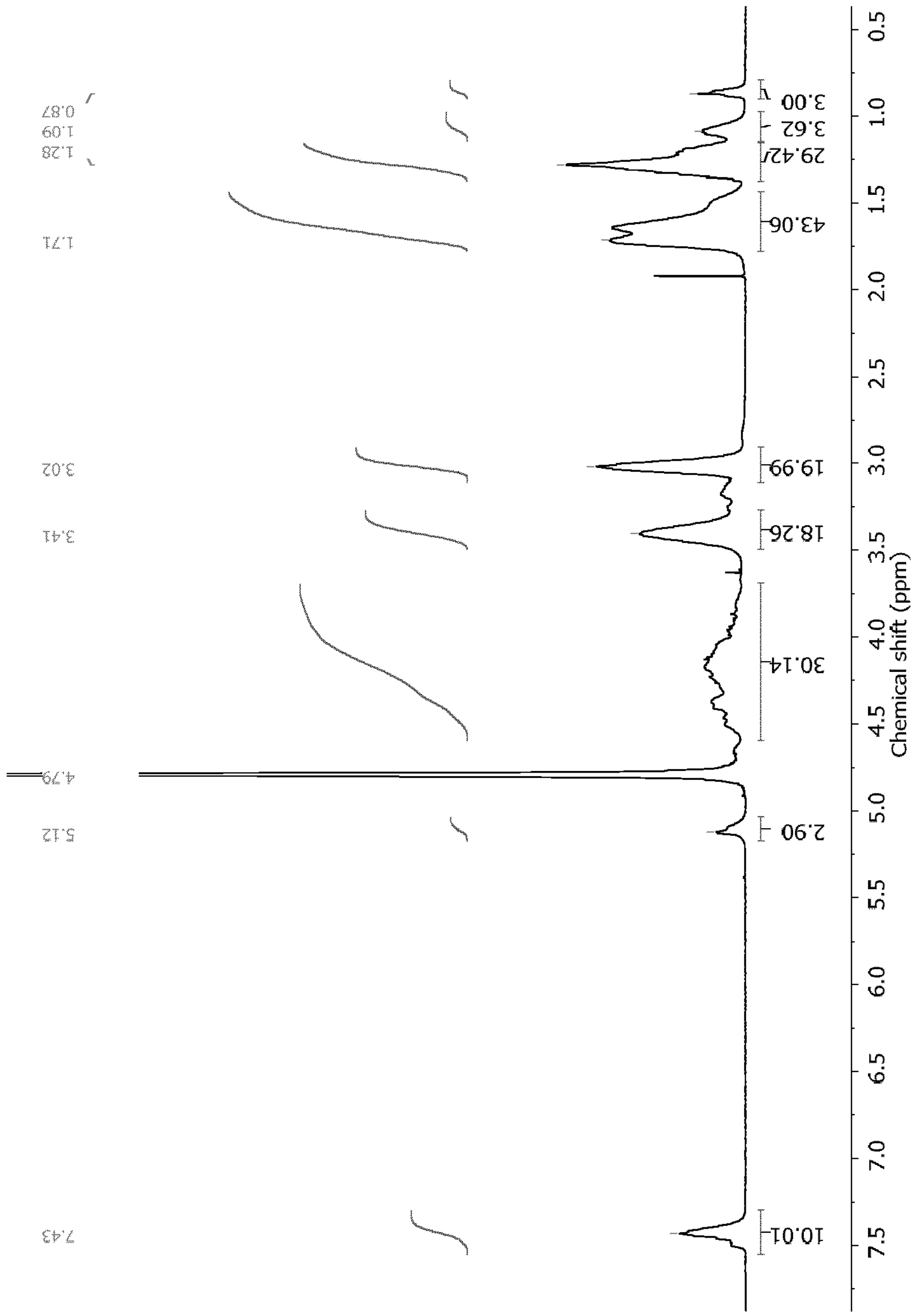

Chemical shift (ppm)
0.87
1.09
1.28
1.71
3.02
3.41
4.79
5.12
7.43
3.00
3.62
29.42
43.06
19.99
18.26
30.14
2.90
10.01

**FIG. 26**

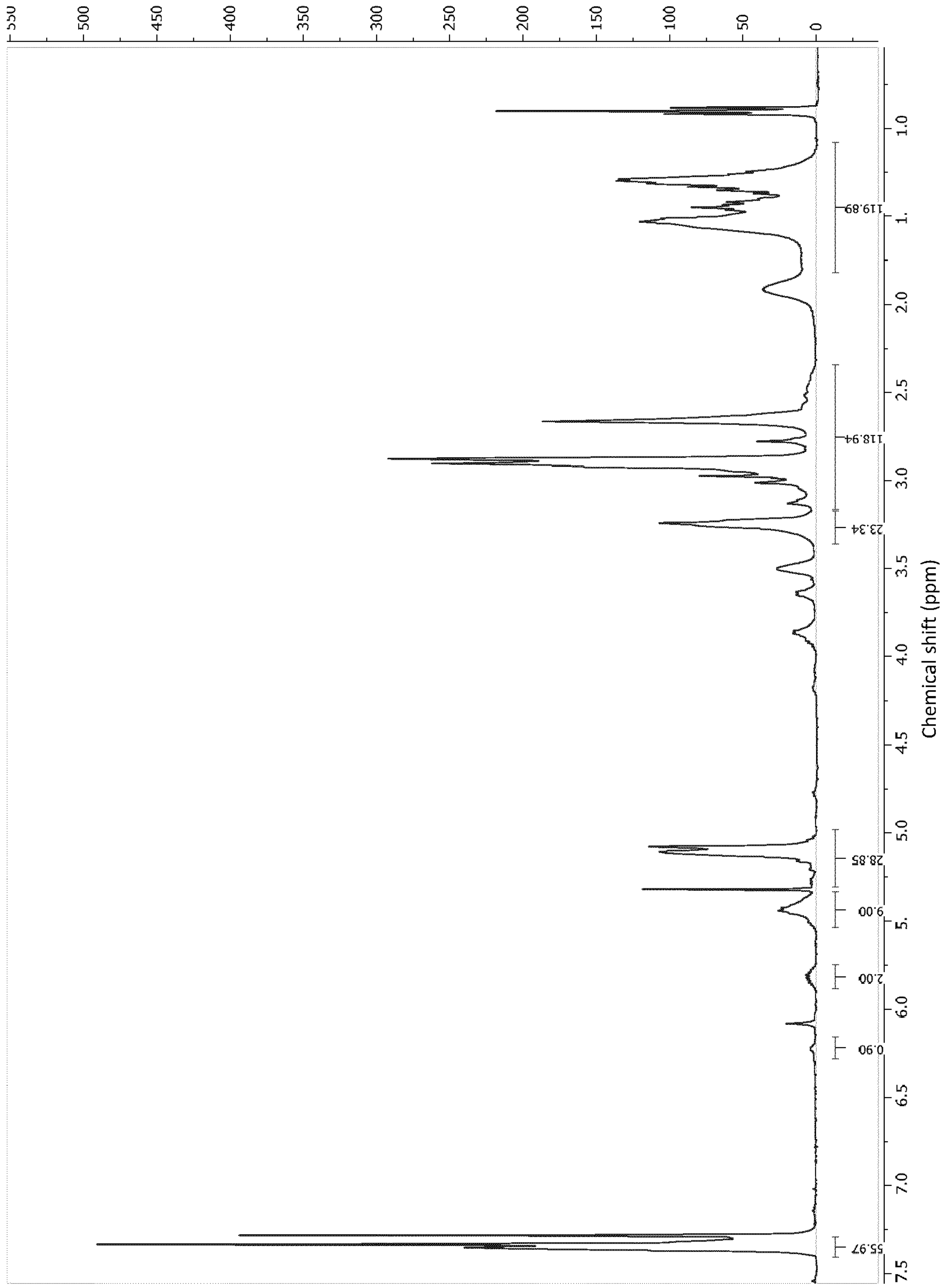

**FIG. 27A**

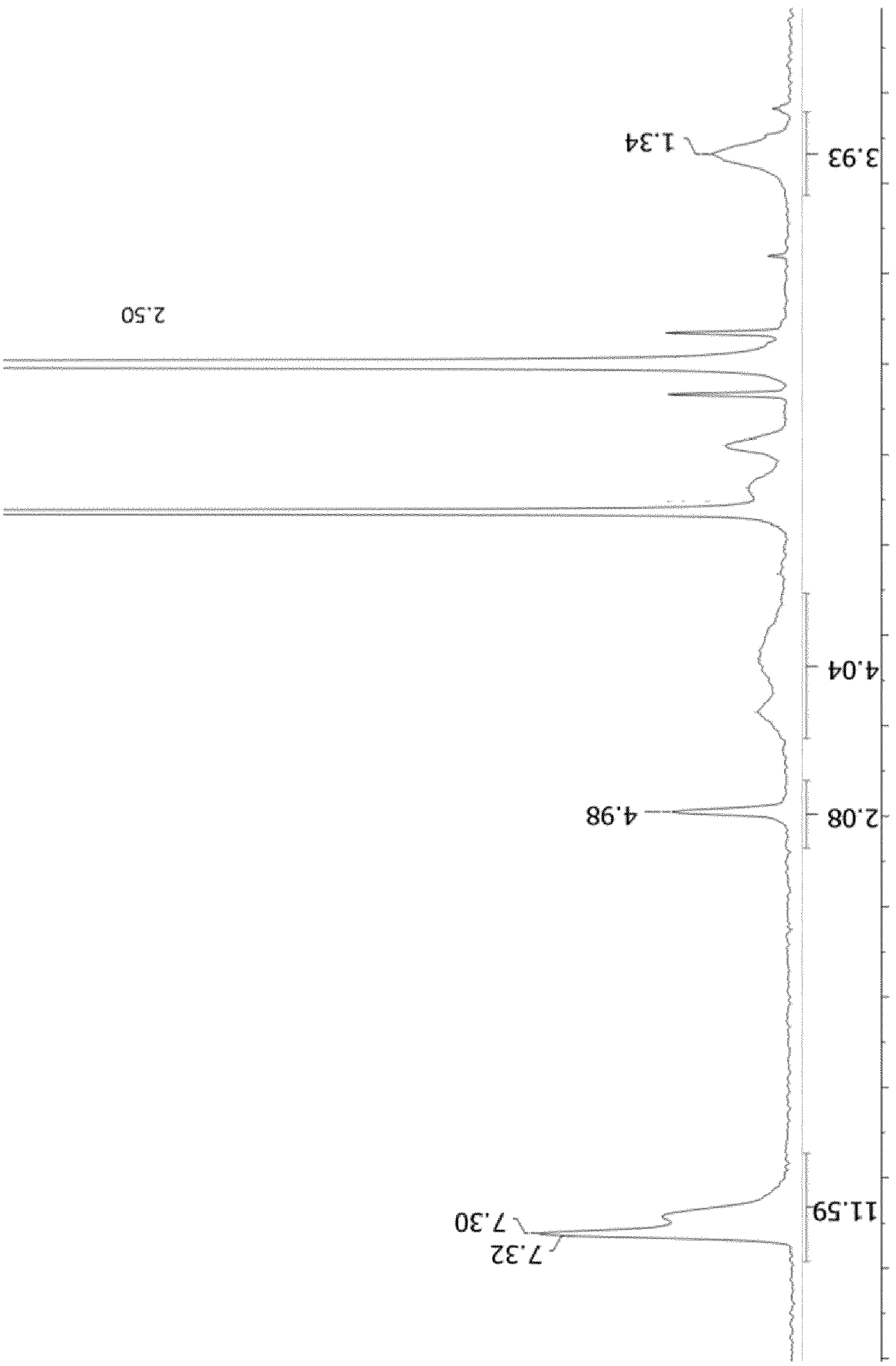
1.34
3.93
2.50
4.04
4.98
2.08
7.30
7.32
11.59
f1 (ppm)
1.0
1.5
2.0
2.5
3.0
3.5
4.0
4.5
5.0
5.5
6.0
6.5
7.0
7.5

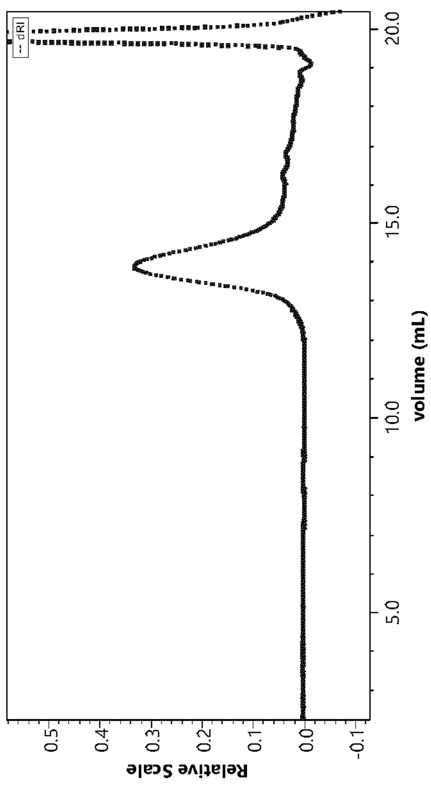


FIG. 27B

FIG. 28A

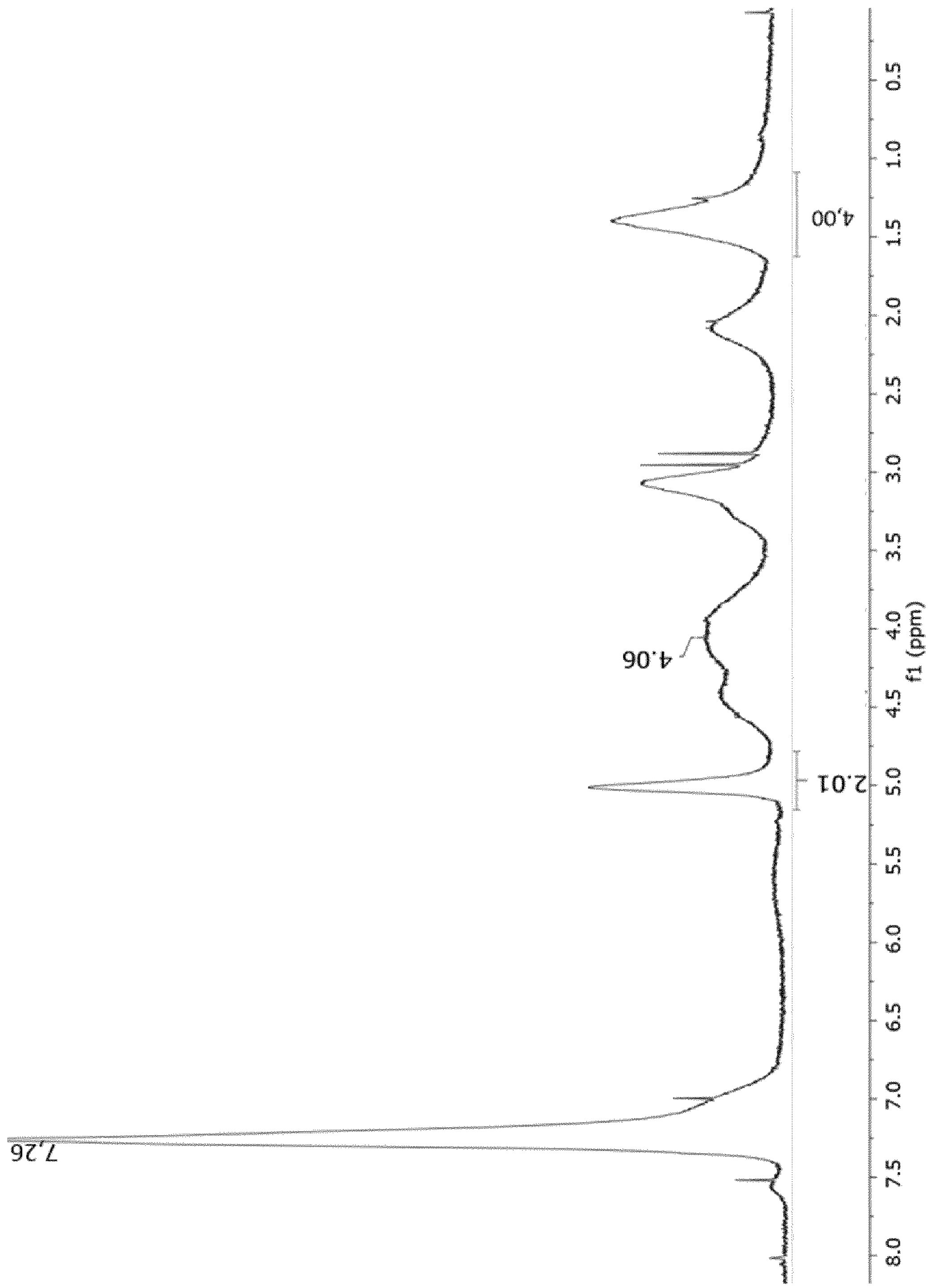

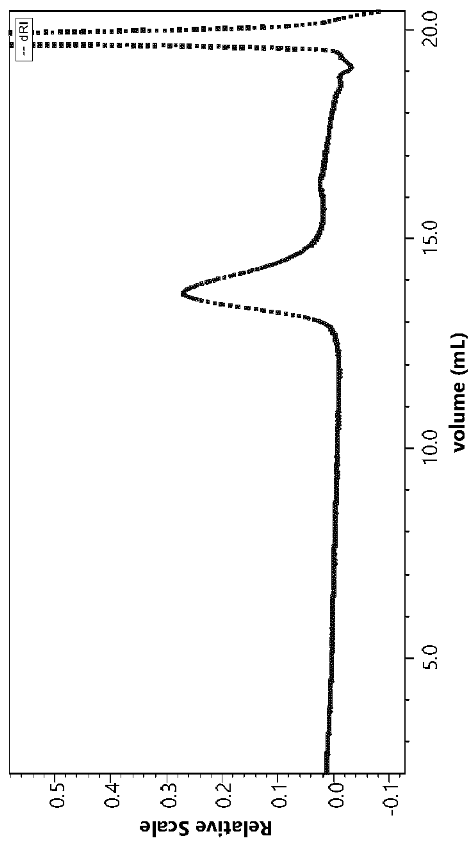


FIG. 28B

**FIG. 29**

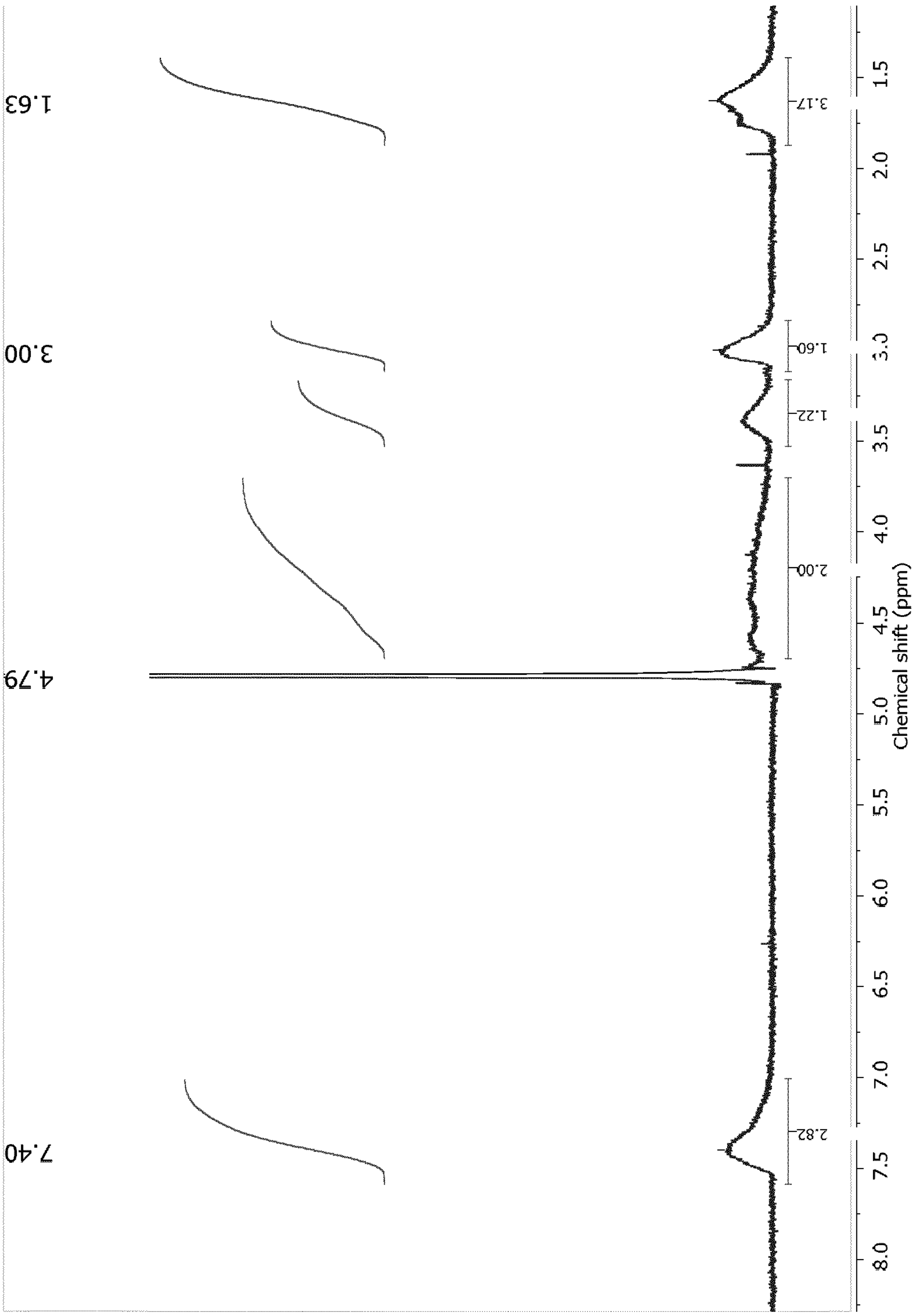
1.63
3.00
4.79
7.40
3.17
1.60
1.22
2.00
2.82
1.5
2.0
2.5
3.0
3.5
4.0
4.5
5.0
5.5
6.0
6.5
7.0
7.5
8.0
Chemical shift (ppm)

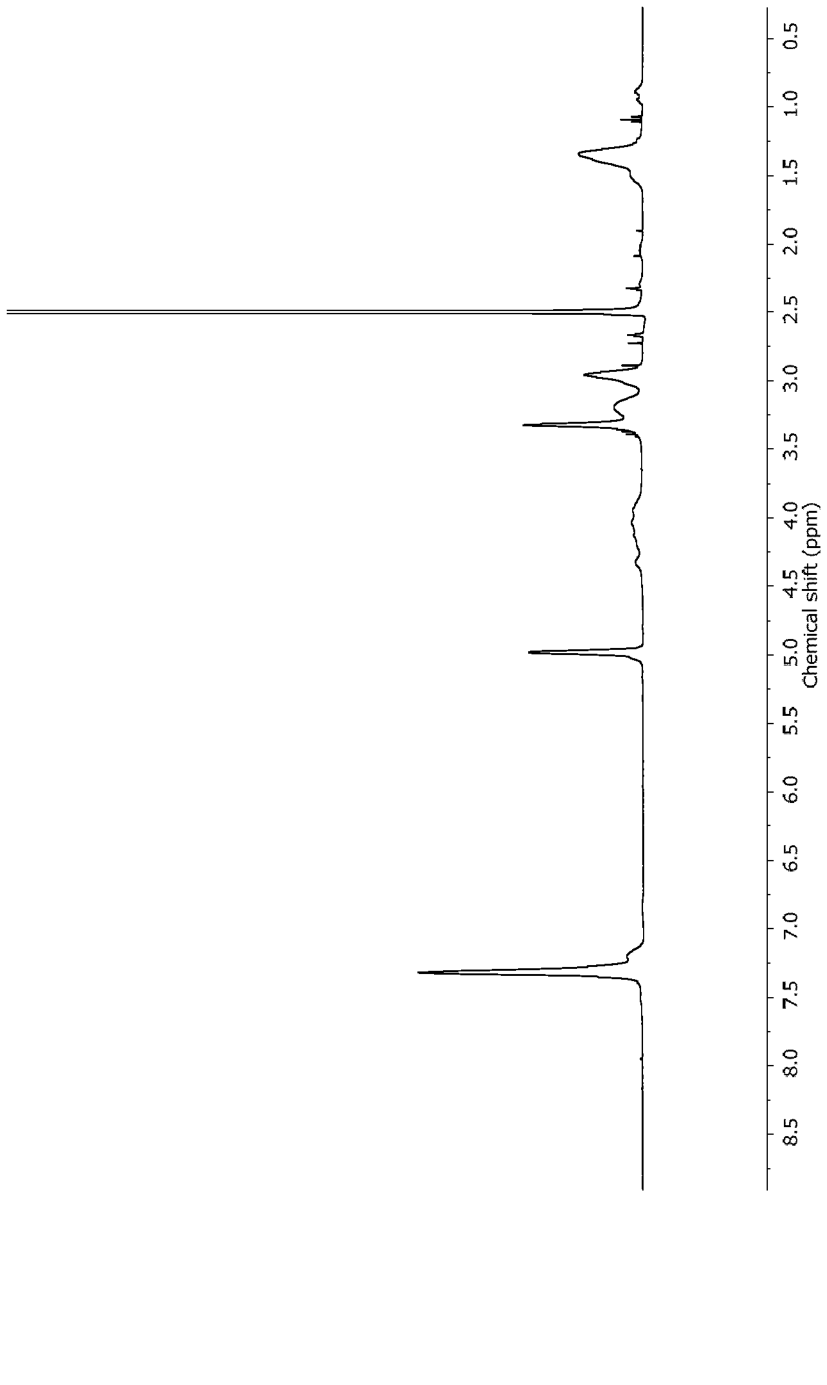
Chemical shift (ppm)
0.5
1.0
1.5
2.0
2.5
3.0
3.5
4.0
4.5
5.0
5.5
6.0
6.5
7.0
7.5
8.0
8.5

FIG. 30A

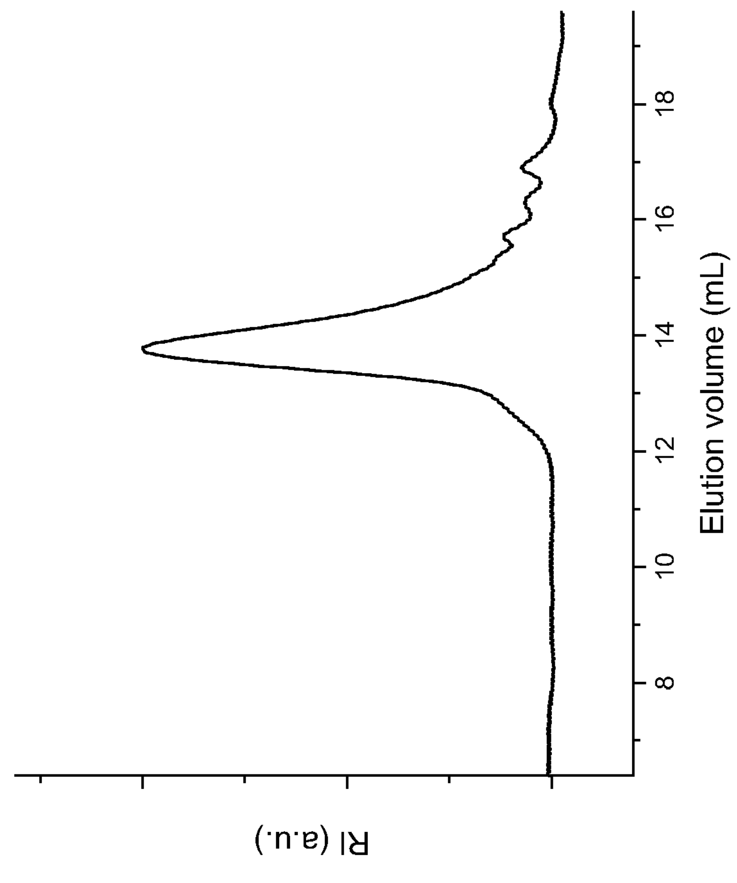


FIG. 30B

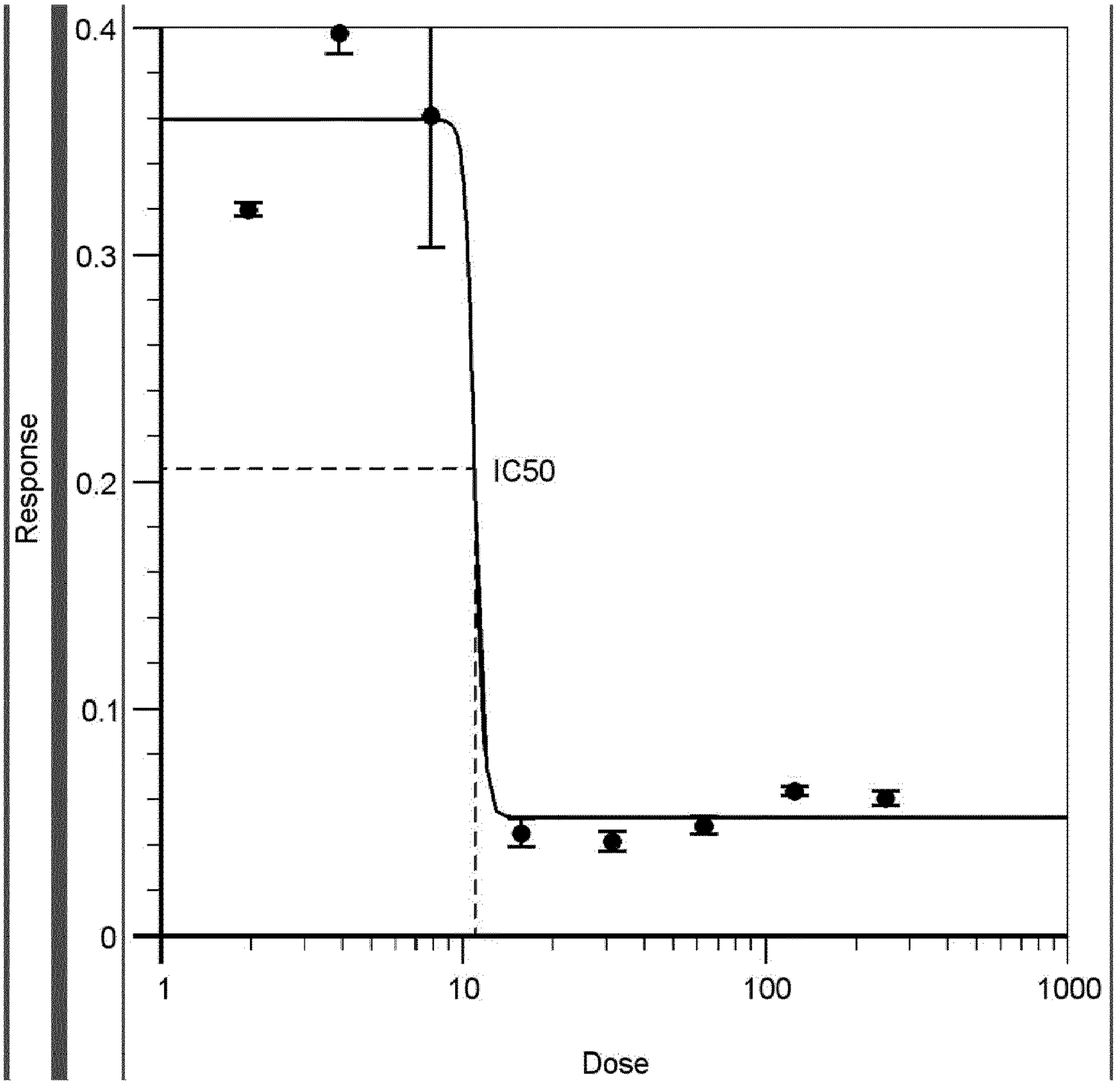


FIG. 31

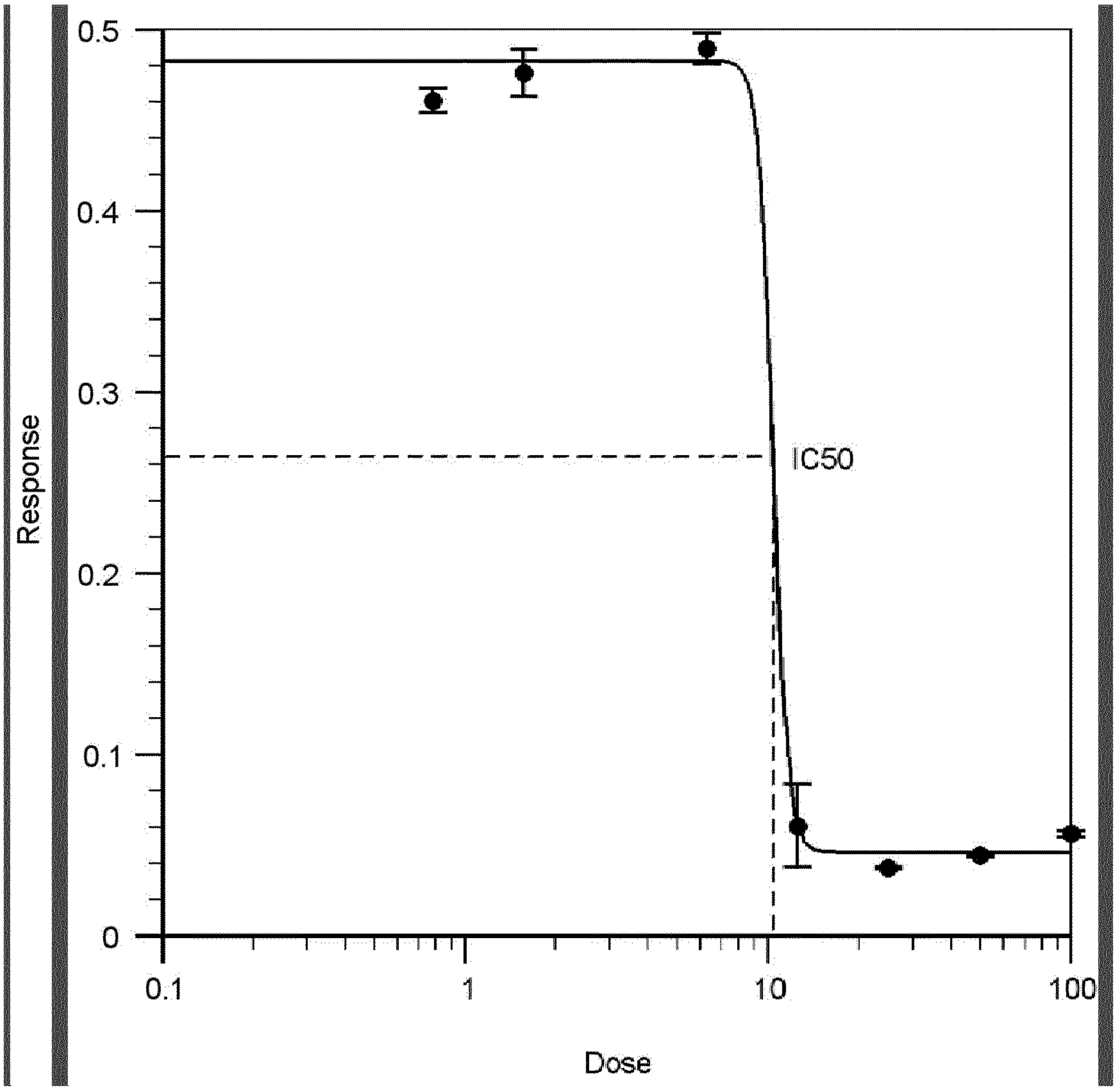


FIG. 32

FIG. 33

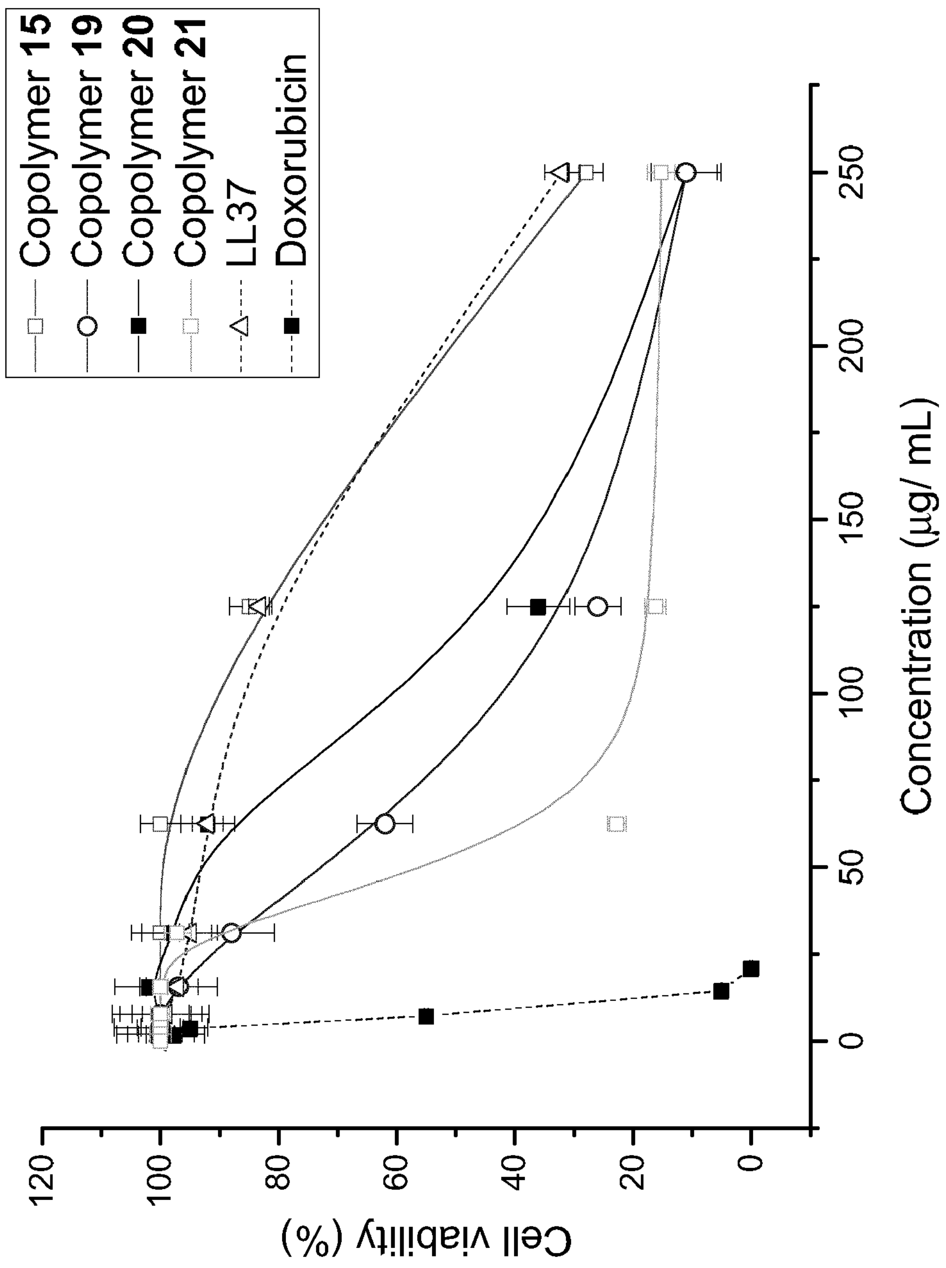
Copolymer 15
Copolymer 19
Copolymer 20
Copolymer 21
LL37
Doxorubicin
Cell viability (%)
0
20
40
60
80
100
120
Concentration (µg/ mL)
0
50
100
150
200
250

**FIG. 34**

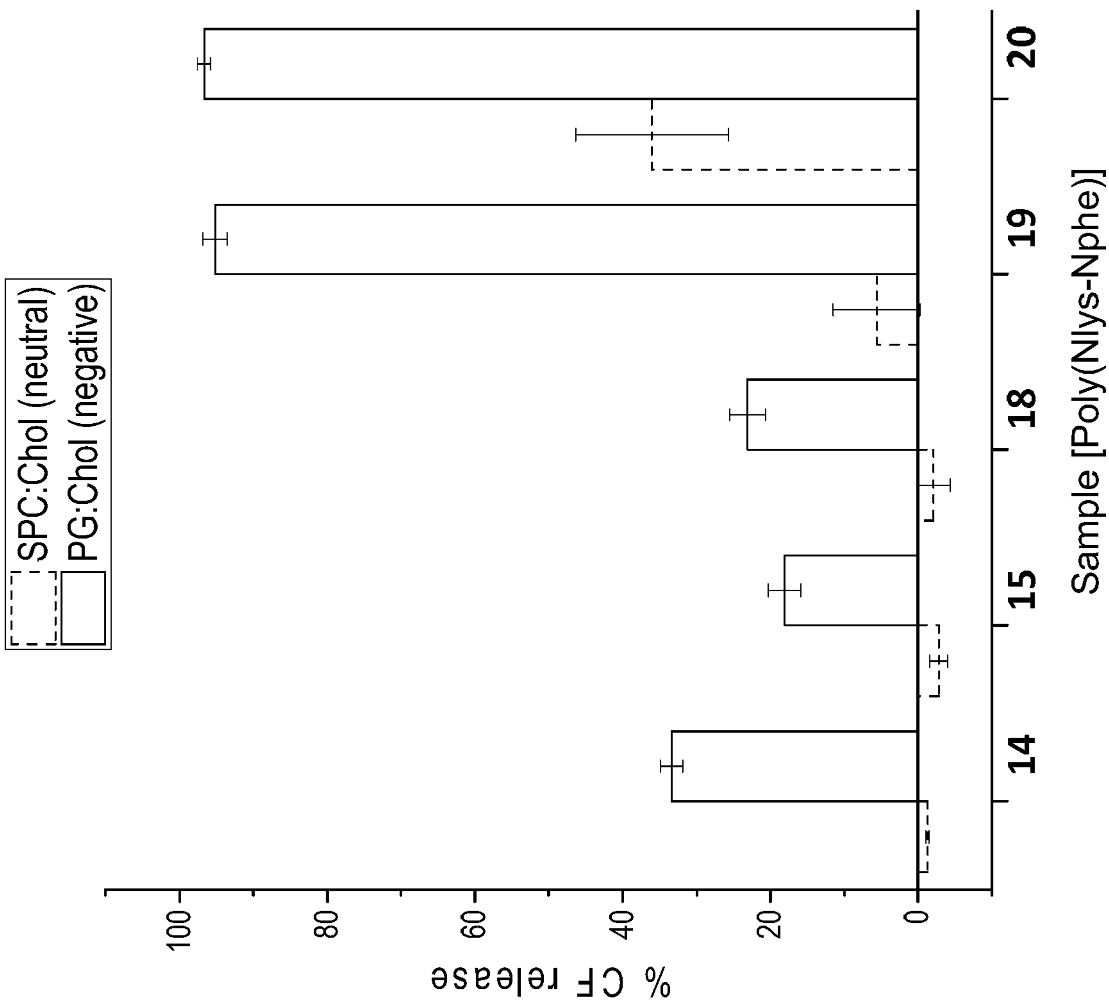
SPC:Chol (neutral)
PG:Chol (negative)
% CF release
0
20
40
60
80
100
14
15
18
19
20
Sample [Poly(Nlys-Nphe)]

FIG. 35

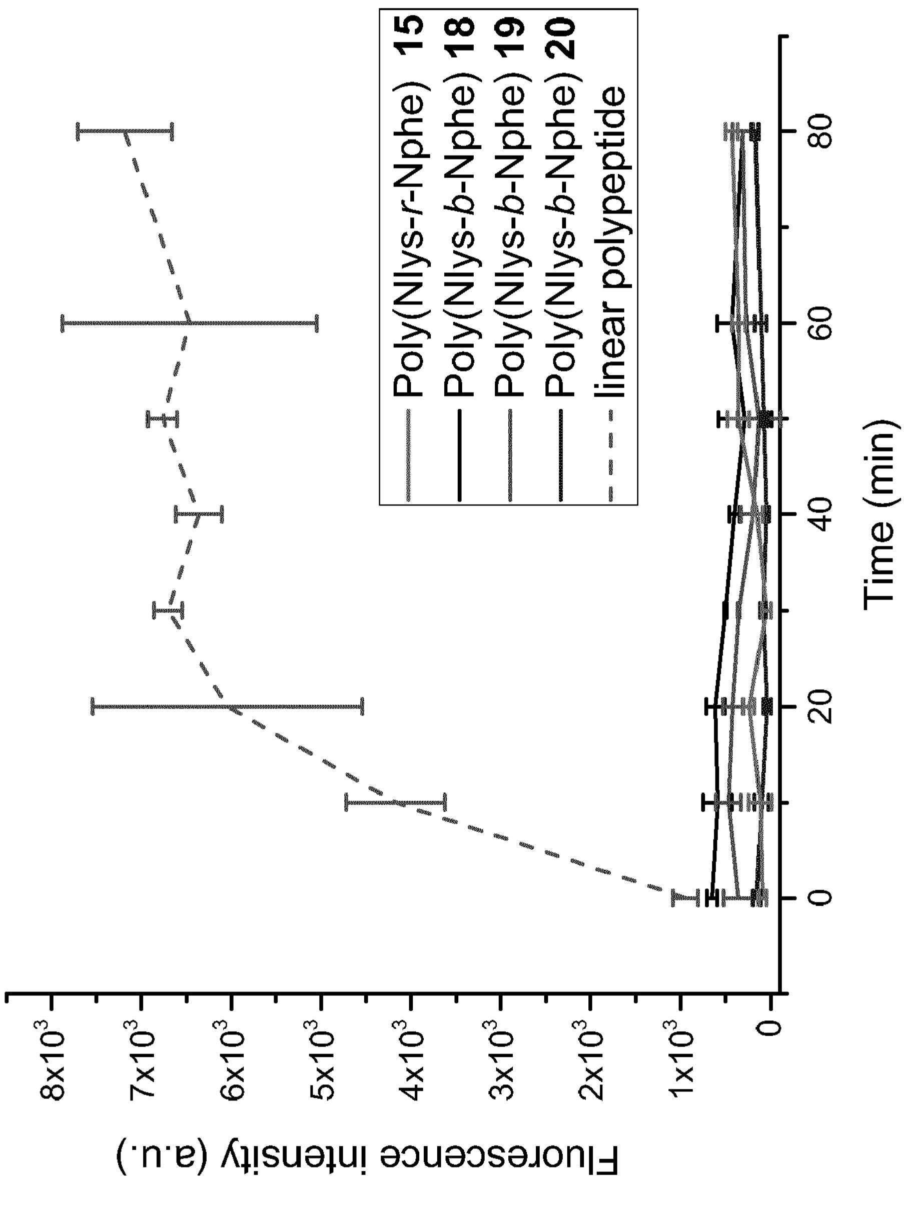
Poly(Nlys-*r*-Nphe) 15
Poly(Nlys-*b*-Nphe) 18
Poly(Nlys-*b*-Nphe) 19
Poly(Nlys-*b*-Nphe) 20
linear polypeptide
Fluorescence intensity (a.u.)
8x10[3]
7x10[3]
6x10[3]
5x10[3]
4x10[3]
3x10[3]
2x10[3]
1x10[3]
0
Time (min)
0
20
40
60
80

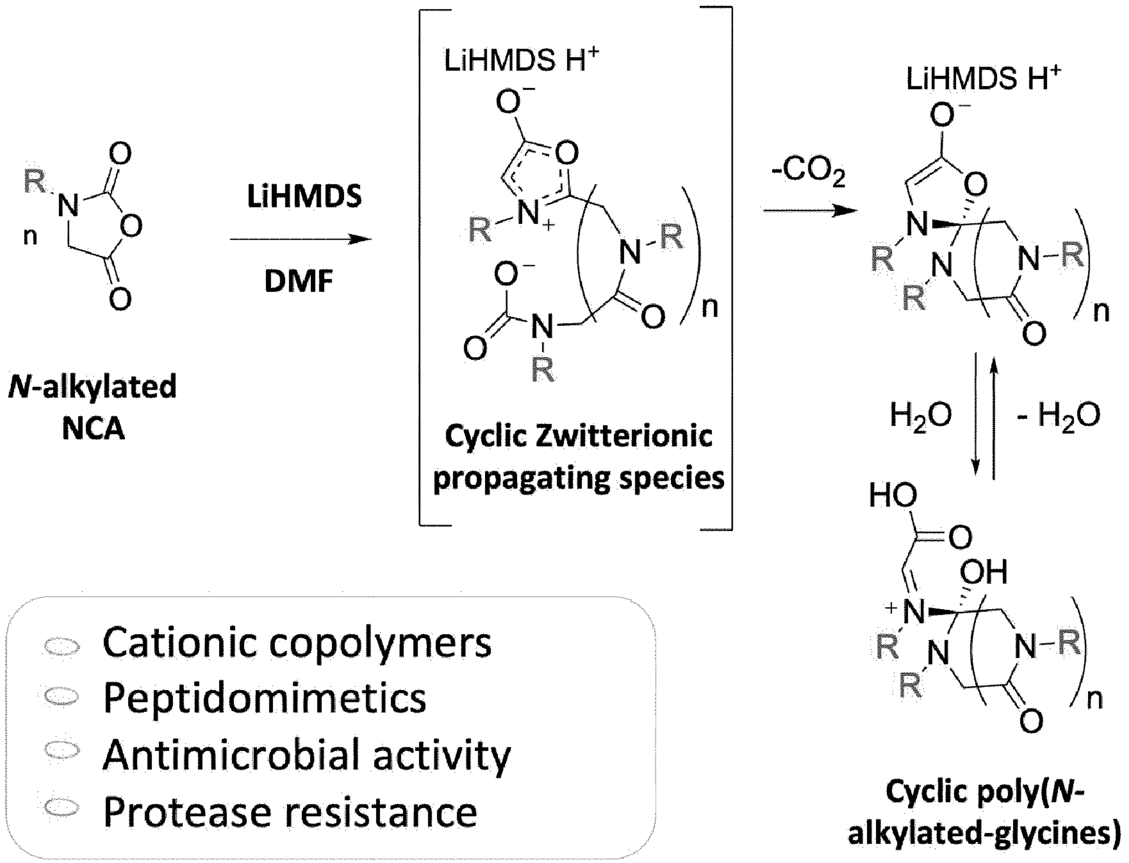
N-alkylated NCA
LiHMDS
DMF
LiHMDS H+
Cyclic Zwitterionic propagating species
-CO2
LiHMDS H+
H2O
- H2O
Cyclic poly(N-alkylated-glycines)
Cationic copolymers
Peptidomimetics
Antimicrobial activity
Protease resistance

FIG. 36

Europäisches Patentamt
European Patent Office
Office européen des brevets

## PARTIAL EUROPEAN SEARCH REPORT

under Rule 62a and/or 63 of the European Patent Convention. This report shall be considered, for the purposes of subsequent proceedings, as the European search report

Application Number
EP 21 30 5198

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MIA LACE HUANG ET AL: "A Comparison of Linear and Cyclic Peptoid Oligomers as Potent Antimicrobial Agents", CHEMMEDCHEM COMMUNICATIONS, vol. 7, no. 1, 23 September 2011 (2011-09-23), pages 114-122, XP055388629, DE ISSN: 1860-7179, DOI: 10.1002/cmdc.201100358 * Scheme 1; pages 119,120; figures 1-3,6; table 1; compound L8 * ----- | 1-19, 24-33 | INV. C07K14/00 A61K47/00 A61P31/04 ADD. A61K38/00 A61L27/00 |
| Y | WO 2020/223581 A1 (MAXWELL BIOSCIENCES INC [US]) 5 November 2020 (2020-11-05) * paragraphs [0006] - [0011], [0044], [0045]; figures 1,2,5,13; table 1 * ----- | 1-19, 24-33 | |
| Y | US 2020/157145 A1 (DUNCAN SCOTT [US]) 21 May 2020 (2020-05-21) * paragraphs [0180], [0413], [0414]; tables A-E * ----- -/-- | 1-19, 24-33 | TECHNICAL FIELDS SEARCHED (IPC) C07K A61K A61L A61P |

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:
see sheet C

1

| Place of search | Date of completion of the search | Examiner |
|---|---|---|
| The Hague | 18 November 2021 | Schmidt-Yodlee, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

page 1 of 2

Europäisches Patentamt
European Patent Office
Office européen des brevets

# PARTIAL EUROPEAN SEARCH REPORT

Application Number
EP 21 30 5198

| Category | Citation of document with indication, where appropriate, of relevant passages | Relevant to claim | CLASSIFICATION OF THE APPLICATION (IPC) |
|---|---|---|---|
| Y | SALAS-AMBROSIO PEDRO ET AL: "Synthetic Polypeptide Polymers as Simplified Analogues of Antimicrobial Peptides", BIOMACROMOLECULES, vol. 22, no. 1, 27 July 2020 (2020-07-27), pages 57-75, XP055822183, US ISSN: 1525-7797, DOI: 10.1021/acs.biomac.0c00797 * pages 64,69; figures 9-11; tables 2,4 * ----- | 1-19, 24-33 | |
| Y | BARRETT BAILEE N. ET AL: "Controlled ring-opening polymerization of N -(3- tert -butoxy-3-oxopropyl) glycine derived N -carboxyanhydrides towards well-defined peptoid-based polyacids", POLYMER CHEMISTRY, vol. 12, no. 10, 5 January 2021 (2021-01-05), pages 1540-1548, XP055822178, ISSN: 1759-9954, DOI: 10.1039/D0PY01395A Retrieved from the Internet: URL:http://pubs.rsc.org/en/content/articlepdf/2021/PY/D0PY01395A> * the whole document * ----- | 1-19 | TECHNICAL FIELDS SEARCHED (IPC) |
| T | SALAS-AMBROSIO PEDRO ET AL: "Cyclic Poly([alpha]-peptoid)s by Lithium bis(trimethylsilyl)amide (LiHMDS)-Mediated Ring-Expansion Polymerization: Simple Access to Bioactive Backbones", JOURNAL OF THE AMERICAN CHEMICAL SOCIETY, vol. 143, no. 10, 2 March 2021 (2021-03-02), pages 3697-3702, XP055822188, ISSN: 0002-7863, DOI: 10.1021/jacs.0c13231 * the whole document * ----- | | |

1

EPO FORM 1503 03.82 (P04C10)

page 2 of 2

Europäisches Patentamt
European Patent Office
Office européen des brevets

# INCOMPLETE SEARCH SHEET C

Application Number
EP 21 30 5198

Claim(s) completely searchable:
20-23

Claim(s) searched incompletely:
1-19, 24-33

Reason for the limitation of the search:

The present claims 1 to 33 relate to an extremely large number of possible compounds, compositions, products and processes. Support and disclosure within the meaning of Articles 84 and 83 EPC are to be found, however, for only a very small proportion of the compounds and their related compositions, products and processes claimed.
It is clear from the examples that a distinct linear and a distinct cyclic copolymer comprising N-aminobutyl-glycine units and N-benzyl-glycine units show antimicrobial activity against Clostridium difficile.
An extrapolation of the distinct copolymer used in the examples to any linear or cyclic copolymer according to general formulae (V linear), (VA macrocyclic) and (VB macrocyclic) comprising N-aminoalkyl (C1-C12)-glycine units and N-benzyl-glycine units appears to be acceptable under Articles 84 and 83 EPC.
Notably, the linear and macrocyclic peptoids as recited in e.g. compound claims 19 and 20 do not even require the presence of two different peptoid units, as x or y may be 0 and x+y is 1.
Non-compliance with the substantive provisions is such that a meaningful search of the whole claimed subject-matter of the claim can not be carried out (Rule 63 EPC and Guidelines B-VIII, 3).
In reply to the invitation to file a statement indicating the subject-matter to be searched, the applicant provided a reworded set of claims and argued in the letter of 24 September 2021 that the new set of claims encompasses a reduced number of possible compounds and methods. Furthermore, some claims have been deleted to address objections with respect to unity of invention.
The applicant has failed to provide convincing arguments as to why an extrapolation of the distinct copolymer used in the examples to any linear copolymer according to general formula (V linear) according to the first identified invention would be justifiable under Articles 83 and 84 EPC. These deficiencies are not regarded to be cured by deleting some of the alternatives from a very long list of possible substituents.
In consequence, the search is carried out for those parts of the claims that refer to a linear peptoid according to claim 19, wherein general formula V linear comprises N-aminoalkyl (C1-C12)-glycine units and N-benzyl-glycine units, and its process, dendritic peptoids, drug conjugates, pharmaceutical compositions and items.
The applicant's attention is drawn to the fact that the application will be further prosecuted on the basis of subject-matter for which a search has been carried out and that the claims should be limited to that subject-matter at a later stage of the proceedings (Rule 63(3) EPC). It is also noted that the claims submitted on 24 September 2021 are not considered as amended claims (Rule 137(1) EPC), but merely as an indication of the subject-matter to be searched in respect of the claims as originally filed (see also official communication of 05 October 2021).

Europäisches Patentamt
European Patent Office
Office européen des brevets

Application Number

EP 21 30 5198

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**19, 24-26(completely); 1-18, 27-33(partially)**

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 21 30 5198

The Search Division considers that the present European patent application does not comply with the requirements of unity of invention and relates to several inventions or groups of inventions, namely:

1. claims: 19, 24-26(completely); 1-18, 27-33(partially)

   linear peptoid/ N-substituted peptidic copolymer having formula (V linear), ist conjugate and dendrimer, process for obtaining said copolymer, conjugate and dendrimer, pharmaceutical composition, use and item

---

2. claims: 1-18, 20-23, 27-33(all partially)

   macrocyclic peptoid/ N-substituted peptidic copolymer having formula (VA macrocyclic), process for obtaining said copolymer, pharmaceutical composition, use and item

---

3. claims: 1-18, 20-23, 27-33(all partially)

   macrocyclic peptoid/ N-substituted peptidic copolymer having formula (VB macrocyclic), process for obtaining said copolymer, pharmaceutical composition, use and item

---

## ANNEX TO THE EUROPEAN SEARCH REPORT ON EUROPEAN PATENT APPLICATION NO.

EP 21 30 5198

This annex lists the patent family members relating to the patent documents cited in the above-mentioned European search report. The members are as contained in the European Patent Office EDP file on The European Patent Office is in no way liable for these particulars which are merely given for the purpose of information.

18-11-2021

| Patent document cited in search report | Publication date | Patent family member(s) | Publication date |
|---|---|---|---|
| WO 2020223581 A1 | 05-11-2020 | CA 3138644 A1 | 05-11-2020 |
| | | EP 3962925 A1 | 09-03-2022 |
| | | WO 2020223581 A1 | 05-11-2020 |
| US 2020157145 A1 | 21-05-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6482799 B

### Non-patent literature cited in the description


- **E. W. MARTIN.** Remington's Pharmaceutical Sciences. Mack Publishing Co, 1975
- **T. W. GREENE.** Protective Groups in Organic Synthesis. John Wiley & Sons, 1991
- **STANFIELD et al.** *Microb Pathog.,* 1987, vol. 3, 155-165
- **FOX et al.** *Am. J. Vet. Res.,* 1987, vol. 48, 85-90
- **RUIZ-PALACIOS.** *Infect. Immun.,* 1981, vol. 34, 250-255
- **HUMPHREY et al.** *J. Infect. Dis.,* 1985, vol. 151, 485-493